# EUROPEAN PATENT APPLICATION

(11) **EP 3 991 752 A1**
(43) Date of publication of application: **04.05.2022**
(21) Application number: 19936127.0
(22) Date of filing: 29.06.2019
(51) Int. Cl.: A61K 47/00, A61P 35/00, A61P 37/02

(54) **CELL-BINDING MOLECULE-TUBULYSIN DERIVATIVE CONJUGATE AND PREPARATION METHOD THEREFOR**

(71) Applicant: Hangzhou Dac Biotech Co., Ltd, Hangzhou City, Zhejiang Province 310018 (CN)
(72) Inventor: ZHAO, Robert, Lexington, Massachusetts 02421 (US); YANG, Qingliang, Hangzhou, Zhejiang 310018 (CN); ZHAO, Linyao, Hangzhou, Zhejiang 310018 (CN); HUANG, Yuanyuan, Hangzhou, Zhejiang 310018 (CN); YE, Hangbo, Hangzhou, Zhejiang 310018 (CN); GAI, Shun, Hangzhou, Zhejiang 310018 (CN); LAI, Juan, Hangzhou, Zhejiang 310018 (CN); LI, Wenjun, Hangzhou, Zhejiang 310018 (CN); BAI, Lu, Hangzhou, Zhejiang 310018 (CN); CAO, Minjun, Hangzhou, Zhejiang 310018 (CN)
(74) Representative: Haseltine Lake Kempner LLP
(86) International application number: PCT/CN2019/093946
(87) International publication number: WO 2021/000067

(57) **Abstract**

The present invention relates to the conjugates of Tubulysin derivatives (anologs) and cell-binding molecules using branched (side-chain) linkers, and the resulting conjugates have better pharmacokinetic properties, and thus can more accurately target and kill abnormal cells. The invention also relates to the conjugation methods of the Tubulysin derivatives (anologs) to cell-binding molecules, and methods for synthesizing the small molecules, and methods of using the conjugates for targeted therapy for cancers, infections and autoimmune diseases. The conjugates of Tubulysin derivatives with long branched linkers demonstrated increased half-life, minimal exposure to non-targeted cells, tissues or organs in system circulation, leading to reduced off-target toxicity.

## Description

### FIELD OF THE INVENTION

The present invention relates to the conjugates of Tubulysin derivatives (anologs) and cell-binding molecules using branched (side-chain) linkers, and the resulting conjugates have better pharmacokinetic properties, and thus can more accurately target and kill abnormal cells. The invention also relates to the conjugation methods of the Tubulysin derivatives (anologs) to cell-binding molecules, and methods for synthesizing the small molecules, and methods of using the conjugates for targeted therapy for cancers, infections and autoimmune diseases.

### BACKGROUND OF THE INVENTION

Antibody-drug conjugates (ADCs) have become one of most promising targeted therapies for cancer as evidenced by the clinical success of brentuximab vedotin (Adcetris) for relapsed/refractory Hodgkin lymphoma (Okeley, N., et al, Hematol Oncol. Clin. North. Am., 2014, 28, 13-25; Gopal, A., et al, Blood, 2015, 125, 1236-43) and ado-trastuzumab emtansine for relapsed HER2+ breast cancer (Peddi, P. and Hurvitz, S., Ther. Adv. Med. Oncol., 2014, 6(5), 202-9; Lambert, J. and Chari, R., J. Med. Chem., 2014, 57, 6949-64) Three components of ADC, monoclonal antibody, cytotoxic payload, linker and the sites of linkage are all important to make a successful ADC (L. Ducry and B Stump, Bioconj. Chem., 2010, 21, 5-13; G.S. Hamilton, Biologicals, 2015, 43, 318-32) The study of each component has been ongoing for about three decades. For a linker, it must be reactive toward certain functional groups of the payload, resulting a stable conjugate while in system circulation and however easily to release the payload upon antigen binding and intracellular uptake, and importantly not to harm the normal tissues if the linker-payload moiety is formed off-target in the circulation. In the sence, the current linker technologies are very limited (Ponte, J. et al., Bioconj. Chem., 2016, 27(7), 1588-98; Dovgan, I., et al. Sci. Rep., 2016, 6, 30835; Ross, P. L. and Wolfe, J. L. J. Pharm. Sci., 105(2), 391-7; Chen, T. et al. J. Pharm. Biomed. Anal., 2016, 117, 304-10)

For early ADCs, which were particularly used for the treatment of liquid tumor, the linker were too labile, leading to the immature release of payload in the circulation and conseq.uent off-target toxicity (Bander, N. H. et al, Clin. Adv. Hematol. Oncol., 2012, 10, 1-16) For the current generation of ADCs, the linkers are more stable, and the cytotoxic agents are also significantly more potent (Behrens, C. R. and Liu, B., mAbs, 2014. 6, 46-53) However, the off-target toxicity so far is still the major challenge in development of ADC drugs (Roberts, S. A. et al, Regul. Toxicol. Pharmacol., 2013, 67, 382-91) For instance, T-DM1 (Kadcyla^{®}), which uses stable (none-cleavable) MCC linker, has shown great benefit in clinical practice to patients who have HER2-positive metastatic breast cancer (mBC) or who have already been treated for mBC and developed tumor recurrence within six months of adjuvant therapy (Peddi, P. and Hurvitz, S., Ther. Adv. Med. Oncol., 2014, 6(5), 202 -209; Piwko C, et al, Clin. Drug Investig., 2015, 35(8), 487-93; Lambert, J. and Chari, R., J. Med. Chem., 2014, 57, 6949-64) But, T-DM1 had failed in clinic trial as first-line treatment for patients with HER2 positive locally advanced unresectable breast cancer or metastatic breast cancer, or as the second line treatment for patients with HER2-positive advanced gastric cancer, due to the limited benefit to patients while comparing its toxicity to the efficacy (Ellis, P. A., et al, J. Clin. Oncol., 2015, 33, (suppl; abstr 507 of 2015 ASCO Annual Meeting); Shen, K. et al, Sci Rep., 2016; 6: 23262; de Goeij, B. E. and Lambert, J. M. Curr. Opin. Immunol., 2016, 40, 14-23; Barrios, C. H. et al, J. Clin. Oncol., 2016, 34 (suppl; abstr 593 of 2016 ASCO Annual Meeting))

To address the issues of off-target toxicity, researchers of ADC chemistry and design are now expanding the scopes of linker, payload and conjugation methods, for example, to use not only sole potent payload, especially to address activity of the linker-payload toward targets/target diseases (Lambert, J. M. Ther. Deliv., 2016, 7, 279-82; Zhao, R. Y. et al, 2011, J. Med. Chem., 54, 3606-23) Nowadays many drug developers in industry and academic institutions are highly focusing on developing novel linkers and methods for site-specific conjugation, which seems to have longer circulation half-life, higher efficacy, potentially less off-target toxicity, and better in vivo pharmacokinetic (PK) properties as well as better batch-to-batch consistency of ADC production (Hamblett, K. J. et al, Clin. Cancer Res., 2004, 10, 7063-70; Adem, Y. T. et al, Bioconj. Chem., 2014, 25, 656-664; Boylan, N. J. Bioconj. Chem., 2013, 24, 1008-1016; Strop, P., et al, Chem. Biol., 2013, 20, 161-67; Wakankar, A. mAbs, 2011, 3, 161-172) These specific conjugation methods reported so far include incorporation of modified antibodies with engineered cysteines (Junutula, J. R. et al. Nat. Biotechnol., 2008, 26, 925-32; Junutula, J. R., et al. Clin. Cancer Res., 2010, 16, 4769; US Patents 8,309,300; 7,855,275; 7,521,541; 7,723,485, WO2008/141044), selenocysteines (Hofer, T., et al. Biochemistry 2009, 48, 12047-57; Li, X., et al. Methods, 2014, 65, 133-8; US Patent 8,916,159 for US National Cancer Institute), cysteine with a perfluoroaromatic tag (Zhang, C. et al. Nat. Chem., 2015, 8, 1-9), thiofucose (Okeley, N. M., et al. Bioconj. Chem.,2013, 24, 1650), non-natural amino acids (Axup, J. Y., et al. Proc. Nat. Acad. Sci. USA., 2012, 109, 16101-6; Zimmerman, E.S., et al., Bioconj. Chem., 2014, 25, 351-361; Wu, P., et al, Proc. Natl. Acad. Sci., 2009, 106, 3000-5; Rabuka, D., et al, Nat. Protoc., 2012, 7, 1052-67; US Patent 8,778,631 and US Pat Appl. 20100184135, WO2010/081110; WO2006/069246, 2007/059312, US Patents 7,332,571, 7,696,312, and 7,638,299; WO2007/130453, US patents 7,632,492 and 7,829,659); re-bridging the reduced intermolecular disulfides by dibromomalemides (Jones, M. W. et al. J. Am. Chem. Soc., 2012, 134, 1847-52), bis-sulfone reagents (Badescu, G. et al. Bioconj. Chem., 2014, 25, 1124-36; WO2013/190272, WO2014/064424), dibromopyridazinediones (Maruani, A. et al. Nat. Commun., 2015, 6, 6645); modifying N-glycans with galactosyltransferase or sialyltransferases (Zhou, Q. et al. Bioconj. Chem., 2014, 25, 510-520; US Pat Appl 20140294867 for Sanofi-Genzyme); enzymatic bioconjugation using formylglycine generating enzyme (FGE) (Drake, P. M. et al. Bioconj. Chem., 2014, 25, 1331-41; Carrico, I. S. et al. US Pat. 7,985,783; 8,097,701; 8,349,910, and US Pat Appl 20140141025, 20100210543), phosphopantetheinyl transferases (PPTases) (Grunewald, J. et al. Bioconj. Chem. 2015, 26, 2554-62), sortase A (Beerli, R. R., et al. PLoS One 2015, 10, e0131177), microbial transglutaminase (mTG) from actinobacterium Streptomyces mobaraensis (Strop, P., Bioconj. Chem., 2014, 25, 855-62; Strop, P., et al., Chem. Biol. 2013, 20, 161-7; US Patent 8,871,908) or microbial transglutaminase recongnizing a glutamine tag (Dennler, P., et al, 2014, Bioconj. Chem., 25, 569-78; Siegmund, V. et al. Angew. Chemie - Int. Ed., 2015, 54, 13420-4; US pat appl 20130189287; US Pat 7,893,019), or enzyme/bacterium which catalyze the formation of an isopeptide bond outside of the protein main chain (Kang, H. J., et al. Science, 2007, 318, 1625-8; Zakeri, B. et al. Proc. Natl. Acad. Sci. USA, 2012, 109, E690-7; Zakeri, B. & Howarth, M. J. Am. Chem. Soc., 2010,132,4526-7)

We have disclosed several conjugation methods of re-bridging a pair of thiols from the reduced inter chain disulfide bonds of a native antibody, such as using bromomaleimide and dibromomaleimide linkers (WO2014/009774), 2,3-disubstituted succinic acid, 2-monosubstituted/2,3-disubstituted fumaric acid or maleic acid linkers (WO2015/155753, WO20160596228), acetylenedicarboxylic acid linkers (WO2015/151080, WO20160596228) or hydrazine linkers (WO2015/151081) The ADCs made with these linkers and methods have demonstrated better therapeutic index than the traditionally unselective cysteine or lysine conjugation methods. Herein we disclose a class of tubulysin conjugates containing long side chain linkers. The long side chain linkers can prevent the antibody-drug conjugates from being hydrolyzed by proteinases or esterases and therefore lead to better stability of the conjugates in the circulation.

Tubulysins as a class of potent cytotoxic agents are well known in the art and they were isolated from natural sources or prepared synthetically according to the known methods (e. g. Balasubramanian, R., et al. J. Med. Chem., 2009, 52, 238-40; Wipf, P., et al. Org. Lett., 2004, 6, 4057-60; Pando, O., et al. J. Am. Chem. Soc., 2011, 133, 7692-5; Reddy, J. A., et al., Mol. Pharmaceutics, 2009, 6, 1518-25; Raghavan, B., et al., J. Med. Chem., 2008, 51, 1530-33; Patterson, A. W., et al., J. Org. Chem., 2008, 73, 4362-9; Pando, O., et al., Org. Lett., 2009, 11 (24), 5567-9; Wipf, P., et al., Org. Lett., 2007, 9 (8), 1605-7; Friestad, G. K., Org. Lett.,2004, 6, 3249-52; Peltier, H. M., et al., J. Am. Chem. Soc., 2006, 128, 16018-9; Chandrasekhar, S., et al, J. Org. Chem., 2009, 74, 9531-4; Liu, Y., et al., Mol. Pharmaceutics, 2012, 9, 168-75; Friestad, G. K., et al., Org. Lett., 2009, 11, 1095-8; Kubicek, K., et al., Angew. Chem. Int. Ed. Engl., 2010.49: 4809-12; Chai, Y, et al., Chem Biol, 2010, 17: 296-309; Ullrich, A., et al., Angew. Chem. Int. Ed. Engl., 2009, 48, 4422-5; Sani, M., et al., Angew. Chem. Int. Ed. Engl., 2007, 46, 3526-9; Domling, A., et al., Angew. Chem. Int. Ed. Engl., 2006, 45, 7235-9; Patent inventions: Zanda, M., et al., Can. Pat. Appl. CA 2710693 (2011); Chai, Y, et al., Eur. Pat. Appl. 2174947 (2010), WO 2010034724; Leamon, C. et al., WO2010033733, WO 2009002993; Ellman, J., et al., PCT WO2009134279; WO 2009012958, US appl. 20110263650, 20110021568; Matschiner, G., et al., WO2009095447; Vlahov, I., et al., WO2009055562, WO 2008112873; Low, P., et al., WO2009026177; Richter, W., WO2008138561; Kjems, J., et al., WO 2008125116; Davis, M.; et al., WO2008076333; Diener, J.; et al., U.S. Pat.Appl. 20070041901, WO2006096754; Matschiner, G., et al., WO2006056464; Vaghefi, F., et al., WO2006033913; Doemling, A., Ger. Offen. DE102004030227, WO2004005327, WO2004005326, WO2004005269; Stanton, M., et al., U.S. Pat. Appl. Publ. 20040249130; Hoefle, G., et al., Ger. Offen. DE10254439, DE10241152, DE10008089; Leung, D., et al., WO2002077036; Reichenbach, H., et al., Ger. Offen. DE19638870; Wolfgang, R., US20120129779; Chen, H., US appl. 20110027274) We previously disclosed the construction of tubulysins conjugates (PCT/IB2012/053554) for targeted treatment of cancer, infection and autoimmune diseases. The present invention of tubulysin conjugates containing long branched (side-chain) linkers can prolong the half-life of the conjugates during the targeted delivery and minimize the exposure to non-target cells, tissues or organs during blood circulation, resulting in less off-target toxicity.

### SUMMARY OF THE INVENTION

The present invention relates to the conjugation of Tubulysin anologs to cell-binding molecules by using branched (side-chain) linkers, and the conjugates have better pharmacokinetic properties, and thus can more accurately target and kill abnormal cells. The invention also relates to the conjugation methods of the Tubulysin anologs and cell-binding agents, the methods of synthesizing Tubulysin molecules used therein, and methods for treating cancer, infection and autoimmune diseases in a targeted manner by using the conjugates.

In one respect, the present invention relates to an antibody-Tubulysin B derivative conjugate, wherein the conjugate has the structure of Formula (I): 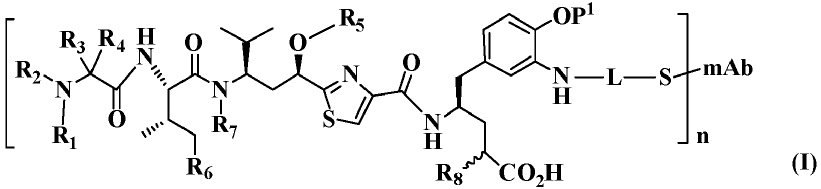 or a pharmaceutically acceptable salt, hydrate or hydrated salt of the structure represented by Formula (I); or a polymorphic crystalline of the structure represented by Formula (I); or an optical isomer of the structure represented by Formula (I), the structure of Formula (I) wherein one or more hydrogen (¹H) atoms substituted by deuterium (²H) atoms, or one or more ¹²C atoms substituted by ¹³C atoms;
wherein P¹ is H, COCH₃, COH, PO(OH)₂, CH₂OPO(OH)₂, SO₂CH₃, C₆H₁₁O₅ (glycosides), CONHCH₃, CON(CH₃)₂, CON(CH₂CH₂)₂NCH₃, CON(CH₂CH₃)₂ or CON(CH₂CH₂)₂CHN(CH₂CH₂)₂CH₂;
R₁, R₂, R₃ and R₄ are independently H, C₁-C₆ alkyl, C₁-C₆ alkenyl, C₁-C₆ alkylether (R₁OR₂), C₁-C₆ alkylcarbonyl (R₁COR₂), C₁-C₆ alkylester (R₁COOR₂), C₁-C₆ alkylcarboxy ((R₁COOH) or C₁-C₆ alkylamido group ((R₁CONHR₂);
or R₁ and R₂, R₁ and R₃, R₂ and R₃, or R₃ and R₄ forme a C₂-C₇ heterocyclic or C₂-C₇ cycloalkyl structure;
R₅ is H, O-C₁-C₆ alkyl, C(O)-H, C(O)-C₁-C₆ linear or branched alkyl, C(O)-NH-C₁-C₆ linear or branched alkyl or C(O)-N(C₁-C₆ linear or branched alkyl)₂;
R₆, R₇ and R₈ are independently H, C₁-C₆ alkyl, C₁-C₆ alkyl ether (R₁OR₂), C₁-C₆ alkylcarbonyl (R₁COR₂), C₁-C₆ alkyl ester (R₁COOR₂), C₁-C₆ alkylcarboxy (R₁COOH) or C₁-C₆ alkylamido group (R₁CONHR₂); preferably R₆, R₇ and R₈ are independently H or CH₃;
mAb is an antibody, antibody fragment, monoclonal antibody, polyclonal antibody, nanobody, prodrug antibody (probody), or an antibody or antibody fragment that is modified by a synthetic molecule or protein;
L is a linker containing a hydrophilic branched chain, which is composed of a C₂-C₁₀₀ peptide unit (1-12 natural or non-natural amino acids), a hydrazone, a disulfide, an ester, an oxime, an amide or a thioether bond.

In an another respect, L of present invention has the structure as below: 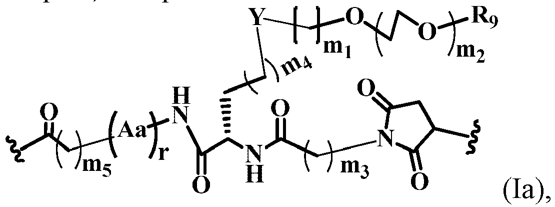 wherin Aa is a L- or D- natural or non-natural amino acid;
r is an integer between 0 and 12; when r is not 0, (Aa)r is a peptide unit composed of the same or different amino acids;
m₁ is an integer between 1 and 18; m₂ is an integer between 1 and 100; m₃ is an integer between 1 and 8; m₄ is an integer between 0 and 8; m₅ is an integer between 1 and 8;
Y is NHC(=O), NHS(O₂), NH(SO), NHS(O₂)NH, NHP(O)(OH)NH or C(O)NH;
R₉ is H, (O=)CR₁, (O=)CNHR₁, R₁COOH, R₁(COCH₂NH)ₘ₂H, R₁(Aa)ᵣ or R₁(COCH₂NCH₃)ₘ₂H; and
R₁, m₂ and (Aa)ᵣ are defined the same as above.

In addition, the cell surface receptor binding molecule mAb can be any forms of cell binding structrue, including peptides or peptide-like structures: an antibody, a single chain antibody; an antibody fragment that binds to the target cell; a monoclonal antibody; a single chain monoclonal antibody; or a monoclonal antibody fragment that binds to the target cell; a chimeric antibody; a chimeric antibody fragment that binds to the target cell; a domain antibody; a domain antibody fragment that binds to the target cell; genetically engineered protein that mimic antibodies; fibronectin binging agent adnectins; designed ankyrin repeat proteins (DARPins); a lymphokine; a hormone; a vitamin; a growth factor; a colony stimulating factor; a nutrient-transport molecule; transferrin; cell surface small molecular ligand; or albumin, polymer, dendrimer with a cell binding molecule attachd; or polymer materials, protain, liposomes, nanoparticles, vesicles, or (viral) capsids containing a cell-binding molecule (binding peptide, protein, antibody or cell surface small molecular ligand) on the surface.

### Preparation of cell-binding molecular-Tubulysin B anolog conjugates

In one embodiment, the synthesis of the cell-binding molecular-Tubulysin B anolog conjugate comprises one or more of the following steps: 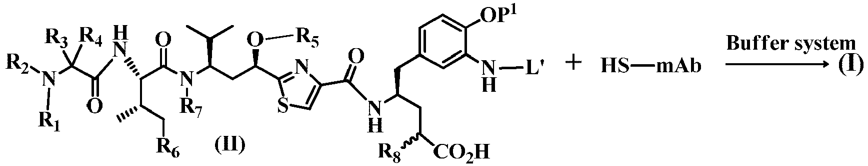 wherein P¹, " 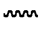 ", R₁, R₂, R₃, R₄, R₅, R₆, R₇ and R₈ in Formula (II) and mAb are defined the same as in Formula (I);
the structures of L' are (II-0) and (II-00): 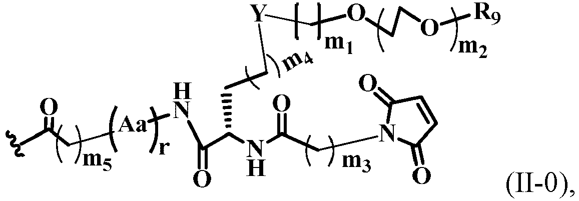 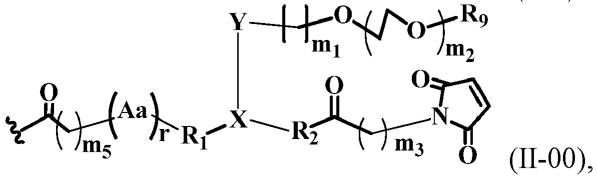 wherein m₁, m₂, m₃, m₄, m₅, Aa, r, Y and R₉ are defined the same as in Formula (I); preferably the structure of L' is: 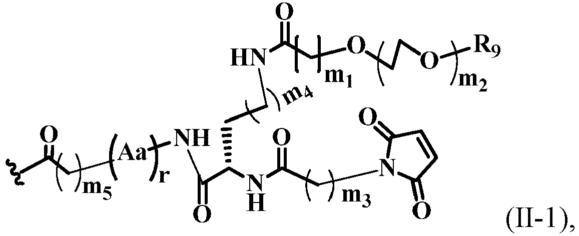 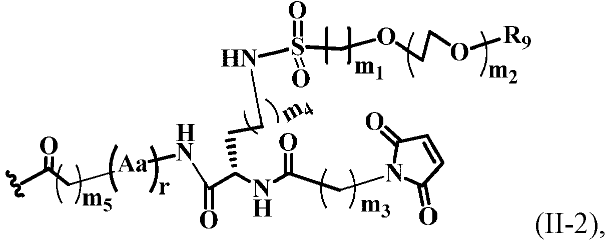 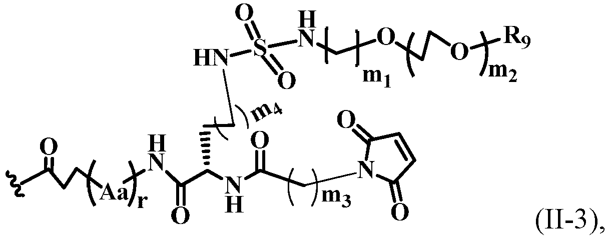 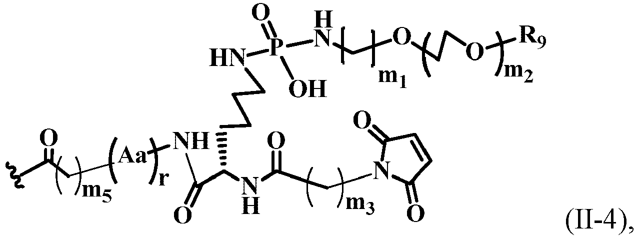 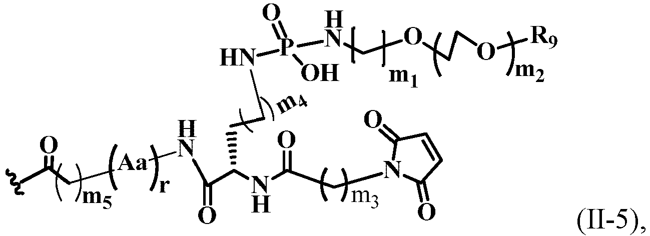 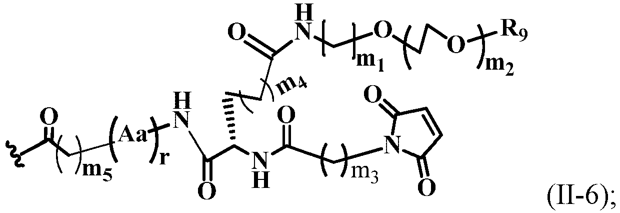 wherein m₁, m₂, m₃, m₄, m₅, Aa, r and R₉ are defined the same as above.

In another specific embodiment, in the process of preparing the above conjugates, the preparation of mAb-SH comprises any method of a)~c):
a) Reduction of the disulfide bonds between heavy and light chains, heavy and heavy chains or intra-disulfide bonds of an antibody, antibody fragment, monoclonal antibody, polyclonal antibody, nanobody, probody, or antibody or antibody fragment modified by synthetic molecules or proteins, by reducing agents (preferably tris (2-carboxyethyl) phosphine (TCEP ), dithiothreitol (DTT), dithioerythritol (DTE), L-glutathione (GSH), 2-mercaptoethylamine (β-MEA), or/and β-mercaptoethanol (β-ME, 2-ME));
b) Preparation of a sulfhydryl group by reaction of the amino group of antibody with Traut's reagent or thiolactone: 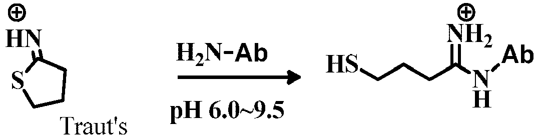 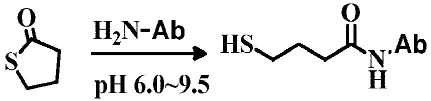
c) Incorporation of an easily reduced disulfide bond into the antibody by biochemical reaction in a buffer system, followed by reduction by TCEP, DTT, GSH, β-MEA or β-ME:

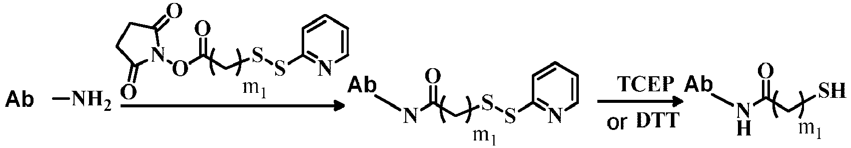

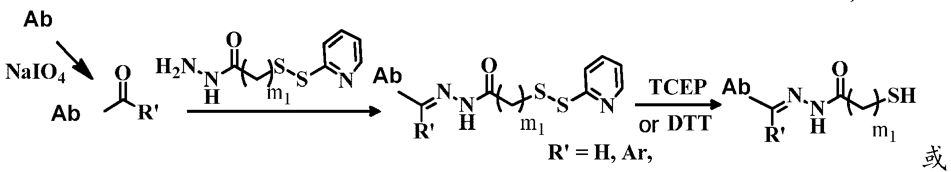

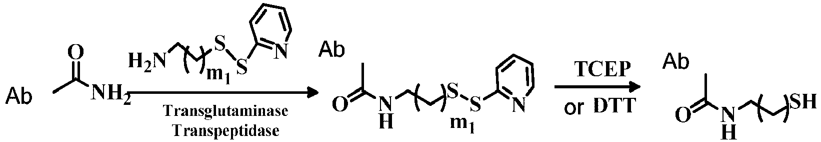

In another specific embodiment, in the process of preparing the conjugates above, wherein the buffer system used in the synthesis of the conjugates is pH 5.0-9.5, 1 mM ~ 1000 mM phosphoric acid, acetic acid, citric acid, boric acid, carbonic acid, barbituric acid, Tris (trimethylaminomethane), benzoic acid or triethanolamine system, or a mixed buffer solution thereof, and contains 0 to 35% water-soluble organic solvent of methanol, ethanol, n-propanol, isopropanol, n-butanol, isobutanol, acetonitrile, acetone, DMF, DMA or DMSO; and the reaction temperature is 0 °C to 45 °C, reaction time is 5 minutes to 96 hours.

In another specific embodiment, in the process of preparing the conjugates above, wherein the conjugates of Formula (I) are obtained by ultrafiltration or column chromatography purification after completion of the conjugation reactions. The purification column comprises a molecular sieve column, a cationic column, an anionic column, a hydrophobic (HIC) column, a reverse phase column or a protein A or G affinity column.

In another specific embodiment, in the process of preparing the above conjugates, the compound of Formula (II) is obtained by condensation reaction of a Tubulysin B derivative of Formula (III) with a compound of Formula (L'): 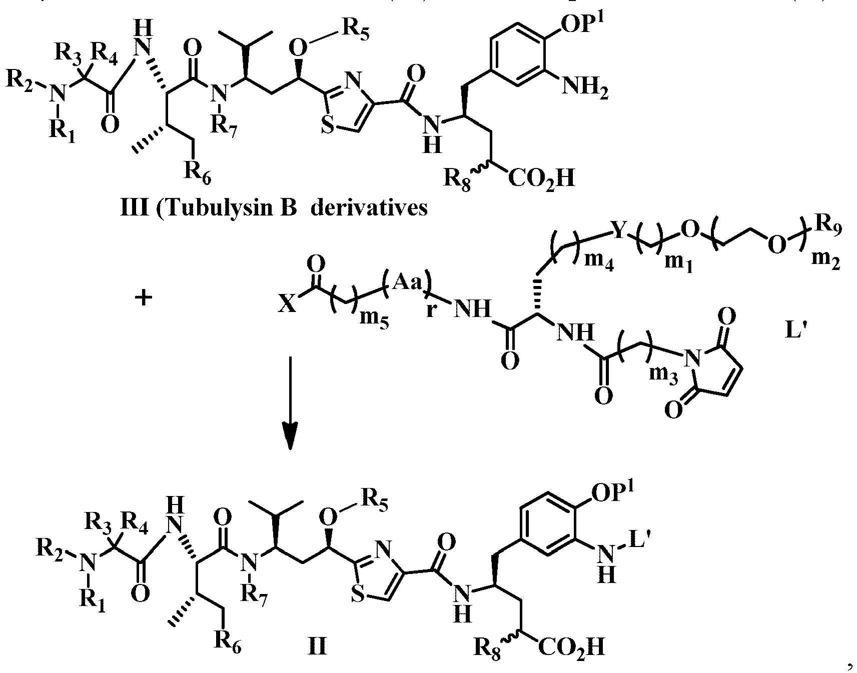 wherein X is OH, halogen (F, Cl, Br, or I), phenoxy, pentachlorophenoxy, trifluoromethanesulfonyl, imidazole, dichlorophenoxy, tetrachlorophenoxy, 1-hydroxybenzotriazole, p-toluenesulfonyl, methanesulfonyl, 2-ethyl-5-phenyl isoxazole -3'- sulfonyl group, 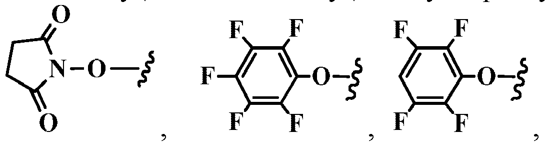 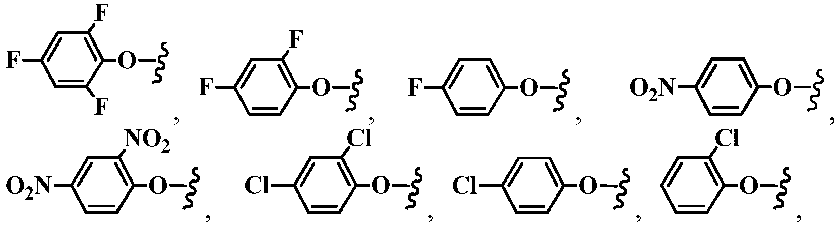 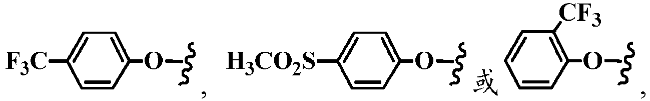 an anhydride formed by itself or with other anhydrides, such as acetyl anhydride and formic anhydride; or a peptide coupling reaction intermediate or a Mitsunobu reaction intermediate;
wherein the condensation reaction is carried out in dichloroethane, DMF, DMA, tetrahydrofuran (THF), DMSO, acetone, isopropanol, *n*-butanol or acetonitrile, or above two or three mixed solvents, containing 1 to 100% pyridine, triethylamine or diisopropylethylamine; with or without inert gas (nitrogen, argon, helium) protection, at -20 to 150 °C, for 5 minutes to 120 hours;
Alternatively, the buffer system is pH 5.0 ~ 9.5, 1 mM ~ 1000 mM phosphoric acid, acetic acid, citric acid, boric acid, carbonic acid, barbituric acid, Tris (trishydroxymethyl aminomethane), benzoic acid or triethanolamine system, or a mixture thereof, containing 0 to 35% miscible organic solvents, such as methanol, ethanol, n-propanol, isopropanol, *n*-butanol, isobutanol, acetonitrile, acetone, DMF, DMA or DMSO. The reaction temperature is 0 to 45 °C and reaction time is from 5 minutes to 96 hours.

Alternatively, wherein the NH₂ group of formula (III) participates in the conjugation reaction in a form of salt, with trifluoroacetic acid, hydrochloride acid, formic acid, acetic acid, sulfuric acid, phosphoric acid, nitric acid, citric acid, succinic acid, benzoic acid or sulfonic acid.

when X is OH, the proceeding of the condensation reaction req.uires a condensation reagent, selected from (N-(3-dimethylaminopropyl)-N'-ethylcarbodiimide) (EDC), dicyclohexylcarbodiimide (DCC), N, N'-diisopropylcarbodiimide (DIC), N-cyclohexyl-N'-(2-morpholino-ethyl)carbodiimide p-toluenesulfonate (CMC or CME-CDI), carbonyldiimidazole (CDI), O-benzotriazole-N, N, N', N'-tetramethylurea tetrafluoroborate (TBTU), O-benzotriazole-tetramethylurea hexafluorophosphate (HBTU), (benzotriazol-1-yloxy)tris(dimethylamino)-phosphonium hexafluorophosphate (BOP), (benzotriazol-1-yloxy) tripyrrolidinophosphonium hexafluorophosphate (PyBOP), diethyl pyrocarbonate (DEPC), N, N, N', N'-tetramethylchlorobenzamidine hexafluorophosphate, 2-(7-oxobenztriazole)-N, N, N', N'-tetramethyluronium hexafluorophosphate (HATU), 1*-*[(dimethylamino*)* (morpholinyl) methylene ] -1 [1,2,3] triazolo [4,5-b] 1-pyridin-3-oxyhexafluorophosphate (HDMA), 2-chloro-1,3-dimethylimidazolium hexafluorophosphate (CIP), chlorotripyrrolidinylphosphonium hexafluorophosphate (PyClOP), bis (tetramethylene) fluoroformamide (BTFFH), N, N, N', N'-tetramethyl-sulfur-(1-oxo-2-pyridinyl) thiuronium hexafluorophosphate, 2-(2-pyridone-1-yl) -1,1,3,3-tetramethylurea tetrafluoroborate (TPTU), sulfur-(1-oxo-2-pyridinyl)-N, N, N', N'-tetramethylthiourea hexafluorophosphate, O-[(ethoxycarbonyl) cyanamide ]-N, N, N', N'-tetramethylthiourea hexafluorophosphate (HOTU), (1-cyano-2-ethoxy-2-oxoiminoyloxy) dimethylaminomorpholinecarbenium hexafluorophosphate (COMU), O-(benzotriazol-1-yl)-N, N, N', N'-bis(tetramethylene)uronium hexafluorophosphate (HBPyU), N-benzyl- N'-cyclohexyl carbodiimide (or on solid support), dipyrrolidino (N-succinimidyloxy) carbenium hexafluorophosphate (HSPyU), 1-(chloro-1-pyrrolidinylmethylene) pyrrolidinium hexafluorophosphate (PyClU), 2-chloro -1,3-dimethylimidazolium tetrafluoroborate (CIB), (benzotriazol-1-yloxy) dipiperidinylcarbon hexafluorophosphate (HBPipU), (N, N, N', N'-tetramethyl-O-(6-chloro-1H-benzotriazol-1-yl)uronium tetrafluoroborate (TCTU), bromotris(dimethylamino)phosphonium hexafluorophosphate (BrOP), 1-*n*-propylphosphorus anhydride (PPACA, T3P^{®}), 2-isocyanoethylmorpholine (MEI), N,N,N',N'-tetramethylurea-oxy-(N-succinimidyl) hexafluorophosphate (HSTU), 2-bromo-1-ethylpyridine tetrafluoroborate (BEP), oxygen-[(ethoxycarbonyl) cyanomethylamine]-N,N,N',N'-tetramethylthiourea tetrafluoroborate (TOTU), 4-(4,6-dimethoxytriazin-2-yl)-4-methylmorpholine hydrochloride (MMTM, DMTMM), 2-succinimidyl -1,1,3,3-tetramethylurea tetrafluoroborate (TSTU), N, N, N', N'-tetramethyl-O-(3,4-dihydro-4-oxo -1, 2) 3-benzotriazine-3-yl) urea tetrafluoroborate (TDBTU), azodicarbonyldipiperidinyl (ADD), bis (4-chlorobenzyl) azodicarboxylate (DCAD), di-tert-butyl azodicarboxylate (DBAD), diisopropyl azodicarboxylate (DIAD) or diethyl azodicarboxylate (DEAD)

In another specific embodiment, in the process of preparing the conjugates above, the synthesis of the Tubulysin B derivatives of formula (III) comprises one or more of the following steps: 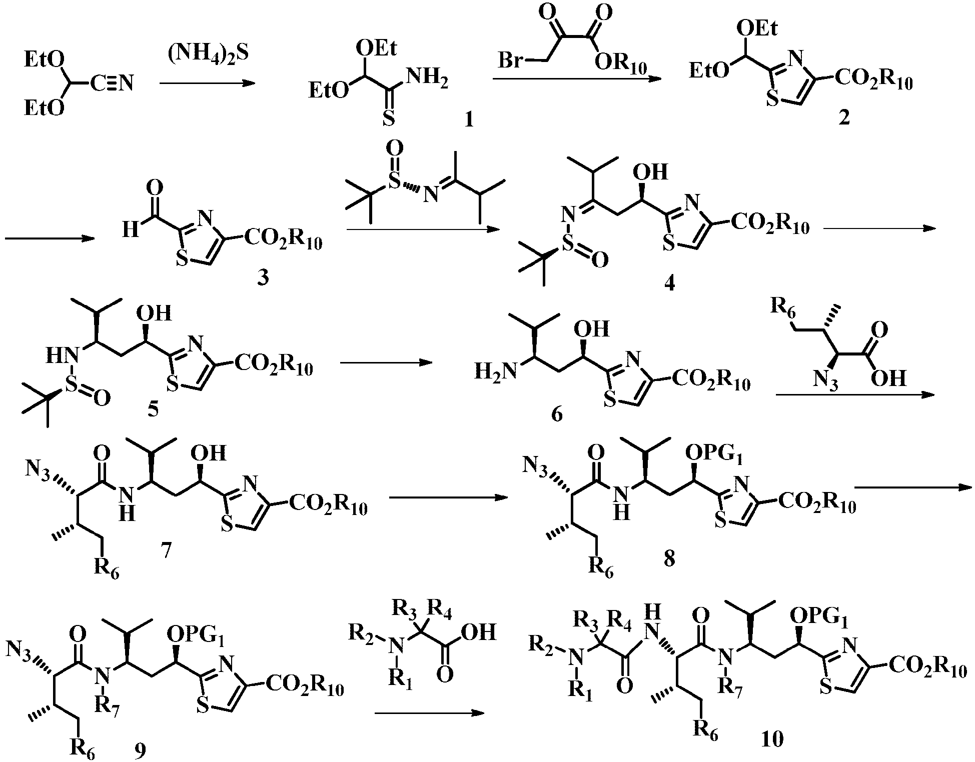 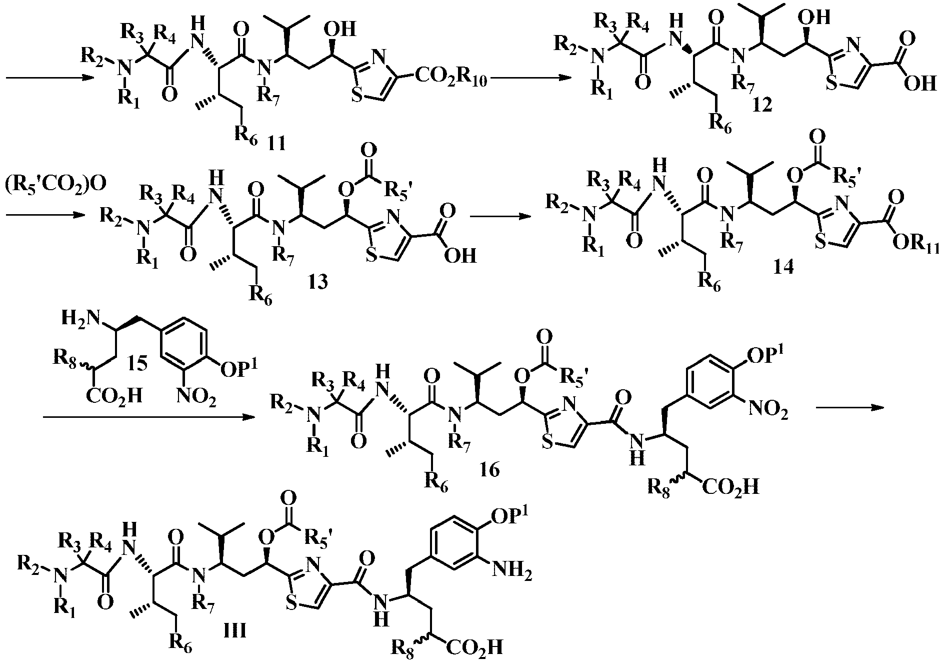 wherein R₅' is H, C₁-C₆ alkyl, C₁-C₆ alkenyl, or C₁-C₆ linear or branched aminoalkyl group; the other groups are defined the same as above.

Preferably, the synthesis of the Tubulysin B derivatives of formula (III) comprises one or more of the following steps:
Step 1. Diethoxyacetonitrile and aqueous ammonium sulfide are stirred at room temperature to yield compound 1, 2,2-diethoxythioacetamide; 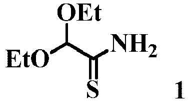
Step 2. Compound 1 and bromopyruvate in an anhydrous solvent (such as anhydrous tetrahydrofuran, dichloromethane, acetonitrile, N, N-dimethylformamide, methanol or isopropanol) are heated and condensed to yield compound **2;** 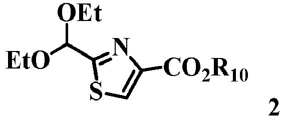
Step 3. Compound 2 is dissolved in a solvent (such as tetrahydrofuran, dichloromethane, ethyl acetate, n-heptane, dioxane, acetonitrile) and hydrolyzed in the presence of a Lewis acid or protonic acid (such as hydrochloric acid, sulfuric acid, phosphoric acid, methanesulfonic acid, formic acid, oxalic acid, acetic acid, *p*-toluenesulfonic acid, pyridinium *p*-toluenesulfonate, AlCl₃, FeCl₃, ZnCl₂, BF₃, BCl₃, BBr₃, TiCl₄, ZnBr₂ or LiBF₄), to yield compound **3;** 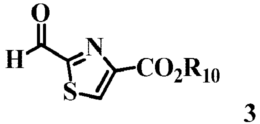
Step 4. The sulfinamide is deprotonated by *n*-butyllithium under low temperature (such as -45 to -78°C), and then condensed with compound **3** in the presence of a Lewis acid, to yield compound **4** (Adol reaction); 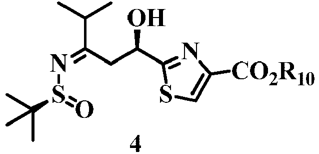 The acids are selected from hydrochloric acid, sulfuric acid, phosphoric acid, methanesulfonic acid, formic acid, oxalic acid, acetic acid, *p*-toluenesulfonic acid, *p*-toluenesulfonic acid pyridine, AlCl₃, FeCl₃, ZnCl₂, BF₃, BCl₃, BBr₃, TiCl₄, ZnBr₂, LiBF₄;
Step 5. Compound **4** is selectively reduced at low temperature (for example, -45 to -78°C) by a reducing reagent (such as NaBH₄, LiBH₄, Na(OAc)₃BH, Na(CN)BH₃ etc.), to yield compound ***5,*** and a Lewis acid (for example Ti(OEt)₄) is added to control the stereochemistry outcome. 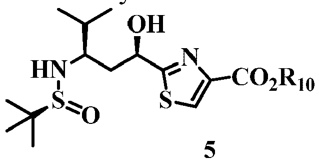
Step 6. Compound ***5*** is dissolved in a solvent (such as methanol, ethanol, isopropanol, tetrahydrofuran or acetonitrile), and *tert*-butylsulfinyl group is removed by acid such as hydrochloric acid, sulfuric acid and phosphoric acid, to yield compound ***6.*** 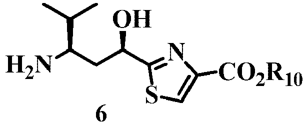
Step 7. In presence of a condensation reagent (such as DIC/HOBt, DCC/HOBt, EDC/HOBt, HATU, BOP, T3P or BrOP), compound **6** and azido acid in a solvent (for example, n-heptane, tetrahydrofuran, dichloromethane, N, N-dimethylformamide) are condensed, to yield compound **7*;***
   Alternatively, azido acid reacts with isobutyl chloroformate in THF, in the presence of an organic base (such as triethylamine, diisopropylethylamine, N-methylmorpholine, etc.), to yield a mixed anhydride, which condenses with the hydrochloride salt of compound ***6*** to afford ***7;***
   Alternatively, azido acid in a solvent (such as *n*-heptane, *n*-hexane, dichloromethane or tetrahydrofuran) reacts with oxalyl chloride, in presence of triethylamine and DMF (catalytic amount), to produce acyl chloride, which then condenses with compound 6 (hydrochloride salt to afford ***7.*** 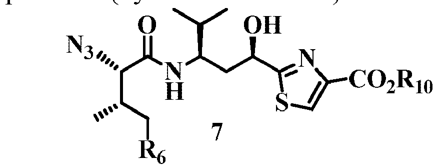
Step 8. In a solvent (such as dichloromethane, tetrahydrofuran or acetonitrile), the hydroxyl group of compound **7** reacts with a hydroxyl protecting reagent (such as using TESCl), in the presence of an organic base (such as imidazole, triethylamine or pyridine), to yield the protected compound **8;** 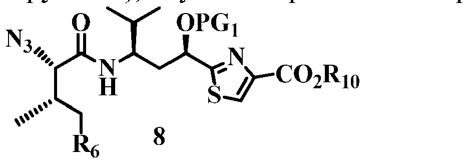
Step 9. Compound **8** in a solvent (such as tetrahydrofuran, dichloromethane or acetonitrile) is deprotonated with added base (such as KHMDS, LiHMDS, NaHMDS, KOtBu, NaH or KH), and then alkylated with iodomethane, bromomethane, dimethyl sulfate, methyl trifluoromethanesulfonate, iodoethane and the like to yield compound **9;** 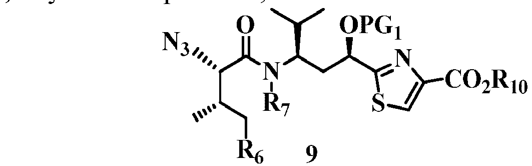
Step 10. Compound 9 is dissolved in a solvent (such as tetrahydrofuran, dichloromethane or ethyl acetate), wherein the azido group is reduced to an amino group under certain conditions, such as H₂ and Pd/C, triphenylphosphine and water (Staudinger reaction) and then condensed with an acid or an acid derivative having similar reactivity, to afford compound **10**; 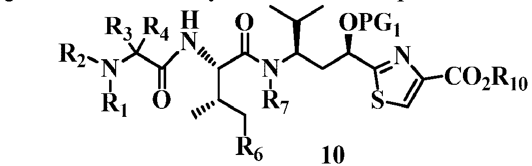
Step 11. The hydroxyl protecting group PG₁ of compound **10** can be deprotected under appropriate conditions (for example, TES protecting group may be deprotected in HCl, THF/MeOH/AcOH, *ⁿ*Bu₄NF or HF-pyridine in THF) to yield compound 11; 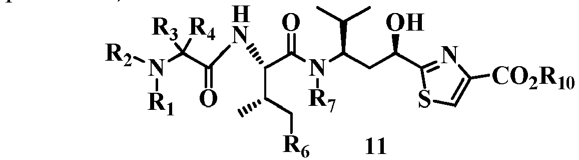
Step 12. The ester compound 11 is converted to acid 12, being subjected to a base (such as LiOH, NaOH or KOH) or other suitable conditions (such as methyl ester can be converted to carboxylic acid by LiCl, LiI, Me₃SiOK, and the like); 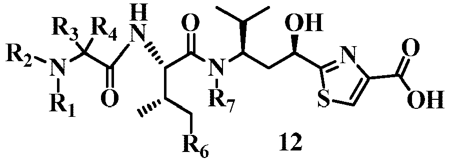
Step 13. In the presence of a base (such as triethylamine, N, N-diisopropylethylamine or pyridine) and a catalyst (such as DMAP), compound **12** reacts with anhydride, such as acetic anhydride, propionic anhydride, iso-propionic anhydride, or acyl halide, such as acetyl halide, propionyl halide, carbamoyl halide, methylcarbamoyl halide, ethylcarbamoyl halide, dimethylcarbamoyl halide, at certain temperature (such as 0°C to 23°C), to yield compound **13,** and the reaction may take place without base or catalyst; 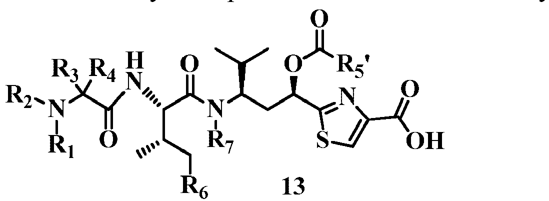
Step 14. Compound **13** condenses with hydroxyl-containing compound such as pentafluorophenol or N-hydroxysuccinimide in the presence of a condensation reagent, such as EDC, DIC, DCC, HATU, HBTU, to yield a reactive ester **14;** 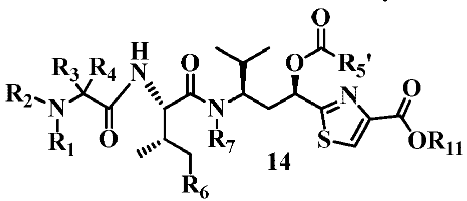
Step 15. Compound **15** and compound **14** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **16.** Optionally a base is not req.uired for the reaction to proceed, but the reaction temperature (from °C to 23°C) and reaction time (from 30 minutes to 18 hours) need to be tightly controlled; 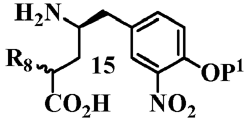 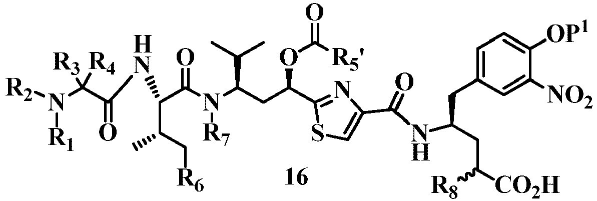
Step 16. The nitro group of compound **16** is reduced to an amino group under reduction conditions, such as H₂ and Pd/C catalyst, hydrazine hydrate and FeCl₃, iron powder and acetic acid, and the like, to yield compound III.

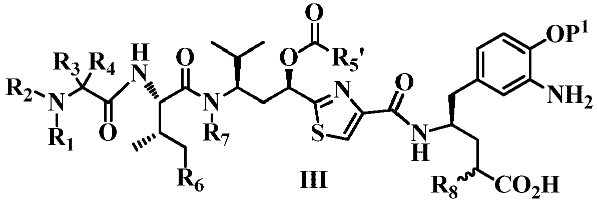

In another specific embodiment, in the process of preparing of the above conjugates, the synthesis of compounds of Formula (L') comprises one or more of the following steps:

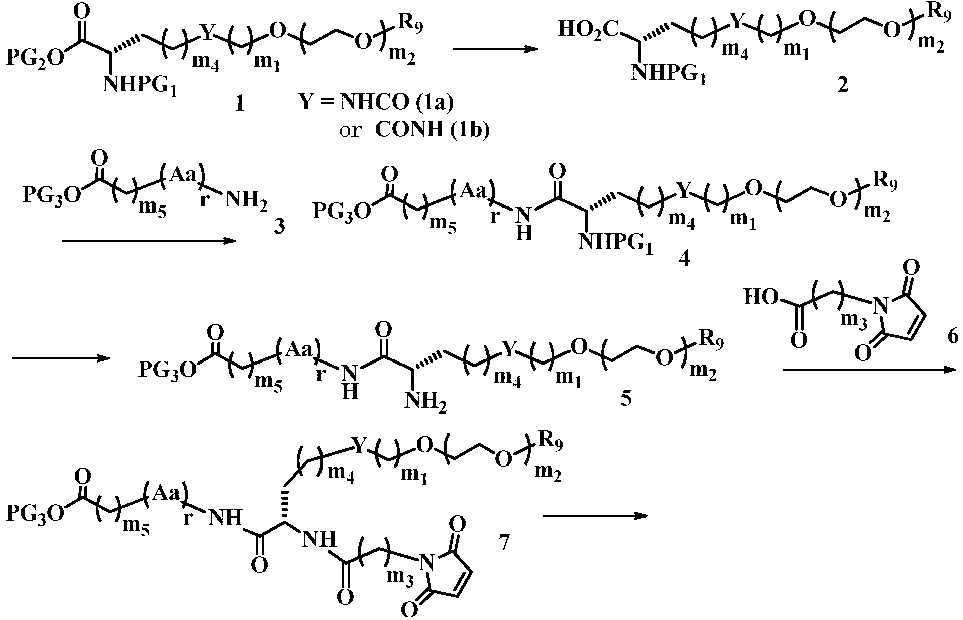

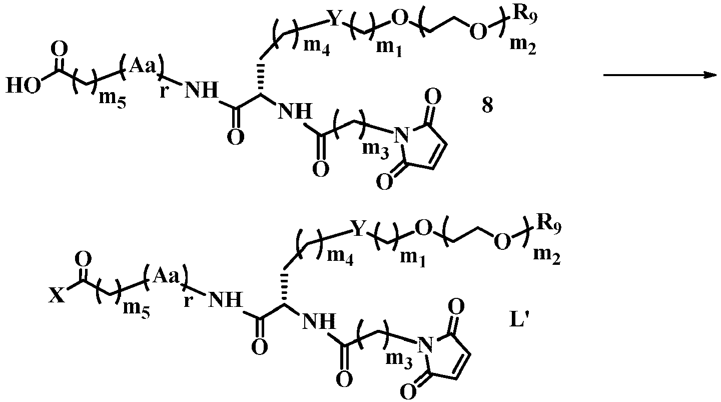

Preferably, the synthesis of the compounds of Formula (L') comprises one or more of the following steps:
Step 1. Compound **1-1** and compound **1-2** condense directly in the presence of a condensation reagent (such as EDC, HATU, DIC or DCC), or by reacting compound **1-2** with pentafluorophenol, nitrophenol or N-hydroxysuccinimide, to yield corresponding active eater in the presence of a condensation reagent such as DIC or EDC, and then reacting with compound **1-1,** to yield **la;**
   Alternatively, compound **1-3** and compound **1-4** condense directly, in the presence of a condensation reagent (such as EDC, HATU, DIC or DCC), or via other indirect condensation reaction routes, to yield compound **1b**; 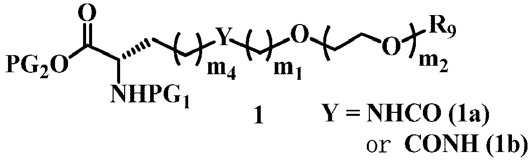 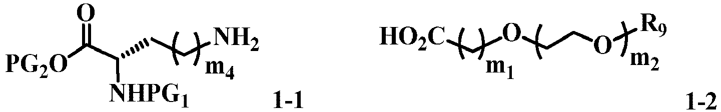 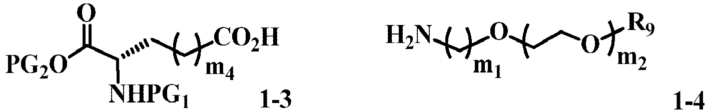
Step 2. The carboxyl protecting group PG₂ of compound **1** is removed by a deprotection reagent (such as to remove tert-butyl ester group by an acid), to yield compound **2;** 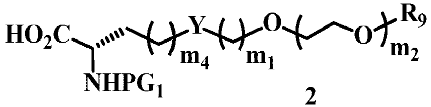
Step 3. An acid compound **2** and an amine compound **3** condense in the presence of a condensation reagent (such as EDC, HATU, DIC or DCC), or via other indirect condensation reaction routes, to yield compound **4;** 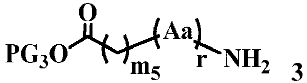 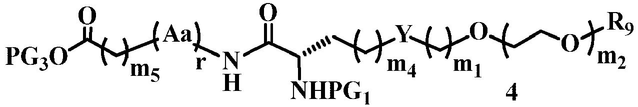
Step 4. The amino protecting group PG₁ of compound **4** is removed under deprotection conditions, such as H₂ and Pd/C catalyst for Cbz protecting group, and acidic conditions for Boc protecting group, to yield compound **5;** 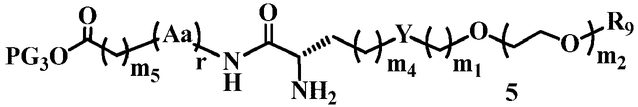
Step 5. Carboxylic acid **6** and amine **5** condense in the presence of a condensation reagent (such as EDC, HATU, DIC or DCC), or via other indirect condensation reaction routes, to yield compound **7;** 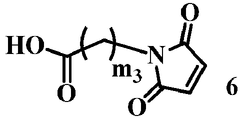 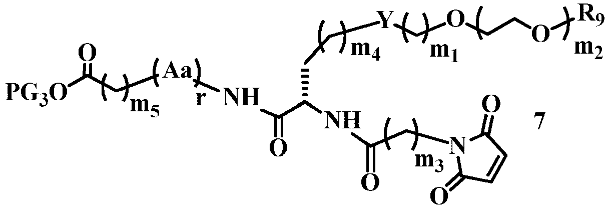
Step 6. The carboxyl protecting group PG₃ of compound **7** is removed under deprotection conditions, such as acid (formic acid, acetic acid, trifluoroacetic acid, hydrochloric acid, phosphoric acid and the like) for tert-butyl ester protecting group, to yield compound **8;** 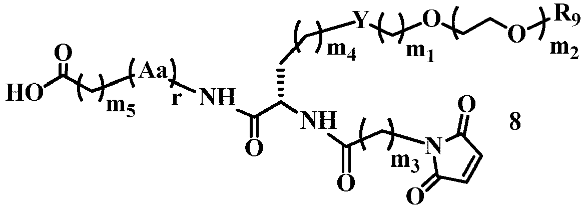
Step 7. Compound **8** reacts with a compound containing a hydroxyl group (such as pentafluorophenol or N-hydroxysuccinimide), in the presence of a condensation reagent (such as EDC, HATU, DIC or DCC), or reacts with other carboxylic acid activating compounds, to yield a reactive ester L'.

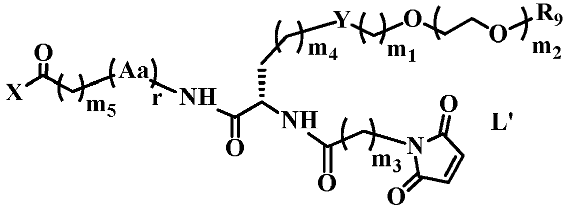

In another specific embodiment, the synthesis of compounds of Formula (L') comprises one or more of the following steps:

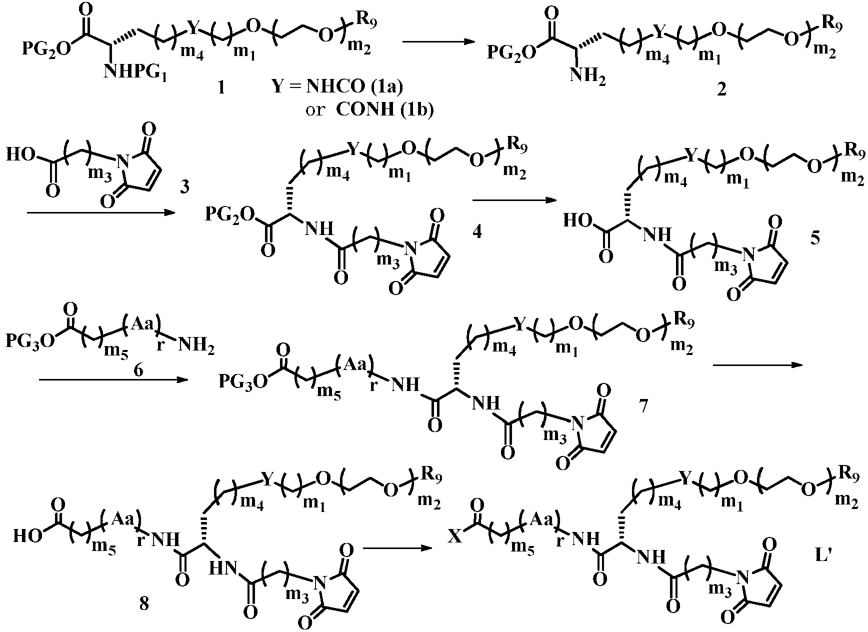

Preferably, **the synthesis of** the compounds of Formula (L') comprises one or more of the following steps:
Step 1. The amino protecting group PG₁ on compound **1** is removed under deprotection condition, such as H₂ and Pd/C catalyst for Cbz protecting group, and acidic conditions for Boc protecting group, to yield compound **2;** 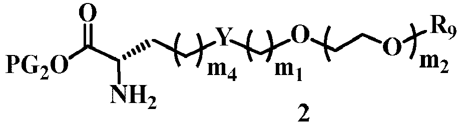
Step 2. Amino compound **2** and carboxylic acid 3 condense in the presence of a condensation reagent (such as EDC, HATU, DIC or DCC), or via other indirect condensation reaction routes, to yield compound **4**; 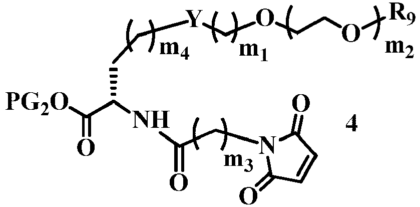
Step 3. The carboxyl protecting group PG₂ of compound **4** is removed under deprotection conditions, such as acid (formic acid, acetic acid, trifluoroacetic acid, hydrochloric acid, phosphoric acid and the like) for the cleavage of tert-butyl ester protecting group, to yield compound **5**; 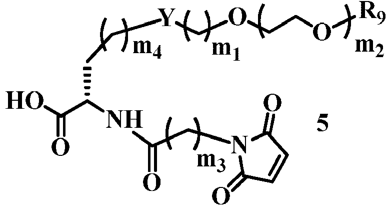
Step 4. Carboxylic acid **5** and amine **6** condense in the presence of a condensation reagent (such as EDC, HATU, DIC or DCC), or via other indirect condensation reaction routes, to yield compound **7;** 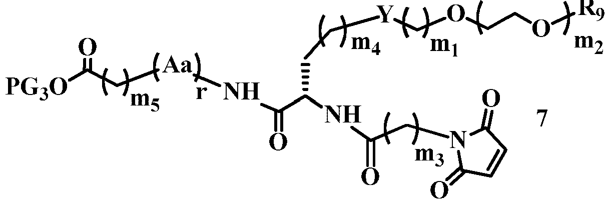
Step 5. The carboxyl protecting group PG₃ of compound **7** is removed under deprotection conditions, such as acid (formic acid, acetic acid, trifluoroacetic acid, hydrochloric acid, phosphoric acid and the like) for the cleavage of tert-butyl ester protecting group, to yield compound **8;** 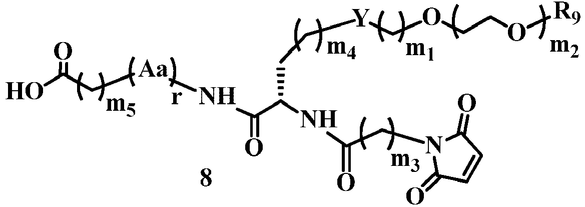
Step 6. Compound **8** reacts with a compound containing a hydroxyl group (such as pentafluorophenol or N-hydroxysuccinimide), in the presence of a condensation reagent (such as EDC, HATU, DIC or DCC), or reacts with other carboxylic acid activating compounds, to yield a reactive ester **9;**

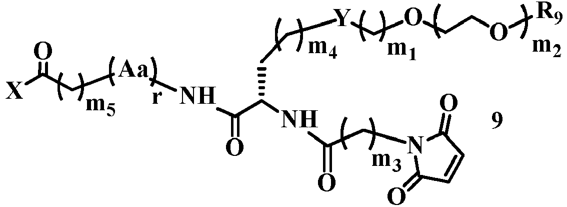

In another specific embodiment, in the process of preparing of the above conjugates, the synthesis of compounds of Formula (II) is achieved by the condensation reaction of compounds of Formula (IV) and Formula (V): 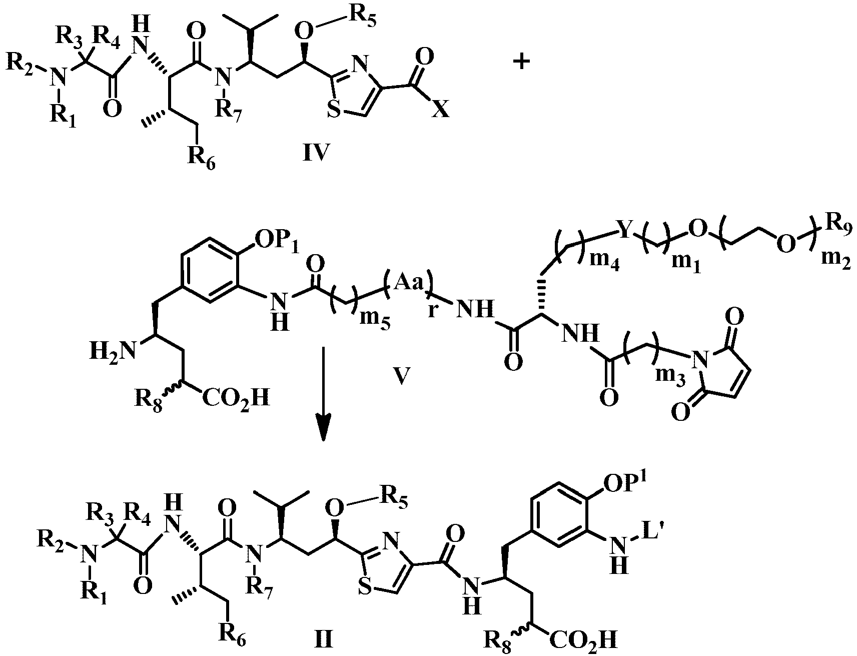 wherein the definition of X and the condensation reaction conditions are the same as above.

The NH₂ group of Formula (V) participates in the conjugation reaction is in a form of salt, preferably with trifluoroacetic acid, hydrochloride acid, formic acid, acetatic acid, sulfuric acid, phosphoric acid, nitric acid, citric acid, succinic acid, benzoic acid or sulfonic acid.

In another specific embodiment, in the process of preparing of the above conjugates, the synthesis of the compound of Formula (IV) comprises one or more of the following steps:

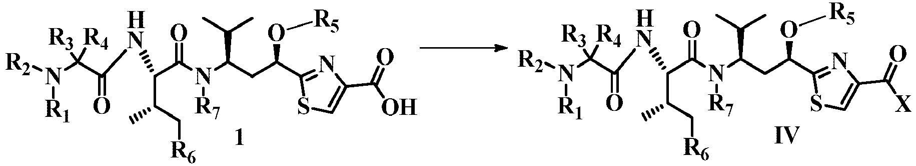

Preferably, the synthesis of compounds of Formula (IV) comprises one or more of the following steps:
Carboxylic acid **1** and the compound with a hydroxyl group (such as pentafluorophenol or N-hydroxysuccinimide) condense in the presence of a condensation reagent (such as EDC, DIC, DCC, HATU or HBTU) to yield a reactive ester;
Alternatively, carboxylic acid **1** reacts with ethyl chloroformate, isobutyl chloroformate, etc., in the presence of an organic base (such as N-methylmorpholine, triethylamine, diisopropylethylamine, etc.) to yield a reactive mixed anhydride;
Alternatively, carboxylic acid **1** reacts with oxalyl chloride, in the presence of an organic base such as trimethylamine, and catalytic amount of DMF (such as 0.01 eq. to 0.5 eq.) to yield an acyl chloride.

In another specific embodiment, in the process of preparing of the above conjugates, the synthesis of the compounds of Formula (V) comprises one or more of the following steps:

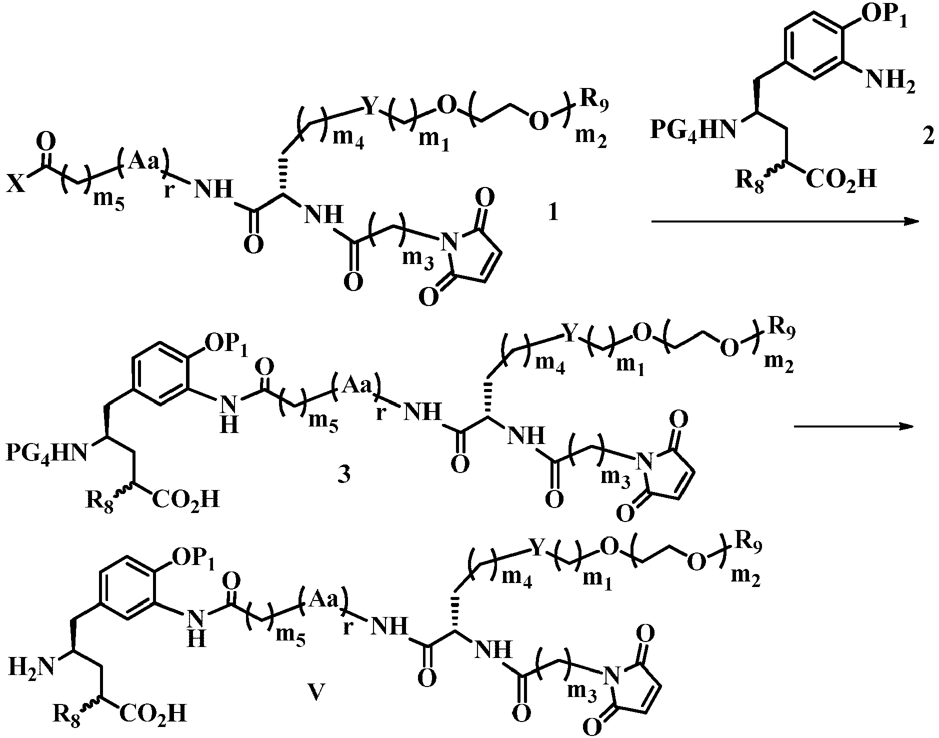

Preferably, the synthesis of compounds of Formula (V) comprises one or more of the following steps:
Step 1. Compound **1** and compound **2** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound 3. Optionally a base is not req.uired for the reaction to proceed, but the reaction temperature and reaction time need to be tightly controlled 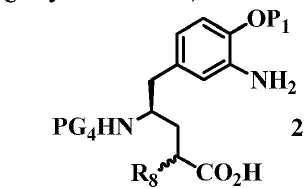 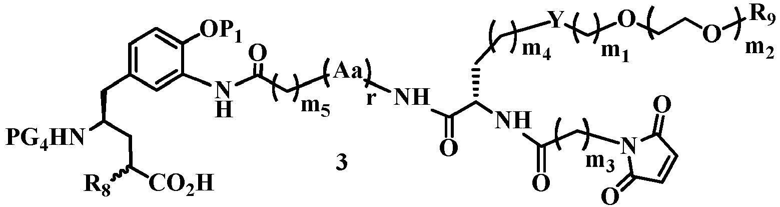
Step 2. The amino protecting group PG₄ of compound **3** is removed under deprotection conditions, such as H₂ and Pd/C catalyst for Cbz protecting group, and acidic conditions for Boc protecting group, to yield compound V;

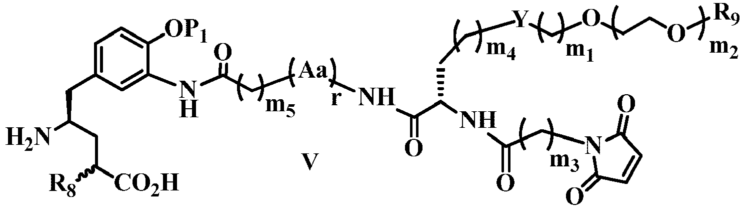

In another specific embodiment, in the process of preparing of the above conjugates, the synthesis of compound **2** comprises one or more of the following steps: 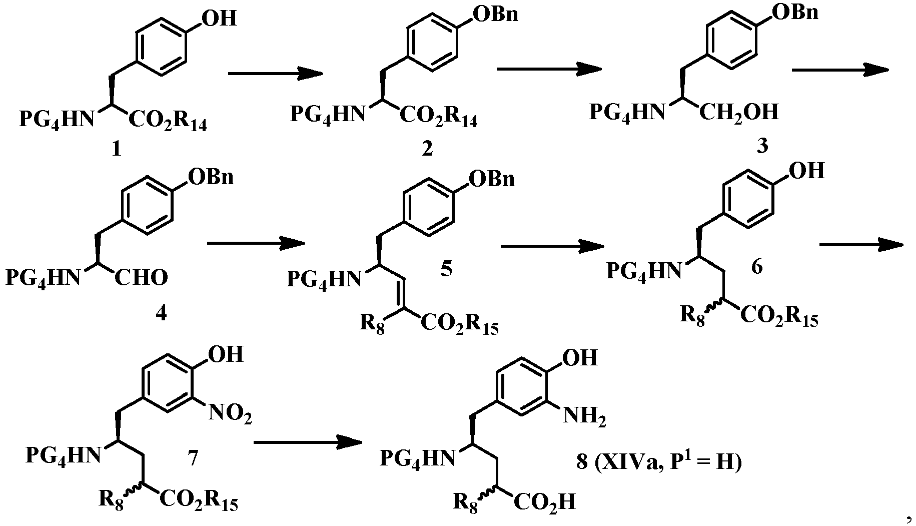 wherein compound **8** (compound XIVa) is compound **2** in the previous embodiment; PG₄ is an amino protecting group.

Preferably, the synthesis of compound **2** comprises one or more of the following steps:
Step 1. To an ester derivative of L-tyrosine **(1)** in an appropriate solvent (such as acetone, tetrahydrofuran, acetonitrile, dichloromethane, etc.) or a solvent mixture of any above solvent and water, is added benzyl chloride, benzyl bromide or other benzyl compound at 0 ~ 60 °C, followed by an organic or inorganic base, such as sodium carbonate, potassium carbonate, sodium bicarbonate, potassium bicarbonate, sodium hydroxide, potassium hydroxide, triethylamine, DBU, sodium hydride and the like, and optionally an appropriate additive, such as sodium iodide or a phase transfer catalyst, such as benzyltriethylammonium chloride (TEBA), tetrabutylammonium bromide (TBAB), tetrabutylammonium ammonium chloride, tetrabutylammonium bisulfate, trioctylmethylammonium chloride, dodecyl trimethyl ammonium chloride, tetradecyl trimethyl ammonium chloride and the like, to yield compound **2;**
Step 2. Compound **2** is dissolved in an organic solvent (such as dichloromethane, tetrahydrofuran, methanol, ethanol, ether and the like), and then reacts with a reducing reagent, such as LiAlH₄, DIBAL, NaBH₄, LiBH₄, sodium bis(2-methoxyethoxy)aluminumhydride (Red-Al), diborane boroethane, etc., optionally in the presence of an addctive (such as I₂, FeCl₃, ZnCl₂, MgCl₂, LiCl or CaCl₂) to tune the activity of the reducing reagent, to yield compound **3;**
Step 3. Alcohol **3** is oxidized, under oxidation conditions such as Swern oxidation (oxalyl chloride, DMSO, triethylamine), Parikh-Doering oxidation (sulfur trioxide), Dess-Martin oxidation and the like, to yield aldehyde **4;**
Step 4. Aldehyde **4** reacts with a phosphate ester (Horner-Wadsworth-Emmons reaction) or a phosphorus ylide (Wittig reaction) to elongate the carbon chain and yield compound **5;**
Step 5. The double bond of compound **5** is reduced, in the presence of a homogeneous or heterogeneous catalyst, wherein the benzyl group is also removed, to yield a stereochemically pure compound, or a mixture of two diastereomers; the heterogeneous catalysts include Pd/C, Pd(OH)₂/C, Pd/BaSO₄, PtO₂, Pt/Al₂O₃, Ru/C, Raney Ni and the like, and the homogeneous asymmetric hydrogenation catalysts include Crabtree catalyst, [Ru (II)-(BINAP) ]-type catalyst, [(Ph₃P)CuH]₆, and the like;
Step 6. Compound 6 is dissolved in an organic solvent, such as tetrahydrofuran, acetonitrile, dichloromethane, and undergoes nitration. The nitration reagents include nitric acid, nitric acid/acetic acid, potassium nitrate/sulfuric acid, tert-butyl nitrite, nitric acid/trifluoroacetic anhydride, NO₂BF₄, nitropyridine, and the like;
Step 7. The nitro group of compound **7** is reduced to an amino group, under a suitable condition, including H₂/Pd/C, Fe or Zn/HOAc, or SnCl₂/HCl.

In another specific embodiment, in the process of preparing of the above conjugates, the synthesis of compound **2** comprises one or more of the following steps: 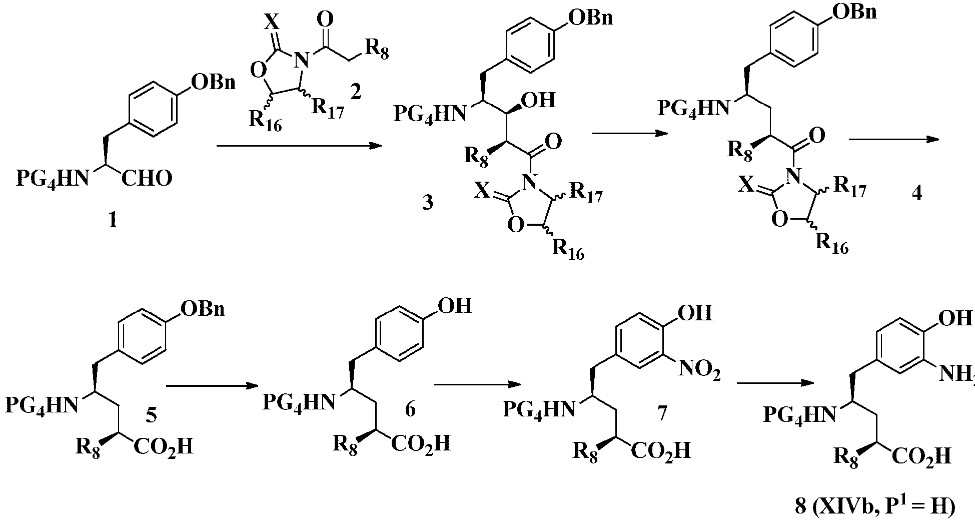 wherein compound **8** (Compound XIVb) is compound **2.**

Preferably, synthesis of compound 2 comprises one or more of the following steps:
Step 1. Compound **1** undergoes Aldol reaction with Evans' chiral N-acyl oxazolidinone or thioketone **2** at -78 °C to -45°C, to yield a stereochemically pure compound **3,** wherein X = O or S, R₁₆ = H, methyl, phenyl, R₁₇ = H, methyl, isopropyl, phenyl, benzyl, and the like;
Step 2. The hydroxyl group of compound **3** is deoxygenated under Barton-McCombie deoxygenation conditions, i.e. the alcohol is first converted to a thiocarbonyl derivative, such as alkyl xanthate, phenyl carbothioate, imidazole carbothioate, and then treated with Bu₃SnH to undergo radical cleavage and afford the dehydrogenation product. The conditions of radical cleavage include *n*-Bu₃SnH/AIBN, *n*-Bu₃SnH/AIBN/*n*-BuOH/PMHS and (Bu₄N)₂S₂O₈/HCO₂Na;
Step 3. Compound **4** is dissolved in tetrahydrofuran and the Evans chiral auxiliary group is cleaved by LiOH/H₂O₂, to yield the corresponding acid **5;**
Step 4. Compound **5** is dissolved in an organic solvent (such as ethyl acetate, methanol, dichloromethane, ethanol or acetic acid, etc.) and hydrogenated in the presence of Pd/C catalyst, wherein the benzyl group is also removed, to yield compound **6;**
Step 5. Compound **6** is dissolved in an organic solvent, such as tetrahydrofuran, acetonitrile, dichloromethane, and undergoes nitration. The nitration reagents include nitric acid, nitric acid/acetic acid, potassium nitrate/sulfuric acid, tert-butyl nitrite, nitric acid/trifluoroacetic anhydride, NO₂BF₄, nitropyridine, and the like;
Step 6 . The nitro group of compound **7** is converted to amino group, under a suitable condition, such as H₂/Pd/C, Fe or Zn/HOAc or SnCl₂/HCl, to yield stereochemically pure compound **8**.

In another specific embodiment, in the process of preparing of the above conjugates, the compound of formula (II) is obtained by condensation reaction of compounds of Formula (VI) and Formula (VII): 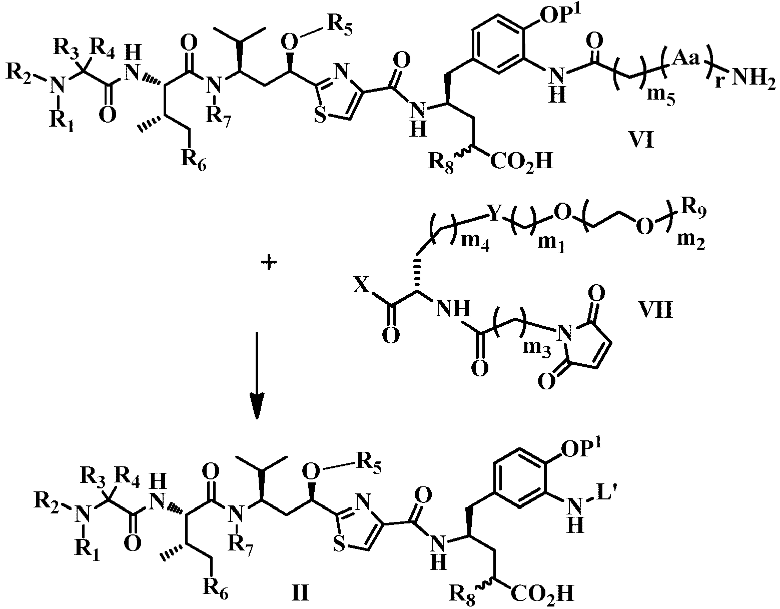 wherein the defination of X and condensation reaction conditions are the same as above;
wherein the synthesis of compounds of formula (VI) comprises one or more of the following steps:

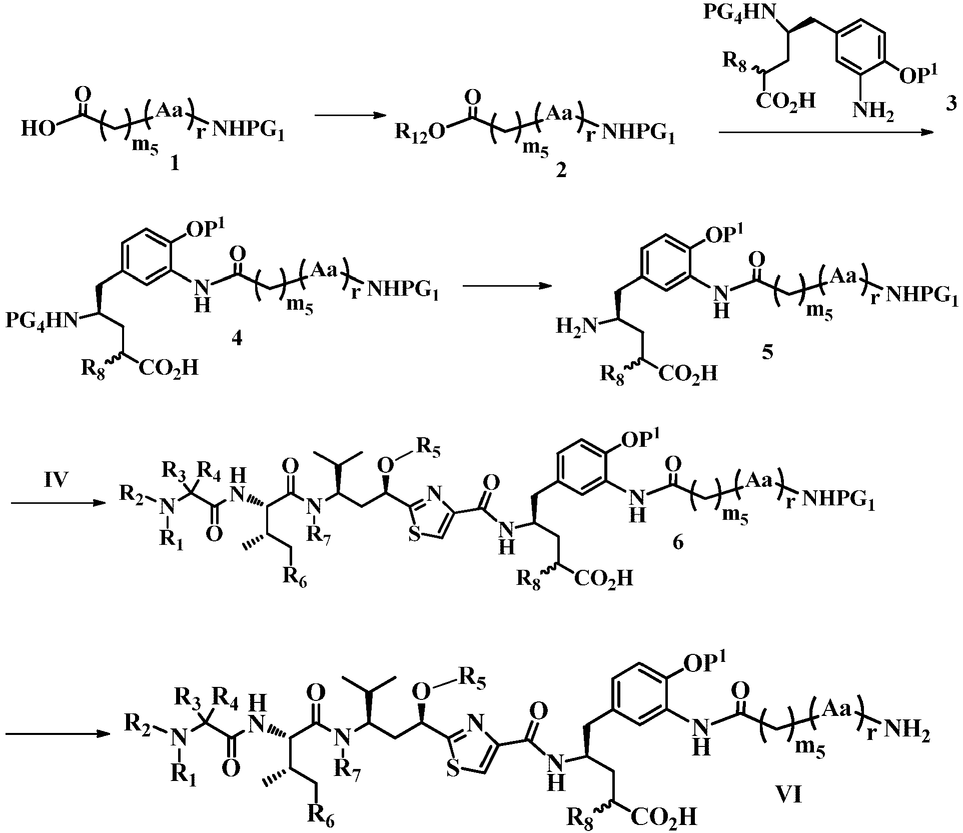

Preferably, the synthesis of compounds of Formula (VI) comprises one or more of the following steps:
Step 1. Compound **1** reacts with the compound containing a hydroxyl group (such as pentafluorophenol or N-hydroxysuccinimide), in the presence of a condensation reagent, to yield a reactive carboxylic acid derivative **2;** 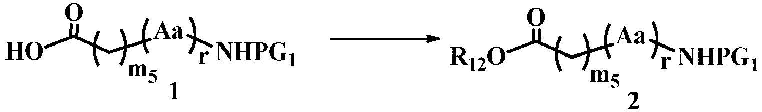
Step 2. Compound **2** and compound **3** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **4.** Optionally a base is not req.uired for the reaction to proceed, but the reaction temperature and reaction time need to be tightly controlled; 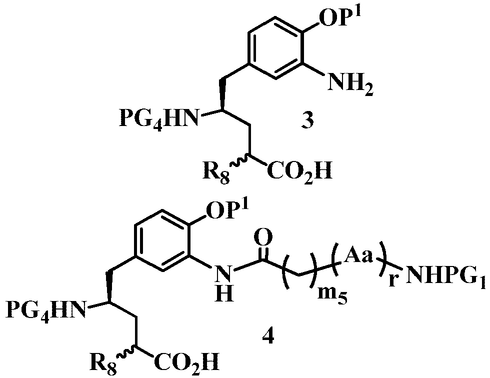
Step 3. The amino protecting group PG₄ of compound **4** is removed selectively under an appropriate deprotection condition, such as H₂ and Pd/C catalyst for Cbz protecting group, and acidic conditions for Boc protecting group, to yield compound **5**; 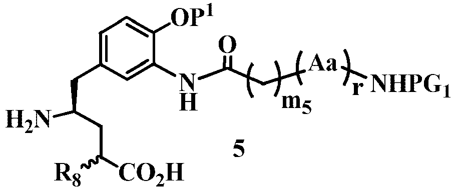
Step 4. Compound **5** and compound of Formula (IV) condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **6**. Optionally a base is not req.uired for the reaction to proceed, but the reaction temperature and reaction time need to be tightly controlled; 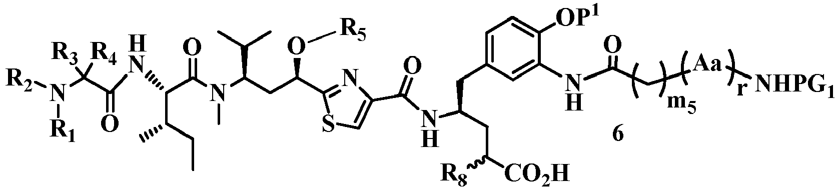
Step 5. The amino protecting group PG₁ of compound **6** is removed under deprotection conditions, such as H₂ and Pd/C catalyst for Cbz protecting group, and acidic conditions for Boc protecting group, to yield compound **VI;**

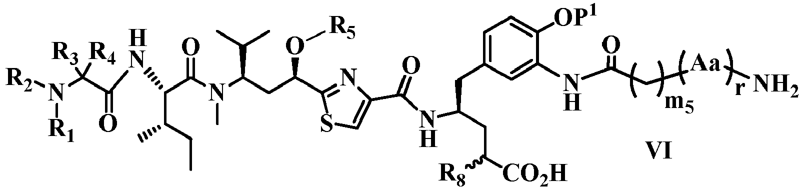

In another specific embodiment, in the process of preparing of the above conjugates, the synthesis of compounds of Formula (VII) comprises one or more of the following steps:

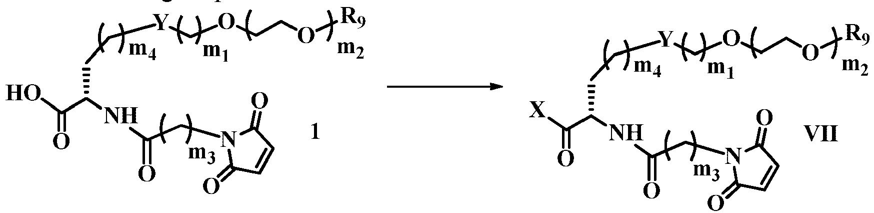

Preferably, the synthesis of compounds of Formula (VII) comprises one or more of the following steps:
Step 1. Carboxylic acid **1** and the compound with a hydroxyl group (such as pentafluorophenol or N-hydroxysuccinimide) condense in the presence of a condensation reagent (such as EDC, HATU, DIC or DCC) to yield a reactive ester;
Alternatively, carboxylic acid **1** reacts with ethyl chloroformate, isobutyl chloroformate, etc., in the presence of an organic base (such as N-methylmorpholine, triethylamine, diisopropylethylamine, etc.) to yield a reactive mixed anhydride;
Alternatively, carboxylic acid **1** reacts with oxalyl chloride, in the presence of an organic base such as trimethylamine, and catalytic amount of DMF (such as 0.01 eq. to 0.5 eq.) to yield an acyl chloride.

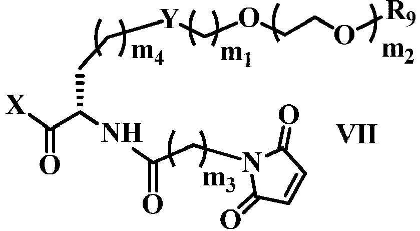

In another specific embodiment, in the process of preparing of the above conjugates, the compound of Formula (II) is obtained by condensation reaction of compounds of Formula (VIII) and Formula (IX): 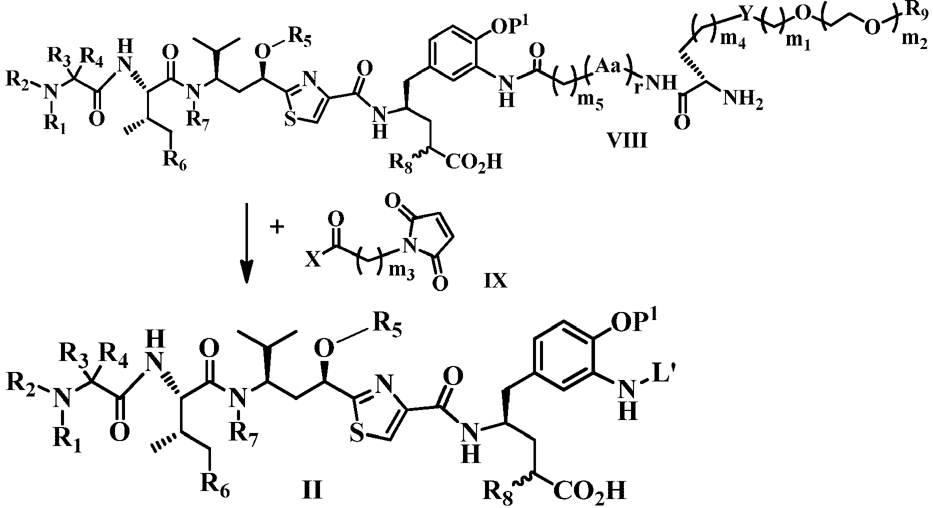 wherein the definition of X and the condensation reaction conditions are the same as above, and the NH₂ group of formula (VIII) participates in the conjugation reaction in a form of salt, with trifluoroacetic acid, hydrochloride acid, formic acid, acetic acid, sulfuric acid, phosphoric acid, nitric acid, citric acid, succinic acid, benzoic acid or sulfonic acid.

In another specific embodiment, the synthesis of Formula (VIII) comprises one or more of the following steps:

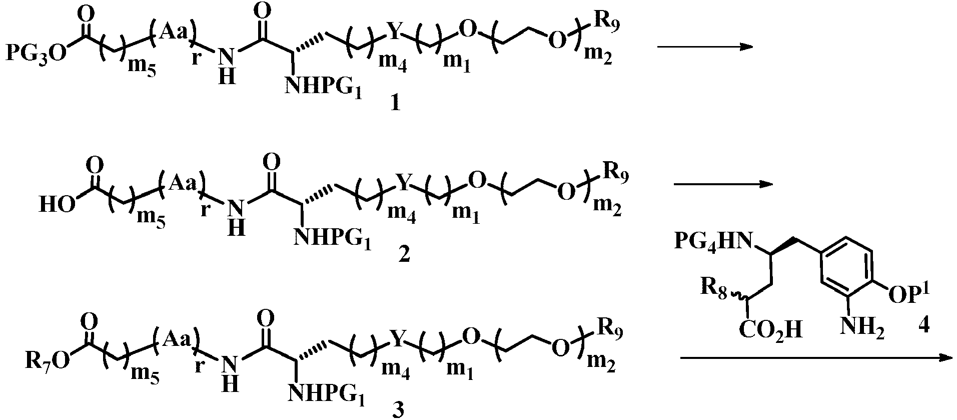

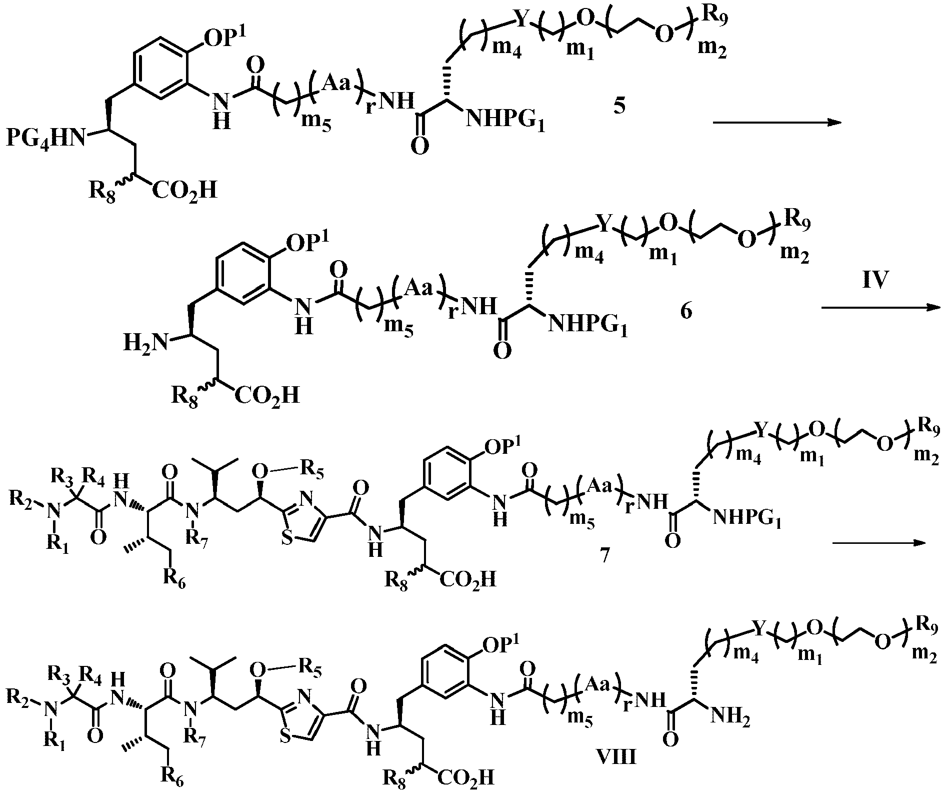

Preferably, the synthesis of compounds of Formula (VIII) comprises one or more of the following steps:
Step 1. The carboxyl protecting group PG₃ of compound **1** is removed under deprotection conditions, such as acid (formic acid, acetic acid, trifluoroacetic acid, hydrochloric acid, phosphoric acid and the like) for the cleavage of tert-butyl ester protecting group, to yield compound 2; 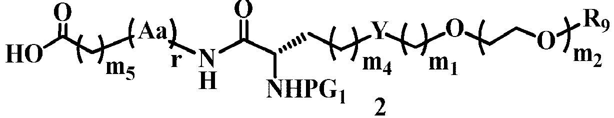
Step 2. Compound **2** reacts with the compound containing a hydroxyl group (such as pentafluorophenol or N-hydroxysuccinimide), in the presence of a condensation reagent (such as EDC, HATU, DIC or DCC), to yield a reactive ester **3**; 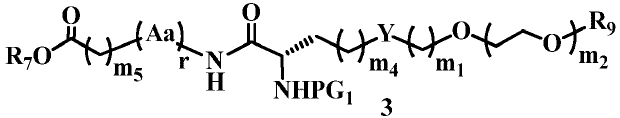
Step 3. Compound **3** and compound **4** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **5**. Optionally a base is not req.uired for the reaction to proceed, but the reaction temperature and reaction time need to be tightly controlled; 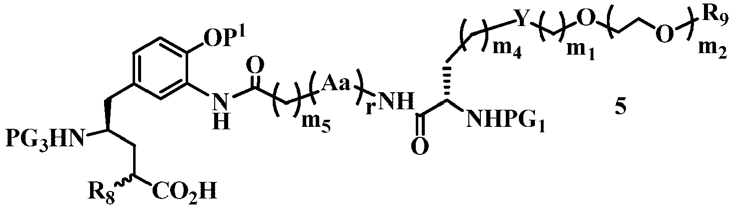
Step 4. The amino protecting group PG₄ of compound **5** is removed under deprotection conditions, such as H₂ and Pd/C catalyst for Cbz protecting group, and acidic conditions for Boc protecting group, to yield compound **6;** 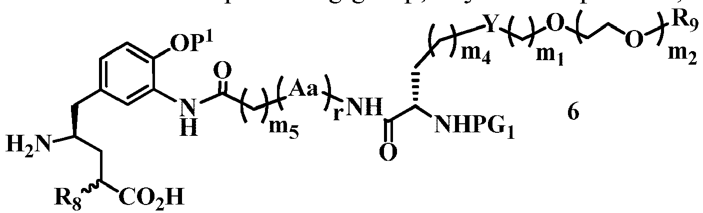
Step 5. Compound **6** and a compound of Formula (IV) condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **7**. Optionally a base is not req.uired for the reaction to proceed, but the reaction temperature and reaction time need to be tightly controlled; 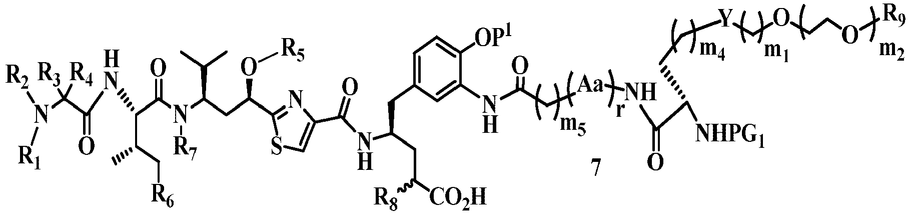
Step 6. The amino protecting group PG₁ of compound **7** is removed under deprotection conditions, such as H₂ and Pd/C catalyst for Cbz protecting group, and acidic conditions for Boc protecting group, to yield compound VIII;

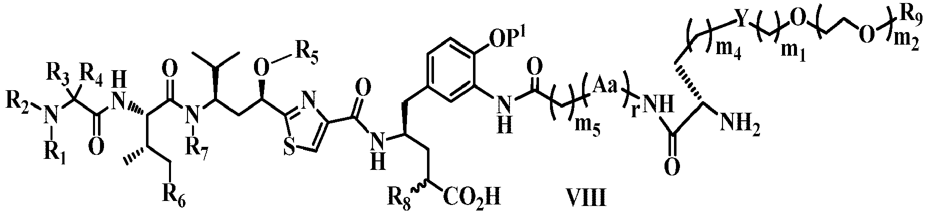

In another specific embodiment, in the process of preparing of the above conjugates, the synthesis of compounds of Formula (IX) comprises one or more of the following steps:
Carboxylic acid **1** and the compound with a hydroxyl group (such as pentafluorophenol or N-hydroxysuccinimide) condense in the presence of a condensation reagent to yield a reactive ester IX;
Alternatively, carboxylic acid **1** reacts with ethyl chloroformate, isobutyl chloroformate, etc., in the presence of an organic base (such as N-methylmorpholine, triethylamine, diisopropylethylamine, etc.) to yield a reactive mixed anhydride IX;
Alternatively, carboxylic acid **1** reacts with oxalyl chloride, in the presence of an organic base such as trimethylamine, and catalytic amount of DMF to yield an acyl chloride IX.

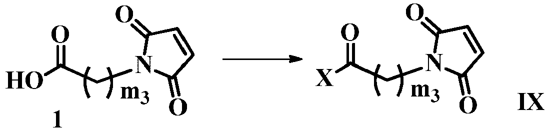

In another specific embodiment, in the process of preparing of the above conjugates, the compound of Formula (II) is obtained by condensation reaction of compounds of Formula (X) and Formula (XI): 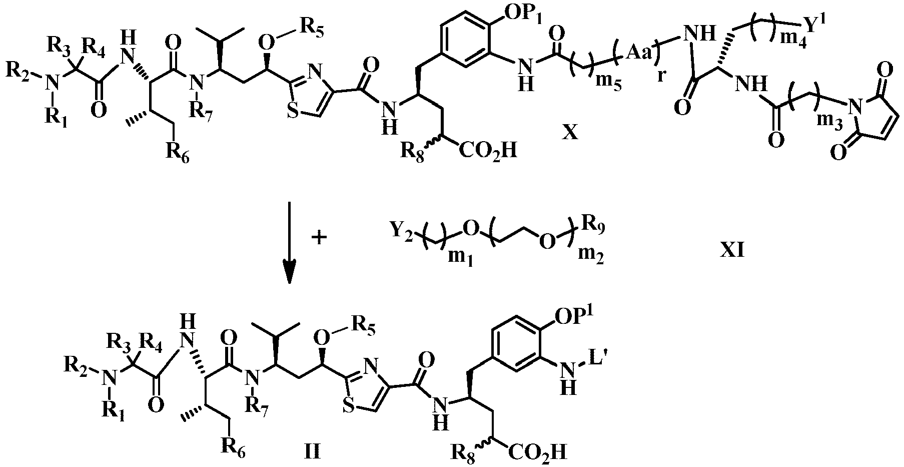 wherein Y¹ and Y² condense; and Y¹ and Y² are respectively NH₂, -⁺NH₃, COOH, COX, SO₂Cl, P(O)Cl₂, NHCOX, NHSO₂Cl, NHP(O)Cl₂, NHP(O)(OH)Cl,

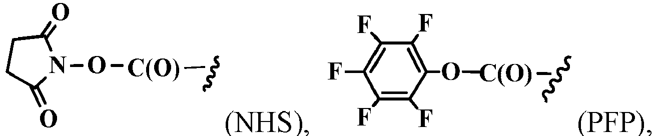

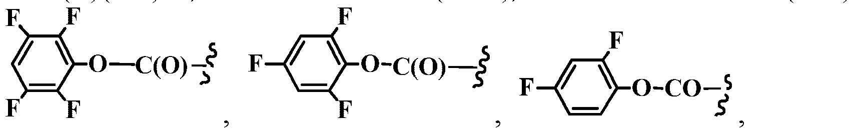

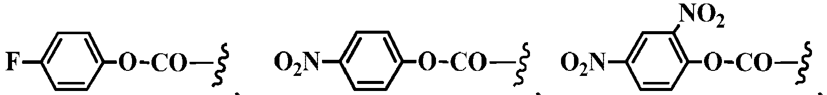

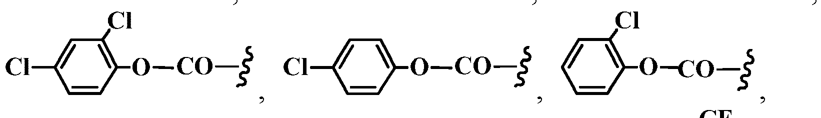

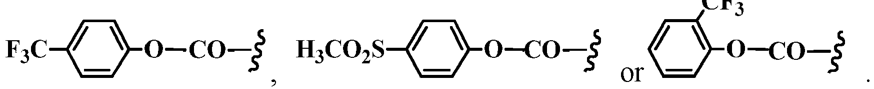

In another specific embodiment, in the process of preparing of the above conjugates, the synthesis of compounds of Formula (X) comprises one or more of the following steps:

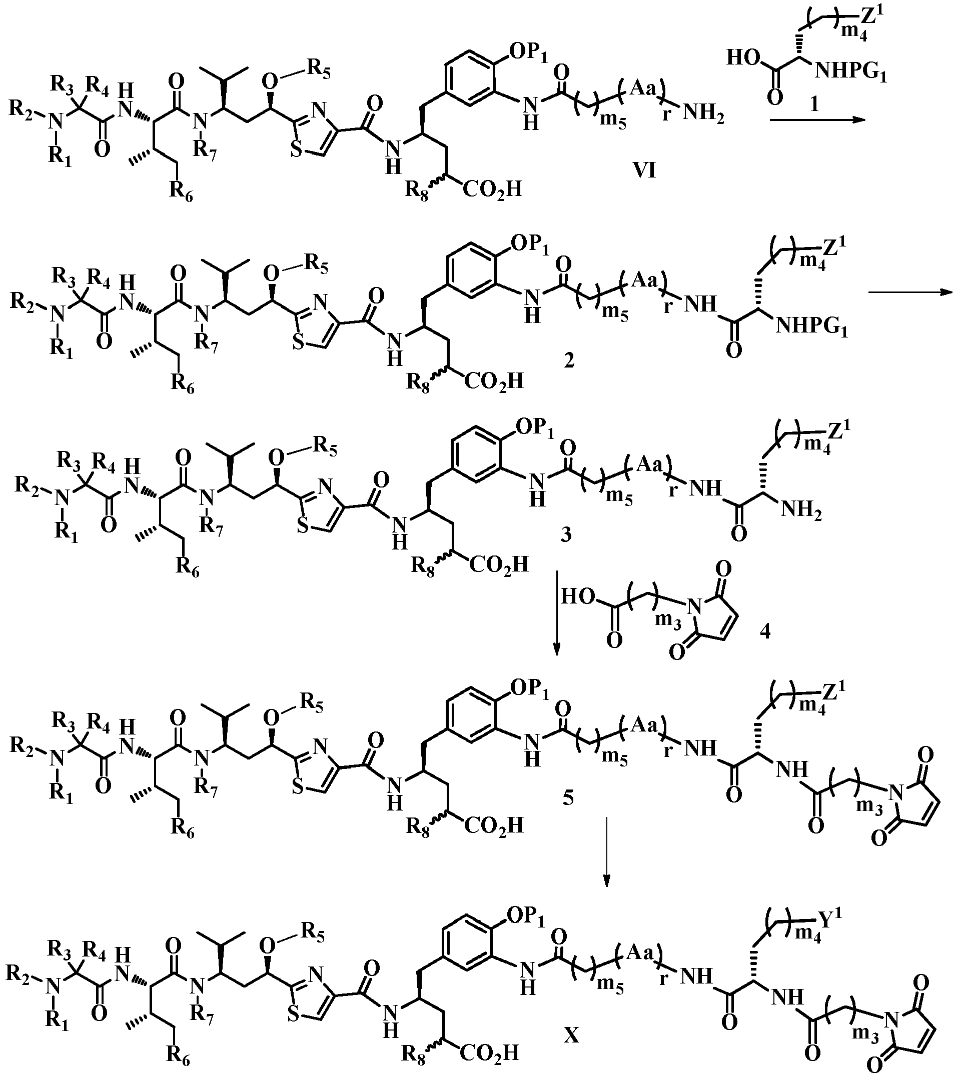

Preferably, the synthesis of compounds of Formula (X) comprises one or more of the following steps:
Step 1. Carboxylic acid **1** and compound **VI** condense in the presence of a condensation reagent (such as EDC, HATU, DIC or DCC), or via other indirect condensation reaction routes, to yield compound **2**, wherein Z¹ is a precursor of Y¹, such as protected amine, protected carboxylic acid, amide, phosphoramide, sulfonamide, carboxylate, phosphate, phosphonate, etc.; 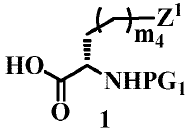 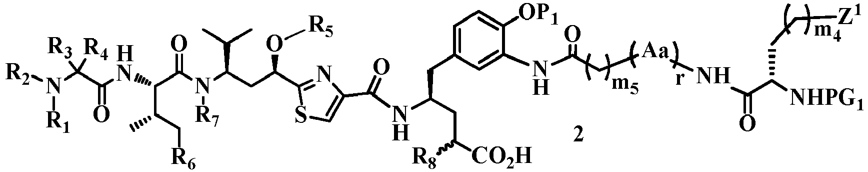
Step 2. The amino protecting group PG₁ on compound **2** is removed under deprotection condition, such as H₂ and Pd/C catalyst for Cbz protecting group, and acidic conditions for Boc protecting group, to yield compound 3; 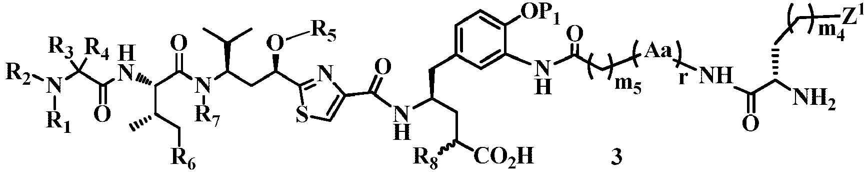
Step 3. Carboxylic acid 4 and amine 3 condense in the presence of a condensation reagent, or via other indirect condensation reaction routes, to yield compound 5; 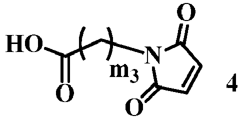 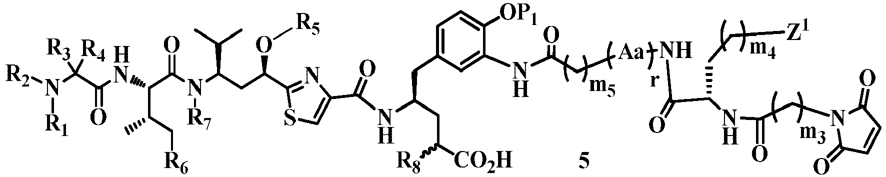
Step 4. The functional group Z¹ of compound **5** is converted to functional group Y¹ by appropriate chemical manipulations, such as deprotection of carboxylic acids and amines, leading to the formation of compound X;

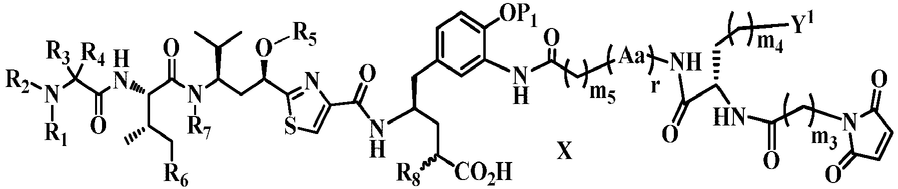

In another specific embodiment, in the process of preparing of the above conjugates, the synthesis of compounds of Formula (XI) comprises one or more of the following steps:

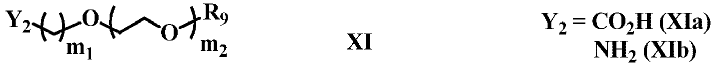

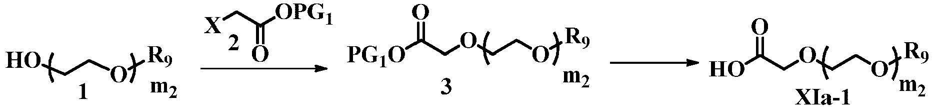

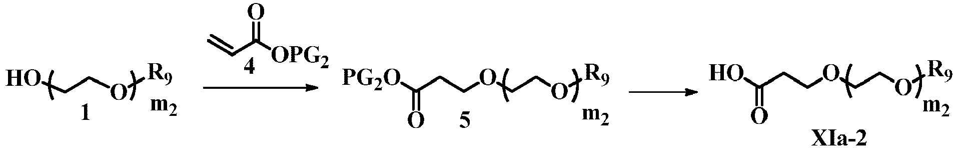

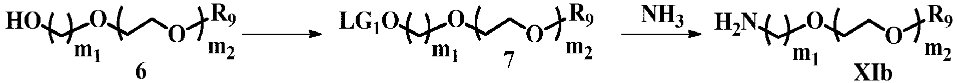

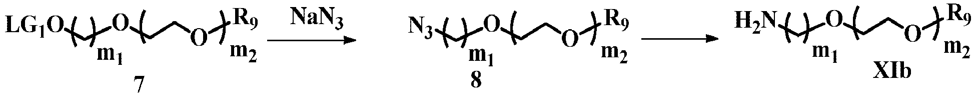

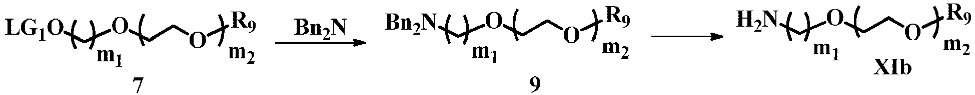

Preferably, the synthesis of compounds of Formula (XI) comprises one or more of the following steps:
Step 1. Compound 1 is dissolved in an organic solvent, such as tetrahydrofuran, dichloromethane, N, N-dimethylformamide, dimethyl sulfoxide, and then deprotonated with a base, such as sodium hydride, sodium, sodium hydroxide, and the like, and then reacted with compound **2** (wherein X is chlorine, bromine, iodine and the like or other leaving groups) at appropriate temperature to yield compound **3**;
Step 2. The carboxyl protecting group PG₁ of compound **3** is removed under deprotection conditions, for example, the tert-butyl ester protecting group can be removed by formic acid, acetic acid, trifluoroacetic acid, hydrochloric acid, phosphoric acid and the like, to yield compound XIa-1;
Step 3. Compound **1** is dissolved in an organic solvent, such as tetrahydrofuran, dichloromethane, N, N-dimethylformamide, dimethyl sulfoxide, and then deprotonated with a base, such as sodium hydride, sodium, sodium hydroxide, and the like, and then reacted with compound **4** at appropriate temperature to yield compound **5**;
Step 4. The carboxyl protecting group PG₁ of compound **5** is removed under deprotection conditions, for example, the tert-butyl ester protecting group can be removed by formic acid, acetic acid, trifluoroacetic acid, hydrochloric acid, phosphoric acid and the like, to yield compound **XIa-2;**
Step 5. Compound 6 is dissolved in an organic solvent, such as tetrahydrofuran, dichloromethane, N, N-dimethylformamide, dimethyl sulfoxide and the like, and in the presence of an appropriate organic base such as triethylamine, N, N-diisopropylethylamine, pyridine and the like, and then reacts with methylsulfonyl chloride, 4-toluenesulfonyl chloride and the like, at 0-5°C to yield compound **7**;
Step 6. Compound **7** and ammonia react in water or an organic solvent, such as methanol, ethanol, acetonitrile, tetrahydrofuran, dioxane and the like, optionally under heat, to yield compound **XIb**;
Step 7. Compound **7** and sodium azide react in an organic solvent, such as tetrahydrofuran, dichloromethane, N, N-dimethylformamide, dimethyl sulfoxide and the like, to yield compound **8**;
Step 8. The azide **8** is reduced, under hydrogenation condition (with Pd/C), or under the condition of triphenylphosphine and water, to give compound **XIb**;
Step 9. Compound **7** and dibenzylamine in an organic solvent, such as tetrahydrofuran, dichloromethane, N, N-dimethylformamide, dimethyl sulfoxide, and the like, preferably N, N-dimethylformamide, are heated to 100°C, to yield compound **9**;
Step 10. Compound **9** is dissolved in an organic solvent, such as ethyl acetate, methanol, ethanol, acetic acid, tetrahydrofuran and the like, and hydrogenated under H₂, in the presence of Pd/C catalyst, to yield the compound **XIb**. Optionally the reaction can be heated to 45°C.

In another specific embodiment, in the process of preparing of the above conjugates, the compound of Formula (II) is obtained by condensation reaction of compounds of Formula (XII) and Formula (XIII): 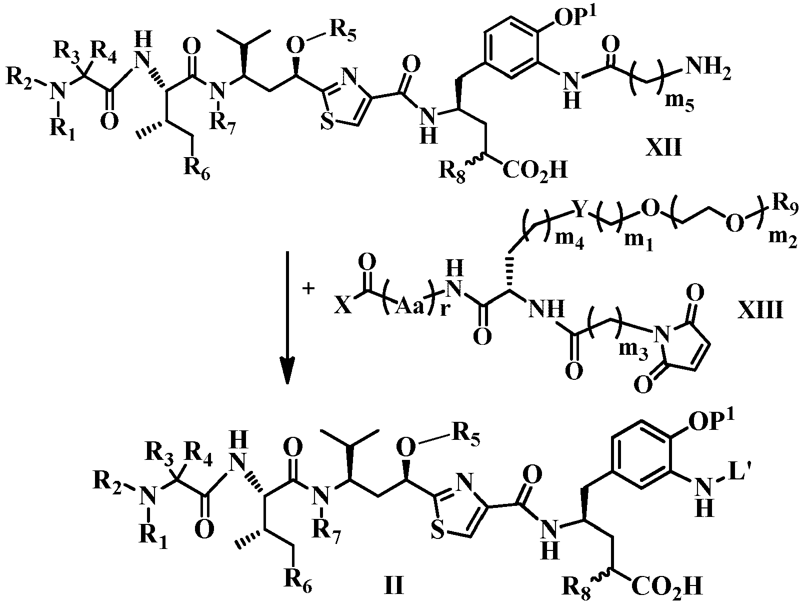 wherein the definition of X and condensation reaction conditions are the same as above.

Preferably, the NH₂ group of Formula (XII) participates in the condensation reaction in a form of salt, with trifluoroacetic acid, hydrochloride acid, formic acid, acetic acid, sulfuric acid, phosphoric acid, nitric acid, citric acid, succinic acid, benzoic acid or sulfonic acid.

In another specific embodiment, in the process of preparing of the above conjugates, the synthesis of the compound of Formula (XII) comprises one or more of the following steps:

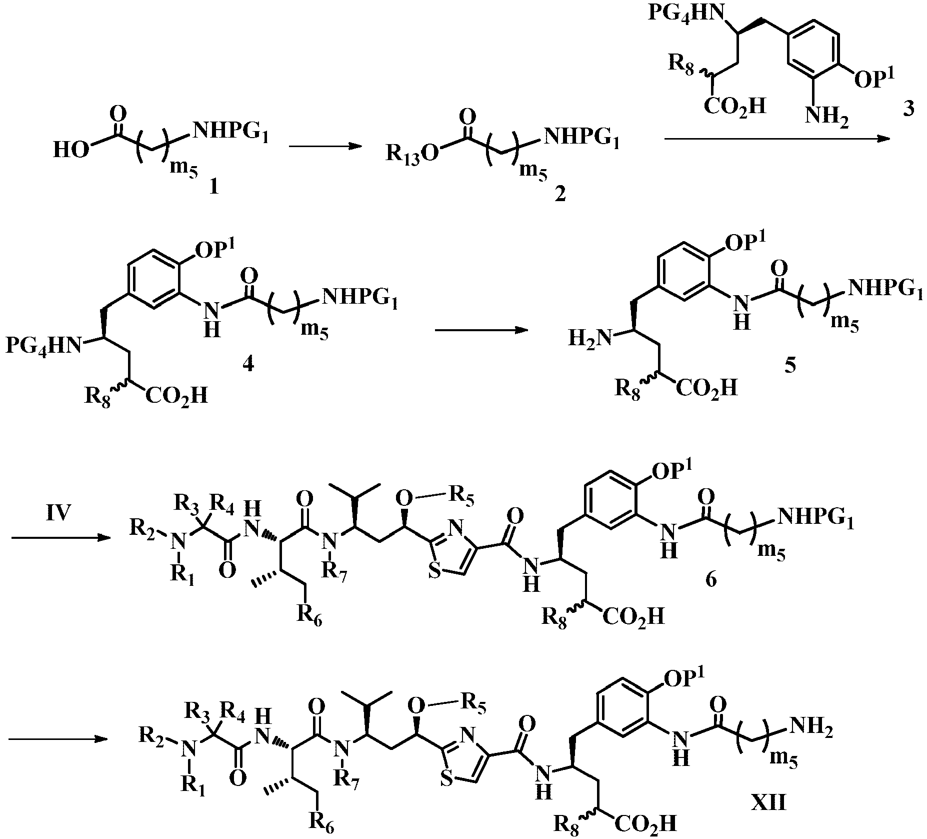

Preferably, the synthesis of compounds of Formula (XII) comprises one or more of the following steps:
Step 1. Compound **1** reacts with the compound containing a hydroxyl group (such as pentafluorophenol or N-hydroxysuccinimide), in the presence of a condensation reagent, to yield a reactive carboxylic acid derivative **2**; 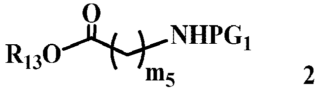
Step 2. Compound **2** and compound **3** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **4**. Optionally a base is not req.uired for the reaction to proceed, but the reaction temperature and reaction time need to be tightly controlled; 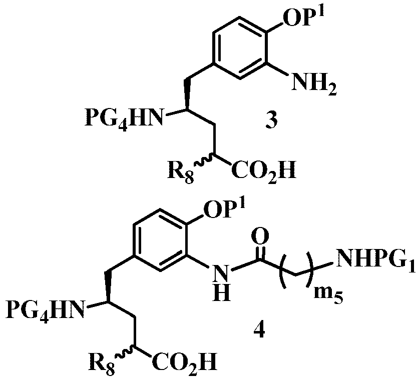
Step 3. The amino protecting group PG₄ of compound **4** is removed selectively under appropriate deprotection conditions, such as H₂ and Pd/C catalyst for Cbz protecting group, and acidic conditions for Boc protecting group, to yield compound **5**; 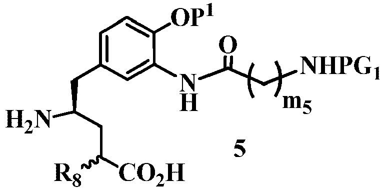
Step 4. Compound **5** and the compound of Formula (IV), condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **6**. Optionally a base is not req.uired for the reaction to proceed, but the reaction temperature and reaction time need to be tightly controlled; 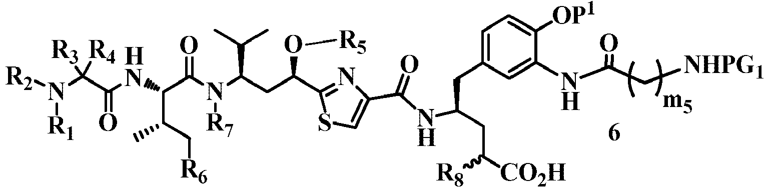
Step 5. The amino protecting group PG₁ of compound **6** is removed under deprotection conditions, such as H₂ and Pd/C catalyst for Cbz protecting group, and acidic conditions for Boc protecting group, to yield compound XII.

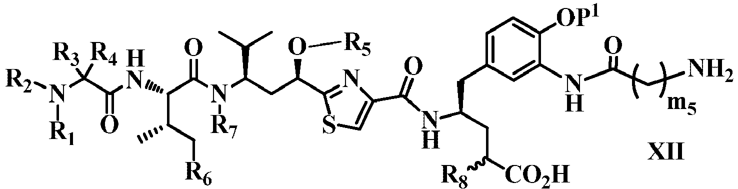

In another specific embodiment, in the process of preparing of the above conjugates, the synthesis of compounds of Formula (XIII) comprises one or more of the following steps:

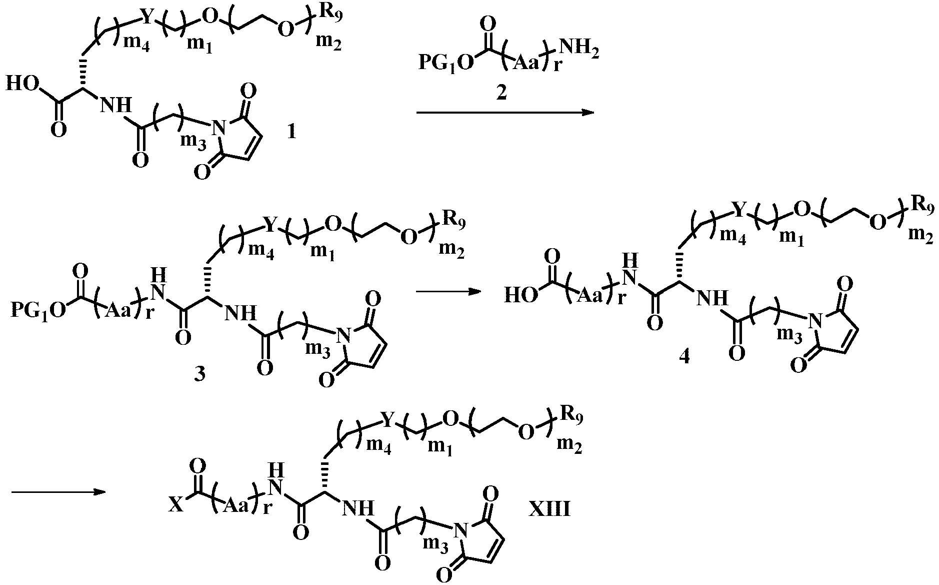

Preferably, the synthesis of compounds of Formula (XIII) comprises one or more of the following steps:
Step 1. Carboxylic acid **1** and amine **2** condense in the presence of a condensation reagent (such as EDC, HATU, DIC or DCC), or via other indirect condensation reaction routes, to yield compound **3;** 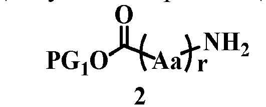 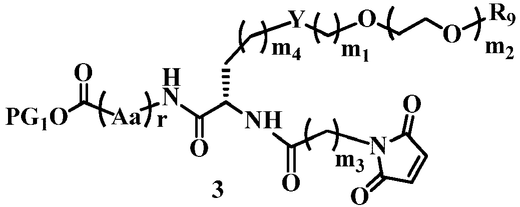
Step 2. The carboxyl protecting group PG₁ of compound **3** is removed under deprotection conditions, such as acid (formic acid, acetic acid, trifluoroacetic acid, hydrochloric acid, phosphoric acid and the like) for the cleavage of tert-butyl ester protecting group, to yield compound **4;** 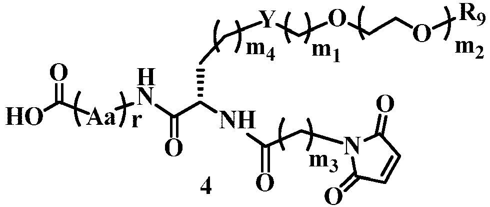
Step 3. Carboxylic acid **4** reacts with the compound containing a hydroxyl group (such as pentafluorophenol or N-hydroxysuccinimide), in the presence of a condensation reagent, to yield a reactive ester **(XIII);**
   Alternatively, carboxylic acid **4** reacts with ethyl chloroformate, isobutyl chloroformate, etc., in the presence of an organic base (such as N-methylmorpholine, triethylamine, diisopropylethylamine, etc.) to yield a reactive mixed anhydride **(XIII);**
   Alternatively, carboxylic acid **4** reacts with oxalyl chloride, in the presence of an organic base such as trimethylamine, and catalytic amount of DMF to yield an acyl chloride **(XIII)**

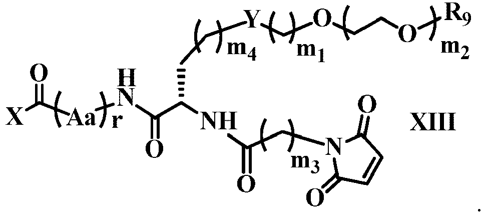

The preferred structure of the compound of formula (II) is as follows:

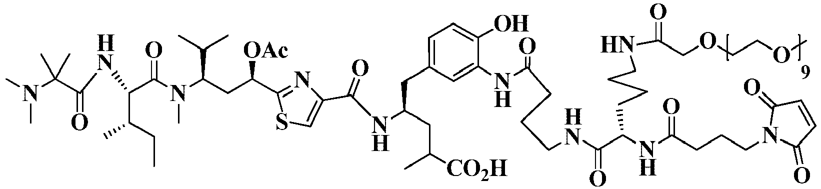

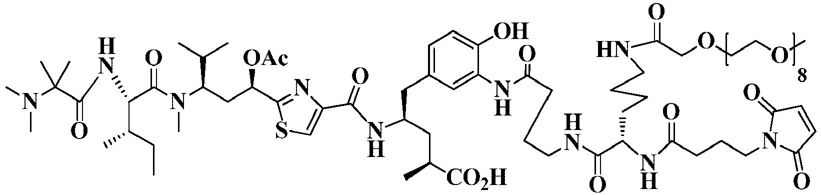

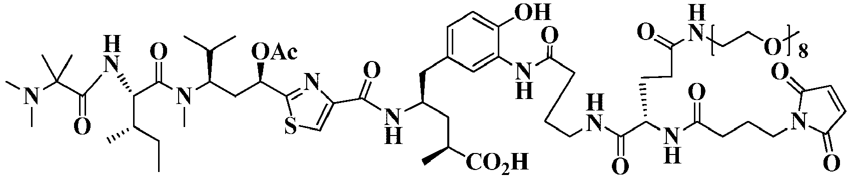

In another specific embodiment, a pharmaceutical composition comprises any of the above-mentioned conjugates or a conjugate prepared by the above compounds with a cell-binding molecule, and pharmaceutically acceptable excipients. Any of the above-mentioned conjugates can be used in the preparation of a medicine for the treatment of cancer, infection or autoimmune diseases.

### BRIEF DESCRIPTION OF THE DRAWINGS

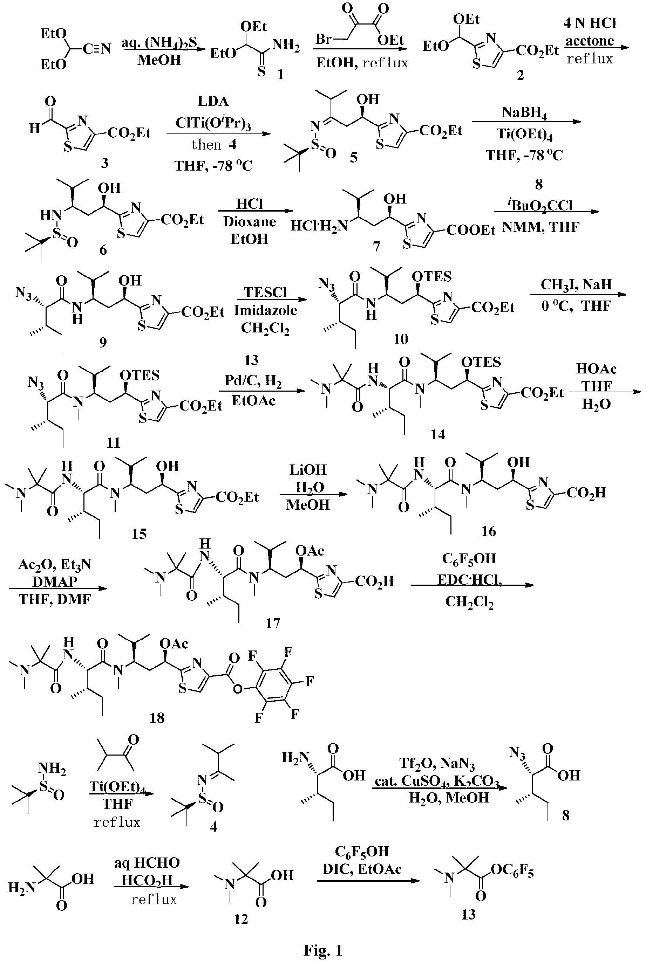

Figure 1 shows the synthesis of compound 13 and 18 of Tubulysin derivatives.
Figure 2 shows the synthesis of intermediate compound 34 of Tubulysin derivatives.

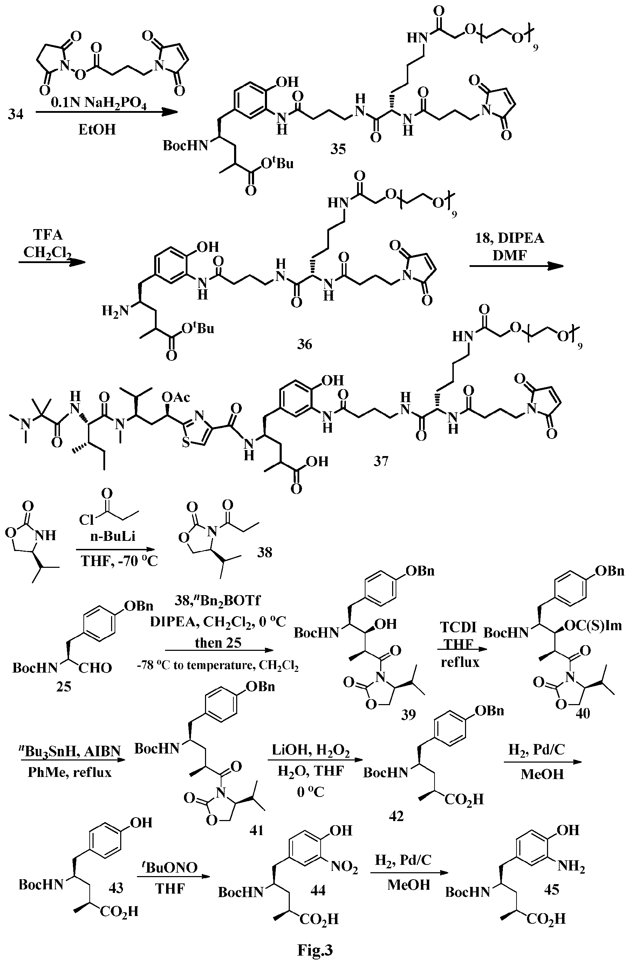

Figure 3 shows the synthesis of intermediate compound 37, 38 and 45 of Tubulysin derivatives.

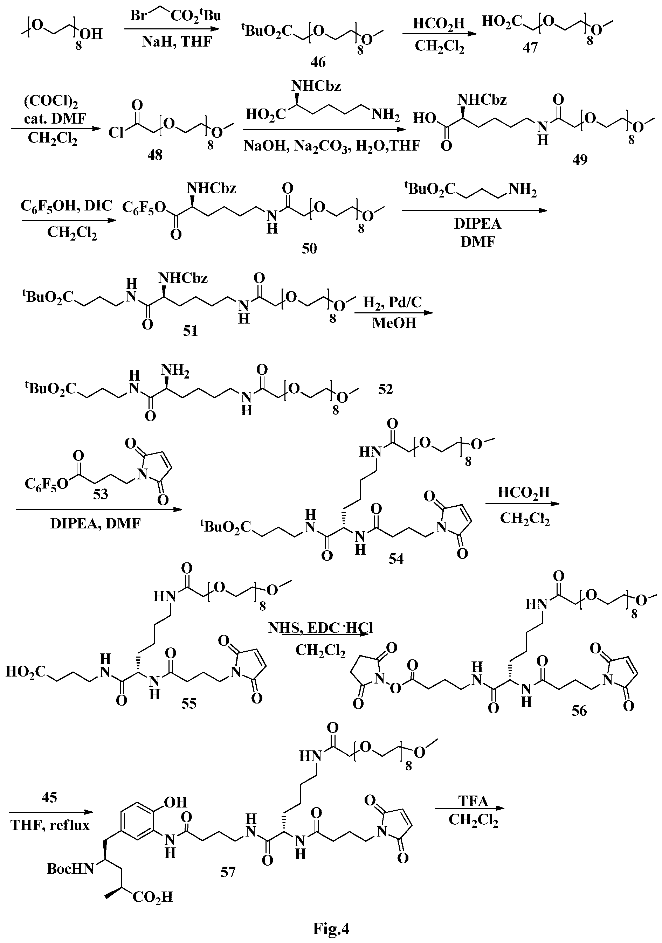

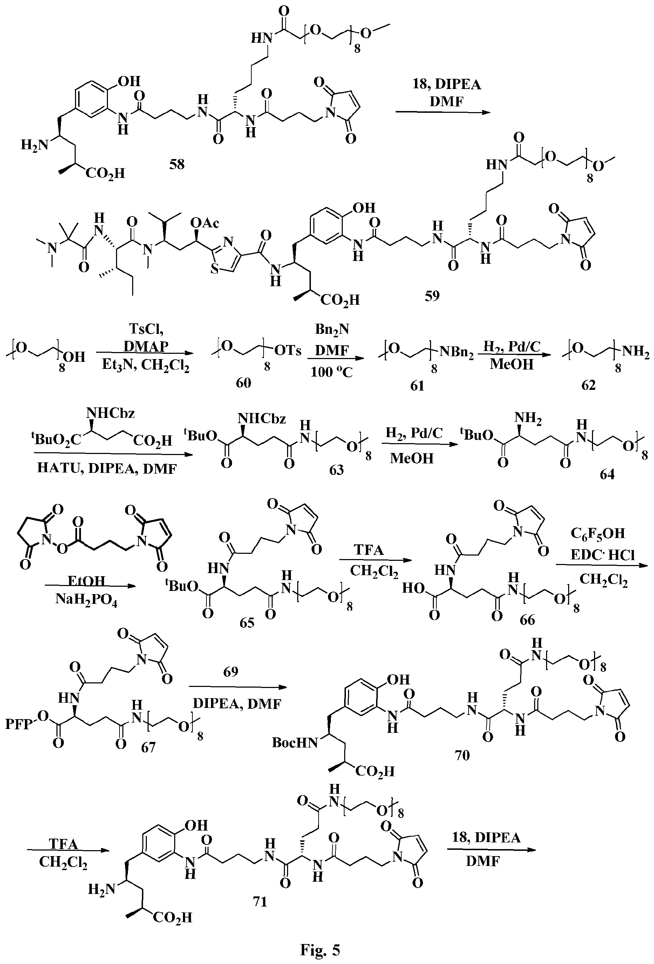

Figure 4 shows the synthesis of intermediat compound 57 of Tubulysin derivatives.

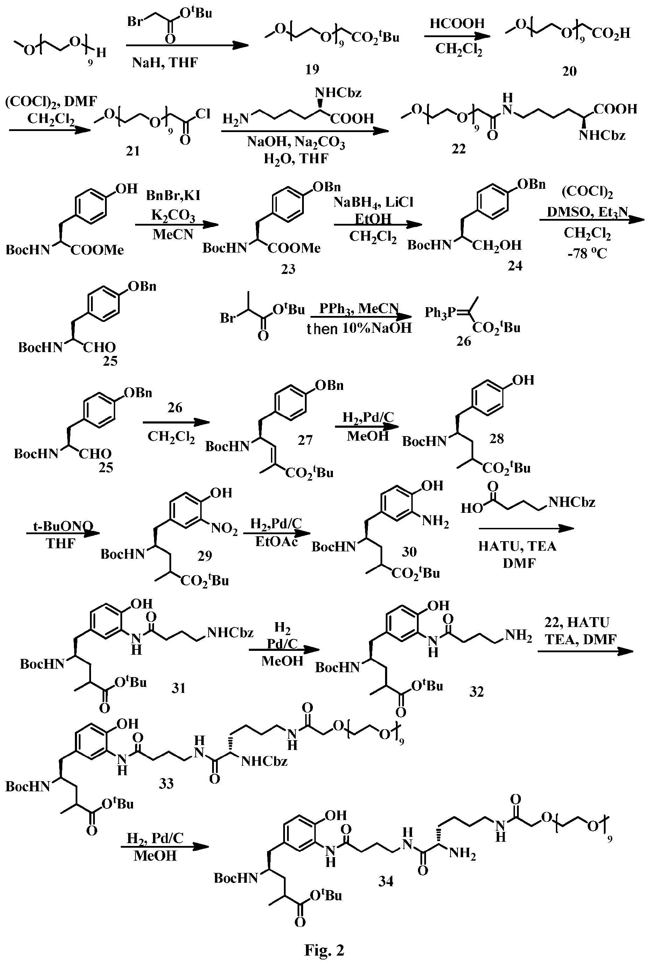

Figure 2 shows the synthesis of intermediate compound 71 of Tubulysin derivatives.

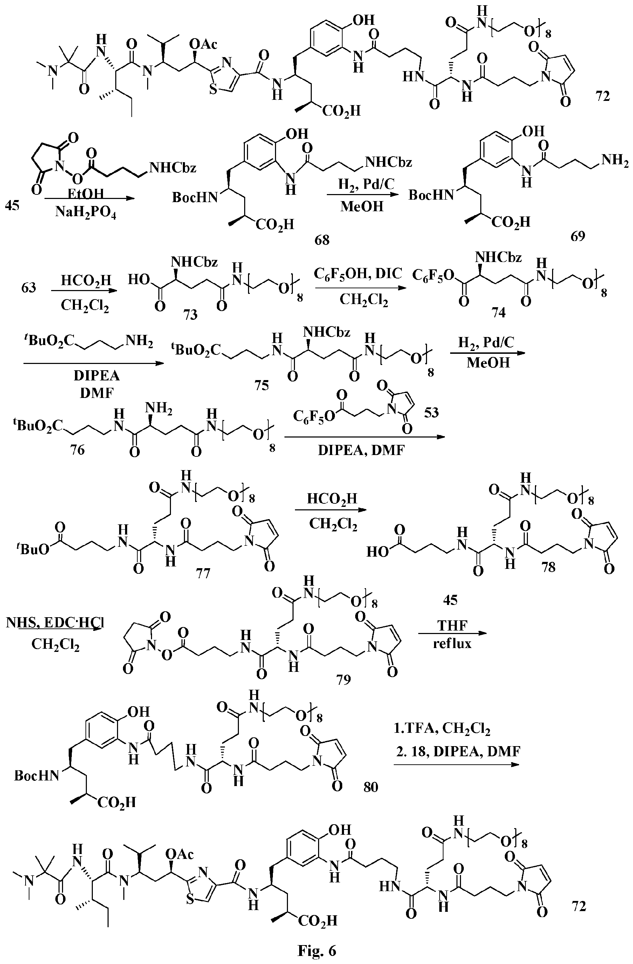

Figure 6 shows the synthesis of Tubulysin derivative 72.

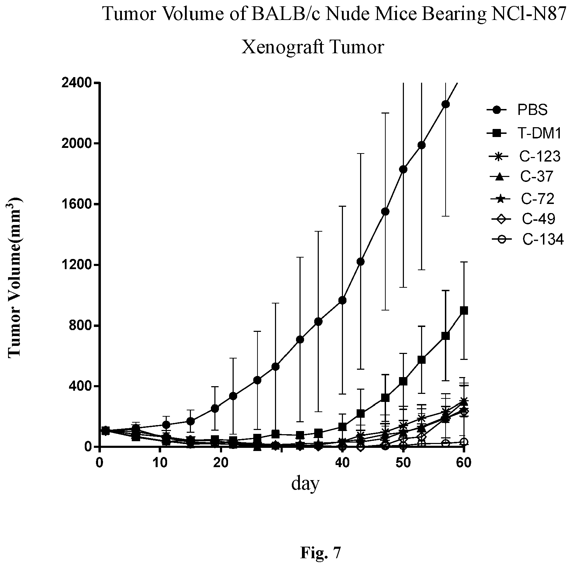

Figure 7 shows the in vivo antitumor activity of conjugates against xenograft tumor in BALB/c nude mice.

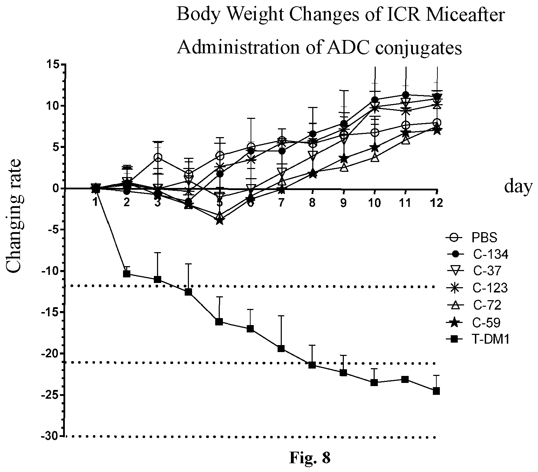

Figure 8 shows the cytotoxicity study of Her2-Tubulysin analog conjugates and the comparision with T-DM1.

### DETAILED DESCRIPTION OF THE INVENTION

### DEFINITIONS

"Alkyl" refers to a linear or cyclic linear or branched aliphatic hydrocarbon containing 1 to 8 carbon atoms. Branched chain refers to a linear alkyl group with one or more lower alkyl groups, such as methyl, ethyl or propyl connected.

"Alkyl groups" include methyl, ethyl, *n*-propyl, *i*-propyl, *n*-butyl, *t*-butyl, *n*-pentyl, 3-pentyl, octyl, nonyl, decyl, cyclopentyl, cyclohexyl, 2,2-dimethylbutyl, 2,3-dimethylbutyl, 2,2-dimethylpentyl, 2,3-dimethylpentyl, 3,3-dimethylpentyl, 2,3,4-trimethylpentyl, 3-methyl-hexyl, 2,2-dimethylhexyl, 2,4-dimethylhexyl, 2,5-dimethylhexyl, 3,5-dimethylhexyl, 2,4-dimethylpentyl, 2-methylheptyl, 3-methylheptyl, *n*-heptyl, isoheptyl, *n*-octyl, and isooctyl.A C₁-C₈ alkyl group can be unsubstituted or substituted with one or more groups including, but not limited to, -C₁-C₈ alkyl,-O-(C₁-C₈ alkyl), -aryl, -C(O)R', -OC(O)R', -C(O)OR', -C(O)NH₂, -C(O)NHR', -C(O)N(R')₂, -NHC(O)R', -SR', -S(O)₂R', -S(O)R', -OH, -halogen, -N₃, -NH₂, -NH(R'), -N(R')₂ and -CN; where each R' is independently selected from -C₁-C₈ alkyl and aryl.

A "C₃-C₈ carbocycle" refers to a 3,4,5,6,7, or 8 membered saturated or unsaturated nonaromatic carbocyclic ring. A C₃-C₈ carbocycle group can be unsubstituted or substituted with one or more groups including, but not limited to, -C₁-C₈ alkyl, -O-(C₁-C₈ alkyl), -aryl, -C(O)R', -OC(O)R', -C(O)OR', -C(O)NH₂, -C(O)NHR', -C(O)N(R')₂, -NHC(O)R', -SR', -S(O)R'-S(O)₂R', -OH, -halogen, -N₃, -NH₂, -NH(R'), -N(R')₂ and -CN; where each R' is independently selected from -C₁-C₈ alkyl and aryl.

A "C₃-C₈ carbocyclic group" refers to one hydrogen atom of C₃-C₈ carbocyclic group was substituted with a chemical bond.

"Alkenyl" refers to an aliphatic hydrocarbon group containing a carbon-carbon double bond which may be straight or branched having 2 to 8 carbon atoms in the chain. Exemplary alkenyl groups include ethenyl, propenyl, n-butenyl, i-butenyl, 3-methylbut-2-enyl, n-pentenyl, hexylenyl, heptenyl, octenyl.

"Alkynyl" refers to an aliphatic hydrocarbon group containing a carbon-carbon triple bond which may be straight or branched having 2 to 8 carbon atoms in the chain. Exemplary alkynyl groups include ethynyl, propynyl, *n*-butynyl, 2-butynyl, 3-methylbutynyl, 5-pentynyl, *n*-pentynyl, hexylynyl, heptynyl, and octynyl.

"Heteroalkyl" refers to an alkyl group containing 2 to 8 carbon atoms and having 1 to 4 carbon atoms substituted with O, S or N.

"Aryl" or Ar refers to an aromatic or hetero aromatic group, composed of one or several rings, comprising three to fourteen carbon atoms(preferentially six to ten carbon atoms) The term of "hetero aromatic group" refers one or several carbons on aromatic group, preferentially one, two, three or four carbon atoms are replaced by O, N, Si, Se, P or S, preferentially by O, S, and N. The term aryl or Ar also refers to an aromatic group, wherein one or several H atoms are replaced independently by -R', -halogen, -OR', or -SR', -NR'R", -N=NR', -N=R', -NR'R", -NO₂, -S(O)R', -S(O)₂R', -S(O)₂OR', -OS(O)₂OR', -PR'R", -P(O)R'R", -P(OR')(OR"), -P(O)(OR')(OR") or -OP(O)(OR')(OR") wherein R', R" are independently H, alkyl, alkenyl, alkynyl, heteroalkyl, aryl, arylalkyl, carbonyl, or pharmaceutical salts.

"Halogen" refers to fluorine, chlorine, bromine or iodine atom; preferably fluorine and chlorine atom.

"Heterocycle" refers to 2 to 8 carbon atoms Ar, a ring system in which 1 to 4 of the ring carbon atoms are independently replaced with a heteroatom from the group of O, N, S, Se, B, Si and P. Preferable heteroatoms are O, N and S. Heterocycles are also described in The Handbook of Chemistry and Physics, 78th Edition, CRC Press, Inc., 1997-1998, p. 225 to 226, the disclosure of which is hereby incorporated by reference. Preferred nonaromatic heterocyclic include epoxy, aziridinyl, thiiranyl, pyrrolidinyl, pyrazolidinyl, imidazolidinyl, oxiranyl, tetrahydrofuranyl, dioxolanyl, tetrahydropyranyl, dioxanyl, dioxolanyl, piperidyl, piperazinyl, morpholinyl, pyranyl, imidazolinyl, pyrrolinyl, pyrazolinyl, thiazolidinyl, tetrahydrothiopyranyl, dithianyl, thiomorpholinyl, dihydropyranyl, tetrahydropyranyl, dihydropyranyl, tetrahydropyridyl, dihydropyridyl, tetrahydropyrimidinyl, dihydrothiopyranyl, azepanyl, as well as the fused systems resulting from the condensation with a phenyl group.

The term "heteroaryl" or aromatic heterocycles refers to a 3 to 14 (preferably 5 to 10 membered) aromatic hetero, mono-, bi-, or multi-cyclic ring. Example s include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazolyl, tetrazolyl, isoquinolyl, benzothienyl, isobenzofuryl, pyrazolyl, carbazolyl, benzimidazolyl, isoxazolyl, pyridyl-*N*-oxide, as well as the fused systems resulting from the condensation with a phenyl group.

"Alkyl", "cycloalkyl", "alkenyl", "alkynyl", "aryl", "heteroaryl", "heterocyclic" and the like refer also to the corresponding "alkylene", "cycloalkylene", "alkenylene", "alkynylene", "arylene", "heteroarylene", "heterocyclene" and the likes which are formed by the removal of two hydrogen atoms.

"Arylalkyl" refers to an acyclic alkyl radical in which one of the hydrogen atoms bonded to a carbon atom, typically a terminal or sp³ carbon atom, is replaced with an aryl radical. Typical arylalkyl groups include, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like.

"Heteroarylalkyl" refers to an acyclic alkyl radical in which one of the hydrogen atoms bonded to a carbon atom, typically a terminal or sp³ carbon atom, is replaced with a heteroaryl radical. Example s of heteroarylalkyl groups are 2-benzimidazolylmethyl, 2-furylethyl.

"Hydroxyl protecting group" refers to methoxymethyl ether (MOM), 2-methoxyethoxymethyl ether (2-MOEOM), tetrahydropyranyl ether, benzyl ether, *p*-methoxybenzyl ether, trimethylsilyl ether, triethylsilyl ether, triisopropylsilyl ether, *t*-butyldimethylsilyl ether, triphenylmethylsilyl ether, acetate ester, substituted acetate esters, Benzoate, benzyl formate, chloroacetate, methoxyacetate, phenoxyacetate,pivaloate, adamantanoate, mesitoate, methanesulfonate and tosylate and *p*-toluenesulfonate.

The "amino acid(s)" can be natural and/or unnatural amino acids, L or D type, preferably alpha-amino acids. Natural amino acids are those encoded by the genetic code, which are alanine, arginine, asparagine, aspartic acid, cysteine, glutamic acid, glutamine, glycine, histidine, isoleucine, leucine, lysine, methionine, phenylalanine, proline, serine, threonine, tyrosine. tryptophan and valine. The unnatural amino acids are derived forms of proteinogenic amino acids. Example s include hydroxyproline, lanthionine, 2-aminoisobutyric acid, dehydroalanine, gamma-aminobutyric acid (the neurotransmitter), ornithine, citrulline, beta-alanine (3-aminopropanoic acid), gamma-carboxyglutamate, selenocysteine (present in many noneukaryotes as well as most eukaryotes, but not coded directly by DNA), pyrrolysine (found only in some archaea and one bacterium), N-formylmethionine (which is often the initial amino acid of proteins in bacteria, mitochondria, and chloroplasts), 5-hydroxytryptophan, L-dihydroxyphenylalanine, triiodothyronine, L-3,4-dihydroxyphenylalanine (DOPA), and O-phosphoserine. The term "amino acid" also includes amino acid analogs and mimetics. Analogs are compounds having the same general H₂N(R)CHCO₂H structure of a natural amino acid, except that the R group is not one found among the natural amino acids. Example s of analogs include homoserine, norleucine, methionine-sulfoxide, and methionine methyl sulfonium. Preferably, an amino acid mimetic is a compound that has a structure different from the general chemical structure of an alpha-amino acid but functions in a manner similar to one. The term "unnatural amino acid" is intended to represent the "D" stereochemical form, the natural amino acids being of the "L" form. When 1~8 amino acids are used in this patent invention, amino acid seq.uence is then preferably a cleavage recognition seq.uence for a protease. Many cleavage recognition seq.uences are known in the art. See, e.g., Matayoshi et al. Science 247: 954 (1990); Dunn et al. Meth. Enzymol. 241: 254 (1994); Seidah et al. Meth. Enzymol. 244: 175 (1994); Thornberry, Meth. Enzymol. 244: 615 (1994); Weber et al. Meth. Enzymol. 244: 595 (1994); Smith et al. Meth. Enzymol. 244: 412 (1994); and Bouvier et al. Meth. Enzymol. 248: 614 (1995); the disclosures of which are incorporated herein by reference. In particular, the seq.uence is selected from the group consisting of Val-Cit, Ala-Val, Ala-Ala, Val-Val, Val-Ala-Val, Lys-Lys, Ala-Asn-Val, Val-Leu-Lys, Cit-Cit, Val-Lys, Ala-Ala-Asn, Asp-Lys, Asp-Glu, Glu-Lys, Lys, Cit, Ser, and Glu.

A "peptide" is formed by combining two or more amino acids with a carboxyl group of another amino acid with a peptide bond (i.e. an amide bond) The two amino acids are referred to as dipeptides by peptide bonds; the three amino acids are referred to as tripeptides by peptide bonds and the like, the three amino acids are referred to as peptide bonds, and the compounds linked by peptide bonds are referred to as tripeptides. Peptides consisting entirely of natural α amino acids are natural peptides (natural proteins) Peptides containing one or more non-natural amino acids or amino acid analogs are non-natural peptides (peptoid compound) The peptide of two or more amino acids is a peptide single unit.

The "glycoside" is a molecule in which a sugar group is bonded through its anomeric carbon to another group via a glycosidic bond. Glycosides can be linked by an O- (an *O*-glycoside), N- (a glycosylamine), S- (a thioglycoside), or C- (a C-glycoside) glycosidic bond. Its empirical formula is C*ₘ*(H₂O)*ₙ* (where m could be different from n, and m and n are < 36) Glycoside herein includes glucose (dextrose), fructose (levulose) allose, altrose, mannose, gulose, iodose, galactose, talose, galactosamine, glucosamine, sialic acid, *N*-acetylglucosamine, sulfoquinovose (6-deoxy-6-sulfo-D-glucopyranose), ribose, arabinose, xylose, lyxose, sorbitol, mannitol, sucrose, lactose, maltose, trehalose, maltodextrins, raffinose, glucuronic acid (glucuronide), and stachyose. It can be in D form or L form, 5-atom cyclic furanose form, 6-atom cyclic pyranose form, or acyclic form, α-isomer (the -OH of the anomeric carbon below the plane of the carbon atoms of Haworth projection), or β-isomer (the -OH of the anomeric carbon above the plane of Haworth projection) Most often used herein are monosaccharides, disaccharides, polyols, or oligosaccharides (containing 3-6 sugar units)

The term "antibody," as used herein, refers to a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce auto-immune antibodies associated with an autoimmune disease. The immunoglobulin disclosed herein can be of any type (e.g. IgG, IgE, IgM, IgD, and IgA), class (e.g., IgG1, IgG2, IgG3, IgG4, IgA1 and IgA2) or subclass of immunoglobulin molecule. The immunoglobulins can be derived from any species. Preferably, however, the immunoglobulin is of human, murine, or rabbit origin. Antibodies can be human, humanized or chimeric antibodies.

The term "specific binding" means that an antibody or antibody derivative will bind to its corresponding target antigen in a highly selective manner, rather than in combination with many other antigens. Generally, the antibody or antibody has an affinity of at least about 1×10⁻⁷M. Preferably, 1×10⁻⁸M to 10⁻⁹M, 10-¹⁰M, 10⁻¹¹M or 10⁻¹²M. The affinity of the predetermined antigen is at least twice the affinity of the non-specific antigen (such as bovine serum albumin, casein)

"Pharmaceutically" or "pharmaceutically acceptable" refer to molecular entities and compositions that do not produce an adverse, allergic or other untoward reaction when administered to an animal, or a human, as appropriate.

"Pharmaceutically acceptable excipient" includes any carriers, diluents, adjuvants, or vehicles, such as preserving or antioxidant agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions as suitable therapeutic combinations.

In the present invention, pharmaceutically acceptable salts refer to salt derivatives of the compounds of the present invention. "pharmaceutical salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, tartaric, citric, methanesulfonic, benzenesulfonic, glucuronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutical salts of the present invention can be synthesized by conventional chemical methods. Generally, such salts can be prepared via reaction the free acidic or basic forms of these compounds with a stoichiometric amount of the appropriate base or acid in water or in an organic solvent, or in a mixture of the two. Non-aqueous media like ether, ethyl acetate, ethanol, isopropanol, or acetonitrile. Lists of suitable salts are found in Remington's Pharmaceutical Sciences, 17th ed., Mack Publishing Company, Easton, PA, 1985, p. 1418, the disclosure of which is hereby incorporated by reference.

The term "pharmaceutically acceptable salt" refers to a pharmaceutically acceptable organic or inorganic salt of a ligand drug conjugate or linker drug conjugate. The conjugate may contain at least one amino group and thus may form an acid addition salt with the amino group, such as nitrate, hydrogen sulfate, phosphate, acid phosphate, isonicotinic acid salt, lactate, salicylate, acid citrate, tartrate, oleate, perchlorate, pantothenate, tartrate, ascorbate, succinate, maleate, cholate, fumarate, gluconate, glucuronic acid salt, gluconic acid salt, formate, benzoate, glutamate, mesylate, ethanesulfonate, benzenesulfonate, p-toluenesulfonate, and bis (2-hydroxynaphthoate) (i.e. 1,1'-methylene bis-(2-hydroxynaphthoate)) Pharmaceutically acceptable salts may include additional molecules, such as the salts of acetate ions, succinate ions, or other counter ions. The counter ion may be any organic or inorganic moiety that stabilizes the charge on the parent compound. Furthermore, a pharmaceutically acceptable salt may have more than one charged atom in its structure. A pharmaceutically acceptable salt of a plurality of charged atoms may have a plurality of counter ions. Thus, a pharmaceutically acceptable salt may have one or more charged atoms and/or one or more counter ions.

"Pharmaceutically acceptable solvate" or "solvate" refer to an association of one or more solvent molecules with ligand drug conjugate or linker drug conjugate. Example s of solvents that form pharmaceutically acceptable solvates include, but are not limited to, water, isopropanol, ethanol, methanol, DMSO, ethyl acetate, acetic acid and ethanolamine.

Hydrate refers to a compound containing water. The water can be connected to other parts with a coordination bond, such as to form a hydrated metal ion complex, or with a covalent bond, such as to form hydrated trichloroacetaldehyde. It also refers to certain compounds and moisture form crystals or liquid molecules under certain temperature, pressure conditions. The water in the hydrate is present in a determined amount, for example, the hydrate of anhydrous Na₂SO₄ is Na₂SO₄·10H₂O. Water in the hydrate has several different binding ways: one is the ligand and is coordinated to the metal ions, known as coordination crystal water; the other is bound to the anion, referred to as anionic crystal water. Water also cannot be directly bound to a cation or anion, but presents in a certain proportion and occupies a certain site in the crystal. This combined form of water is referred to as lattice water, typically containing 12 water molecules. Some crystalline compounds also contain water, but do not have a certain proportion. A salt of a hydrate refers to a pharmaceutically acceptable salt formed on the basis of the hydrate.

The optical isomers, also known as enantiomers, enantiomers, optical isomers, mirror isomers, enantiomers or chiral isomers, cannot mirror completely overlapping molecules with each other. When a substance contains one chiral carbon atom, there are two optical isomers, which have a relationship between the physical and mirror images, and thus are also referred to as enantiomers. Enantiomers have eq.ual optical spin capacity, but the direction of rotation is opposite, and its physical and chemical properties may be similar. Molecules containing two identical property carbon atoms have three optical isomers. When several different chiral atoms are contained in the molecule, the number of optical isomers thereof is 2ⁿ, and n is the number of different chiral atoms. Equal amounts of the two substances, such as the optical isomers, are uniformly mixed, and the optically active components cancel each other to form a racemate.

Example s of a "mammal" or "animal" include, but are not limited to, a human, rat, mouse, guinea pig, monkey, pig, goat, cow, horse, dog, cat, bird and fowl. In an exemplary embodiment, the patient or subject is a person.

"Administering" or "administration" refers to any mode of transferring, delivering, introducing or transporting a pharmaceutical drug or other agent to a subject. Such modes include oral administration, topical contact, intravenous, intraperitoneal, intramuscular, intralesional, intranasal, subcutaneous or intrathecal administration. Also contemplated by the present invention is utilization of a device or instrument in administering an agent. Such device may utilize active or passive transport and may be slow-release or fast-release delivery device.

The following abbreviations may be used herein and have the indicated definitions: Boc, tert-butoxy carbonyl; BroP, bromotrispyrrolidinophosphonium hexafluorophosphate; CDI, 1,1'-carbonyldiimidazole; DCC, dicyclohexylcarbodiimide; DCE, dichloroethane; dichloromethane , dichloromethane; DIAD, diisopropylazodicarboxylate; DIBAL-H, diisobutylaluminium hydride; DIPEA, diisopropylethylamine; DEPC, diethyl phosphorocyanidate; DMA, N,N-dimethyl acetamide; DMAP, 4-(N, N-dimethylamino)pyridine; DMF, N,N-dimethylformamide; DMSO, dimethylsulfoxide; DTT, dithiothreitol; EDC, 1-(3-dimethylaminopropyl)-3-ethylcarbodiimide hydrochloride; ESI-MS, electrospray mass spectrometry; HATU, O-(7-azabenzotriazol-1-yl)-N, N, N', N'-tetramethyluronium hexafluorophosphate; HOBt, 1-hydroxybenzotriazole; HPLC, high pressure liquid chromatography; NHS, N-Hydroxysuccinimide; MMP, 4-methylmorpholine; PAB, p-aminobenzyl; PBS, phosphate-buffered saline (pH 7.0~7.5); PEG, polyethylene glycol; SEC, size-exclusion chromatography; TCEP, tris(2-carboxyethyl)phosphine; TFA, trifluoroacetic acid; THF, tetrahydrofuran; Val, valine.

### Specific embodiments

Specific embodiments of the present invention will be described in more detail below with reference to the drawings. While specific embodiments of the present invention have been shown in the drawings and the following examples, it is to be understood that the invention may be embodied in various forms and should not be limited by the embodiments set forth herein. Rather, these embodiments are provided to enable a more thorough understanding of the present invention, and to fully convey the scope of the invention to those skilled in the art.

The subject matter of the present invention can be embodied in many different forms and should not be construed as limited to the embodiments set forth herein. Indeed, many modifications and other embodiments of the present invention having the benefit of the teachings presented in the description included herein will occur to those skilled in the art to which this disclosure pertains. Therefore, it is to be understood that the subject matter of the present disclosure is not limited to the specific embodiments disclosed, and that modifications and other embodiments are intended to be included within the scope of the disclosed subject matter.

Although specific terms are used herein, they are used in a generic and descriptive sense only and not for purposes of limitation. Unless defined otherwise, all technical and scientific terms used herein have the same meaning as commonly understood by one of ordinary skill in the art to which this disclosure pertains.

Compounds are described using standard nomenclature. Unless defined otherwise, all technical and scientific terms used herein have the same meaning as commonly understood by one of ordinary skill in the art to which this invention belongs.

The term "a" or "one" does not denote a limitation of the number, but rather indicates the presence of at least one referenced item. Recitation of numerical ranges are merely intended as a shorthand method of referring individually to each separate value falling within the range, unless otherwise indicated herein, and each separate value is incorporated into the specification as if it were individually recited herein. All ranges of endpoints are included within the range and can be combined independently. All methods described herein may be performed in a suitable order unless otherwise indicated herein or clearly contradicted by context. The use of instances or exemplary language (eg, "such as") is intended to better illustrate the invention unless otherwise stated, and does not constitute a limitation on the scope of the invention.

The present invention includes the use of compounds of formula (I) and compounds having at least one desired atomic isotope substitution, in an amount higher than the natural abundance (ie, enrichment) of isotopes. Isotopes are atoms with the same atomic number but different mass numbers, i.e. the same number of protons but different numbers of neutrons. Isotope substitutions, such as deuterium substitution, may be partial or complete. Partial deuterium substitution means that at least one hydrogen is replaced by deuterium. In certain embodiments, the isotope is enriched at any preferred location by 90%, 95%, or 99% or more. In one embodiment, deuterium is enriched in the req.uied position by 90%, 95% or 99%.

It should be noted that certain terms are used in the description and claims to refer to particular components. Those skilled in the art will appreciate that the skilled person may refer to the same component with different nouns. The specification and claims do not serve as a means for distinguishing components in terms of differences in terms, but rather are used as a criterion for distinguishing between components. As mentioned throughout the specification and claims, "comprising" or "comprising" is an open term, and is therefore to be interpreted as "including but not limited to". The specification is subseq.uently described as implementing the preferred embodiments of the present invention, and the description is for the purpose of illustrating the general principles of the description and is not intended to limit the scope of the present invention. The scope of the invention is defined by the appended claims.

The term "C₁-C₆" means a group containing from 1 to 6 carbons.

The term "hydrophilic branched linker" means that the main framework is a peptide unit (1 to 12 natural or non-natural amino acids) of C₂-C₁₀₀, a hydrazone bond group, a disulfide group, an ester group, an oxime group, an amide group, or a thioether bond group.

The term "pharmaceutically acceptable salt" means a salt of a compound suitable for use in a pharmaceutical formulation. The compound has one or more basic groups, the salt can be an acid addition salt, such as sulfate, hydrobromate, tartrate, methanesulfonate, maleate, citrate, phosphate, acetate, alginate, hydroiodic acid, nitrate, hydrochloride, lactate, methyl sulfate, fumarate, benzoate, succinate, methanesulfonate, lactobate, octanoate, tosylate, and the like. The compound has one or more acidic groups, the salt can be a salt such as a calcium salt, a potassium salt, a magnesium salt, a meglumine salt, an ammonium salt, a zinc salt, a piperazine salt, an aminobutanetriol salt, a lithium salt, a choline salt, a diethylamine salt, a 4-phenylcyclohexylamine salt, a benzatine salt, a sodium salt, a tetramethylammonium salt, and the like. Polymorphic crystalline forms and solvates are also included within the scope of the present invention.

The pharmaceutically acceptable salts of the present invention can be made by conventional chemical methods. Generally, these salts may be formed by the addition of other suitable same eq.uivalents of base or acid in a mixed solution of the free acid or base of the compound of the present invention or an organic solution or both. The non-aqueous phase reaction medium is generally diethyl ether, ethyl acetate, ethanol, isopropanol or acetonitrile. The list of applicable salts can be found in Remington's Pharmaceutical Sciences, 17th ed. Mack. Publishing Company, Easton, PA. 1985, p. 1418.

Pharmaceutically acceptable excipients include all carriers, diluents, adjuvants or forming agents, such as preservatives, antioxidants, fillers, disintegrants, wetting agents, emulsifiers, suspending agents, solvents, dispersing media, coatings, antibacterial agents, antifungal agents, isotonic and absorption delaying agents, and the like. In the field of medicine, the addition of these adjuvants in active pharmaceutical ingredients is a very common practice. Unless the auxiliary material is not compatible with the active component of the drug, the auxiliary material is added to the pharmaceutical ingredient and is not the same. To achieve good results, the active auxiliary component can also be added to the pharmaceutical ingredient.

In the present invention, " 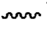 " of Formula (I) refers to chiral carbon atom site, which is selected from pure R, pure S or R/S in different propotions.

All stereoisomers as pure compounds and mixtures thereof are included within the scope of the present invention unless a particular stereoisomer is particularly pointed out (for example, by a thick or dotted bond at the relevant center in the formula, by describing a double bond in the formula having an E or Z configuration, or by using a stereochemistry designation nomenclature) Unless otherwise stated, individual enantiomers, diastereomers, geometric isomers, and combinations and mixtures thereof are inclueded by the present invention.

Those skilled in the art would understand that the compounds may have the form of tautomeric forms (such as ketones and enol forms), resonant forms and zwitterionic forms, which are eq.uivalent to those depicted in the structural formula used herein, and the structural formula includes such tautomeric, resonant, or zwitterionic forms.

Preparation of antibodies used in the present invention includes in vivo or in vitro procedures or combinations thereof. Methods for preparation polyclonal anti-receptor peptide antibodies are well-known in the art, such as in U.S. Pat. No. 4,493,795 (to Nestor et al) A monoclonal antibody is typically made by fusing myeloma cells with the spleen cells from a mouse that has been immunized with the desired antigen (Köhler, G.; Milstein, C. Nature 1975, 256: 495-7) The detailed procedures are described in Antibodies--A Laboratory Manual, Harlow and Lane, eds., Cold Spring Harbor Laboratory Press, New York (1988), which is incorporated herein by reference. Particularly monoclonal antibodies are prepared by immunizing mice, rats, hamsters or any other mammal with the antigen of interest such as the intact target cell, antigens isolated from the target cell, whole virus, attenuated whole virus, and viral proteins. Splenocytes are typically fused with myeloma cells using polyethylene glycol (PEG) 6000. Fused hybrids are selected by their sensitivity to HAT (hypoxanthine-aminopterin-thymine) Hybridomas producing a monoclonal antibody useful in practicing this invention are identified by their ability to immunoreacted specified receptors or inhibit receptor activity on target cells.

A monoclonal antibody used in the present invention can be prepared by initiating a monoclonal hybridoma culture comprising a nutrient medium containing a hybridoma that secretes antibody molecules of the appropriate antigen specificity. The culture is maintained under conditions and for a time period sufficient for the hybridoma to secrete the antibody molecules into the medium. The antibody-containing medium is then collected. The antibody molecules can then be further isolated by well-known techniques, such as using protein-A affinity chromatography; anion, cation, hydrophobic, or size exclusive chromatographies (particularly by affinity for the specific antigen after protein A, and sizing column chromatography); centrifugation, differential solubility, or by any other standard technique for the purification of proteins.

Culture medium used for the preparation of these compositions are both well-known in the art and commercially available and include synthetic culture media. An exemplary synthetic medium is Dulbecco's minimal essential medium (DMEM; Dulbecco et al., Virol. 8, 396 (1959)) supplemented with 4.5 gm/l glucose, 20 mM glutamine, 20% fetal calf serum, an anti-foaming agent, such as polyoxyethylene-polyoxypropylene block copolymer.

In addition, antibody-producing cell lines can also be created by techniques other than fusion, such as direct transformation of B lymphocytes with oncogenic DNA, or transfection with an oncovirus, such as Epstein-Barr virus (EBV, also called human herpesvirus 4 (HHV-4)) or Kaposi's sarcoma-associated herpesvirus (KSHV) See, U.S. Pat. Nos. 4,341,761; 4,399,121; 4,427,783; 4,444,887; 4,451,570; 4,466,917; 4,472,500; 4,491,632; 4,493,890. A monoclonal antibody may also be produced via an anti-receptor peptide or peptides containing the carboxyl terminal as described well-known in the art. See Niman et al., Proc. Natl. Acad. Sci. USA, 80: 4949-53 (1983); Geysen et al., Proc. Natl. Acad. Sci. USA, 82: 178-82 (1985); Lei et al. Biochemistry 34(20): 6675-88, (1995) Typically, the anti-receptor peptide or a peptide analog is used either alone or conjugated to an immunogenic carrier, as the immunogen for producing anti-receptor peptide monoclonal antibodies.

There are also a number of other well-known techniques for preparing monoclonal antibodies as binding molecules of this invention. Particularly useful are methods of making fully human antibodies. One method is phage display technology which can be used to select a range of human antibodies binding specifically to the antigen using methods of affinity enrichment. Phage display has been thoroughly described in the literature and the construction and screening of phage display libraries are well known in the art, see, e.g., Dente et al, Gene. 148(1):7-13 (1994); Little et al, Biotechnol Adv. 12(3): 539-55 (1994); Clackson et al., Nature 352: 264-8 (1991); Huse et al., Science 246: 1275-81 (1989)

Monoclonal antibodies derived by hybridoma technique from another species (such as mouse) should be humanized. The modified antibodies when infused into humans. Among the more common methods of humanization of antibodies are complementarity-determining region grafting and resurfacing. The modified antibody can greatly reduce the immune side response of the heterologous antibody to the human body. These methods have been extensively described, see e.g. U.S. Pat. Nos. 5,859,205 and 6,797,492; Liu et al, Immunol Rev. 222: 9-27 (2008); Almagro et al, Front Biosci. 13: 1619-33 (2008); Lazar et al, Mol Immunol. 44(8): 1986-98 (2007); Li et al, Proc. Natl. Acad. Sci. USA. 103(10): 3557-62 (2006) each incorporated herein by reference. Fully human antibodies can also be prepared by immunizing transgenic mice, rabbits, monkeys, or other mammals, carrying large portions of the human immunoglobulin heavy and light chains, with an immunogen. Example s of such mice are: the Xenomouse (Abgenix/Amgen), the HuMAb-Mouse (Medarex/BMS), the VelociMouse (Regeneron), see U.S. Pat. Nos. 6,596,541, 6,207,418, 6,150,584, 6,111,166, 6,075,181, 5,922,545, 5,661,016, 5,545,806, 5,436,149 and 5,569,825. In human therapy, murine variable regions and human constant regions can also be fused to construct called "chimeric antibodies" that are considerably less immunogenic in man than murine mAbs (Kipriyanov et al, Mol Biotechnol. 26: 39-60 (2004); Houdebine, Curr Opin Biotechnol. 13: 625-9 (2002), each incorporated herein by reference) In addition, site-directed mutagenesis in the variable region of an antibody can result in an antibody with higher affinity and specificity for its antigen (Brannigan et al, Nat Rev Mol Cell Biol. 3: 964-70, (2002)); Adams et al, J Immunol Methods. 231: 249-60 (1999)) and exchanging constant regions of a mAb can improve its ability to mediate effector functions of binding and cytotoxicity.

Antibodies immunospecific for a malignant cell antigen can also be obtained commercially or produced by any method known to one of skill in the art such as, e.g., chemical synthesis or recombinant expression techniques. The nucleotide seq.uence encoding antibodies immune-specific for a malignant cell antigen can be obtained commercially, e.g., from the GenBank database or a database like it, the literature publications, or by routine cloning and seq.uencing.

In addition to antibodies, peptides or proteins can also be used as binding molecules to block, attack or otherwise interact with receptors or epitopes corresponding to the surface of target cells. As long as these peptides or proteins can specifically bind to specific epitopes or their corresponding receptors, they do not necessarily belong to the immunoglobulin family. These peptides can also be isolated by a technique similar to phage display antibody (Szardenings, J receive signal transfer res. 2003; 23 (4): 307-49) The peptides obtained from random peptide libraries are similar to the invention of antibodies and antibody fragments. Polypeptide or protein molecules can maintain the specificity of antigen binding by connecting their binding molecules with some macromolecules or mediators. These macromolecules and media include albumin, polymers, liposomes, nanoparticles or dendrimers.

Example s of antibodies used for conjugation of drugs for treating cancers, autoimmune diseases, and infectious diseases include( but are not limited to): 3F8 (anti-GD2), Abagovomab (anti CA-125), Abciximab (anti CD41 (integrin α-IIB), Adalimumab (anti-TNF-a), Adecatumumab (anti-EpCAM, CD326), Afelimomab (anti-TNF-a); Afutuzumab (anti-CD20), Alacizumab pegol (anti-VEGFR2), ALD518 (anti-IL-6), Alemtuzumab :(Campath, MabCampath, anti- CD52), Altumomab (anti-CEA), Anatumomab (anti-TAG-72), Anrukinzumab (IMA-638, anti-IL-13), Apolizumab (anti-HLA-DR), Arcitumomab (anti-CEA), Aselizumab (anti-L-selectin (CD62L), Atlizumab (tocilizumab, Actemra, RoActemra, anti-IL-6 receptor), Atorolimumab (anti-Rhesus factor), Bapineuzumab (anti-β-amyloid), Basiliximab (Simulect, antiCD25 (α chain of IL-2 receptor), Bavituximab (anti-phosphatidylserine), Bectumomab (LymphoScan, anti-CD22), Belimumab (Benlysta, LymphoStat-B, anti-BAFF), Benralizumab (anti-CD125), Bertilimumab (anti-CCL11 (eotaxin-1)), Besilesomab (Scintimun, anti-CEA-related antigen), Bevacizumab (Avastin, anti-VEGF-A), Biciromab (FibriScint, anti-fibrin II β chain), Bivatuzumab (anti-CD44 v6), Blinatumomab (BiTE, anti-CD19), Brentuximab (cAC10, anti-CD30 TNFRSF8), Briakinumab (anti-IL-12, IL-23) Canakinumab (Ilaris, anti-IL-1), Cantuzumab (C242, anti-CanAg), Capromab, Catumaxomab (Removab, anti-EpCAM, anti-CD3), CC49 (anti-TAG-72), Cedelizumab (anti-CD4), Certolizumab pegol (Cimzia anti-TNF-a), Cetuximab (Erbitux, IMC-C225, anti-EGFR), Citatuzumab bogatox (anti-EpCAM), Cixutumumab (anti-IGF-1), Clenoliximab (anti-CD4), Clivatuzumab (anti-MUCl), Conatumumab (anti-TRAIL-R2), CR6261 (anti-Influenza A hemagglutinin), Dacetuzumab (anti-CD40), Daclizumab (Zenapax, anti-CD25 (α chain of IL-2 receptor)), Daratumumab (anti-CD38 (cyclic ADP ribose hydrolase), Denosumab (Prolia, anti-RANKL), Detumomab (anti-B-lymphoma cell), Dorlimomab, Dorlixizumab, Ecromeximab (anti-GD3 ganglioside), Eculizumab (Soliris, anti-C5), Edobacomab (anti-endotoxin), Edrecolomab (Panorex, MAb17-1A, anti-EpCAM), Efalizumab (Raptiva, anti-LFA-1 (CD11a), Efungumab (Mycograb, anti-Hsp90), Elotuzumab (anti-SLAMF7), Elsilimomab (anti-IL-6), Enlimomab pegol (anti-ICAM-1 (CD54)), Epitumomab (anti-episialin), Epratuzumab (anti-CD22), Erlizumab (anti-ITGB2 (CD18)), Ertumaxomab (Rexomun, anti-HER2/neu, CD3), Etaracizumab (Abegrin, anti-integrin αᵥβ₃), Exbivirumab ( anti-hepatitis B surface antigen), Fanolesomab (NeutroSpec, anti-CD15), Faralimomab (anti-interferon receptor), Farletuzumab (anti-folate receptor 1), Felvizumab (anti-respiratory syncytial virus), Fezakinumab (anti-IL-22), Figitumumab (anti-IGF-1 receptor), Fontolizumab (anti-IFN-y), Foravirumab (anti-rabies virus glycoprotein), Fresolimumab (anti-TGF-β), Galiximab (anti-CD80), Gantenerumab (anti-β amyloid), Gavilimomab (anti-CD147 (basigin)), Gemtuzumab (anti-CD33), Girentuximab (anti-carbonic anhydrase 9), Glembatumumab (CR011, anti-GPNMB), Golimumab (Simponi, anti-TNF-a), Gomiliximab (anti-CD23 (IgE receptor)), Ibalizumab (anti-CD4), Ibritumomab (anti-CD20), Igovomab (Indimacis-125, anti-CA-125), Imciromab (Myoscint, anti-cardiac myosin), Infliximab (Remicade, anti-TNF-a), Intetumumab (anti-CD51), Inolimomab (anti-CD25 (α chain of IL-2 receptor)), Inotuzumab (anti-CD22), Ipilimumab (anti-CD152), Iratumumab (anti- CD30 (TNFRSF8)), Keliximab (anti-CD4), Labetuzumab (CEA-Cide, anti-CEA), Lebrikizumab (anti-IL-13), Lemalesomab (anti-NCA-90 (granulocyte antigen)), Lerdelimumab (anti-TGF beta 2), Lexatumumab (anti-TRAIL-R2), Libivirumab (anti-hepatitis B surface antigen), Lintuzumab (anti-CD33), Lucatumumab (anti-CD40), Lumiliximab (anti- CD23 (IgE receptor), Mapatumumab (anti-TRAIL-R1), Maslimomab (anti- T-cell receptor), Matuzumab (anti-EGFR), Mepolizumab (Bosatria, anti-IL-5), Metelimumab (anti-TGF β 1), Milatuzumab (anti-CD74), Minretumomab (anti-TAG-72), Mitumomab (BEC-2, anti-GD3 ganglioside), Morolimumab (anti-Rhesus factor), Motavizumab (Numax, anti-respiratory syncytial virus), Muromonab-CD3 (Orthoclone OKT3, anti-CD3), Nacolomab (anti-C242), Naptumomab (anti-5T4), Natalizumab (Tysabri, anti-integrin α₄), Nebacumab (anti-endotoxin), Necitumumab (anti-EGFR), Nerelimomab (anti-TNF-a), Nimotuzumab (Theracim, Theraloc, anti-EGFR), Nofetumomab, Ocrelizumab (anti-CD20), Odulimomab (Afolimomab, anti-LFA-1 (CD11a)), Ofatumumab (Arzerra, anti-CD20), Olaratumab (anti-PDGF-R α), Omalizumab (Xolair, anti-IgE Fc region), Oportuzumab (anti-EpCAM), Oregovomab (OvaRex, anti-CA-125), Otelixizumab (anti-CD3), Pagibaximab (anti-lipoteichoic acid), Palivizumab (Synagis, Abbosynagis, anti-respiratory syncytial virus), Panitumumab (Vectibix, ABX-EGF, anti-EGFR), Panobacumab (anti-Pseudomonas aeruginosa), Pascolizumab (anti-IL-4), Pemtumomab (Theragyn, anti-MUC1), Pertuzumab (Omnitarg, 2C4,anti-HER2/neu), Pexelizumab (anti-C5), Pintumomab (anti-adenocarcinoma antigen), Priliximab (anti-CD4), Pritumumab (anti-vimentin), PRO 140 (anti-CCR5), Racotumomab (1E10, anti-(N-glycolylneuraminic acid (NeuGc, NGNA)-gangliosides GM3)), Rafivirumab (anti-rabies virus glycoprotein), Ramucirumab (anti-VEGFR2), Ranibizumab (Lucentis, anti-VEGF-A), Raxibacumab (anti-anthrax toxin, protective antigen), Regavirumab (anti-cytomegalovirus glycoprotein B), Reslizumab (anti-IL-5), Rilotumumab (anti-HGF), Rituximab (MabThera, Rituxanmab, anti-CD20), Robatumumab (anti-IGF-1 receptor), Rontalizumab (anti-IFN-a), Rovelizumab (LeukArrest, anti-CD11, CD18), Ruplizumab (Antova, anti-CD154 (CD40L)), Satumomab (anti-TAG-72), Sevirumab (anti-cytomegalovirus), Sibrotuzumab (anti-FAP), Sifalimumab (anti-IFN-a), Siltuximab (anti-IL-6), Siplizumab (anti-CD2), (Smart) MI95 (anti-CD33), Solanezumab (anti-beta amyloid), Sonepcizumab (anti-sphingosine-1-phosphate), Sontuzumab (anti-episialin), Stamulumab (anti-myostatin), Sulesomab (LeukoScan, anti-NCA-90 (granulocyte antigen)), Tacatuzumab (anti-α-fetoprotein), Tadocizumab (anti-integrin α_{IIb},β₃), Talizumab (anti-IgE), Tanezumab (anti-NGF), Taplitumomab (anti-CD19), Tefibazumab (Aurexis, (anti-clumping factor A)), Telimomab, Tenatumomab (anti-tenascin C), Teneliximab (anti-CD40), Teplizumab (anti-CD3), TGN1412 (anti-CD28), Ticilimumab (Tremelimumab, anti-CTLA-4), Tigatuzumab (anti-TRAIL-R2), TNX-650 (anti-IL-13), Tocilizumab (Atlizumab, Actemra, RoActemra, anti-IL-6 receptor), Toralizumab (anti-CD154 (CD40L)), Tositumomab (anti-CD20), Trastuzumab (Herceptin, anti-HER2/neu), Tremelimumab (anti-CTLA-4), Tucotuzumab celmoleukin (anti-EpCAM), Tuvirumab (anti-hepatitis B virus), Urtoxazumab (anti- Escherichia coli), Ustekinumab (Stelara, anti-IL-12, IL-23), Vapaliximab (anti-AOC3 (VAP-1)), Vedolizumab (anti-integrin α₄β₇), Veltuzumab (anti-CD20), Vepalimomab (anti-AOC3 (VAP-1)), Visilizumab (Nuvion, anti-CD3), Vitaxin (anti-vascular integrin avb3), Volociximab (anti-integrin α₅β₁), Votumumab (Humrespect, anti-tumor antigen CTAA16.88), Zalutumumab (HuMax-EGFr, anti-EGFR), Zanolimumab (HuMax-CD4, anti-CD4), Ziralimumab (anti-CD147 (basigin)), Zolimomab (anti-CD5), Etanercept (Enbrel^{®}), Alefacept (Amevive^{®}), Abatacept (Orencia^{®}), Rilonacept (Arcalyst), 14F7 (anti-IRP-2 (Iron Regulatory Protein 2)), 14G2a (anti-GD2 ganglioside, from Nat. Cancer Inst., for melanoma and solid tumors), J591 (anti-PSMA, from Weill Cornell Medical School, for prostate cancers), 225.28S (anti-HMW-MAA (High molecular weight-melanoma-associated antigen)), Sorin Radiofarmaci S.R.L. (Milan, Italy, for melanoma), COL-1 (anti-CEACAM3, CGM1, from Nat. Cancer Inst. USA, for colorectal and gastric cancers), CYT-356 (Oncoltad^{®}, for prostate cancers), HNK20 (OraVax Inc., for respiratory syncytial virus), ImmuRAIT (from Immunomedics, for NHL), Lym-1 (anti-HLA-DR10, from Peregrine Pharm., for Cancers), MAK-195F (anti-TNF (tumor necrosis factor, TNFA, TNF-α, TNFSF2), from Abbott/Knoll, for Sepsis toxic shock), MEDI-500 (T10B9, anti-CD3, TRαβ (T cell receptor alpha/beta) complex, from Medlmmune Inc, for Graft-versus-host disease), RING SCAN (anti-TAG 72 (tumour associated glycoprotein 72), from Neoprobe Corp., for Breast, Colon and Rectal cancers), Avicidin (anti-EPCAM (epithelial cell adhesion molecule)), anti-TACSTD1 (Tumor-associated calcium signal transducer 1), anti-GA733-2 (gastrointestinal tumor-associated protein 2), anti-EGP-2 (epithelial glycoprotein 2), anti-KSA, KS1/4 antigen, M4S, tumor antigen 17-1A, CD326 (from NeoRx Corp., for Colon, Ovarian, Prostate cancers and NHL), LymphoCide (Immunomedics, NJ), Smart ID10 (Protein Design Labs), Oncolym (Techniclone Inc, CA), Allomune (BioTransplant, CA), anti-VEGF (Genentech, CA), CEAcide (Immunomedics, NJ), IMC-1C11 (ImClone, NJ) and Cetuximab (ImClone, NJ) .

Other antibodies used to bind antigen include (but are not limited to): Aminopeptidase N (CD13), Annexin A1, B7-H3 (CD276, various cancers), CA125 (ovarian), CA15-3 (carcinomas), CA19-9 (carcinomas), L6 (carcinomas), Lewis Y (carcinomas), Lewis X (carcinomas), alpha fetoprotein (carcinomas), CA242 (colorectal), placental alkaline phosphatase (carcinomas), prostate specific antigen (prostate), prostatic acid phosphatase (prostate), epidermal growth factor (carcinomas), CD2 (Hodgkin's disease, NHL lymphoma, multiple myeloma), CD3 ε (T cell lymphoma, lung, breast, gastric, ovarian cancers, autoimmune diseases, malignant ascites), CD19 (B cell malignancies), CD20 (non-Hodgkin's lymphoma), CD22 (leukemia, lymphoma, multiple myeloma, SLE), CD30 (Hodgkin's lymphoma), CD33 (leukemia, autoimmune diseases), CD38 (multiple myeloma), CD40 (lymphoma, multiple myeloma, leukemia (CLL)), CD51 (metastatic melanoma, sarcoma), CD52 (leukemia), CD56 (small cell lung cancers, ovarian cancer, Merkel cell carcinoma, and the liquid tumor, multiple myeloma), CD66e (cancers), CD70 (metastatic renal cell carcinoma and non-Hodgkin lymphoma), CD74 (multiple myeloma), CD80 (lymphoma), CD98 (cancers), mucin (carcinomas), CD221 (solid tumors), CD227 (breast, ovarian cancers), CD262 (NSCLC and other cancers), CD309 (ovarian cancers), CD326 (solid tumors), CEACAM3 (colorectal, gastric cancers), CEACAM5 (carcinoembryonic antigen, CEA, CD66e) (breast, colorectal and lung cancers), DLL3 (delta-like-3), DLL4 (delta-like-4), EGFR (epidermal growth factor receptor, various cancers), CTLA4 (melanoma), CXCR4 (CD184, heme tumor, solid tumors), Endoglin (CD105, solid tumors), EPCAM (epithelial cell adhesion molecule; bladder, head, neck, colon, NHL prostate, and ovarian cancers), ERBB2 (epidermal growth factor receptor 2; lung, breast, prostate cancers), FCGR1 (autoimmune diseases), FOLR (folate receptor, ovarian cancers), GD2 ganglioside (cancers), G-28 (a cell surface antigen glyvolipid; melanoma), GD3 idiotype (cancers), Heat shock proteins (cancers), HER1 (lung, stomach cancers), HER2 (breast, lung and ovarian cancers), HLA-DR10 (NHL), HLA-DRB (NHL, B cell leukemia), human chorionic gonadotropin (carcinoma), IGF1R (insulin-like growth factor 1 receptor, solid tumors, blood cancers), IL-2 receptor (interleukin 2 receptor, T-cell leukemia and lymphomas), IL-6R (interleukin 6 receptor, multiple myeloma, RA, Castleman's disease, IL6 dependent tumors), Integrins (ανβ3, α5β1, α6β4, αllβ3, α5β5, αvβ5, for various cancers), MAGE-1 (carcinomas), MAGE-2 (carcinomas), MAGE-3 (carcinomas), MAGE 4 (carcinomas), anti-transferrin receptor (carcinomas), p97 (melanoma), MS4A1 (membrane-spanning 4-domains subfamily A member 1; Non-Hodgkin's B cell lymphoma, leukemia), MUC1 or MUC1-KLH (breast, ovarian, cervix, bronchus and gastrointestinal cancer), MUC16 (CA125) (ovarian cancers), CEA (colorectal), gp100 (melanoma), MART1 (melanoma), MPG (melanoma), MS4A1 (membrane-spanning 4-domains subfamily A; small cell lung cancers, NHL), Nucleolin, Neu oncogene product (carcinomas), P21 (carcinomas), Paratope of anti-(N-glycolylneuraminic acid) (breast, melanoma cancers), PLAP-like testicular alkaline phosphatase (ovarian, testicular cancers), PSMA (prostate tumors), PSA (prostate), ROBO4, TAG 72 (tumour associated glycoprotein 72; AML, gastric, colorectal, ovarian cancers), T cell transmembrane protein (cancers), Tie (CD202b), TNFRSF10B (tumor necrosis factor receptor superfamily member 10B; cancers), TNFRSF13B (tumor necrosis factor receptor superfamily member 13B; multiple myeloma, NHL, other cancers, RA and SLE), TPBG (trophoblast glycoprotein; renal cell carcinoma), TRAIL-R1 (tumor necrosis apoprosis inducing ligand receptor 1; lymphoma, NHL, colorectal, lung cancers), VCAM-1 (CD106, melanoma), VEGF, VEGF-A, VEGF-2 (CD309) (various cancers) Some other tumor associated antigens recognized by antibodies have been reviewed (Gerber, et al, mAbs 1:3, 247-53 (2009); Novellino et al, Cancer Immunol Immunother. 54(3), 187-207 (2005) Franke, et al, Cancer Biother Radiopharm. 2000, 15, 459-76)

There are many other antigens, including other different clusters (CD1, CD1a, CD1b, CD1c, CD1d, CD1e, CD2, CD3, CD3d, CD3e, CD3g, CD4, CD5, CD6, CD7, CD8, CD8a, CD8b, CD9, CD10, CD11a, CD11b, CD11c, CD11d, CD12w, CD14, CD15, CD16, CD16a, CD16b, CDw17, CD18, CD19, CD20, CD21, CD22, CD23, CD24, CD25, CD26, CD27, CD28, CD29, CD30, CD31, CD32, CD32a, CD32b, CD33, CD34, CD35, CD36, CD37, CD38, CD39, CD40, CD41, CD42, CD42a, CD42b, CD42c, CD42d, CD43, CD44, CD45, CD46, CD47, CD48, CD49b, CD49c, CD49c, CD49d, CD49f, CD50, CD51, CD52, CD53, CD54, CD55, CD56, CD57, CD58, CD59, CD60, CD60a, CD60b, CD60c, CD61, CD62E, CD62L, CD62P, CD63, CD64, CD65, CD65s, CD66, CD66a, CD66b, CD66c, CD66d, CD66e, CD66f, CD67, CD68, CD69, CD70, CD71, CD72, CD73, CD74, CD75, CD75s, CD76, CD77, CD78, CD79, CD79a, CD79b, CD80, CD81, CD82, CD83, CD84, CD85, CD85a, CD85b, CD85c, CD85d, CD85e, CD85f, CD85g, CD85g, CD85i, CD85j, CD85k, CD85m, CD86, CD87, CD88, CD89, CD90, CD91, CD92, CD93, CD94, CD95, CD96, CD97, CD98, CD99, CD100, CD101, CD102, CD103, CD104, CD105, CD106, CD107, CD107a, CD107b, CD108, CD109, CD110, CD111, CD112, CD113, CD114, CD115, CD116, CD117, CD118, CD119, CD120, CD120a, CD120b, CD121, CD121a, CD121b, CD122, CD123, CD123a, CD124, CD125, CD126, CD127, CD128, CD129, CD130, CD131, CD132, CD133, CD134, CD135, CD136, CD137, CD138, CD139, CD140, CD140a, CD140b, CD141, CD142, CD143, CD144, CD145, CDw145, CD146, CD147, CD148, CD149, CD150, CD151, CD152, CD153, CD154, CD155, CD156, CD156a, CD156b, CD156c, CD156d, CD157, CD158, CD158a, CD158b1, CD158b2, CD158c, CD158d, CD158e1, CD158e2, CD158f2, CD158g, CD158h, CD158i, CD158j, CD158k, CD159, CD159a, CD159b, CD159c, CD160, CD161, CD162, CD163, CD164, CD165, CD166, CD167, CD167a, CD167b, CD168, CD169, CD170, CD171, CD172, CD172a, CD172b, CD172g, CD173, CD174, CD175, CD175s, CD176, CD177, CD178, CD179, CD179a, CD179b, CD180, CD181, CD182, CD183, CD184, CD185, CD186, CDw186, CD187, CD188, CD189, CD190, CD191, CD192, CD193, CD194, CD195, CD196, CD197, CD198, CD199, CDw198, CDw199, CD200, CD201, CD202, CD202(a, b), CD203, CD203c, CD204, CD205, CD206, CD207, CD208, CD209, CD210, CDw210a, CDw210b, CD211, CD212, CD213, CD213a1, CD213a2, CD214, CD215, CD216, CD217, CD218, CD218a, CD218, CD21b9, CD220, CD221, CD222, CD223, CD224, CD225, CD226, CD227, CD228, CD229, CD230, CD231, CD232, CD233, CD234, CD235, CD235a, CD235b, CD236, CD237, CD238, CD239, CD240, CD240ce, CD240d, CD241, CD242, CD243, CD244, CD245, CD246, CD247, CD248, CD249, CD250, CD251, CD252, CD253, CD254, CD255, CD256, CD257, CD258, CD259, CD260, CD261, CD262, CD263, CD264, CD265, CD266, CD267, CD268, CD269, CD270, CD271, CD272, CD273, CD274, CD275, CD276, CD277, CD278, CD279, CD281, CD282, CD283, CD284, CD285, CD286, CD287, CD288, CD289, CD290, CD291, CD292, CD293, CD294, CD295, CD296, CD297, CD298, CD299, CD300, CD300a, CD300b, CD300c, CD301, CD302, CD303, CD304, CD305, CD306, CD307, CD307a, CD307b, CD307c, CD307d, CD307e, CD307f, CD308, CD309, CD310, CD311, CD312, CD313, CD314, CD315, CD316, CD317, CD318, CD319, CD320, CD321, CD322, CD323, CD324, CD325, CD326, CD327, CD328, CD329, CD330, CD331, CD332, CD333, CD334, CD335, CD336, CD337, CD338, CD339, CD340, CD341, CD342, CD343, CD344, CD345, CD346, CD347, CD348, CD349, CD350, CD351, CD352, CD353, CD354, CD355, CD356, CD357, CD358, CD359, CD360, CD361, CD362, CD363, CD364, CD365, CD366, CD367, CD368, CD369, CD370, CD371, CD372, CD373, CD374, CD375, CD376, CD377, CD378, CD379, CD381, CD382, CD383, CD384, CD385, CD386, CD387, CD388, CD389, CRIPTO, CR, CR1, CRGF, CRIPTO, CXCR5, LY64, TDGF1, 4-1BB, APO2,ASLG659,BMPR1B, 4-1BB, 5AC, 5T4), APO2, ASLG659, BMPR1B (bone morphogenetic protein receptor), CRIPTO, annexin A1, nucleolus, Endoglin (CD105), ROBO4, aminopeptidase N, delta-like-3 (DLL3 ), delta-like-4 (DLL4 ),VEGFR-2 (CD309), CXCR4 (CD 184), Tie2, B7-H3, WT1, MUC1, LMP2, HPV E6 E7, EGFRvIII, HER-2/neu, idiotype, MAGE A3, P53 nonmutant, NY-ESO- 1, GD2, CEA, MelanA/MART1, Napi3b (NAPI-3B, NPTIIb, SLC34A2, solute carrier family 34 member 2, Type II sodium-dependent phosphorus transport 3b), Ras mutation, gp100, p53 mutant, proteinase 3(PR1), BCR-abl, tetratocarcinoma derived growth factor, EphA receptor, EphB receptor, EGFR, EGFRvIII, ETBR (endothelin), HER2/neu, HER3, HLA-DOB (MHC class II molecule IA antigen), Integrin, IRTA2, MPF(MPF, MSLN, SMR, Megakaryocyte enhancing factor, mesothelin), CRIPTO, Sema 5b ( FLJ10372, KIAA1445, Mm42015, SEMA5B, 5EMAG, semaphoring 5 bHlog, sdema domain, seven platelet repeats, cytoplasmic region), PSCA, STEAP1 (6 transmembrane epithelial prostate antigens) and STEAP2 (HGNC 8639, IPCA-1, PCANP1, STAMP1, STEAP2, STMP, prostate cancer), tyrosinase, survivin, hTERT, sarcoma translocation breakpoint, EphA2, PAP, ML-IAP, AFP, EpCAM, ERG (TMPRSS2 ETS fusion gene), NA17, PAX3, ALK, androgen receptor, cyclin B1, polysialic acid, MYCN, RhoC, TRP- 2, GD3, fucose ganglioside, mesothelin, PSMA, MAGE A1, sLe (a), CYP1B1, PLAC1, GM3, BORIS, Tn, GloboH, ETV6-AML, NY-BR-1, RGS5, SART3, STn, carbonic anhydrase IX, PAX5, OY-TES1, sperm protein 17, LCK, HMWMAA, AKAP-4, SSX2, XAGE 1, B7H3, legume protein, Tie 2, Trop2, VEGFR2, MAD -CT-1, FAP, PDGFR-β, MAD-CT-2, fos protein-associated antigen 1.

The conjugates of this invention are used for the targeted treatment of cancers. The targeted cancers include, but are not limited to, adrenocortical carcinoma, anal cancer, bladder cancer, brain tumor (adult, brain stem glioma, childhood, cerebellar astrocytoma, cerebral astrocytoma, ependymoma, medulloblastoma, supratentorial primitive neuroectodermal and pineal tumors, visual pathway and hypothalamic glioma), breast cancer, carcinoid tumor, gastrointestinal, carcinoma of unknown primary, cervical cancer, colon cancer, endometrial cancer, esophageal cancer, extrahepatic bile duct cancer, Ewings family of tumors (PNET), extracranial germ cell tumor, eye cancer, intraocular melanoma, gallbladder cancer, gastric cancer (stomach), germ cell tumor, extragonadal, gestational trophoblastic tumor, head and neck cancer, hypopharyngeal cancer, islet cell carcinoma, kidney cancer (renal cell cancer), laryngeal cancer, leukemia (acute lymphoblastic, acute myeloid, chronic lymphocytic, chronic myelogenous, hairy cell), lip and oral cavity cancer, liver cancer, lung cancer (non-small cell, small cell), lymphoma (aids-related, central nervous system, cutaneous T-cell, hodgkin's disease, non-hodgkin's disease), malignant mesothelioma, melanoma, Merkel cell carcinoma, metasatic squamous neck cancer with occult primary, multiple myeloma, and other plasma cell neoplasms, mycosis fungoides, myelodysplastic syndrome, myeloproliferative disorders, nasopharyngeal cancer, neuroblastoma, oral cancer, oropharyngeal cancer, osteosarcoma, ovarian cancer (epithelial, germ cell tumor, low malignant potential tumor), pancreatic cancer (exocrine, islet cell carcinoma), paranasal sinus and nasal cavity cancer, parathyroid cancer, penile cancer, pheochromocytoma cancer, pituitary cancer, plasma cell neoplasm, prostate cancer rhabdomyosarcoma, rectal cancer, renal cell cancer (kidney cancer), renal pelvis and ureter (transitional cell), salivary gland cancer, sezary syndrome, skin cancer, skin cancer (cutaneous T-cell lymphoma, Kaposi's sarcoma, melanoma), small intestine cancer, soft tissue sarcoma, stomach cancer, testicular cancer, thymoma (malignant), thyroid cancer, urethral cancer, uterine cancer (sarcoma), unusual cancer of childhood, vaginal cancer, vulvar cancer, Wilms' tumor.

The conjugates of this invention are used for the treatment or prevention of an autoimmune disease. The autoimmune diseases include, but are not limited to, achlorhydra autoimmune active chronic hepatitis, acute disseminated encephalomyelitis, acute hemorrhagic leukoencephalitis, Addison's disease, Agammaglobulinemia, alopecia areata, amyotrophic lateral sclerosis, ankylosing spondylitis, anti-GBM/TBM nephritis, antiphospholipid syndrome, antisynthetase syndrome, arthritis, atopic allergy, atopic dermatitis, autoimmune aplastic anemia, autoimmune cardiomyopathy, autoimmune hemolytic anemia, autoimmune hepatitis, autoimmune inner ear disease, autoimmune lymphoproliferative syndrome, autoimmune peripheral neuropathy, autoimmune pancreatitis, autoimmune polyendocrine syndrome Types I, II, & III, autoimmune progesterone dermatitis, autoimmune thrombocytopenic purpura, autoimmune uveitis, Balo disease/Balo concentric sclerosis, Bechets syndrome, BergeR's disease, Bickerstaff's encephalitis, Blau syndrome, Bullous pemphigoid, Castleman's disease, Chagas disease, chronic fatigue immune dysfunction Syndrome, chronic inflammatory demyelinating polyneuropathy, chronic recurrent multifocal ostomyelitis, chronic lyme disease, chronic obstructive pulmonary disease, Churg-Strauss syndrome, cicatricial pemphigoid, coeliac disease, Cogan syndrome, cold agglutinin disease, complement component 2 deficiency, cranial arteritis, CREST syndrome, Crohns disease (a type of idiopathic inflammatory bowel diseases), Cushing's Syndrome, cutaneous leukocytoclastic angiitis, Dego' s disease, Dercum' s disease, dermatitis herpetiformis, dermatomyositis, diabetes mellitus type 1, diffuse cutaneous systemic sclerosis, DressleR's syndrome, discoid lupus erythematosus, eczema, endometriosis, enthesitis-related arthritis, eosinophilic fasciitis, epidermolysis bullosa acquisita, erythema nodosum, essential mixed cryoglobulinemia, Evan's syndrome, fibrodysplasia ossificans progressiva, fibromyalgia, fibromyositis, fibrosing aveolitis, gastritis, gastrointestinal pemphigoid, giant cell arteritis, glomerulonephritis, Goodpasture's syndrome, Graves' disease, Guillain-Barre syndrome, Hashimoto's encephalitis, Hashimoto's thyroiditis, haemolytic anaemia, Henoch-Schonlein purpura, herpes gestationis, hidradenitis suppurativa, Hughes syndrome (See antiphospholipid syndrome), hypogamma-globulinemia, idiopathic inflammatory femyelinating disease, idiopathic pulmonary fibrosis, idiopathic thrombocytopenic purpura (See autoimmune thrombocytopenic purpura), IgA nephropathy (Also BergeR's disease), inclusion body myositis, inflammatory demyelinating polyneuopathy, interstitial cystitis, irritable bowel syndrome, juvenile idiopathic arthritis, juvenile rheumatoid arthritis, Kawasaki's disease, Lambert-Eaton myasthenic syndrome, leukocytoclastic vasculitis, lichen planus, lichen sclerosus, linear IgA disease (LAD), Lou Gehrig's disease (Also Amyotrophic lateral sclerosis), lupoid hepatitis, lupus erythematosus, majeed syndrome, Ménière's disease, microscopic polyangiitis, Miller-Fisher syndrome, mixed monnective tissue disease, morphea, Mucha-Habermann disease, Muckle-Wells syndrome, multiple myeloma, multiple sclerosis, myasthenia gravis, myositis, narcolepsy, neuromyelitis optica (Devic's disease), neuromyotonia, occular cicatricial pemphigoid, opsoclonus myoclonus syndrome, ord thyroiditis, palindromic rheumatism, PANDAS (pediatric autoimmune neuropsychiatric disorders associated with streptococcus), paraneoplastic cerebellar degeneration, paroxysmal nocturnal hemoglobinuria, Parry Romberg syndrome, Parsonnage-Turner syndrome, pars planitis, pemphigus, pemphigus vulgaris, pernicious anaemia, perivenous encephalomyelitis, POEMS syndrome, polyarteritis nodosa, polymyalgia rheumatica, polymyositis, primary biliary cirrhosis, primary sclerosing cholangitis, progressive inflammatory neuropathy, psoriasis, psoriatic arthritis, pyoderma gangrenosum, pure red cell aplasia, Rasmussen's encephalitis, Raynaud phenomenon, relapsing polychondritis, ReiteR's syndrome, restless leg syndrome, retroperitoneal fibrosis, rheumatoid arthritis, rheumatoid fever, sarcoidosis, schizophrenia, Schmidt syndrome, Schnitzler syndrome, scleritis, scleroderma, Sjögren's syndrome, spondyloarthropathy, sticky blood syndrome, Still's disease, stiff person syndrome, subacute bacterial endocarditis, Susac's syndrome, sweet syndrome, sydenham chorea, sympathetic ophthalmia, Takayasu's arteritis, temporal arteritis (giant cell arteritis), Tolosa-Hunt syndrome, transverse myelitis, ulcerative colitis (a type of idiopathic inflammatory bowel diseases), undifferentiated connective tissue disease, undifferentiated spondyloarthropathy, vasculitis, vitiligo, WegeneR's granulomatosis, Wilson's syndrome, Wiskott-Aldrich syndrome.

In another specific embodiment, the antigen-binding molecules used for the conjugate for the treatment or prevention of an autoimmune disease include, but are not limited to: anti-elastin antibody; Abys against epithelial cells antibody; anti-basement membrane collagen Type IV protein antibody; anti-nuclear antibody; anti ds DNA; anti ss DNA, anti cardiolipin antibody IgM, IgG; anti-celiac antibody; anti phospholipid antibody IgK, IgG; anti SM antibody; anti mitochondrial antibody; thyroid antibody; microsomal antibody, T-cells antibody; thyroglobulin antibody, anti SCL-70; anti-Jo; anti-systemic lupus erythematosus antibody; anti-parietal cell antibody; anti-histone antibody; anti RNP; C-ANCA; P-ANCA; anti centromere; anti-Fibrillarin, and anti GBM antibody, anti-ganglioside antibody; anti-desmogein 3 antibody; anti-p62 antibody; anti-sp100 antibody; anti-mitochondrial (M2) antibody; rheumatoid factor antibody; anti-MCV antibody; anti-topoisomerase antibody; anti-neutrophil cytoplasmic (CANCA) antibody.

In certain preferred embodiments, the binding molecule for the conjugate in the present invention, can bind to a receptor or receptor complex expressed on an activated lymphocyte which is associated with an autoimmune disease. The receptor or receptor complex can comprise an immunoglobulin gene superfamily member (e.g. CD2, CD3, CD4, CD8, CD19, CD20, CD22, CD28, CD30, CD33, CD37, CD38, CD56, CD70, CD79, CD79b, CD90, CD125, CD137, CD138, CD147, CD152/CTLA-4, PD-1, or ICOS), a TNF receptor superfamily member (e.g. CD27, CD40, CD95/Fas, CD134/OX40, CD137/4-1BB, INF-R1, TNFR-2, RANK, TACI, BCMA, osteoprotegerin, Apo2/TRAIL-R1, TRAIL-R2, TRAIL-R3, TRAIL-R4, and 30 APO-3), an integrin, a cytokine receptor, a chemokine receptor, a major histocompatibility protein, a lectin (C-type, S-type, or I-type), or a complement control protein.

In another specific embodiment, the useful cell binding ligands that are immunospecific to a viral or a microbial antigen are humanized or human monoclonal antibodies. As used herein, the term "viral antigen" includes, but is not limited to, any viral peptide, polypeptide protein (e.g. HIV gp120, HIV nef, RSV F glycoprotein, influenza virus neuraminidase, influenza virus hemagglutinin, HTLV tax, herpes simplex virus glycoprotein (e.g. gB, gC, gD, and gE) and (hepatitis B surface antigen) that is capable of eliciting an immune response. As used herein, the term "microbial antigen" includes, but is not limited to, any microbial peptide, polypeptide, protein, saccharide, polysaccharide, or lipid molecule (e.g., a bacteria, fungi, pathogenic protozoa, or yeast polypeptides including, e.g., LPS and capsular polysaccharide 5/8) that is capable of eliciting an immune response. Example s of antibodies available 1 for the viral or microbial infection include, but are not limited to, Palivizumab which is a humanized anti-respiratory syncytial virus monoclonal antibody for the treatment of RSV infection; PRO542 which is a CD4 fusion antibody for the treatment of HIV infection; Ostavir which is a human antibody for the treatment of hepatitis B virus; PROTVIR which is a humanized IgG.sub.1 antibody for the treatment of cytomegalovirus; and (anti-LPS) antibodies.

The conjugates of this invention can be used in the treatment of infectious diseases. These infectious diseases include, but are not limited to, acinetobacter infections, actinomycosis, african sleeping sickness (african trypanosomiasis), aids (acquired immune deficiency syndrome), amebiasis, microsporidiosis, anthrax, argentine hemorrhagic fever, ascariasis, aspergillosis, astrovirus infection, babesiosis, bacillus cereus infection, bacterial pneumonia, bacterial vaginosis, bacteroides infection, balantidiasis, baylisascaris infection, bk virus infection, black piedra, blastocystis hominis infection, blastomycosis, bolivian hemorrhagic fever, borrelia infection, botulism (and infant botulism), brazilian hemorrhagic fever, brucellosis, burkholderia infection, buruli ulcer, calicivirus infection (norovirus and sapovirus), campylobacteriosis, candidiasis (moniliasis; thrush), cat-scratch disease, cellulitis, chagas disease (american trypanosomiasis), chancroid, chickenpox, chlamydia, chlamydophila pneumoniae infection, cholera, chromoblastomycosis, liver fluke disease, clostridium difficile infection, coccidioido-mycosis, colorado tick fever, common cold (acute viral rhinopharyngitis; acute coryza), creutzfeldt-jakob disease, crimean-congo hemorrhagic fever, cryptococcosis, cryptosporidiosis, skin larval migration, cyclosporidium infection, cysticercosis, cytomegalovirus infection, dengue fever, binuclear amebiasis, diphtheria, diphyllobothriasis, dracunculiasis, ebola hemorrhagic fever, echinococcosis, ehrlichiosis, enterobiasis (pinworm infection), enterococcus infection, enterovirus infection, epidemic typhus, erythema infectiosum (fifth disease), acute rash in children, gingerworm disease, fatal familial insomnia, filariasis, food poisoning by clostridium perfringens, free-living amebic infection, fusobacterium infection, gas gangrene (clostridial myonecrosis), gothicillosis, gistmann-strauss syndrome, giardiasis, eq.uine meliosis, palate oral nematode disease, gonorrhea, granuloma inguinale (donovanosis), group A streptococcal infection, group B streptococcal infection, haemophilus influenzae infection, hand, foot and mouth disease (HFMD), hantavirus pulmonary syndrome, helicobacter pylori infection, hemolytic-uremic syndrome, hemorrhagic fever with renal syndrome, hepatitis A, hepatitis B, hepatitis C, hepatitis D, hepatitis E, herpes simplex, histoplasmosis, hookworm infection, human bocavirus infection, human ewingii ehrlichiosis, human granulocytic anaplasmosis, human metapneumovirus infection, human monocytic ehrlichiosis, human papillomavirus infection, human parainfluenza virus infection, hymenolepiasis, epstein-barr virus infectious mononucleosis (mono), influenza, isosporiasis, kawasaki disease, keratitis, kingella kingae infection, kuru, lassa fever, legionellosis (Legionnaires' disease), legionellosis (pontiac fever), leishmaniasis, leprosy, leptospirosis, listeriosis, lyme disease (lyme borreliosis), lymphatic filariasis (elephantiasis), lymphocytic choriomeningitis, malaria, marburg hemorrhagic fever, measles, melioidosis (Whitmore's disease), meningitis, meningococcal disease, metagonimiasis, microsporidiosis, molluscum contagiosum, mumps, murine typhus (endemic typhus), mycoplasma pneumonia, mycetoma, myiasis, neonatal conjunctivitis (ophthalmia neonatorum), Creutzfeldt-Jakob disease (vCJD, nvCJD), Nocardiosis, onchocerciasis (river blindness), paracoccidioidomycosis (south american blastomycosis), paragonimiasis, pasteurellosis, pediculosis capitis (head lice), pediculosis corporis (body lice), pediculosis pubis (pubic lice, crab lice), pelvic inflammatory disease, pertussis (Whooping cough), plague, pneumococcal infection, pneumocystis pneumonia, pneumonia, poliomyelitis, prevotella infection, primary amoebic meningoencephalitis, progressive multifocal leukoencephalopathy, psittacosis, Q fever, rabies, rat-bite fever, respiratory syncytial virus infection, rhinosporidiosis, rhinovirus infection, rickettsial infection, rickettsial-pox, rift valley fever, rocky mountain spotted fever, rotavirus infection, rubella, salmonellosis, SARS (severe acute respiratory syndrome), scabies, schistosomiasis, sepsis, shigellosis (bacillary dysentery), shingles (herpes zoster), smallpox (variola), sporotrichosis, staphylococcal food poisoning, staphylococcal infection, nematodes, syphilis, tapeworm disease, tetanus (closed teeth disease), tinea bursa, tinea capitis, tinea corporis, tinea cruris, tinea hand, pityriasis nigricans, tinea pedis, onychomycosis, tinea versicolor, toxoplasmosis (eye larval migration), toxoplasmosis (visceral larval migration)), toxoplasmosis, trichinosis, trichomoniasis, whipworm infection, tuberculosis, tularemia, ureolytic mycoplasma urea infection, venezuelan eq.uine encephalitis, venezuelan hemorrhagic fever, viral pneumonia, sini rouge, white sarcoidosis (white sarcoidosis), pseudotuberculosis infection, yersinia disease, yellow fever, zygomycosis.

The cell binding molecule described in this invention that are against pathogenic strains including, but are not limit, acinetobacter baumannii, actinomyces israelii, actinomyces gerencseriae and propionibacterium propionicus, trypanosoma brucei, HIV (human immunodeficiency virus), entamoeba histolytica, anaplasma genus, bacillus anthracis, arcanobacterium haemolyticum, junin virus, ascaris lumbricoides, aspergillus genus, astroviridae family, babesia genus, bacillus cereus, multiple bacteria, bacteroides genus, balantidium coli, baylisascaris genus, BK virus, piedraia hortae, blastocystis hominis, blastomyces dermatitides, machupo virus, borrelia genus, clostridium botulinum, sabia, brucella genus, usually burkholderia cepacia and other burkholderia species, mycobacterium ulcerans, caliciviridae family, campylobacter genus, usually candida albicans and other candida species, bartonella henselae, group A streptococcus and staphylococcus, trypanosoma cruzi, haemophilus ducreyi, varicella zoster virus (VZV), chlamydia trachomatis, chlamydophila pneumoniae, vibrio cholerae, fonsecaea pedrosoi, clonorchis sinensis, clostridium difficile, coccidioides immitis and coccidioides posadasii, Colorado tick fever virus, rhinoviruses, coronaviruses, CJD prion, Crimean-Congo hemorrhagic fever virus, cryptococcus neoformans, cryptosporidium genus, ancylostoma braziliense; multiple parasites, cyclospora cayetanensis, taenia solium, cytomegalovirus, dengue viruses (DEN-1, DEN-2, DEN-3 and DEN-4), flaviviruses, dientamoeba fragilis, corynebacterium diphtheriae, diphyllobothrium, dracunculus medinensis, Ebola virus, echinococcus genus, ehrlichia genus, enterobius vermicularis, enterococcus genus, enterovirus genus, rickettsia prowazekii, parvovirus B19, human herpesvirus 6 and human herpesvirus 7, fasciolopsis buski, fasciola hepatica and fasciola gigantica, ffi prion, filarioidea superfamily, clostridium perfringens, fusobacterium genus, clostridium perfringens; other clostridium species, geotrichum candidum, GSS prion, giardia intestinalis, burkholderia mallei, gnathostoma spinigerum and gnathostoma hispidum, neisseria gonorrhoeae, klebsiella granulomatis, streptococcus pyogenes, streptococcus agalactiae, haemophilus influenzae, enteroviruses, mainly coxsackie a virus and enterovirus 71, Sinnombre virus, helicobacter pylori, escherichia coli O157:H7, bunyaviridae family, hepatitis A virus, hepatitis B virus, hepatitis C virus, hepatitis D virus, hepatitis E virus, herpes simplex virus 1, herpes simplex virus 2, histoplasma capsulatum, ancylostoma duodenale and necator americanus, hemophilus influenzae, human bocavirus, ehrlichia ewingii, anaplasma phagocytophilum, human metapneumovirus, ehrlichia chaffeensis, human papillomavirus, human parainfluenza viruses, hymenolepis nana and hymenolepis diminuta, epstein-barr virus, orthomy-xoviridae family, isospora belli, kingella kingae, klebsiella pneumoniae, klebsiella ozaenas, klebsiella rhinoscleromotis, kuru prion, lassa virus, legionella pneumophila, legionella pneumophila, leishmania genus, mycobacterium leprae and mycobacterium lepromatosis, leptospira genus, Listeria monocytogenes, borrelia burgdorferi and other borrelia species, wuchereria bancrofti and brugia malayi, lymphocytic choriomeningitis virus (LCMV), plasmodium genus, marburg virus, measles virus, burkholderia pseudomallei, neisseria meningitides, metagonimus yokagawai, microsporidia phylum, molluscum contagiosum virus (MCV), mumps virus, rickettsia typhi, mycoplasma pneumoniae, numerous species of bacteria (actinomycetoma) and fungi (eumycetoma), parasitic dipterous fly larvae, chlamydia trachomatis and neisseria gonorrhoeae, vCJD prion, nocardia asteroides and other nocardia species, onchocerca volvulus, paracoccidioides brasiliensis, paragonimus westermani and other paragonimus species, pasteurella genus, pediculus humanus capitis, pediculus humanus corporis, phthirus pubis, bordetella pertussis, yersinia pestis, streptococcus pneumoniae, pneumocystis jirovecii, poliovirus, prevotella genus, naegleria fowleri, JC virus, chlamydophila psittaci, coxiella burnetii, rabies virus, streptobacillus moniliformis and spirillum minus, respiratory syncytial virus, rhinosporidium seeberi, rhinovirus, rickettsia genus, rickettsia akari, rift valley fever virus, Rickettsia rickettsii, rotavirus, rubella virus, salmonella genus, sars coronavirus, sarcoptes scabiei, schistosoma genus, shigella genus, varicella zoster virus, variola major or variola minor, sporothrix schenckii, staphylococcus genus, staphylococcus genus, staphylococcus aureus, streptococcus pyogenes, strongyloides stercoralis, treponema pallidum, taenia genus, clostridium tetani, trichophyton genus, trichophyton tonsurans, trichophyton genus, epidermophyton floccosum, trichophyton rubrum, and trichophyton mentagrophytes, trichophyton rubrum, hortaea werneckii, trichophyton genus, malassezia genus, toxocara canis or toxocara cati, toxoplasma gondii, trichinella spiralis, trichomonas vaginalis, trichuris trichiura, mycobacterium tuberculosis, francisella tularensis, ureaplasma urealyticum, venezuelan eq.uine encephalitis virus, vibrio colerae, guanarito virus, west nile virus, trichosporon beigelii, yersinia pseudotuberculosis, yersinia enterocolitica, yellow fever virus, mucorales order (mucormycosis) and entomophthorales order (entomophthora-mycosis), pseudomonas aeruginosa, campylobacter (vibrio) fetus, aeromonas hydrophila, edwardsiella tarda, yersinia pestis, shigella dysenteriae, shigella flexneri, shigella sonnei, salmonella typhimurium, treponema pertenue, treponema carateneum, borrelia vincentii, borrelia burgdorferi, leptospira icterohemorrhagiae, pneumocystis carinii, brucella abortus, brucella suis, brucella melitensis, mycoplasma spp., rickettsia prowazeki, rickettsia tsutsugumushi, clamydia spp., pathogenic fungi (aspergillus fumigatus, candida albicans, histoplasma capsulatum); protozoa (entomoeba histolytica, trichomonas tenas, trichomonas hominis, tryoanosoma gambiense, trypanosoma rhodesiense, leishmania donovani, leishmania tropica, leishmania braziliensis, pneumocystis pneumonia, plasmodium vivax, plasmodium falciparum, plasmodium malaria or helminiths (schistosoma japonicum, schistosoma mansoni, schistosoma haematobium, and hookworms)

Other antidodies used in this invention for the treatment of viral disease include, but are not limited to, antibodies against antigens of the pathogenic viruses, which include, but are not limited to poxyiridae, herpesviridae, adenoviridae, papovaviridae, enteroviridae, picornaviridae, parvoviridae, reoviridae, retroviridae, influenza viruses, parainfluenza viruses, mumps, measles, respiratory syncytial virus, rubella, arboviridae, rhabdoviridae, arenaviridae, Non-A/Non-B hepatitis virus, rhinoviridae, coronaviridae, rotoviridae, oncovirus [such as, HBV (hepatocellular carcinoma), HPV (cervical cancer, anal cancer), Kaposi's sarcoma-associated herpesvirus (Kaposi's sarcoma), epstein-barr virus (nasopharyngeal carcinoma, Burkitt's lymphoma, primary central nervous system lymphoma), MCPyV (merkel cell cancer), SV40 (simian virus 40), HCV (hepatocellular carcinoma), HTLV-I (adult T-cell leukemia/lymphoma)]; immune disorders caused virus [such as human immunodeficiency virus (AIDs)]; central nervous system virus [such as JCV (progressive multifocal leukoencephalopathy), MEV (subacute sclerosing panencephalitis), LCV (lymphocytic choriomeningitis), arbovirus encephalitis, orthomyxoviridae (probable) (encephalitis lethargica), RV (rabies), chandipura virus, herpesviral meningitis, ramsay hunt syndrome type II, poliovirus (poliomyelitis, post-polio syndrome), HTLV-1 (tropical spastic paraparesis)]; cytomegalovirus (cytomegalovirus retinitis, HSV (herpetic keratitis)); cardiovascular virus [such as CBV (pericarditis, myocarditis)]; respiratory system/acute viral nasopharyngitis/viral pneumonia [epstein-barr virus (EBV infection/infectious mononucleosis), cytomegalovirus; SARS coronavirus (severe acute respiratory syndrome); orthomyxoviridae: influenzavirus A/B/C (influenza/avian influenza); paramyxovirus: human parainfluenza viruses (parainfluenza); RSV(human respiratory syncytialvirus); HMPV]; digestive system virus [MUV (mumps), cytomegalovirus (cytomegalovirus esophagitis); adenovirus (adenovirus infection); rotavirus, norovirus, astrovirus, coronavirus; HBV (hepatitis B virus), CBV, HAV (hepatitis A virus), HCV (hepatitis C virus), HDV (hepatitis D virus), HEV (hepatitis E virus), HGV (hepatitis G virus)]; urogenital virus [such as BK virus, MUV (mumps)].

According to a further purpose, the invention comprises the above conjugates binding with other feasible drug carriers as therapeutic drugs for cancers and autoimmune diseases. The methods for treating cancers and autoimmune diseases include *in vitro, in vivo* or *ex vivo* therapy. An example of *in vitro* therapy includes *in vitro* treatment of the culture cells by a drug, killing all cells other than cells that express a target antigen, or killing cells that express undesired antigens. One example *of ex vivo* therapy is to treat hematopoietic stem cells *in vitro,* kill diseased or malignant cells and transfer back into the patient. For example, the clinical practise to remove tumour cells or lymphoid cells *ex vivo* from bone marrow to treat cancers or autoimmune diseases, or to remove T cells and other lymphoid cells *ex vivo* from allogeneic bone marrow or tissue prior to transplant in order to prevent graft-versus-host reactions, can be carried out as follows: bone marrow is harvested from the patient or other individual and then incubated in medium containing serum to which is added the conjugate of the invention, concentrations range from about 1 pM to 0.1 mM, for about 15 minutes to about 48 hours at about 37 °C. The exact conditions of concentration and time of incubation (=dose) are readily determined by the skilled clinicians. After incubation the bone marrow cells are washed with medium containing serum and returned to the patient by i.v. infusion according to known methods. In circumstances where the patient receives other treatment such as a course of ablative chemotherapy or systemic radiation therapy between the time of harvest of the marrow and reinfusion of the treated cells, the treated marrow cells are stored frozen in liquid nitrogen using standard medical eq.uipment.

For clinical *in vivo* use, the conjugate of the invention will be supplied as solutions or as a lyophilized solid that can be redissolved in sterile water for injection. Example s of suitable protocols of conjugate administration are as follows: conjugates are given weekly for 4~12 weeks as an i.v. bolus. Bolus doses are given in 50 to 500 ml of normal saline to which human serum albumin (e.g. 0.5 to 1 mL of a concentrated solution of human serum albumin, 100 mg/mL) can optionally be added. Dosages will be about 50 µg to 20 mg/kg of body weight per week, i.v. (range of 10 µg to 200 mg/kg per injection) 4~12 weeks after treatment, the patient may receive a second course of treatment. Specific clinical protocols with regard to route of administration, excipients, diluents, dosages, times, etc., can be determined by the skilled clinicians.

Example s of medical conditions that can be treated according to the *in vivo* or *ex vivo* methods of killing selected cell populations include malignancy of any types of cancer, autoimmune diseases, graft rejections, and infections (viral, bacterial or parasite)

The amount of a conjugate which is req.uired to achieve the desired biological effect, will vary depending upon a number of factors, including the chemical characteristics, the potency, and the bioavailability of the conjugates, the type of disease, the species to which the patient belongs, the diseased state of the patient, the route of administration, all factors which dictate the req.uired dose amounts, delivery and regimen to be administered.

In general terms, the conjugates of this invention may be provided in an aqueous physiological buffer solution containing 0.1 to 10% w/v conjugates for parenteral administration. Typical dose ranges are from 1 µg/kg to 0.1 g/kg of body weight daily, a preferred dose range is from 0.01 mg/kg to 20 mg/kg of body weight daily, an eq.uivalent dose in a human. The preferred dosage of drug to be administered is likely to depend on such variables as the type and extent of progression of the disease or disorder, the overall health status of the particular patient, the relative biological efficacy of the compound selected, the formulation of the compound, the route of administration (intravenous, intramuscular, or other), the pharmacokinetic properties of the conjugates by the chosen delivery route, and the speed (bolus or continuous infusion) and schedule of administrations (number of repetitions in a given period of time)

The conjugates of the present invention are capable of being administered in unit dose forms, wherein the term "unit dose" means a single dose which is capable of being administered to a patient, and which can be readily handled and packaged, remaining as a physically and chemically stable unit dose comprising either the active conjugate itself, or as a pharmaceutically acceptable composition, as described hereinafter. As such, typical total daily dose ranges are from 0.01 to 100 mg/kg of body weight. By way of general guidance, unit doses for humans range from 1 mg to 3000 mg per day. Preferably, the unit dose range is from 1 to 500 mg administered one to four times a month and even more preferably from 10 mg to 500 mg daily. Conjugates provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasal, particularly in the form of powders, nasal drops, or aerosols; or dermally, for example, topically in ointments, creams, lotions, gels or sprays, or via transdermal patches. The compositions may conveniently be administered in unit dosage form and may be prepared by any of the methods well known in the pharmaceutical art, e.g., as listed in Remington: The Science and Practice of Pharmacy, 21th ed.; Lippincott Williams & Wilkins: Philadelphia, PA.

Preferred formulations include pharmaceutical compositions in which a compound of the present invention is formulated for oral or parenteral administration. For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferred dosage forms. Preferred tablets contain lactose, cornstarch, magnesium silicate, croscarmellose sodium, povidone, magnesium stearate, talcum, or any combination thereof.

Liquid preparations for parenteral administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on RingeR's dextrose, and the like. Other potentially useful parenteral delivery systems for these active compounds include ethylene-vinyl acetate copolymer particles, osmotic pumps, implantable infusion systems, and liposomes.

Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, such as cocoa butter, and may include a salicylate. Formulations for topical invention to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

In a specific embodiment, the conjugates of this invention are administered concurrently with the other known or will be known therapeutic agents such as the chemotherapeutic agent, the radiation therapy, immunotherapy agents, autoimmune disorder agents, anti-infectious agents or the other antibody-drug conjugates, resulting in a *synergistic effect* for effective treatment or prevention of a cancer, or an autoimmune disease, or an infectious disease. In another specific embodiment, the *synergistic* drugs or radiation therapy are administered prior or subseq.uent to administration of a conjugate, in one respect at least an hour, 12 hours, a day, a week, a month, in further respects several months.

The synergistic agents are preferably selected from one or several of the following drugs:
1) Chemotherapeutic agents: a) alkylating agents: such as [nitrogen mustards (phenylbutyric acid nitrogen mustard, cyclophosphamide, ifosfamide, mechlorethamine, melphalan, ethyl cyclophosphamide); nitrosoureas (carmustine, lomustine); alkylsulphonates (busulfan, treosulfan); triazenes (dacarbazine); platinum containing compounds (carboplatin, cisplatin, oxaliplatin); b) plant alkaloids, such as vinca alkaloids (vincristine, vinblastine, vindesine, vinorelbine); taxane compounds (paclitaxel, taxotere); c) DNA topoisomerase inhibitors, such as [epipodophyllins (9-aminocamptothecin, camptothecin, etoposide, etoposide, etoposide phosphate, irinotecan, teniposide, topotecan); mitomycins (mitomycin c)]; d) anti-metabolites, such as [anti-folate: DHFR inhibitors (methotrexate, trimethoate); IMP dehydrogenase inhibitors (mycophenolic acid, carboxamidothiazole, ribavirin, eicar); ribonucleotide reductase inhibitors (hydroxyurea, deferoxamine)]; [pyrimidine analogs: uracil analogs (5-fluorouracil, doxifluridine, floxuridine, ratitrexed(tomudex)); cytosine analogs (cytarabine, fludarabine); purine analogs (azathioprine, mercaptopurine, thioguanine)]; e) hormonal therapies: such as receptor antagonists: [anti-estrogen (megestrol, raloxifene, tamoxifen); LHRH agonists (goscrclin, leuprolide acetate); anti-androgens (bicalutamide, flutamide)]; retinoids/deltoids: [vitamin D3 analogs (CB 1093, EB 1089 kh 1060, vitamin D2); photodynamic therapies (verteporfin, phthalocyanine, photosensitizer PC4, demethoxy-hypocrellin A); cytokines (interferon-alpha, interferon-gamma, tumor necrosis factor (TNFS), human proteins containing a TNF domain)]; f) kinase inhibitors, such as bibw 2992 (anti-egfr/erb2), imatinib, gefitinib, pegaptanib, sorafenib, dasatinib, sunitinib, erlotinib, nilotinib, lapatinib, axitinib, pazopanib. vandetanib, e7080 (anti-VEGFR2), moritinib, meditinib, pranatinib, ponatinib (ap24534), HQP1351, bafitinib (INNO-406), bosutinib (SKI-606), sunitinib, cabotinib, volitinib, vermodec, iniparib, ruxolitinib, CYT387, axitinib, tivozanib, sorafenib, bevacizumab, cetuximab, trastuzumab, ranibizumab, panitumumab, ispinesib; g) others, such as gemcitabine, epoxomicins (e. g. carfilzomib), bortezomib, thalidomide, lenalidomide, pomalidomide, tosedostat, zybrestat, PLX4032, STA-9090, stimuvax, allovectin-7, xegeva, provenge, yervoy, isoprenylation inhibitors (such as lovastatin), dopaminergic neurotoxins (such as 1-methyl-4-phenylpyridinium ion), cell cycle inhibitors (such as staurosporine), actinomycins (such as actinomycin D, dactinomycin), bleomycins (such as bleomycin A2, bleomycin B2, peplomycin), anthracyclines (such as daunorubicin, doxorubicin (adriamycin), idarubicin, epirubicin, pirarubicin, zorubicin, mtoxantrone, mdr inhibitors (such as verapamil), Ca²⁺ ATPase inhibitors (such as thapsigargin), histone deacetylase inhibitors (vorinostat, romidepsin, panobinostat, valproic acid, mocetinostat (MGCD0103), belinostat, PCI-24781, entinostat, SB939, resminostat, givinostat, AR-42, sulforaphane, trichostatin A, thapsigargin, celecoxib, glitazones, epigallocatechin gallate, disulfiram, salinosporamide A.
2) An anti-autoimmune disease agent includes, but is not limited to, cyclosporine, cyclosporine A, aminocaproic acid, azathioprine, bromocriptine, chlorambucil, chloroquine, cyclophosphamide, glucocorticoid (e.g. hormone drugs, betamethasone, budesonide, hydrocortisone, flunisolide, fluticasone propionate, fluocortolone danazol, dexamethasone, Triamcinolone acetonide, beclometasone propionate), dehydroepiandrosterone, enanercept, hydroxychloroquine, infliximab, meloxicam, methotrexate, mofetil, mycophenylate, sirolimus, tacrolimus, prednisone.
3) An anti-infectious disease agent includes, but is not limited to, a) aminoglycosides: amikacin, astromicin, gentamicin (netilmicin, sisomicin, isepamicin), hygromycin B, kanamycin (amikacin, arbekacin, bekanamycin, dibekacin, tobramycin), neomycin (framycetin, paromomycin, ribostamycin), netilmicin, spectinomycin, streptomycin, tobramycin, verdamicin; b) amphenicols: azidamfenicol, chloramphenicol, florfenicol, thiamphenicol; c) ansamycins: geldanamycin, herbimycin; d) carbapenems: biapenem, doripenem, ertapenem, imipenem/cilastatin, meropenem, panipenem; e) cephems: carbacephem (loracarbef), cefacetrile, cefaclor, cefradine, cefadroxil, cefalonium, cefaloridine, cefalotin or cefalothin, cefalexin, cefaloglycin, cefamandole, cefapirin, cefatrizine, cefazaflur, cefazedone, cefazolin, cefbuperazone, cefcapene, cefdaloxime, cefepime, cefminox, cefoxitin, cefprozil, cefroxadine, ceftezole, cefuroxime, cefixime, cefdinir, cefditoren, cefepime, cefetamet, cefmenoxime, cefodizime, cefonicid, cefoperazone, ceforanide, cefotaxime, cefotiam, cefozopran, cephalexin, cefpimizole, cefpiramide, cefpirome, cefpodoxime, cefprozil, cefquinome, cefsulodin, ceftazidime, cefteram, ceftibuten, ceftiolene, ceftizoxime, ceftobiprole, ceftriaxone, cefuroxime, cefuzonam, cephamycin (cefoxitin, cefotetan, cefmetazole), oxacephem (flomoxef, latamoxef); f) glycopeptides: bleomycin, vancomycin (oritavancin, telavancin), teicoplanin (dalbavancin), ramoplanin; g) glycylcyclines: e. g. tigecycline; daptomycin; g) β-lactamase inhibitors: penam (sulbactam, tazobactam), clavam (clavulanic acid); i) lincosamides: clindamycin, lincomycin; j) lipopeptides: daptomycin, A54145, calcium-dependent antibiotics (CDA); k) macrolides: azithromycin, cethromycin, quinerythromycin, clarithromycin, dirithromycin, erythromycin, flurithromycin, josamycin, ketolide (telithromycin, cethromycin, quinerythromycin), midecamycin, miocamycin, oleandomycin, rifamycins (rifampicin, rifampin, rifabutin, rifapentine), rokitamycin, roxithromycin, spectinomycin, spiramycin, tacrolimus (FK506), troleandomycin, telithromycin; l) monobactams: aztreonam, tigemonam; m) oxazolidinones: linezolid; n) penicillins: amoxicillin, ampicillin (pivampicillin, hetacillin, bacampicillin, metampicillin, talampicillin), azidocillin, azlocillin, benzylpenicillin, benzathine benzylpenicillin, benzathine phenoxymethylpenicillin, clometocillin, procaine benzylpenicillin, carbenicillin (carindacillin), cloxacillin, dicloxacillin, epicillin, flucloxacillin, mecillinam (pivmecillinam), mezlocillin, meticillin, nafcillin, oxacillin, penamecillin, penicillin, pheneticillin, phenoxymethylpenicillin, piperacillin, propicillin, sulbenicillin, temocillin, ticarcillin; o) polypeptides: bacitracin, colistin, polymyxin B; p) quinolones: alatrofloxacin, balofloxacin, ciprofloxacin, clinafloxacin, danofloxacin, difloxacin, enoxacin, enrofloxacin, floxin, garenoxacin, gatifloxacin, gemifloxacin, grepafloxacin, trovafloxacin, levofloxacin, lomefloxacin, marbofloxacin, moxifloxacin, nadifloxacin, norfloxacin, orbifloxacin, ofloxacin, pefloxacin, trovafloxacin, grepafloxacin, sitafloxacin, sparfloxacin, temafloxacin, tosufloxacin, trovafloxacin; q) streptogramins: pristinamycin, quinupristin/dalfopristin); r) sulfonamides: mafenide, prontosil, sulfacetamide, sulfamethizole, sulfanilimide, sulfasalazine, sulfisoxazole, trimethoprim, trimethoprim-sulfamethoxazole (Bactrim); s) steroid antibacterials, e.g. fusidic acid; t) tetracyclines: doxycycline, chlortetracycline, clomocycline, demeclocycline, lymecycline, meclocycline, metacycline, minocycline, oxytetracycline, penimepicycline, rolitetracycline, tetracycline, glycylcyclines (e.g. tigecycline); u) other types of antibiotics: annonacin, arsphenamine, bactoprenol inhibitors (bacitracin), DADAL/AR inhibitors (cycloserine), dictyostatin, discodermolide, eleutherobin, epothilone, ethambutol, etoposide, faropenem, fusidic acid, furazolidone, isoniazid, laulimalide, metronidazole, mupirocin, mycolactone, NAM synthesis inhibitors (e. g. fosfomycin), nitrofurantoin, paclitaxel, platensimycin, pyrazinamide, quinupristin/dalfopristin, rifampicin (rifampin), tazobactam tinidazole, uvaricin;
4) Anti-viral drugs: a) entry/fusion inhibitors: aplaviroc, maraviroc, vicriviroc, gp41 (enfuvirtide), PRO 140, CD4 (ibalizumab); b) integrase inhibitors: raltegravir, elvitegravir, globoidnan A; c) maturation inhibitors: bevirimat, vivecon; d) neuraminidase inhibitors: oseltamivir, zanamivir, peramivir; e) nucleosides and nucleotides: abacavir, aciclovir, adefovir, amdoxovir, apricitabine, brivudine, cidofovir, clevudine, dexelvucitabine, didanosine (ddI), elvucitabine, emtricitabine (FTC), entecavir, famciclovir, fluorouracil (5-FU), 3'-fluoro-substituted 2', 3'-dideoxynucleoside analogues (e.g. 3'-fluoro-2',3'-dideoxythymidine (FLT) and 3'-fluoro-2',3'-dideoxyguanosine (FLG), fomivirsen, ganciclovir, idoxuridine, lamivudine (3TC), 1-nucleosides (e.g. *β*-1-thymidine and *β*-1-2'-deoxycytidine), penciclovir, racivir, ribavirin, stampidine, stavudine (d4T), taribavirin (viramidine), telbivudine, tenofovir, trifluridine valaciclovir, valganciclovir, zalcitabine (ddC), zidovudine (AZT); f) non-nucleosides: amantadine, ateviridine, capravirine, diarylpyrimidines (etravirine, rilpivirine), delavirdine, docosanol, emivirine, efavirenz, foscarnet (phosphonoformic acid), imiquimod, interferon alfa, loviride, lodenosine, methisazone, nevirapine, NOV-205, peginterferon alfa, podophyllotoxin, rifampicin, rimantadine, resiquimod (R-848), tromantadine; g) protease inhibitors: amprenavir, atazanavir, boceprevir, darunavir, fosamprenavir, indinavir, lopinavir, nelfinavir, pleconaril, ritonavir, saquinavir, telaprevir (VX-950), tipranavir; h) other types of anti-virus drugs: abzyme, arbidol, calanolide A, ceragenin, cyanovirin-N, diarylpyrimidines, epigallocatechin gallate (EGCG), foscarnet, griffithsin, taribavirin (viramidine), hydroxyurea, KP-1461, miltefosine, pleconaril, portmanteau inhibitors, ribavirin, seliciclib.
5) Other immunotherapy drugs: imiquimod, interferon (e.g.,α,β), granulocyte colony-stimulating factors, interleukin (IL-1, IL-35), antibodies (e.g., trastuzumab, pertuzumab, bevacximab, altuximab, paximab, dacryximab, olarbazin), protein-binding drugs (e.g., Abraxane), an antibody-binding drug selected from calicheamicin derivatives, maytansinoids derivatives (DM 1 and DM 4), CC-1065 and duocarmycin, effective paclitaxel derivatives, doxorubicin and auristatin anti-mitotic drug (such as trastuzumab-DM 1, inotuzumab, brentuximab vedotin, glembatumumab vedotin, lorvotuzumab mertansine, AN-152 LMB 2, TP-38, VB 4-845, cantuzumab mertansine, AVE 9633, SAR 3419, CAT-8015 (anti-CD22), IMGN 388, IMGN 529, IMGN 853, milauzumab-doxorubicin, SGN-75 (anti-CD70), anti-CD22-MCC-DM 1).

For a further purpose, the present invention also relates to the preparation of ADCs. The compounds of the present invention can be prepared in a number of ways well known to those skilled in the art. The antimitotic compounds can be synthesized, for example, by the methods described in the examples, or variations thereof as appreciated by the skilled artisan. The appropriate modifications and substitutions will be readily apparent and well known or readily obtainable from the scientific literature to those skilled in the art. In particular, such methods can be found in Richard C. Larock, Comprehensive Organic Transformations, 2nd Edition, Wiley Publishers, 1999.

In the synthetic reactions of the present invention, it may be necessary to protect reactive functional groups, for example hydroxy, amino, imino, thio or carboxy groups, to avoid their unwanted participation in the reactions. Conventional protecting groups may be used in accordance with standard practice, for example, as in P. G. Wuts and T.W. Greene, Greene's Protective Groups in Organic Synthesis, 4th Edition, Wiley-Interscience, 2006.

Some reactions can be carried out in a suitable acidic or base solutions. The acid, base, and solvent of such reactions are not particularly limited as long as there is no side effect, and any conventional acid, base, and solvent can be used herein. Moreover, these reactions can be performed within a wide range of temperatures. However, the reaction temperature that is relatively easy to operate is typically between -80°C and 150°C (preferably between room temperature and 100°C). The time req.uired for the reaction may also have a large range of variations, of course depending on a variety of factors, in particular the reaction temperature and the nature of the solvent used. Generally, for a relatively ideal reaction, the reaction time of 3 to 20 hours is preferred.

The operation after completion of the reaction can be performed according to a conventional method. For example, the reaction product may be recovered by steaming the solvent out of the reaction system. Alternatively, if necessary, after the solvent is evaporated, the residue can be poured into the water, and then extracted with an organic solvent which is immiscible with the water. Finally, after the extraction solvent is evaporated, to yield the reaction product. In addition, if there is a need for higher purity, various common methods can be used for further purification, such as recrystallization, sedimentation, or various chromatographic methods. Generally, the column chromatography and prep-TLC are more common.

### EXAMPLES

The invention is further described in the following examples, which are not intended to limit the scope of the invention. Cell lines described in the following examples were maintained in culture according to the conditions specified by the American Type Culture Collection (ATCC) or Deutsche Sammlung von Mikroorganismen und Zellkulturen GmbH, Braunschweig, Germany (DMSZ), or The Shanghai Cell Culture Institute of Chinese Acadmy of Science, unless otherwise specified. Cell culture reagents were obtained from Invitrogen Corp., unless otherwise specified. All anhydrous solvents were commercially obtained and stored in Sure-Seal bottles under nitrogen. All other reagents and solvents were purchased as the highest grade available and used without further purification. The preparative HPLC separations were performed with Varain PreStar HPLC. NMR spectra were recorded on Bruker 500 MHz Instrument. Chemical shifts are reported in parts per million (ppm) referenced to tetramethylsilane at 0 ppm and coupling constants (J) are reported in Hz. The mass spectral data were acquired on a Waters Xevo Q Tof mass spectrum eq.uipped with Waters Acquity UPLC separations module and Acquity TUV detector.

### Example 1 Synthesis of compound 1

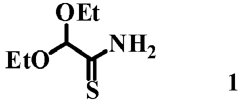

In a 10-L reactor, 2,2-diethoxyacetonitrile (1.00 kg, 7.74 mol, 1.0 eq.) in methanol (6.0 L) was mixed with (NH₄)₂S (48% aqueous solution, 1.41 kg, 9.29 mol, 1.2 eq.) at room temperature. The internal temperature increased to 33 °C and then dropped back to room temperature After stirring overnight, the reaction mixture was concentrated under vacuum and the residue was taken up in ethyl acetate (5 L) and washed with saturated NaHCO₃ solution (4 x 1.0 L). The aqueous layer was back-extracted with ethyl acetate (5 x 1.0 L). The organic phases were combined and washed with brine (3 L), dried over anhydrous Na₂SO₄ and concentrated. The resulting solid was collected by vacuum filtration and washed with petroleum ether. The filtrate was concentrated and triturated with petroleum ether to yield a few crops of white or light yellow solid. All crops were combined to give 1.1 kg of desired product (87% yield). ¹H NMR (500 MHz, CDCl₃) δ 7.81 (d, *J* = 71.1 Hz, 2H), 5.03 (s, 1H), 3.73 (dq, *J* = 9.4, 7.1 Hz, 2H), 3.64 (dq, *J* = 9.4, 7.0 Hz, 2H), 1.25 (t, *J* = 7.1 Hz, 6H).

### Example 2 Synthesis of compound 2

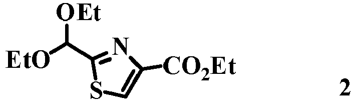

In a 5-L 3-neck round bottle flask, eq.uipped with a reflux condenser and an additional funnel, ethyl bromopyruvate (80% purity, 404 mL, 2.57 mol) was added over 30 min. to a mixture of molecular sieves (3A, 500 g) and thioamide (350 g, 2.14 mol, 1.0 eq.) in 3 L of EtOH. During addition, the internal temperature increased slightly. The reaction mixture was then heated to reflux and stirred for 30 min. After cooling to room temperature the reaction mixture was filtered over Celite and the filter cake washed with ethyl acetate. The filtrate was concentrated under vacuum. Two batches of the crude product were combined and mixed with silica gel (1.5 kg) and loaded on a silica gel (10 kg packed) column and eluted with ethyl acetate/petroleum ether (10-20% ) to give thiazole carboxylate as a brown oil (509 g, 92% yield).

### Example 3 Synthesis of compound 3

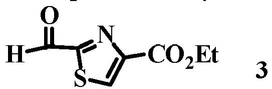

A solution of acetal (300 g, 1.16 mol) in acetone (3.0 L) was heated to reflux and 4 N HCl (250 mL) was added over 1.0 h to the refluxing solution. TLC analysis indicated complete consumption of the starting material. The reaction mixture was concentrated under reduced pressure and phases were separated. The organic phase was diluted with ethyl acetate (1.5 L) and washed with saturated NaHCO₃ solution (1.0 L), water (1.0 L) and brine (1.0 L), and then dried over anhydrous Na₂SO₄. All of the aqueous phases were combined and extracted with ethyl acetate. The extracts were combined and dried over anhydrous Na₂SO₄. The organic solutions were filtered and concentrated under reduced pressure. The crude product was triturated with petreolum ether and diethyl ether (5:1) and the resulting solid was collected by vacuum filtration and washed with petreolum ether and ethyl acetate (10:1). The filtrate was concentrated and chromatographed using 0-15% ethyl acetate/petreolum ether to give another crop of desired product. All white to light yellow solids were combined and weighed 40 g (43% yield). ¹H NMR (500 MHz, CDCl₃) δ 10.08 - 10.06 (m, 1H), 8.53 - 8.50 (m, 1H), 4.49 (q, *J* = 7.1 Hz, 2H), 1.44 (t, *J* = 7.1 Hz, 3H). MS ESI m/z calcd for C₇H₈NO₃S [M+H]⁺ 186.01; found 186.01.

### Example 4 Synthesis of compound 4

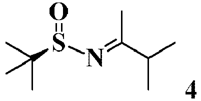

To a solution of (S)-2-methylpropane-2-sulfinamide (100 g, 0.825 mol) in 1L of THF were added Ti(OEt)₄ (345 mL, 1.82 mol) and 3-methyl-2-butanone (81 mL, 0.825 mol) under N₂ at room temperature The reaction mixture was refluxed for 16 h, then cooled to room temperature and poured onto ice-water (1L). The mixture was filtered and the filter cake was washed with ethyl acetate. The organic layer was separated, dried over anhydrous Na₂SO₄ and concentrated to give a residue which was purified by vacuum distillation (15-20 torr, 95 °C) to afforded product **4** (141 g, 90% yield) as a yellow oil. ¹H NMR (500 MHz, CDCl₃) δ 2.54 - 2.44 (m, 1H), 2.25 (s, 3H), 1.17 (s, 9H), 1.06 (dd, *J* = 6.9, 5.1 Hz, 6H). MS ESI m/z calcd for C₉H₁₉NaNOS [M+Na]⁺ 212.12; found 212.11.

### Example 5 Synthesis of compound 5

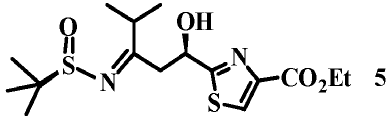

At -78 °C, to a solution of diisopropylamine (264 mL, 1.87 mol) in dry THF (1L) was added *n*-butyllithium (2.5 M, 681 mL, 1.70 mol) under N₂. The reaction mixture was warmed to 0 °C over 30 min and then cooled back to -78 °. Compound **10** (258 g, 1.36mol) was added, and then rinsed with THF (50 mL). The reaction mixture was stirred for 1 h before ClTi(O*ⁱ*Pr)₃ (834 g, 3.17 mol) in THF (1.05 L) was added dropwise. After stirring for 1 h, compound **4** (210 g, 1.13 mol) dissolved in THF (500 mL) was added dropwise in about 1 hours and the resulting reaction mixture was stirred for 3 h. The completion of the reaction was indicated by TLC analysis. The reaction was quenched by a mixture of acetic acid and THF (v/v 1:1, 300 mL), then poured onto brine (2 L), extracted with ethyl acetate (8 × 1L). The organic phase was washed with water and brine, dried over anhydrous Na₂SO₄, filtered and concentrated. The residue was purified by column chromatography (dichloromethane/ethyl acetate/petroleum ether 2:1:2) to afforded the compound **5** (298 g, 74% yield) as a colorless oil. ¹H NMR (500 MHz, CDCl₃) δ 8.13 (s, 1H), 6.63 (d, *J* = 8.2 Hz, 1H), 5.20 - 5.11 (m, 1H), 4.43 (q, *J* = 7.0 Hz, 2H), 3.42 - 3.28 (m, 2H), 2.89 (dt, *J* = 13.1, 6.5 Hz, 1H), 1.42 (t, *J* = 7.1 Hz, 3H), 1.33 (s, 9H), 1.25 - 1.22 (m, 6H). MS ESI m/z calcd for C₁₆H₂₆NaN₂O₄S₂ [M+Na]⁺ 397.13, found 397.11.

### Example 6 Synthesis of compound 6

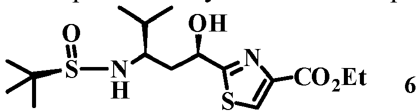

A solution of compound **5** (509 g, 1.35 mol) dissolved in THF (200 mL) was cooled to -78 °C. Ti(OEt)₄ (570 mL, 2.72 mol) was added slowly. After completion of the addition, the mixture was stirred for 1 h, before NaBH₄ (51.3 g, 1.36 mol) was added in portions over 90 min. The reaction mixture was stirred at -78 °C for 3 h. TLC analysis showed starting material still being remained. EtOH (50 mL) was added slowly, and the reaction was stirred for 1.5 h and then poured onto brine (2 L, with 250 mL HOAc) and warmed to room temperature After filtration over Celite, the organic phase was separated and washed with water and brine, dried over anhydrous Na₂SO₄, filtered, and concentrated. The residue was purified by column chromatography (ethyl acetate/petroleum ether 1:1) to deliver product **6** (364 g, 71% yield) as a white solid. ¹H NMR (500 MHz, CDCl₃) δ 8.10 (s, 1H), 5.51 (d, *J* = 5.8 Hz, 1H), 5.23 - 5.15 (m, 1H), 4.41 (q, *J* = 7.0 Hz, 2H), 3.48 - 3.40 (m, 1H), 3.37 (d, *J* = 8.3 Hz, 1H), 2.29 (t, *J* = 13.0 Hz, 1H), 1.95 - 1.87 (m, 1H), 1.73 - 1.67 (m, 1H), 1.40 (t, *J* = 7.1 Hz, 3H), 1.29 (s, 9H), 0.93 (d, *J* = 7.3 Hz, 3H), 0.90 (d, *J* = 7.2 Hz, 3H). MS ESI m/z calcd for C₁₆H₂₈NaN₂O₄S₂ [M+Na]⁺ 399.15, found 399.14.

### Example 7 Synthesis of compound 7

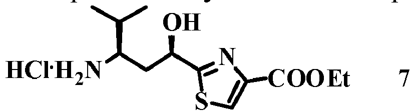

To a solution of compound **6** (600 g, 1.60 mol) in ethanol (590 mL) was added 4 N HCl in dioxane (590 mL) slowly at 0 °C. The reaction was allowed to warm to room temperature and stirred for 2.5 h. A white precipitate crushed out and was collected by filtration and washed with ethyl acetate. The filtrate was concentrated and triturated with ethyl acetate. Two crops of white solid were combined and weighed 446 g (90% yield)

### Example 8 Synthesis of compound 8

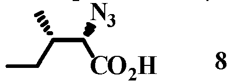

NaN₃ (740 g, 11.4 mol) was dissolved in water (2.0 L) and dichloromethane (2.0 L) was added and cooled at 0 °C, to which Tf₂O(700 mL, 4.10 mol) was added over 1.5 h. After addition was completed, the reaction was stirred at 0 °C for 3 h. The organic phase was separated and the aqueous phase was extracted with dichloromethane (2 × 500 mL) The combined organic phase was washed with saturated NaHCO₃ solution (3 × 1.0 L) This dichloromethane solution of triflyl azide was added to a mixture of (L)-isoleucine (300 g, 2.28 mol), K₂CO₃ (472 g, 3.42 mol), CuSO₄·5H₂O (5.7 g, 22.8 mmol) in water (3.0 L) and methanol (3.0 L) at room temperature During addition, the internal temperature increased slightly. And the mixture was then stirred at room temperature for 16 h. The organic solvents were removed under reduced pressure and the aqueous phase was acidified to pH 6-6.5 with concentrated HCl (about 280 mL added) and then diluted with phosphate buffer (0.25 M, pH 6.2, 6.0 L), washed with ethyl acetate (6 × 2.0 L) to remove the sulfonamide by-product. The solution was acidified to pH 3 with concentrated HCl (about 400 mL added), extracted with ethyl acetate (4 × 2.0 L) The combined organic layers were washed with brine (2.0 L) and dried over anhydrous Na₂SO₄, filtered and concentrated to give product **8** (320 g, 89% yield) as a light yellow oil. ¹H NMR (500 MHz, CDCl₃) δ 12.01 (s, 1H), 3.82 (d, *J* = 5.9 Hz, 1H), 2.00 (ddd, *J* = 10.6, 8.6, 5.5 Hz, 1H), 1.54 (dqd, *J* = 14.8, 7.5, 4.4 Hz, 1H), 1.36 - 1.24 (m, 1H), 1.08 - 0.99 (m, 3H), 0.97 - 0.87 (m, 3H)

### Example 9 Synthesis of compound 9

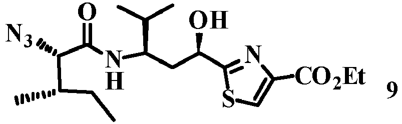

Azido-Ile-OH (**8**, 153g, 0.97 mol, 2.0 eq.) was dissolved in THF (1.5 L) and cooled to 0 °C, to which NMM (214 mL, 1.94 mol) and isobutylchloroformate (95 mL, 0.73 mol) were added in sequence. The reaction was stirred at 0 °C for 1.0 h. Compound 7 (150 g, 0.49 mmol) was added in portions. After stirring at 0 °C for 30 min, the reaction was warmed to room temperature and stirred for 2 h. Water was added at 0 °C to quench the reaction and the resulting mixture was extracted with ethyl acetate for three times. The combined organic layers were washed with IN HCl, saturated NaHCO₃ and brine, dried over anhydrous Na₂SO₄, filtered and concentrated. The residue was purified by column chromatography (0-30% ethyl acetate/petroleum ether) to give a white solid (140 g, 70% yield) ¹H NMR (500 MHz, CDCl₃) δ 8.14 (s, 1H), 6.57 (d, *J* = 8.9 Hz, 1H), 4.91 (d, *J =* 11.1 Hz, 1H), 4.44 (dd, *J* = 13.2, 6.3 Hz, 2H), 4.08 - 3.95 (m, 2H), 2.21 (dd, *J* = 24.4, 11.5 Hz, 2H), 1.90 - 1.79 (m, 3H), 1.42 (t, *J* = 6.6 Hz, 3H), 1.37 - 1.27 (m, 2H), 1.11 (d, *J* = 6.4 Hz, 3H), 1.01 - 0.94 (m, 9H) MS ESI m/z calcd for C₁₈H₃₀N₅O₄S [M+H]⁺ 412.19, found 412.19.

### Example 10 Synthesis of compound 10

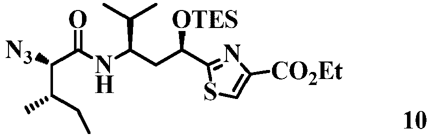

To a solution of compound **9** (436 g, 1.05 mol, 1.0 eq.) in CH₂Cl₂ (50 mL) was added imidazole (94 g, 1.37 mmol, 1.3 eq.), followed by chlorotriethylsilane (222 mL, 1.32 mol) at 0 °C. The reaction mixture was allowed to warm to room temperature over 1 hour and stirred for an additional hour. Brine was added to the reaction mixture, the organic layer was separated and the aqueous layer was extracted with ethyl acetate. The combined organic phase was dried, filtered, concentrated under reduced pressure, and purified by column chromatography with a gradient of (15-35% ethyl acetate/petroleum ether) to afford product **10** (557.4 g, 95% yield) as a colorless oil. ¹H NMR (500 MHz, CDCl₃) δ 8.12 (s, 1H), 6.75 (d, *J* = 8.0 Hz, 1H), 5.20 - 5.12 (m, 1H), 4.44 (q, *J* = 7.0 Hz, 2H), 4.06 - 3.97 (m, 1H), 3.87 (d, *J* = 3.8 Hz, 1H), 2.14 (d, *J* = 3.8 Hz, 1H), 2.01 - 1.91 (m, 3H), 1.42 (t, *J* = 7.1 Hz, 3H), 1.34 - 1.25 (m, 2H), 1.06 (d, *J* = 6.8 Hz, 3H), 1.00 - 0.93 (m, 18H), 0.88 (dd, *J* = 19.1, 6.8 Hz, 6H). MS ESI m/z calcd for C₂₄H₄₄N₅O₄SSi [M+H]⁺ 526.28, found 526.28.

### Example 11 Synthesis of compound 11

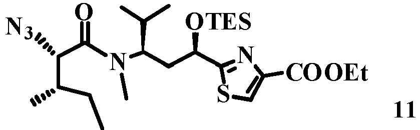

To a solution of **10** (408 g, 0.77 mol) and methyl iodide (145 mL, 2.32 mol) in THF (4 L) was added sodium hydride (60% dispersion in mineral oil, 62.2 g, 1.55 mol) at 0 °C . The resulting mixture was stirred at 0 °C overnight and then poured onto ice-water cooled saturated ammonium chloride (5 L) with vigorous stirring. The mixture was then extracted with ethyl acetate (3 × 500 mL) and the organic layers were dried, filtered, concentrated and purified by column chromatography with a gradient of (15-35% ethyl acetate/petroleum ether) to afford product **11** (388 g, 93% yield) as a light yellow oil. ¹H NMR (500 MHz, CDCl₃) δ 8.09 (s, 1H), 4.95 (d, *J* = 6.6 Hz, 1H),4.41 (q, *J* = 7.1 Hz, 2H), 3.56 (d, *J* = 9.5 Hz, 1H), 2.98 (s, 3H), 2.27 - 2.06 (m, 4H), 1.83 - 1.70 (m, 2H), 1.41 (t, *J* = 7.2 Hz, 3H), 1.29 (ddd, *J* = 8.9, 6.8, 1.6 Hz, 3H), 1.01 (d, *J* = 6.6 Hz, 3H), 0.96 (dt, *J* = 8.0, 2.9 Hz, 15H), 0.92 (d, *J* = 6.6 Hz, 3H), 0.90 (d, *J* = 6.7 Hz,3H). MS ESI m/z calcd for C₂₅H₄₆N₅O₄SSi [M+H]⁺ 540.30, found 540.30.

### Example 12 Synthesis of compound 12

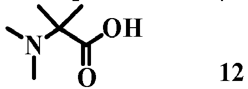

A mixture of 2-amino-2-methylpropanoic acid (500 g, 4.85 mol), aqueous formaldehyde (37%, 1.0 L, 12.1 mol) and formic acid (1.0 L) was heated to reflux (80 °C) for 3.0 h. 6 N HCl (850 mL) was then added at room temperature and the reaction mixture was concentrated. The resulting solid was collected by filtration with washing of ethyl acetate for three times (1.0 L). The solid was dissolved in water (1.5 L) and neutralized to pH 7.0 with 4 N NaOH (about 1.0 L solution). The solution was concentrated and co-evaporated with ethanol (2.0 L) to remove residual water. MeOH (2.0 L) was added to the residue and the solid (NaCl) was filtered off with washing of ethyl acetate. The filtrate was concentrated under reduced pressure to give a white solid 639.2 g, which contains some NaCl and was used without further treatment.

### Example 13 Synthesis of compound 13

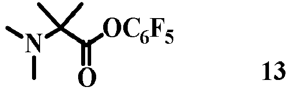

To a solution of **12** (97 g, 0.74 mol) in ethyl acetate (1L) were added pentafluorophenol (163 g, 0.88 mol) and DIC (126 mL, 0.81 mol). The reaction mixture was stirred at room temperature for 24 h, and then filtered over Celite. The filter pad was washed with 10 mL of ethyl acetate. The filtrate was used immediately without further purification or concentration.

### Example 14 Synthesis of compound 14

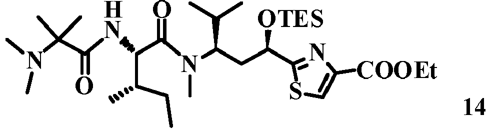

To the ethyl acetate solution of pentafluorophenyl ester **13,** compound **11** (200 g, 0.37 mol) and dry Pd/C (10 wt%, 10 g) were added. The reaction mixture was stirred under hydrogen atmosphere (1 atm) for 27 h, and then filtered through a plug of Celite, with washing of the filter pad with ethyl acetate. The combined organic portions were concentrated and purified by column chromatography with a gradient of 0-5% methanol in ethyl acetate to deliver compound **14** (184 g, 79% yield). MS ESI m/z calcd for C₃₁H₅₈N₄O₅SSi [M+H]⁺ 627.39, found 627.39.

### Example 15 Synthesis of compound 15

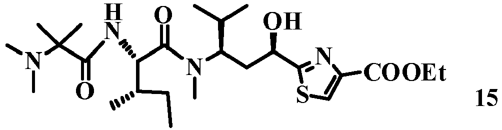

Compound **14** (200 g, 0.32 mmol) was dissolved in AcOH/water/THF (v/v/v 3:1:1, 638 mL), and stirred at room temperature for 4 days. After the reaction was concentrated, toluene was added and concentrated again; this step was repeated two times to afford compound **15,** which was used directly in the next step. MS ESI m/z calcd for C₂₅H₄₅N₄O₅S [M+H]⁺ 513.30, found 513.30.

### Example 16 Synthesis of compound 16

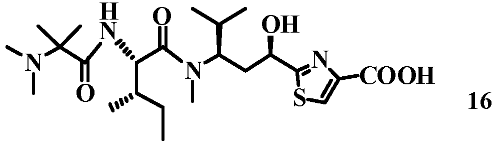

An aqueous solution of LiOH (0.4 N, 600 mL, 2.55 mol) was added to a solution of compound **15** (160 g, 0.319 mol, 1.0 eq.) in MeOH (1.2 L) at 0 °C. The reaction mixture was stirred at room temperature for 2 h and then concentrated. Column chromatography (pure CH₂Cl₂ to 80:20:1 CH₂Cl₂/MeOH/NH₄OH) afforded compound **16** (140 g, 91% yield for two steps) as an amorphous solid. MS ESI m/z calcd for C₂₃H₄₀N₄O₅S [M+H]⁺ 485.27, found 485.27.

### Example 17 Synthesis of compound 17

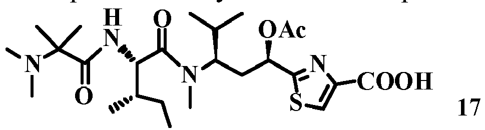

A solution of compound **16** (143 g, 0.30 mol) and DMAP (0.36 g, 2.95 mmol) in anhydrous THF (1.4 L) and anhydrous DMF (75 mL) was cooled to 0 °C, to which TEA (82.2 mL, 0.59 mmol) and acetic anhydride (56 mL, 0.59 mmol) were added. The reaction mixture was allowed to warm to room temperature and stirred for 24 h, and then concentrated. Column chromatography (5-50% MeOH/dichloromethane ) delivered compound **17** (147 g, 95% yield) as an amorphous solid. ¹H NMR (500 MHz, DMSO) δ 8.37 (s, 1H), 7.63 (d, *J* = 9.5 Hz, 1H), 5.54 (dd, *J* = 11.2, 2.5 Hz, 1H), 4.64 (dd, *J* = 9.4, 7.2 Hz, 1H), 4.34 (s, 1H), 2.95 (s, 3H), 2.27-2.19 (m, 1H), 2.19-2.12 (m, 1H), 2.11 (s, 6H), 2.08 (s, 3H), 1.82-1.66 (m, 2H), 1.54-1.42 (m, 1H), 1.10 (s, 3H), 1.06 - 0.95 (m, 1H), 0.99 (s, 3H), 0.91 (d, *J* = 6.5 Hz, 3H), 0.88 (d, *J* = 6.7 Hz, 3H), 0.83 (t, *J* = 7.4 Hz, 3H), 0.65 (d, *J* = 6.6 Hz, 3H). ¹³C NMR (126 MHz, DMSO) δ 175.35, 172.78, 169.70, 169.58, 162.23, 148.03, 128.29, 69.51, 63.00, 55.10, 52.37, 38.86, 36.46, 33.83, 29.25, 28.82, 23.64, 21.09, 20.60, 19.96, 19.40, 18.38, 15.65, 10.77. MS ESI m/z calcd for C₂₅H₄₄N₄O₆S [M+H]⁺ 527.28, found 527.28.

### Example 18 Synthesis of compound18

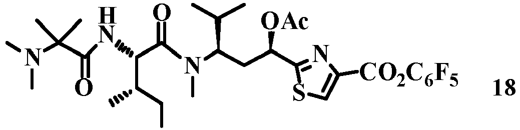

To a solution of compound **17** (41.0 g, 77.9 mmol, 1.0 eq.) in anhydrous dichloromethane (600 mL) was added EDC ·HCl (44.8 g, 233 mmol, 3.0 eq.) and pentafluorophenol (35.9 g, 194 mmol, 2.5 eq.) at room temperature under N₂. The mixture was stirred at room temperature for 2 h, and washed with brine (300 mL), dried over anhydrous Na₂SO₄,filtered, concentrated and purified by SiO₂ column chromatography (25-100% ethyl acetate/hexanes) to give a white solid (43.0 g, 80% yield). ¹H NMR (500 MHz, DMSO) δ 9.06 (s, 1H), 7.65 (d, *J* = 9.4 Hz, 1H), 5.60 (dd, *J* = 11.0, 2.8 Hz, 1H), 4.64 (dd, *J* = 9.4, 7.2 Hz, 1H), 4.35 (s, 1H), 2.97 (s, 3H), 2.34 - 2.16 (m, 2H), 2.12 (s, 6H), 2.11 (s, 3H), 1.88 - 1.65 (m, 2H), 1.57 - 1.37 (m, 1H), 1.11 (s, 3H), 1.06 - 0.96 (m, 1H), 1.00(s, 3H), 0.92 (d, *J* = 6.5 Hz, 3H), 0.88 (d, *J* = 6.7 Hz, 3H), 0.83 (t, *J* = 7.4 Hz, 3H), 0.66 (d, *J* = 6.6 Hz, 3H). ¹³C NMR (126 MHz, DMSO) δ 175.24, 172.78, 171.75, 169.81, 156.32, 141.69, 141.56, 139.71, 138.59, 136.60, 134.68, 69.49, 63.11, 55.16, 52.41, 38.83, 36.40, 33.64, 29.42, 28.82, 23.62, 21.01, 20.55, 19.93, 19.39, 18.35, 15.62, 10.73. MS ESI m/z calcd for C₃₁H₄₂F₅N₄O₆S [M+H]⁺: 693.3, found:693.3.

### Example 19 Synthesis of compound 19

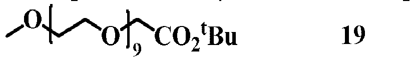

NaH (60%,8.0g,200mmol) was added to a solution of HO-PEG₉-OMe (42.8 g, 100 mmol) in THF (1.0 L). After stirring at room temperature for 30 min, *tert-butyl* 2-bromoacetate (48.8 g, 250 mmol) was added to the mixture, and stirred at room temperature for 1 h. The mixture was then poured onto ice water, extracted with dichloromethane, and the organic layer was washed with brine, dried over anhydrous Na₂SO₄. Purification by column chromatography (0% to 5% MeOH:dichloromethane) yielded compound **19** as a yellow oil (32 g,59% yield).

### Example 20 Synthesis of compound 20

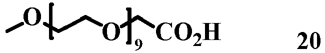

Compound **432** (40.0 g, 73.8 mmol) was dissolved in dichloromethane (400 mL), and then formic acid (600 mL) was added. The resulting solution was stirred at 25 °C overnight. All volatiles were removed under vacuum, which afforded the title product as yellow oil (36.0 g, theoretical yield). ESI m/z calcd for C₂₁H₄₃O₁₂ [M+H]⁺: 487.27, found 487.24.

### Example 21 Synthesis of compound 21

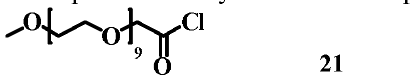

To the solution of compound **20** (36.0 g, 73.8 mmol) dissolved in dichloromethane (640 mL), (COCl)₂ (100 mL) and DMF (52 g, 0.74 mmol) were added. The resulting solution was stirred at room temperature for 4 h. All volatiles were removed under vacuum to yield the title product as a yellow oil.

### Example 22 Synthesis of compound 22

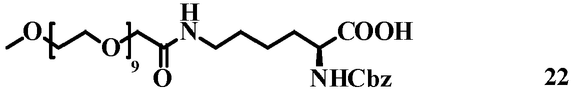

Z-L-Lys-OH (41.4 g, 147.6 mmol), Na₂CO₃ (23.4 g, 221.4 mmol) and NaOH (5.9 g, 147.6 mmol) were dissolved in water (720mL) The mixture was cooled to 0 °C, to which a solution of compound **21** (37.2 g, 73.8 mmol) in THF (20 mL) was added. The resulting mixture was stirred at room temperature for 1 h. THF was removed under vacuum, and concentrated HCl was added to the aqueous solution until pH reached 3 under ice cooling. After extraction with dichloromethane, the organic layer was washed with brine, dried over Na₂SO₄ and concentrated to give the title product as yellow oil (55 g, 99% yield). ESI m/z calcd for C₃₅H₆₀N₂O₁₅ [M+H]⁺: 749.40, found 749.39.

### Example 23 Synthesis of compound 23

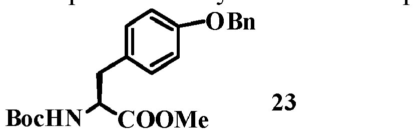

To a mixture of Boc-L-Tyr-OMe (2.2 kg, 7.45 mol), K₂CO₃ (1.54 kg, 11.2 mol) and KI (48 g, 0.29 mol) in acetone (8.8 L) was added benzyl bromide (1.33 kg, 7.78 mol) slowly. The mixture was then refluxed overnight. Water (8L) was added and the reaction mixture was extracted with ethyl acetate (2×4 L) The combined organic layers were washed with water (4 L) and brine (4L), dried over anhydrous Na₂SO₄, filtered, concentrated and triturated with petroleum ether to give a white solid **98** (2.73 kg, 95%yield). ¹H NMR (500 MHz, CDCl₃) δ 7.43 (d, *J* = 7.0 Hz, 2H), 7.38 (t, *J* = 7.4 Hz, 2H), 7.32 (t, *J* = 7.2 Hz, 1H), 7.04 (d, *J* = 8.5 Hz, 2H), 6.91 (d, *J* = 8.6 Hz, 2H), 5.04 (s, 2H), 4.55 (d, *J* = 6.9 Hz, 1H), 3.71 (s, 3H), 3.03 (qd, *J* = 14.0, 5.8 Hz, 2H), 1.43 (s, 9H). ESI: m/z: calcd for C₂₂H₂₈NO₅ [M+H]+: 386.19, found 386.19.

### Example 24 Synthesis of compound 24

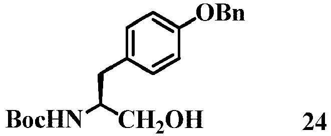

To a mixture of 2.4 L of ethanol and 2.4 L of dichloromethane was added NaBH₄ (122 g, 3.2 mol) and LiCl (136 g, 3.2 mol) was cooled to 0 °C, and then compound **23** (616 g, 1.6 mol) in dichloromethane (2.4 L) was added. After the completion of addition, 2.4 L of dichloromethane was added to the reaction. The reaction was allowed to warm to room temperature, with a lot of bubbles being generated, and then stirred overnight. The reaction mixture was diluted with water (6 L), stirred for 30 minutes, the aqueous layer was extracted with dichloromethane (2 L × 2), the combined organic phase was washed with water (2 L) and brine (2 L), dried, filtered, concentrated to afford a white solid 542 g (95% yield).

### Example 25 Synthesis of compound 25

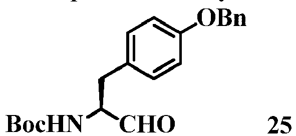

(COCl)₂ (1.02 kg, 8.0 mol) was dissolved in CH₂Cl₂ (4 L), and cooled to -75 °C, DMSO (1.25 kg, 16 mol) in dichloromethane (400 mL) was added dropwise while keeping the temperature under -65 °C. The mixture was stirred for 30 minutes after the addition is completed, and then compound **24** (1.90 kg, 5.33 mol) in dichloromethane (8 L) was added dropwise. After the completion of addition, the temperature of the solution increased to about -65°C. After stirring for 30 minutes, triethylamine (1.62 kg, 16 mol) was added dropwise while maintaining the temperature below -50 °C. After stirring for 15 minutes, the reaction was allowed to warm to about -30 °C in about 1 h, and TLC monitoring showed that the reaction was complete. Water (6 L) was added to the reaction mixture, stirred and layers were separated. The aqueous layer was washed with dichloromethane (2 L). The combined organic layers was washed with 10% HCl (4 L) and brine (2 L), dried, filtered and concentrated. The concentrated solution was triturated with 5:1 petroleum ether/ethyl acetate, and filtered under vacuum to yield 1.36 kg (72% yield) of a light yellow solid.

### Example 26 Synthesis of compound 26

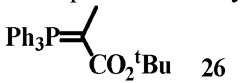

A solution of *tert*-butyl 2-bromopropionate (255 g, 1.22 mol) and triphenylphosphine (320 g, 1.22 mol) was stirred at room temperature for 18 hours. After the acetonitrile was removed under reduced pressure, toluene was added to precipitate the white solid. After pouring the toluene, the white solid was dissolved in dichloromethane (1 L) and transferred to the separatory funnel. 10% aqueous NaOH (1L) was added to the funnel, and the organic layer immediately turned yellow after shaking. The organic layer was separated and the aqueous layer was extracted with dichloromethane (1L) once. The dichloromethane layers were combined and washed with brine (400 mL) once, then dried over Na₂SO₄, filtered and concentrated, to give the ylide **26** as a yellow solid (280 g, 58%).

### Example 27 Synthesis of compound 27

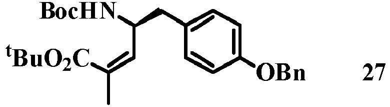

Compound **25** (450 g, 1.27 mol) was dissolved in dry CH₂Cl₂ (3L), to which *tert-butyl* ester ylide **26** (546 g, 1.40 mmol) was added and the solution was stirred at room temperature overnight and determined complete by TLC. Purification by column chromatography (10-50% ethyl acetate/petroleum ether) afforded compound **27** (444 g,75% yield) as a white solid. ESI m/z calcd for C₂₈H₃₈NO₅ M+H⁺: 468.27 found 468.22.

### Example 28 Synthesis of compound 28

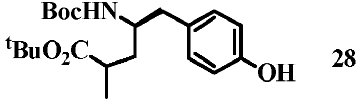

Compound 27 (63 g,0.13mol) was dissolved in methanol (315 mL) and hydrogenated (1 atm H₂) with Pd/C catalyst (10 wt%, 6.3 g) at room temperature overnight. The catalyst was filtered off and the filtrate was concentrated under reduced pressure to afford compound 28 (45.8 g,93% yield).

### Example 29 Synthesis of compound 29

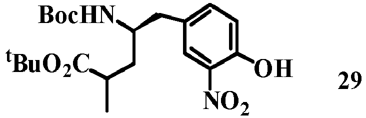

To a solution of compound **28** (390 g,1.03 mol) in THF (4 L), *tert*-butyl nitrite (1.06 kg,10.3 mol) was added at room temperature and the reaction was stirred overnight. After removal of THF, the residue was purified by column chromatography (10-50% ethyl acetate/hexanes) to afford compound **29** (314 g, 72% yield) as a light yellow solid.

### Example 30 Synthesis of compound 30

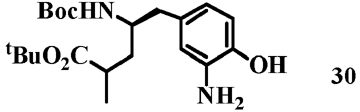

To a solution of **30** (166 g, 0.392 mol) in ethyl acetate (500 mL) was added Pd/C (10 wt%, 16 g) under nitrogen, and the reaction flask was evacuated and purged with hydrogen. After 3 cycles of evacuation and refilling, the reaction mixture was stirred under hydrogen (1 atm) at room temperature for 16 h and then filtered over Celite and concentrated to afford product **30** (146 g, 97% yield) as a light yellow foam. ¹H NMR (400 MHz, CDCl₃) δ 6.62 (d, J = 7.9 Hz, 1H), 6.55 (s, 1H), 6.43 (d, J = 7.3 Hz, 1H), 4.39 (dd, J = 53.0, 44.2 Hz, 1H), 3.77 (s, 4H), 2.72 - 2.29 (m, 3H), 1.83 - 1.58 (m, 1H), 1.40 (d, J = 7.6 Hz, 18H), 1.24 (s, 1H), 1.06 (t, J = 5.7 Hz, 3H). MS ESI m/z calcd for C₂₁H₃₅N₂O₅ [M+H]⁺ 394.25, found 395.25.

### Example 31 Synthesis of compound 31

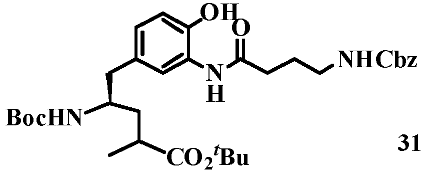

HATU (39.9 g, 105 mmol) was added to a solution of 4-(((benzyloxy)carbonyl)amino) butanoic acid (26.1 g, 110 mmol) in DMF (300 mL). After stirring at room temperature for 30 min, the mixture was added to a solution of compound **30** (39.4 g, 100 mmol) and TEA (20.2 g, 200 mmol) in DMF (300 mL). The resulting mixture was stirred at room temperature for 2 h. Water was then added, extracted with ethyl acetate, the organic layer was washed with brine, dried over Na₂SO₄. Purification by column chromatography (20% to 70% ethyl acetate/petroleum ether) yielded the title product as a white solid (45 g, 73% yield). ESI m/z calcd for C₃₃H₄₈N₃O₈ [M+H]⁺: 614.34, found 614.15.

### Example 32 Synthesis of compound 32

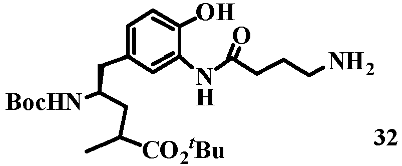

Compound **31** (100 g,163mmol) was dissolved in methanol (500 mL), mixted with Pd/C catalyst (10 wt%, 10 g) and hydrogenated (1 atm) at room temperature overnight. The catalyst was filtered off and the filtrate was concentrated under reduced pressure to afford compound **32** (75.8 g, 97% yield) as a brown foamy solid. ¹H NMR (400 MHz, CDCl₃) δ 7.11 (s, 1H), 6.83 (d, J = 10.3 Hz, 2H), 5.04 - 4.52 (m, 6H), 3.90 - 3.56 (m, 1H), 2.81 (d, J = 5.3 Hz, 2H), 2.63 (dd, J = 12.5, 6.1 Hz, 2H), 2.54-2.26 (dd, J = 14.0, 7.6 Hz, 4H), 1.94-1.64 (m, 3H), 1.44 - 1.36 (m, 18H), 1.08 (d, J = 6.9 Hz, 3H). ESI m/z calcd for C₂₅H₄₂N₃O₆ [M+H]⁺: 480.30, found 480.59.

### Example 33 Synthesis of compound 33

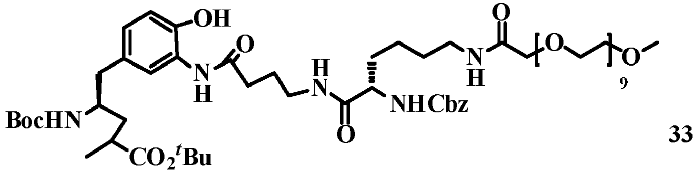

To a solution of compound **22** (130 g, 174 mmol) in DMF (500 mL) were added TEA (66 mL, 474 mmol) and HATU (72 g, 190 mmol) in sequence at 0 °C. Then the reaction mixture was warmed to room temperature and stirred for 2 h. A solution of compound **32** (75.8 g, 158 mmol) in DMF (500 mL) was added to the above solution at 0 °C, and the reaction mixture was stirred at room temperature for 1 h. The reaction mixture was poured into water (4 L), the aqueous layer was extracted with ethyl acetate (3 × 500mL), and the organic layers were combined and washed with brine (2 L), dried over Na₂SO₄, concentrated and the crude product **33** (190 g) was used in the next step directly. ESI m/z: calcd for C₆₀H₁₀₀N₅O₂₀ [M+H]⁺: 1210.69, found 1210.69.

### Example 34 Synthesis of compound 34

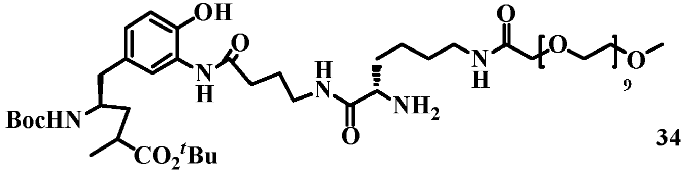

The crude product **33** (190 g) from the previous reaction was dissolved in methanol (900 mL), mixed with Pd/C catalyst (10 wt%, 19 g) and hydrogenated (1 atm) at room temperature overnight. The catalyst was filtered off and the filtrate was concentrated under reduced pressure, and the crude compound was purified by SiO₂ column with a gradient of(0-10% MeOH/dichloromethane) to give a brown oil product (105 g,62% yield over two steps). ESI m/z calcd for C₅₂H₉₅N₅O₁₈ [M+H]⁺: 1077.65, found 1077.65.

### Example 35 Synthesis of compound 35

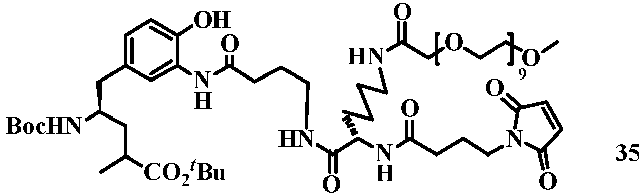

To a solution of compound **34** (105 g, 97.1 mmol) in EtOH (5.3 L) were added N-succinimidyl 4-maleimidobutyrate (54.4 g, 194.2 mmol) and 0.1M NaH₂PO₄ solution (1.1 L) at room temperature. Then the reaction mixture was stirred at room temperature overnight. EtOH was then evaporated under vacuum and the residue was poured onto water (3L). The aqueous solution was extracted with ethyl acetate (4 × 500mL), and the organic layers were combined and washed with brine (2 L), dried over Na₂SO₄, concentrated and the crude product was purified by SiO₂ column with a gradient of (0-10% MeOH/dichloromethane) to afford a yellow oil (100 g, 83% yield). ¹H NMR (500 MHz, DMSO) δ 9.53 (s, 0.7H), 9.52 (s, 0.3H), 9.22 (s, 0.7H), 9.21 (s, 0.3H), 7.95-7.87 (m, 2H), 7.65 (t, *J* = 5.9 Hz, 1H), 7.51-7.44 (m, 1H), 6.99 (s, 2H), 6.77 - 6.66 (m, 2H), 6.65-6.57 (m, 1H), 4.13 (dt, *J* = 5.4, 8.1 Hz, 1H), 3.84 (s, 2H), 3.55 (s, 2H), 3.52 (s, 2H), 3.51 - 3.45 (m, 30H), 3.42 (dd, *J* = 5.8, 3.7 Hz, 2H), 3.38 (t, *J* = 6.9 Hz, 2H), 3.23 (s, 3H), 3.14 - 3.01 (m, 4H), 2.64 - 2.44 (m, 1H), 2.41 - 2.22 (m, 4H), 2.16 - 2.04 (m, 2H), 1.76 - 1.64 (m, 4H), 1.64 - 1.52 (m, 2H), 1.52 - 1.35 (m, 2H), 1.37 (s, 3H), 1.35 (s, 6H), 1.31 (s, 9H), 0.97 (t, *J* = 8.5 Hz, 3H). ¹³C NMR (126 MHz, DMSO) δ 175.35 (minor), 174.88, 171.69, 171.42, 171.29, 171.10, 169.04, 155.19 (minor), 155.05, 145.97, 134.47, 129.36, 126.00, 125.20, 122.92, 115.69, 79.32 (minor), 79.17, 77.35 (minor), 77.27, 71.29, 70.25, 69.95, 69.79, 69.60, 69.53, 58.06, 52.57, 50.13, 49.55 (minor), 41.25, 38.12, 37.94, 37.45, 36.84, 36.80 (minor), 33.38, 32.37, 31.86, 28.96, 28.28, 28.23, 27.69, 27.57, 25.42, 24.19, 22.86, 18.04, 16.49 (minor). ESI m/z calcd for C₆₀H₁₀₁N₆O₂₁ [M+H]⁺: 1241.7, found 1241.8.

### Example 36 Synthesis of compound 36

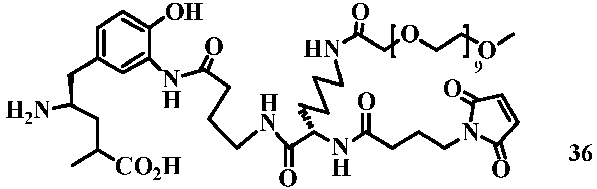

Compound **35** (31.5 g, 25.4 mmol) was dissolved in dichloromethane (15 mL), and then TFA (15 mL) was added. The reaction mixture was stirred at room temperature for 3 h, then concentrated on rotovap, and under vacuum oil pump. The crude product was stirred with ether (200 mL), and the product layer was seperated and concentrated on rotovap, and under vacuum oil pump until no change of weight, to give compound **36** (36.0 g, with trace solvent). ¹H NMR (500 MHz, DMSO) δ 9.18 (s, 1H), 7.97-7.87 (m, 2H), 7.79 (s, 2H), 7.71 - 7.61 (m, 2H), 7.00 (s, 2H), 6.85-6.73 (d, *J* = 5.4 Hz, 2H), 4.17 - 4.07 (m, 1H), 3.84 (s, 2H), 3.55 (s, 2H), 3.52 (s, 2H), 3.50 (s, 30H), 3.42 (dd, *J* = 5.8, 3.7 Hz, 2H), 3.39 (t, *J* = 7.0 Hz, 2H), 3.32-3.23 (m, 1H), 3.23 (s, 3H), 3.14 - 3.01 (m, 4H), 2.82-2.71 (m, 1H), 2.71 - 2.61 (m, 1H), 2.58 - 2.50 (m, 1H), 2.38 (t, *J* = 7.3 Hz, 2H), 2.11 (dt, *J* = 7.8, 3.0 Hz, 2H), 1.88 - 1.77 (m, 1H), 1.76 - 1.64 (m, 4H), 1.59 (dt, *J* = 15.1, 5.8 Hz, 1H), 1.53 - 1.32 (m, 4H), 1.31 - 1.11 (m, 2H), 1.05 (d, *J* = 7.0 Hz, 2.1H), 1.00 (d, *J* = 6.9 Hz, 0.9H). ¹³C NMR (126 MHz, DMSO) δ 176.78 (minor), 176.55, 171.74, 171.42, 171.34, 171.12, 169.07, 146.63 (minor), 146.57, 134.49, 126.51, 126.31, 125.19, 122.85, 115.80, 71.31, 70.27, 69.96, 69.81, 69.61, 69.55, 58.07, 54.93 (minor), 52.64, 50.72, 50.13 (minor), 38.30 (minor), 38.14, 37.95, 36.85, 35.75, 35.38 (minor), 34.87, 34.81(minor), 33.46, 32.38, 31.83, 28.98, 25.40, 24.21, 22.89, 17.49, 16.74 (minor). ESI m/z calcd for C₅₁H₈₅N₆O₁₉[M+H]⁺: 1085.6, found 1085.4.

### Example 37 Synthesis of compound 37

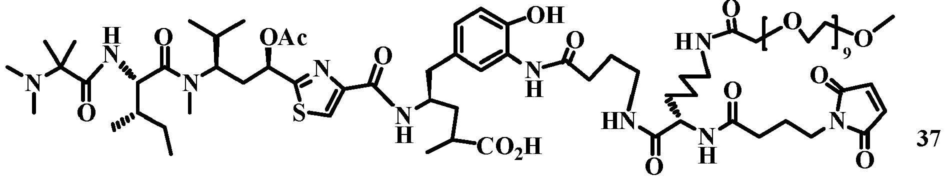

Compound **36** (36.0 g, 25.4 mmol) in DMF (60 mL) was added to the reaction flask and cooled to 5°C in an ice water bath. A solution of compound **18** (19.3 g, 27.9 mmol) in DMF (150 mL) was added, and then DIPEA (25 mL, 139 mmol) was added dropwise. After completion, the water bath was removed, and the reaction mixture was warmed up to room temperature, and stirred for 18 hours. The reaction mixture was concentrated on a high vacuum oil pump until no solvent was distilled out, and the residue was diluted with dichloromethane, cooled in an ice water bath to 5 °C, formic acid was added dropwise, until pH was adjusted to 3.0-4.0. The mixture was concentrated again and the residue was loaded on a silica gel column and eluted with hexanes/ethyl acetate/formic acid and dichloromethane/MeOH/formic acid, and appropriate fractions were concentrated to yield a crude product. The crude product was dissolved in water/MeOH/formic acid, and then further purified by preparative HPLC, eluted with water/acetonitrile/formic acid. The fractions were concentrated and diluted with water, transfered to lyophilizer flasks equally. After lyophilization, a light yellow foamy solid (24 g, 60% yield) was obtained. ¹H NMR (500 MHz, DMSO) δ 9.60 (bs, 1H), 9.20 (s, 1H), 8.19 (s, 0.33H), 8.17 (s, 0.67H), 8.02 (d, J = 9.0 Hz, 0.33H), 7.98 (d, J = 9.0 Hz, 0.67H), 7.94 - 7.83 (m, 2H), 7.65 (t, J = 5.8 Hz, 1H), 7.63 (s, 1H), 7.56 (s, 0.33H), 7.55 (s, 0.67H), 6.99 (s, 2H), 6.82 - 6.74 (m, 1H), 6.74 - 6.67 (m, 1H), 5.62 - 5.54 (m, 1H), 4.69 - 4.59 (m, 1H), 4.39 (s, 1H), 4.25 - 4.05 (m, 2H), 3.85 (s, 2H), 3.55 (s, 2H), 3.52 (s, 2H), 3.50 (s, 30H), 3.44-3.36 (m, 4H), 3.23 (s, 3H), 3.14 - 3.02 (m, 4H), 2.96 (s, 3H), 2.83 - 2.71 (m, 1H), 2.71 - 2.57 (m, 1H), 2.44 - 2.32 (m, 3H), 2.32 - 2.14 (m, 2H), 2.14 - 2.05 (m, 2H), 2.12 (s, 6H), 2.09 (s, 3H), 1.99 - 1.65 (m, 7H), 1.64 - 1.53 (m, 2H), 1.53 - 1.32 (m, 5H), 1.31 - 1.14 (m, 2H), 1.11 (s, 3H), 1.05 (d, J = 7.2 Hz, 2H), 1.03 (d, J = 6.9 Hz, 1H), 0.99 (s, 3H), 0.93 (d, J = 6.4 Hz, 3H), 0.86 (d, J = 6.7 Hz, 3H), 0.82 (t, J = 7.3 Hz, 3H), 0.67 (d, J = 6.4 Hz, 3H). ¹³C NMR (126 MHz, DMSO) δ 177.52 (minor), 177.01, 175.44, 172.75, 171.69, 171.43, 171.29, 171.09, 169.74, 169.55 (minor), 169.46, 169.04, 159.92 (minor), 159.88, 149.86, 149.74 (minor), 146.07, 134.46, 129.13 (minor), 129.08, 126.12 (minor), 126.07, 125.18, 124.28 (minor), 124.11, 122.95, 122.86 (minor), 115.67, 71.30, 70.26, 69.96, 69.80, 69.60, 69.54, 69.48, 63.01, 58.06, 55.09, 52.58, 52.39, 49.03, 47.96 (minor), 40.35, 38.89, 38.11, 37.94, 37.39, 36.84, 36.53, 36.02, 35.78 (minor), 33.87, 33.38, 32.38, 31.86, 29.02, 28.97, 25.37, 24.19, 23.60, 22.86, 21.15, 20.62, 19.99, 19.41, 18.34, 18.11, 16.17 (minor), 15.69, 10.81. ESI m/z calcd for C₇₆H₁₂₅N₁₀O₂₄S [M+H]⁺ 1593.9, found 1593.8.

### Example 38 Synthesis of compound 38

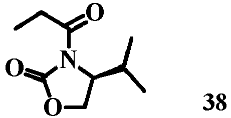

To a solution of (S)-4-isopropyloxazolidin-2-one (400 g, 3.09 mol, 1.0 eq.) in anhydrous THF (8 L), at about -70 °C was added *n*-BuLi (2.5 M in hexanes, 1.36 L, 3.4 mol, 1.1 eq.) under N₂. The mixture was stirred at -70 °C for 1 h, and then propionyl chloride (315 g, 3.4 mol, 1.1 eq.) was added slowly. After the addition was completed, the mixture was stirred at -70 °C for another 1 h, and slowly warmed to room temperature. The reaction mixture was added to an ice-cold saturated ammonium chloride solution (7 L) and extracted with ethyl acetate (3 × 2 L). The combined organic layers were washed with water (2 L) and brine (2 L), dried over anhydrous Na₂SO₄, filtered, concentrated and purified by column chromatography (3 kg silica gel, pure petroleum ether to 5:1 petroleum ether/ethyl acetate) to give the title compound as a colorless oil (500 g, 87% yield). MS ESI m/z calcd for C₉H₁₆NO₃ [M+H]⁺ 186.10, found 186.10.¹H NMR (400 MHz, CDCl₃) δ 4.48 - 4.39 (m, 1H), 4.27 (t, *J* = 8.7 Hz, 1H), 4.21 (dd, *J =* 9.1, 3.1 Hz, 1H), 3.06 - 2.82 (m, 2H), 2.38 (dtd, *J* = 14.0, 7.0, 4.0 Hz, 1H), 1.17 (t, *J* = 7.4 Hz, 3H), 0.90 (dd, *J* = 17.0, 7.0 Hz, 6H).

### Example 39 Synthesis of compound 39

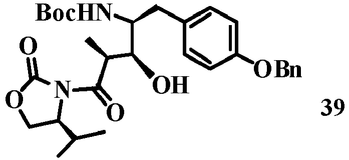

To a solution of compound **38** (92.6 g, 0.50 mol) in anhydrous dichloromethane (1.5 L) was added DIPEA (70.5 g, 0.54 mol) at room temperature. The mixture was cooled to -10 °C and *n*-Bu₂BOTf (1.0 M in dichloromethane, 500 mL) was added under N₂. The temperature of reaction mixture was maintained below 0 °C during addition. The reaction was then stirred at 0 °C for 1 h and then cooled to -78°C, to which compound **25** (161 g, 0.45 mol) in dichloromethane (1 L) was added dropwise. The mixture was stirred at -78 °C for 2 h and then warmed slowly to room temperature and stirred overnight. Phosphate buffer solution (0.1M, pH 7.0, 2 L) was added. After phase separation, the aqueous phase was further extracted with dichloromethane (2 × 500 mL). The combined organic layers were dried over anhydrous Na₂SO₄, filtered and concentrated. The crude product was re-dissolved in methanol (2 L) and cooled to 0 °C, then treated with H₂O₂ (30% aqueous solution, 500 mL) and stirred for 1 h. The methanol was removed by rotary evaporation and water (3 L) was added. The resulting mixture was extracted with dichloromethane (3 × 800 mL). The combined organic layers were washed with water (500 mL), saturated NaHCO₃ (500 mL) and brine (500 mL), dried over anhydrous Na₂SO₄, filtered and concentrated. The residue was mixted with 400 g silica gel and purified by column chromatography (pure petroleum ether to 5:1 petroleum ether/ethyl acetate) to give the title compound as a foamy solid (150 g, 60% yield). ¹H NMR (400 MHz, CDCl₃) δ 7.36 (ddd, *J* = 24.2, 14.2, 7.1 Hz, 5H), 7.12 (d, *J* = 8.4 Hz, 2H), 6.90 (d, *J* = 8.5 Hz, 2H), 5.02 (s, 2H), 4.69 (d, *J* = 9.0 Hz, 1H), 4.45 (d, *J* = 4.1 Hz, 1H), 4.33 (t, *J* = 8.4 Hz, 1H), 4.15 (d, *J* = 8.6 Hz, 1H), 3.90 (dd, *J* = 16.6, 8.0 Hz, 1H), 3.85 - 3.77 (m, 2H), 2.81 (d, *J* = 7.6 Hz, 2H), 2.27 (dd, *J* = 11.4, 6.7 Hz, 1H), 1.35 (s, 9H), 0.89 (dd, *J* = 14.3, 6.9 Hz, 6H). MS ESI m/z calcd for C₃₀H₄₁N₂O₇ [M+H]⁺ 541.28, found 541.30.

### Example 40 Synthesis of compound 40

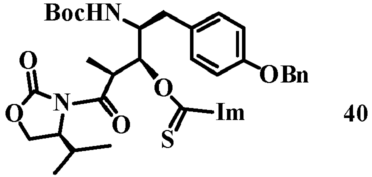

Compound **39** (200 g, 0.37 mol) was discolved in anhydrous THF (3.5 L), then dithiocarbonylimidazole was added (198 g, 1.11 mol) and refluxed 8h under N₂, dithiocarbonylimidazole(65 g, 0.37 mol) was added again, then the mixture was stirred at room temperature overnight. The mixure was cooled back to room temperure, concentrated under reduced pressure and purified by SiO₂ column chromatography (pure petroleum ether to 5:1 petroleum ether/ethyl acetate) to give an oil (170 g, 83% yield). ¹H NMR (400 MHz, CDCl₃) δ 8.41 (s, 1H), 7.67 (s, 1H), 7.36 (dt, *J* = 16.0, 6.9 Hz, 6H), 7.09 (s, 1H), 7.05 (d, *J* = 8.4 Hz, 2H), 6.86 (d, *J* = 8.4 Hz, 2H), 6.32 (d, *J* = 9.5 Hz, 1H), 5.01 (s, 2H), 4.56 - 4.43 (m, 2H), 4.32 (ddd, *J* = 16.2, 15.6, 7.8 Hz, 3H), 4.19 (d, *J* = 8.7 Hz, 1H), 2.96 (dd, *J* = 14.6, 4.4 Hz, 1H), 2.49 (dd, *J* = 14.5, 10.5 Hz, 1H), 2.29 (td, *J* = 13.4, 6.7 Hz, 1H), 1.73 (s, 1H), 1.29 (s, 9H), 0.91 (dd, *J* = 13.9, 6.9 Hz, 6H). MS ESI m/z calcd for C₃₄H₄₃N₄O₇S[M+H]⁺ 651.27, found 651.39.

### Example 41 Synthesis of compound 41

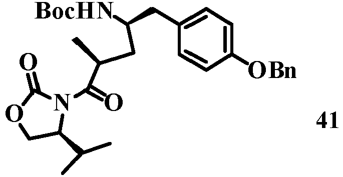

To a solution of compound **40** (210 g, 323 mmol) in anhydrous toluene (30 mL) was added *n*-Bu₃SnH (182 g, 646 mmol) and azodiisobutyronitrile (0.5 g, 3.23 mmol) in sequence under N₂. The mixture was refluxed for 2.5 h, cooled to room temperature and then cconcentrated and purified by SiO₂ column chromatography (pure petroleum ether to 5:1 petroleum ether/ethyl acetate) to give a colorless oil (141 g,83%yield). ¹H NMR (400 MHz, CDCl₃) δ 7.36 (ddd, *J* = 24.5, 14.5, 7.1 Hz, 5H), 7.08 (d, *J* = 8.5 Hz, 2H), 6.90 (d, *J* = 8.5 Hz, 2H), 5.04 (d, *J* = 5.1 Hz, 2H), 4.48 (d, *J* = 4.2 Hz, 1H), 4.33 (t, *J* = 8.4 Hz, 1H), 4.22 (d, *J* = 9.7 Hz, 1H), 4.15 (d, *J* = 8.8 Hz, 1H), 3.81 (s, 2H), 2.73 (dd, *J* = 14.1, 5.9 Hz, 1H), 2.61 (dd, *J* = 14.0, 7.2 Hz, 1H), 2.29 (dq, *J* = 13.5, 6.8 Hz, 1H), 2.11 - 2.00 (m, 1H), 1.60 (dd, *J* = 15.2, 6.2 Hz, 2H), 1.35 (s, 9H), 1.20 (d, *J* = 6.9 Hz, 3H), 0.89 (dd, *J* = 14.0, 6.9 Hz, 6H). MS ESI m/z calcd for C₃₀H₄₁N₂O₆ [M+H]⁺ 525.28, found 525.37.

### Example 42 Synthesis of compound 42

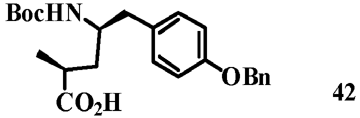

To a solution of compound **41**(208 g, 390 mmol) in THF (30 mL) and H₂O (0.7 L)were added LiOH (0.192 g, 4.58 mmol, 2.0 eq.) in H₂O₂ (30% aqueous solution, 1.4 mL, 6.0 eq.). After 3 h of stirring at the temperature lower than 5 °C, sodium bisulfite solution (1.5 M, 2L) and 2N HCl were added until pH 4 was reached. The mixture was extracted with ethyl acetate (3 × 800 mL) and the combined organic phrases were washed with water (500ml) and brine (500ml), dried over anhydrous Na₂SO₄, filtered, concentrated and purified by SiO₂ column chromatography (pure petroleum ether to 3:1 hexanes/ethyl acetate ) to give an oil (158 g, 96% yield). ¹H NMR (400 MHz, CDCl₃) δ 7.46 - 7.28 (m, 5H), 7.07 (d, *J* = 7.7 Hz, 2H), 6.91 (d, *J* = 7.8 Hz, 2H), 5.04 (s, 2H), 4.52 (d, *J* = 8.5 Hz, 1H), 3.87 (d, *J* = 41.8 Hz, 1H), 2.82 - 2.43 (m, 3H), 1.85 (t, *J* = 12.2 Hz, 1H), 1.41 (s, 9H), 1.17 (d, *J* = 6.9 Hz, 3H). MS ESI m/z calcd for C₂₄H₃₂NO₅ [M+H]⁺ 414.22, found 414.21.

### Example 43 Synthesis of compound 43

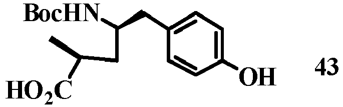

A mixture of compound **42** (158 g, 0.38 mmol) and Pd/C (10%, 0.25 g) in methanol (1.5 L) was hydrogenated under 1 atm H₂ pressure for 16 h and then filtered through Celite. The filtrate was concentrated to afford an oil (123 g, 100% yield). ¹H NMR (400 MHz, CDCl₃) δ 7.00 (d, *J* = 7.5 Hz, 2H), 6.80 (s, 2H), 4.51 (d, *J* = 9.0 Hz, 1H), 3.88 (s, 1H), 2.66 (dd, *J* = 65.6, 22.6 Hz, 4H), 1.88 (t, *J* = 12.2 Hz, 1H), 1.42 (s, 9H), 1.14 (d, *J* = 6.6 Hz, 3H). MS ESI m/z calcd for C₁₇H₂₆NO₅ [M+H]+: 324.17, found 324.16.

### Example 44 Synthesis of compound 44

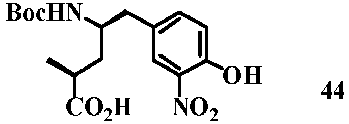

To a solution of compound **43** (113 g, 0.35 mol) in THF (10 mL) was added *t*-BuONO (360 g, 3.5 mol). The reaction was stirred at room temperature for 3 h, concentrated and purified by SiO₂ column chromatography (pure petroleum ether to 2:1 petroleum ether/ethyl acetate) to give a yellow solid (85 g, 61% yield). ¹H NMR (400 MHz, DMSO) δ 12.00 (s, 1H), 10.68 (s, 1H), 7.67 (s, 1H), 7.34 (d, *J* = 8.4 Hz, 1H), 7.03 (d, *J* = 8.4 Hz, 1H), 6.69 (d, *J* = 8.9 Hz, 1H), 3.56 (d, *J* = 3.8 Hz, 1H), 2.67 (dd, *J* = 13.5, 5.1 Hz, 1H), 2.41 (dd, *J* = 13.8, 6.6 Hz, 1H), 1.78 - 1.65 (m, 1H), 1.27 (s, 9H), 1.18 (s, 1H), 1.05 (d, *J* = 7.1 Hz, 3H). MS ESI m/z calcd for C₁₇H₂₅N₂O₇ [M+H]⁺ 369.15, found 369.14.

### Example 45 Synthesis of compound 45

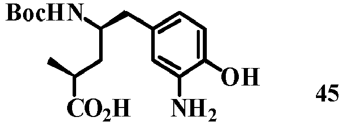

A mixture of compound **44** (80 g, 217 mmol) and Pd/C (10 wt%, 2.0g) in methanol (500 mL) was hydrogenated (1 atm H₂) at room temperature for 1 h, and then filtered through Celite. The filtrate was concentrated to afford a white soild (73 g, 93% yield). MS ESI m/z calcd for C₁₇H₂₇N₂O₅ [M+H]+ 339.18, found 339.17. ¹H NMR (400 MHz, MeOD) δ 6.60 (d, *J* = 7.9 Hz, 2H), 6.44 (d, *J* = 7.3 Hz, 1H), 3.71 (d, *J* = 6.3 Hz, 1H), 2.62 - 2.37 (m, 3H), 1.83 (ddd, *J* = 13.7, 9.9, 3.7 Hz, 1H), 1.39 (s, 9H), 1.13 (d, *J* = 7.1 Hz, 3H)

### Example 46 Synthesis of compound 46

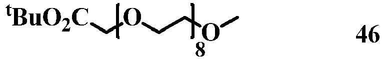

Octadecanol monomethyl ether (115.2 g, 0.3 mol) was dissolved in dry tetrahydrofuran (3L) and sodium hydride (60 wt %, 24 g, 0.6 mol) was added at room temperature. After stirring for 1 hour, *tert*-butyl bromide (146.3 g, 0.75 mol) was added, and stirred for another 1 hour. The reaction mixture was poured onto 4 L dichloromethane, and then mixed with 2 kg of crushed ice, the aqueous phase was separated, extracted with 1 L dichloromethane. The combined organic phases were washed with water, concentrated and purified by column chromatography (20% petroleum ether/ethyl acetate, and then 0 to 5% MeOH/dichloromethane) to afford 108 g of the title compound (72% yield).

### Example 47 Synthesis of compound 47

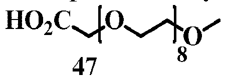

To mixture of anhydrous formic acid (1 L) and CH₂Cl₂ (500 mL) was added compound **46** (210 g, 0.422 mol). The mixture was stirred overnight at room temperature and concentrated to afford 200 g of the product (100% yield).

### Example 48 Synthesis of compound48

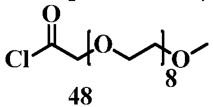

Compound **48** (198 g, 0.422 mol) was dissolved in 2.6 L CH₂Cl₂, and then 0.5 mL of DMF and oxalyl chloride (275 mL) were added dropwise at room temperature. The reaction was stirred for 3 hours and concentrated to afford 210 g of the product.

### Example 49 Synthesis of compound 49

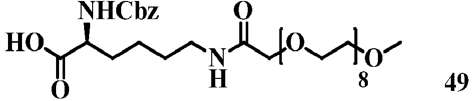

Cbz-L-lysine (236.3 g, 0.844 mol), Na₂CO₃ (89.5 g, 0.844 mol) and NaOH (33.8 g, 0.844 mol) were mixed in 1.6 L water and cooled to 0 °C in an ice salt bath. Compound **48** (210 g, crude, 0.422 mol) in THF (160 mL) was added dropwise, and then the mixture was stirred at room temperature for 1 hour. 1 L ethyl acetate was added, and the aqueous phase was separated, pH was adjusted to 3-4 with concentrated hydrochloric acid. The combined organic phase was washed with brine, and dried over anhydrous Na₂SO₄, filtered and concentrated to afford product 290 g (97% yield).

### Example 50 Synthesis of compound 50

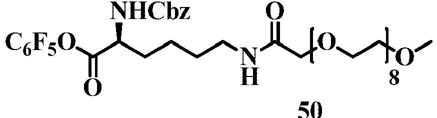

Compound **49** (182.5 g, 0.26 mol) was dissolved in 2 L dichloromethane. Pentafluorophenol (95.4 g, 0.52 mol) and DIC (131 g, 1.04 mol) were added at room temperature. The reaction was stirred for 1 hour, and the product was concentrated to give a crude product 430 g.

### Example 51 Synthesis of compound 51

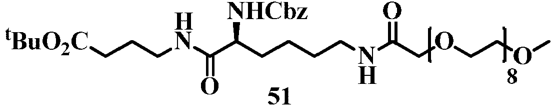

*Tert*-butyl 4-aminobutyric acid (62 g, 0.39 mol) was dissolved in 1.5 L DMF, cooled with ice water, and DIPEA (134.2 g, 1.04 mol) was added, and then compound **50** (430 g, crude, 0.26 mol) was added slowly at 10°C to 20 °C. The reaction mixture was stired at room temperature for 1 hour, concentrated, diluted with dichloromethane, washed with water, and extracted with dichloromethane. The organic phases were combined and washed with 0.2 N HCl, brine, dried over anhydrous Na₂SO₄, filtered and concentrated, purified by column chromatography (25% to 100% ethyl acetate/petroleum ether, 0 to 5% MeOH/dichloromethane) to give 180 g product, 82% yield.

### Example 52 Synthesis of compound 52

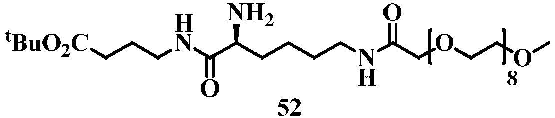

Compound **51** (78 g, 92.3 mmol) and Pd/C (10 wt %, 13g) were mixed in 500 mL methanol. The mixture was stirred under a H₂ balloon at room temperature overnight, concentrated and purified by column chromatography (0 to 20% MeOH/dichloromethane ) to afford 70.2 g product (92% yield).

### Example 53 Synthesis of compound 53

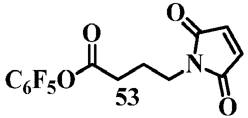

Compound **52** (17.3 g, 94.2 mmol) was dissolved in 500 mL dichloromethane. Pentafluorophenol (34.7 g, 188.5 mmol) and DIC (47.5 g, 377 mmol) were added in sequence at room temperature. The reaction was stirred for 1 hour and concentrated to give a crude product (105 g).

### Example 54 Synthesis of compound 54

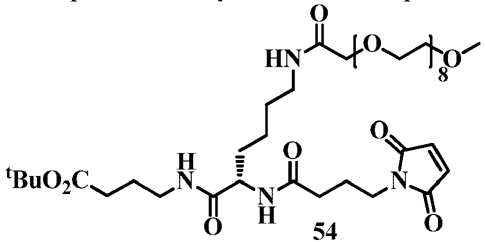

Compound **52** (67 g, 94.2 mmol) was dissolved in 0.75 L DMF, cooled by ice water, then DIPEA (48.6 g, 376.8 mmol) was added. To the mixture was added compound **53** (105 g, crude, 94.2 mmol) slowly at 10 °C to 20 °C. After the reaction mixture was stirred at room temperature for 1 hour, it was concentrated, diluted with dichloromethane, washed with water, and the water layer was extracted with dichloromethane. The combined organic phase was washed with 0.2 N HCl, and brine, dried over anhydrous Na₂SO₄, filtered, concentrated, and purfied by column chromatography (50% -100% ethyl acetate/petroleum ether, 10% MeOH/dichloromethane ) to give a product (80.5 g, 98% yield).

### Example 55 Synthesis of compound 55

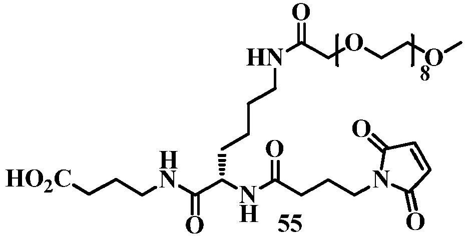

To the mixture of anhydrous formic acid (400 mL) and dichloromethane (200 mL) was added compound **54** (80.5 g, 91.9 mmol). The reaction mixture was stirred overnight at room temperature, concentrated, diluted with dichloromethane, washed with brine, dried over anhydrous Na₂SO₄, filtered, and purified by column chromatography (0% to 20% MeOH/dichloromethane ) to yield 70 g of product (93% yield).

### SpecifiExample 56 Synthesis of compound 56

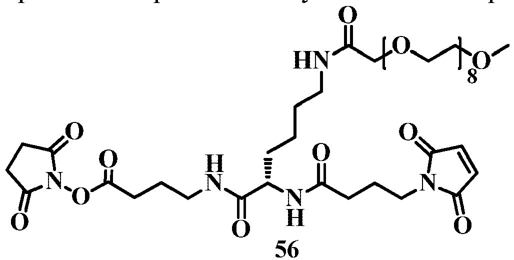

To a solution of compound **55** (104 g, 0.127 mol) in dichloromethane (1000 mL) were added N-hydroxysuccinimide (16g, 0.14 mol) and EDC·HCl (37 g, 0.2 mol) in sequence at room temperature. The reaction mixture was stirred at room temperature for 1h, washed with brine, dried over anhydrous Na₂SO₄, filtered and concentrated to give product (120 g, 100% yield).

### Example 57 Synthesis of compound 57

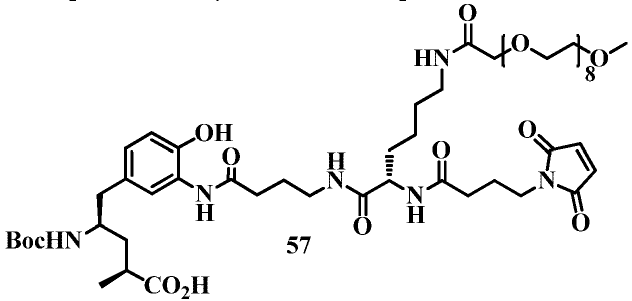

A mixture of compound **56** (120 g, 127 mmol) and compound **45** (45.0 g, 133 mmol) in 1 L tetrahydrofuran was heated and refluxed overnight, diluted with dichloromethane (2 L), washed with brine, dried over anhydrous Na₂SO₄, filtered and concentrated. 1.5 L water and 1.5 L ethyl acetate were added to the crude product, stirred for half an hour, and the aqueous layer was separated, and the ethyl acetate layer was extracted twice with water (600 mL × 2). The aqueous phases were combined, and washed with ethyl acetate (700 mL) to remove impurities. To the aqueous phase was added sodium chloride to saturation, and the resulting aqueous solution was extracted with dichloromethane (1 L × 2), and the dichloromethane phases were combined, dried over anhydrous Na₂SO₄, filtered, concentrated and purified by column chromatography (0% to 20% MeOH/dichloromethane ) to yield 83.6 g product (58% yield). ¹H NMR (600 MHz, DMSO ) δ 9.23 (s, 1H), 7.97-7.83 (m, 1H), 7.64 (t, J = 5.8 Hz, 1H), 7.46 (s, 1H), 6.99 (s, 1H), 6.73 (s, 1H), 6.61 (d, J = 8.7 Hz, 0 H), 4.14 (dt, J = 5.4, 8.3 Hz, 1H), 3.84 (d, J = 5.4, 8.3 Hz, 1H), 3.84 (t, J = 5.4, 8.3 Hz, 1H), 3.51-3.46 (m, 26m), 3. 42 (dd, J = 5.7, 3.8 Hz, 2H), 3.39 (t, J = 7.0 Hz, 2H), 3.23 (t, J = 13.5, 7.0 Hz, 1H), 2.45 (dd, J = 13.5, 7.0 Hz, 1H), 2.41-2.32 (m, 3), 2.11 (td, J = 7.1, 1.9 Hz, 2H), 1.75-1.64 (m, 4H), 1.64-1.54 (m, 1H), 1.52-1.43 (m, 1H), 1.43-1.35 (m, 2H), 1.32 (s, 9g), 1.32-1.15 (m, 4H), 1 02 (d, J = 7.1 Hz, 3H). ¹³C NMR (151 MHz, DMSO) δ 177.18, 171.68, 171.43, 171.28, 171.08, 169.02, 155.15, 145.99, 134.45, 129.41, 125.91, 125.30, 123.04, 1115.71, 77.29, 71.29, 70.25, 69.95, 69.79, 69.59, 69.53, 58.05, 52.57, 50.33, 40.71, 38.12, 37.93, 37.64, 36.83, 35.92, 33.35, 32 37 31.85, 28.95, 28.26, 27.86 (minor), 25.39, 24.18, 22.85, 18.10. ESI m/z calcd for C₅₄H₈₉N₆O₂₀ [M+H]⁺ 1141.6, found1141.9.

### Example 58 Synthesis of compound 58

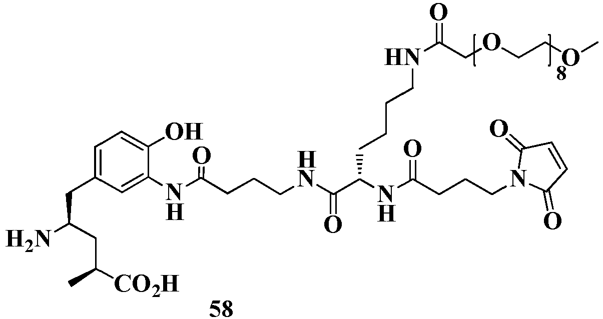

Compound **57** (59.0 g, 51.7 mmol) was dissolved in dichloromethane (345 mL), to which trifluoroacetic acid (172 mL) was added. After the addition was completed, the reaction was stirred at room temperature for 2 hours, concentrated on a rotavap until no solvent was evaporated, and then concentrated on an oil vacuum pump until no weight change, to give a crude product **58** (75.5 g, containing solvent). ¹H NMR (600 MHz, DMSO) δ 9.82 (s, 1H), 9.18 (s, 1H), 7.95 - 7.88 (m, 2H), 7.83 (s, 2H), 7.67 (s, 1H), 7.66 (t, *J* = 6.1 Hz, 1H), 6.99 (s, 2H), 6.84 - 6.78 (m, 2H), 4.16 - 4.09 (m, 1H), 3.84 (s, 2H), 3.55 (s, 2H), 3.52 (s, 2H), 3.52 - 3.44 (m, 26H), 3.42 (dd, *J* = 5.6, 3.9 Hz, 2H), 3.39 (t, *J* = 7.0 Hz, 2H), 3.33 - 3.25 (m, 1H), 3.23 (s, 3H), 3.14 - 3.02 (m, 4H), 2.76 (dd, *J* = 13.9, 6.1 Hz, 1H), 2.65 (dd, *J* = 13.9, 6.1 Hz, 1H), 2.55 (dq, *J* = 14.0, 7.0 Hz, 1H), 2.38 (t, *J* = 7.3 Hz, 2H), 2.14 - 2.08 (m, 2H), 1.82 (ddd, *J* = 14.1, 8.7, 5.5 Hz, 1H), 1.74 - 1.65 (m, 4H), 1.64 - 1.55 (m, 1H), 1.52 - 1.43 (m, 2H), 1.43 - 1.34 (m, 2H), 1.30 - 1.13 (m, 2H), 1.05 (d, *J* = 7.0 Hz, 3H). ¹³C NMR (151 MHz, DMSO) δ 176.54, 171.73, 171.43, 171.34, 171.11, 169.07, 146.59, 134.48, 126.50, 126.33, 125.19, 122.85, 115.84, 71.30, 70.27, 69.96, 69.81, 69.61, 69.55, 58.06, 52.65, 50.72, 38.31, 38.13, 37.95, 36.85, 35.75, 34.88, 33.45, 32.38, 31.82, 28.97, 25.40, 24.20, 22.88, 17.48. ESI m/z calcd for C₄₉H₈₁N₆O₁₈ [M+H]⁺ 1041.6, found1041.7.

### Example 59 Synthesis of compound 59

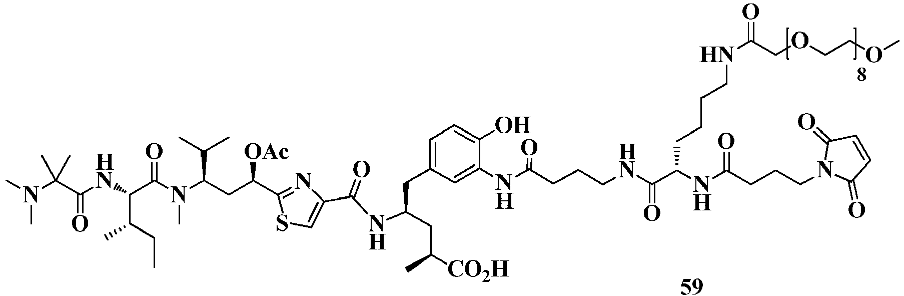

Compound **58** (75.5 g, in 51.7 mmol) in DMF (160 mL) was added to the reaction flask and cooled to 5°C in an ice water bath. A solution of compound **18** (35.8 g, 51.7 mmol) in DMF (240 mL) was added and then DIPEA (36.8 g, 285 mmol) was added dropwise. After completion, the water bath was removed, and the reaction mixture was warmed up to room temperature, and stirred for 18 hours. The reaction mixture was concentrated on a high vacuum oil pump until no solvent was distilled out, and the residue was diluted with dichloromethane, cooled in an ice water bath to 5 °C, to which formic acid was added dropwise, until pH was adjusted to 3.0-4.0. The mixture was concentrated again and the residue was loaded on a silica gel column and eluted with hexanes/ethyl acetate/formic acid and dichloromethane/MeOH/formic acid, and appropriate fractions were concentrated to yield a crude product. The crude product was dissolved in water/MeOH/formic acid, and then further purified by preparative HPLC, eluted with water/acetonitrile/formic acid. The fractions were concentrated and diluted with water, transfered to lyophilizer flasks equally. After lyophilization, a light yellow foamy solid (48 g, 60% yield) was obtained. ¹H NMR (600 MHz, DMSO) δ 9.20 (s, 1H), 8.17 (s, 1H), 8.03-7.95 (m, 1H), 7.95 - 7.86 (m, 2H), 7.77-7.61 (m, 2H), 7.55 (s, 1H), 6.98 (s, 2H), 6.77 (d, *J* = 8.1 Hz, 1H), 6.71 (d, *J* = 8.1 Hz, 1H), 5.58 (d, *J* = 10.7 Hz, 1H), 4.65 (dd, *J* = 9.3, 7.1 Hz, 1H), 4.40 (s, 1H), 4.18 - 4.08 (m, 2H), 3.85 (s, 2H), 3.55 (s, 2H), 3.52 (s, 2H), 3.51 - 3.47 (m, 26H), 3.44 - 3.36 (m, 4H), 3.23 (s, 3H), 3.14 - 3.02 (m, 4H), 2.96 (s, 3H), 2.75 (dd, *J* = 13.5, 6.7 Hz, 1H), 2.63 (dd, *J* = 13.5, 6.7 Hz, 1H), 2.43 - 2.32 (m, 3H), 2.31 - 2.23 (m, 1H), 2.23 - 2.09 (m, 3H), 2.13 (s, 6H), 2.09 (s, 3H), 1.88 - 1.73 (m, 3H), 1.75 - 1.65 (m, 4H), 1.65 - 1.53 (m, 2H), 1.53 - 1.44 (m, 3H), 1.44 - 1.34 (m, 2H), 1.31 - 1.15 (m, 2H), 1.12 (s, 3H), 1.05 (d, *J* = 6.9 Hz, 3H), 1.01 (s, 3H), 0.93 (d, *J* = 6.4 Hz, 3H), 0.86 (d, *J* = 6.6 Hz, 3H), 0.82 (t, *J* = 7.3 Hz, 3H), 0.67 (d, *J* = 6.1 Hz, 3H). ¹³C NMR (151 MHz, DMSO) δ 177.05, 175.28, 172.76, 171.74, 171.47, 171.34, 171.11, 169.78, 169.49, 169.09, 159.91, 149.88, 146.10, 134.47, 129.10, 126.11, 125.23, 124.12, 122.97, 115.72, 71.33, 70.30, 69.99, 69.83, 69.63, 69.57, 69.52, 63.21, 58.08, 55.05, 52.63, 52.49, 49.07, 40.37, 38.89, 38.14, 37.97, 37.43, 36.87, 36.50, 36.06, 33.91, 33.41, 32.41, 31.87, 29.06, 28.99, 25.40, 24.22, 23.66, 22.88, 21.08, 20.63, 20.00, 19.43, 18.41, 18.13, 15.68, 10.81. ESI m/z calcd for C₇₄H₁₂₁N₁₀O₂₃S[M+H]⁺ 1549.8,found1550.2.

### Example 60 Synthesis of compound 60

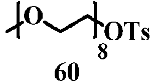

Octadecanol monomethyl ether (10 g, 26 mmol, 1.0 eq.) was dissolved in 100 mL of anhydrous dichloromethane, DMAP (32 mg, 0.26 mmol, 0.01 eq.) was added, and then triethylamine (10.5 g, 104 mmol, 4.0 eq.) and TSCL (14.9 g, 78 mmol, 3.0 eq.) were added dropwise over an ice bath. After stirring for 10 minutes, the reaction was warmed to room temperature, and stirred overnight, washed with 1 N HCl (100 mL), water (100 mL) and brine (100 mL), dried over anhydrous Na₂SO₄, concentrated on a rotavap. The residue was dissolved with a small amount of dichloromethane, and then loaded on a column and eluted with 5% -100% ethyl acetate/petroleum ether and 1%-3% MeOH/dichloromethane to afford a yellow oil (11.6 g, 83% yield). ESI m/z calcd for C₂₄H₄₃O₁₁S [M+H]⁺ 539.2, found539.2.

### Example 61 Synthesis of compound 61

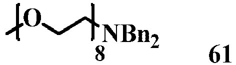

Compound **60** (11.6 g, 21.5 mmol, 1.0 eq.) was dissolved in 20 mL of anhydrous DMF, dibenzylamine (5.5 g, 27.8 mmol, 1.5 eq.) was added and the reaction mixture was stirred at 100°C overnight, diluted with 300 mL of dichloromethane, washed with water (300 mL × 3) and brine (300 mL), dried over anhydrous Na₂SO₄, concentrated on a rotavap. The residue was dissolved with a small amount of dichloromethane, loaded on a column and eluted with 5% -100% ethyl acetate/petroleum ether to afford a light yellow oil (8.2, 66% g). ESI m/z calcd for C₃₁H₅₀NO₈ [M+H]⁺ 564.3, found564.3.

### Example 62 Synthesis of compound 62

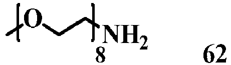

To a solution of compound **61** (8.6 g, 15.2 mmol, 1.0 eq.) in 100 mL of anhydrous MeOH was added Pd/C (0.9 g, 10 wt %), and the mixture was heated under hygrogen to reflux and stirred overnight. The reaction mixture was filtered, washed with methanol and concentrated on a rotavap to give 5.3 g of colorless oil, 90% yield. ESI m/z calcd for C₁₇H₃₈NO₈ [M+H]⁺ 384.3, found384.3.

### Example 63 Synthesis of compound 63

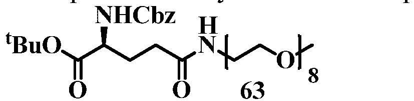

To a solution of Z-L-glutamic acid tert-butyl ester (0.96 g, 2.86 mmol) and compound **62** (1.1 g, 2.86 mmol) in DMF (201 mL) were added HATU (1.2 g, 3.15 mmol) and DIPEA (1 mL, 6.3 mmol) at 0°C. The mixture was warmed to room temperature and stirred for 1 hour, and then poured onto ice water, extracted with dichloromethane (3 × 50 mL), the combined organic phase was washed with water (30 mL), saturated sodium bicarbonate (30 mL), brine (30 mL), dried over Na₂SO₄, filtered and concentrated, and purified by silica gel column (mobile phase: MeOH/CH₂Cl₂) to give product **63** (1.5 g, 75% yield). ESI m/z calcd for C₃₄H₅₉N₂O₁₃ [M+H]⁺ 703.4, found 703.4.

### Example 64 Synthesis of compound 64

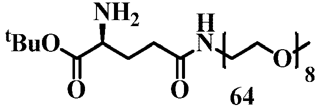

To a solution of compound **63** (0.66 g, 0.93 mmol) in methanol (10 mL) was added Pd/C (10 wt %, 60 mg), and the reaction was stirred under H₂ balloon for 2 hours, then filtered and concentrated to give compound **64** (450 mg, 85% yield). ESI m/z calcd for C₂₆H₅₃N₂O₁₁ [M+H]⁺ 569.4, found569.4.

### Example 65 Synthesis of compound 65

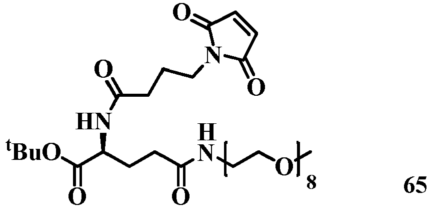

To a solution of compound **64** (0.45 g, 0.79 mmol) and N-succinimidyl 4-maleimidobutyrate (0.33 g, 1.18 mmol) in ethanol (5 mL) was added NaH₂PO₄ (0.110 mmol), and the reaction mixture was stirred overnight at room temperature. The majority of the ethanol was removed under reduced pressure, and the residue was diluted with water (20 mL), extracted with dichloromethane (3 × 30 mL). The organic phases were combined, washed with water (20 mL) and brine (20 mL), dried over Na₂SO₄, filtered and concentrated, and the crude product was purified by silica gel column (mobile phase: MeOH/dichloromethane ) to give compound **65** (380 mg, 65% yield). ESI m/z calcd for C₃₄H₆₀N₃O₁₄ [M+H]⁺ 734.4, found734.4.

### Example 66 Synthesis of compound 66

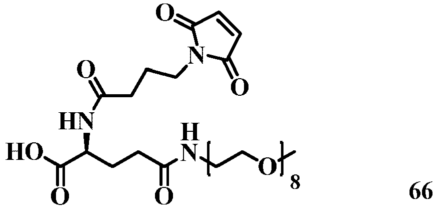

To a solution of compound **65** (230 mg, 0.31 mmol) in dichloromethane (2 mL) was added trifluoroacetic acid (2 mL), the reaction mixture was stirred at room temperature for 1 hour. The mixture was concentrated, and then co-evaporated 3 times with dichloromethane, and dried on an oil pump to afford compound 66 (208 mg, 100% yield). ESI m/z calcd for C₃₀H₅₂N₃O₁₄ [M+H]⁺ 678.3, found 678.3.

### Example 67 Synthesis of compound 67

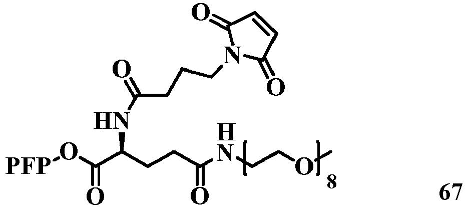

To a solution of compound **66** (208 mg, 0.3 mmol) in dichloromethane (5 mL) were added pentafluorophenol (113 mg, 0.6 mmol) and EDC·HCl (117 mg, 0.6 mmol). After addition, the mixture was stirred overnight at room temperature, diluted with dichloromethane (20 mL), washed with water (5 mL), dried over Na₂SO₄ and concentrated to afford compound **67** (252 mg, 100% yield). ESI m/z calcd for C₃₆H₅₁F₅N₃O₁₄ [M+H]⁺ 844.3, found844.3.

### Example 68 Synthesis of compound 68

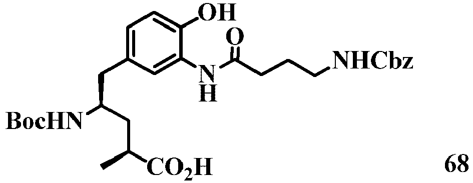

To a solution of compound **45** (7.3 g, 21.7 mmol) and N-Boc-alanine hydroxysuccinimide ester (7.2 g, 21.7 mmol) in ethanol (300 mL) was added 0.1 N of NaH₂PO₄ (150 mL). The reaction mixture was stirred overnight at room temperature, and then concentrated, diluted with water (100 mL) and extracted with ethyl acetate (3 × 50 mL). The combined organic phase was washed with brine (50 mL), dried over Na₂SO₄, filtered and concentrated, and purified by silica gel column (mobile phase: ethyl acetate/PE) to give compound **68** (6.7 g, 55% yield). ESI m/z calcd for C₂₉H₄₀N₃O₈[M+H]⁺ 558.3, found558.3.

### Example 69 Synthesis of compound 69

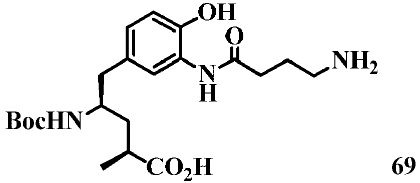

To a solution of compound **68** (6.7 g, 12 mmol) in methanol (100 mL) was added Pd/C (10 wt %, 0.67 g), and the mixture was stirred under H₂ balloon for 2 hours, then filtered and concentrated to give compound **69** (5 g, 100% yield). ESI m/z calcd for C₂₁H₃₄N₃O₆ [M+H]⁺ 424.2, found424.2.

### Example 70 Synthesis of compound70

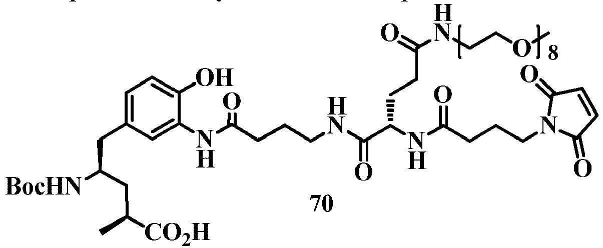

To a solution of compound **67** (252 mg, 0.3 mmol) and compound **69** (190 mg, 0.45 mmol) in DMF (5 mL) was added DIPEA (0.13 mL, 0.75 mmol) at 0°C. After completion, the mixture was slowly warmed to room temperature and stirred for 1 hour. The mixture was diluted with water and extracted with dichloromethane (3 × 10 mL), then the combined organic phase was washed with water (10 mL), 1 N HCl (10 mL) and brine (10 mL), dried with Na₂SO₄, filtered and concentrated. The crude product was purified by silica gel column (mobile phase:MeOH/dichloromethane) to afford compound **70** (180 mg, 55% yield). ESI m/z calcd for C₅₁H₈₃N₆O₁₉ [M+H]⁺ 1083.6, found 1083.6.

### Example 71 Synthesis of compound 71

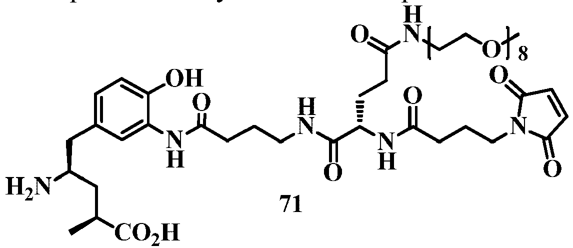

To a solution of compound **70** (8.2 g, 7.6 mmol) in dichloromethane (56.8 mL) was added trifluoroacetic acid (18.9 mL), and the reaction mixture was stirred for 2 hours at room temperature, then co-evaporated twice with dichloromethane and dried on an oil pump. To the residue was added ether (100 mL) and the mixture was stirred vigorously for 1 hour, settled, and the upper layer was discarded. The residue was taken to repeat the operation twice, and then concentrated on a rotavap and dried on an oil pump to give compound **71** (9.2 g, >100% yield). ESI m/z calcd for C₄₆H₇₅N₆O₁₇ [M+H]⁺ 983.51, found 983.37.

### Example 72 Synthesis of compound 72

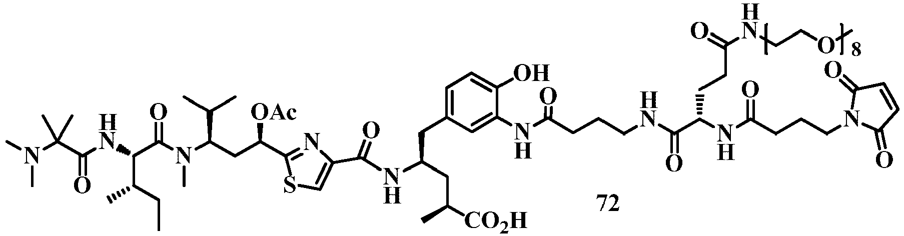

Compound **71** (8.2 g, 7.6 mmol) was dissolved in DMF (80 mL), the reaction flask was cooled to 0-5°C in an ice-water bath, a solution of compound **18** (5.2 g, 7.6 mmol) in DMF (201 mL) was added, followed by DIPEA (4 mL, 22.8 mmol) slowly. The speed of adding DIPEA should be controlled, in order to maintain the reaction temperature between 5°C and 10°C. After that, the ice water bath was removed, and the reaction was warmed to room temperature and stirred for 1.5 hours. The reaction mixture was concentrated and diluted with dichloromethane, cooled in an ice water bath, formic acid was added dropwise, until pH was adjusted to 3.0-4.0. The mixture was concentrated again and purified on a silica gel column and eluted with 20-100% ethyl acetate/petroleum ether and 0-20% MeOH/dichloromethane (containing 0.1% formic acid) to afford a crude product (11.44 g). The crude product was purified on preparative HPLC (20-30% acetonitrile/water (each containing 0.1% formic acid)), the fractions were concentrated and lyophilized to afford compound **72** (6.8 g, yield 60%). ESI m/z calcd for C₇₁H₁₁₅N₁₀O₂₂S [M+H]⁺ 1491.78, found1492.01.

### Example 73 Synthesis of compound 73

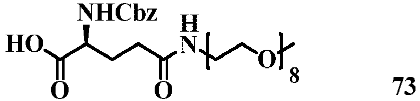

To a mixture of anhydrous formic acid (500 mL) and dichloromethane (250 mL) was added compound **63** (22.4 g, 0.03 mol). The mixture was stirried overnight at room temperature and concentrated to give a product (19 g, 100% yield).

### Example 74 Synthesis of compound 74

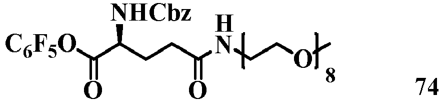

Compound **73** (19.0 g, 0.03 mol) was dissolved in dichloromethane (200 mL), to which pentafluorophenol (11.0 g, 0.06 mol) and DIC (15.1 g, 0.12 mol) were added at room temperature. The reaction was stirred for 1 hour and concentrated to give crude product 45 g.

### Example 75 Synthesis of compound 75

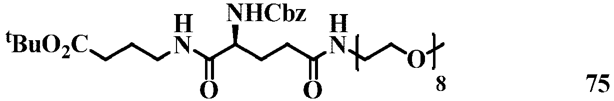

*Tert*-butyl 4-aminobutyric acid (6.40 g, 0.04 mol) was dissolved in 500 mL DMF and cooled with ice water, DIPEA (15.5 g, 0.12 mol) was added, and then compound **74** (45 g, crude, 0.03 mol) was slowly added at 10°C to 20 °C. The reaction mixture was stirred at room temperature for 1 hour, concentrated, diluted with dichloromethane, washed with water, and the aqueous phase was extracted with dichloromethane. The combined organic phase was washed with 0.2 N HCl, brine, dried over anhydrous Na₂SO₄, filtered, concentrated, and then purified by column chromatography (25% -100% ethyl acetate/petroleum ether, 0-5% MeOH/dichloromethane ) to give a product (19.5 g, 83% yield).

### Example 76 Synthesis of compound 76

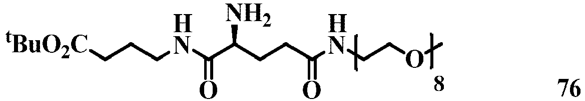

Compound **75** (19.5 g, 24.8 mmol) and Pd/C (10 wt %, 5g) was added in 200 mL methanol, and the mixture was stirred under a H₂ balloon at room temperature overnight, and then filtrated, concentrated to afford 16.7 g of product (100% yield).

### Example 77 Synthesis of compound 77

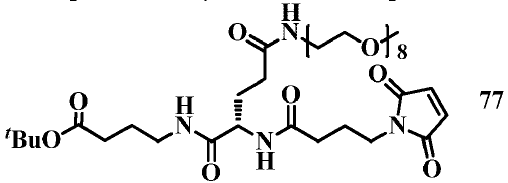

Compound **76** (16.7 g, 24.8 mmol) was dissovled in 200 mL DMF, cooled with ice water, DIPEA (12.9 g, 0.10 mol) was added. The mixture was kept at 10 °C to 20 °C, to which compound **53** (30 g, crude, 24.8 mmol) was slowly added. The mixture was stirred at room temperature for 1 hour, concentrated, diluted with dichloromethane, washed with water, and the aqueous phase was extracted with dichloromethane. The combined organic phase was washed with 0.2 N HCl, brine, dried over anhydrous Na₂SO₄, filtered and concentrated, purified by column chromatography (50%-100% ethyl acetate/petroleum ether, 10% MeOH/dichloromethane ) to give a product (20 g, 98% yield).

### Example 78 Synthesis of compound 78

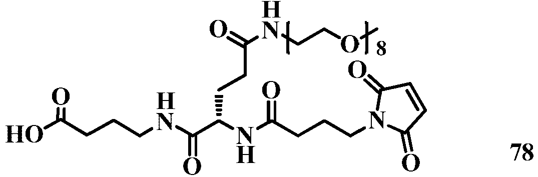

Compound **77** (16.8 g, 20.5 mmol) was dissolved in dichloromethane (60 mL) and anhydrous formic acid (120 mL) was added. The reaction was stirred overnight, concentrated, diluted with ethyl acetate (150 mL), extracted with water (300 mL). The organic phase was discarded, solid sodium chloride was added to the aqueous phase to reach saturation. The aqueous solution was then extracted with dichloromethane (200 mL × 2), and the dichloromethane phase was collected, dried over anhydrous Na₂SO₄, filtered, concentrated, and purified by column chromatography (0-20% methanol/dichloromethane) to give compound **78** (16.4 g, yield > 100%, containing formic acid). ESI m/z calcd for C₃₄H₅₉O₁₅N₄ [M+H]⁺ 763.39, found 763.29.

### Example 79 Synthesis of compound 79

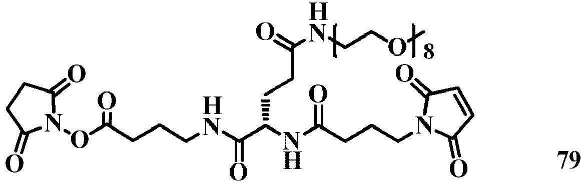

Compound **78** (15.6 g, 20.5 mol) was dissolved in dichloromethane (200 mL) at room temperature, to which N-hydroxysuccinimide (NHS, 3.7 g, 32.3 mol) and EDC·HCl (8.3 g, 43 mol) were added in sequence. The mixture was stirred at room temperature for 30 minutes, washed with brine, dried over anhydrous Na₂SO₄,filtered and concentrated to give compound **79** (17.6 g, 100% yield). ESI m/z calcd for C₃₈H₆₂O₁₇N₅ [M+H]⁺ 860.41, found 860.29.

### Example 80 Synthesis of compound 80

### Method I

Compound **79** (8.8 g, 10.2 mmol) and compound **45** (3.5 g, 10.2 mmol) were dissolved in 200 mL of tetrahydrofuran. The mixture was heated to reflux, stirred overnight, concentrated, and then water (300 mL) and ethyl acetate (100 mL) were added to the crude product, stirred, and the aqueous phase was separated. To the aqueous phase was added sodium chloride to reach saturation, and the solution was extracted with dichloromethane (2 × 150 mL). The combined dichloromethane was dried over anhydrous Na₂SO₄, filtered and concentrated, purified by column chromatography (0-20% MeOH/dichloromethane) to give compound **80** (4.0 g, yield 36%). ESI m/z: calcd for C₅₁H₈₃O₁₉N₆[M+H]⁺ 1083.56, found 1083.47.

### Method II

Compound **79** (4.4 g, 5.12 mmol) and compound **45** (1.73 g, 5.12 mmol) were dissolved in 100 mL of EtOH. Then 0.1 N NaH₂PO₄ solution was added (20 mL) and the mixture was stirred overnight. After concentration, water (200 mL) and ethyl acetate (100 mL) were added to the residue, stirred, and the aqueous phase was separated. To the aqueous phase was added sodium chloride to reach saturation, and the solution was extracted with dichloromethane (2 × 100 mL). The combined dichloromethane was dried over anhydrous Na₂SO₄, filtered and concentrated, purified by column chromatography (0-20% MeOH/dichloromethane) to give compound **80** (2.0 g, yield 36%).

### Method III

Compound **79** (4.4 g, 5.12 mmol) and compound **45** (1.73 g, 5.12 mmol) were dissolved in 100 mL of acetonitrile. Then 0.1 N NaH₂PO₄ solution was added (20 mL) and the mixture was stirred overnight. After concentration, water (200 mL) and ethyl acetate (100 mL) were added to the residue, stirred, and the aqueous phase was separated. To the aqueous phase was added sodium chloride to reach saturation, and the solution was extracted with dichloromethane (2 × 100 mL). The combined dichloromethane was dried over anhydrous Na₂SO₄, filtered and concentrated, purified by column chromatography (0-20% MeOH/dichloromethane) to give compound **80** (2.2 g, yield 40%).

### Example 81 Synthesis of compound 73

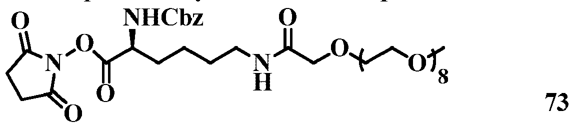

Compound **49** (20 g, 28.4 mmol, 1.0 eq.) was dissolved in 350 mL of anhydrous dichloromethane and cooled in an ice water bath. NHS (3.9 g, 34.1 mmol, 1.2 eq.) and EDC (27 g, 142.0 mmol, 5.0 eq.) were added in sequnce. The reaction was stirred at room temperature overnight and then washed with water (200 mL × 2), brine (200 mL × 1), dried over anhydrous Na₂SO₄, and concentrated. The residue was dissolved in a small amount of dichloromethane and loaded on a silica gel column and eluted with 2: 49: 49 to 4: 48: 48 MeOHl/ethyl acetate/dichloromethane . The product was obtained as a yellow oil (14.2 g, 62% yield). ESI m/z calcd for C₃₇H₆₀N₃O₁₆ [M+H]⁺:802.4,found:802.4.

### Example 82 Synthesis of compound 74

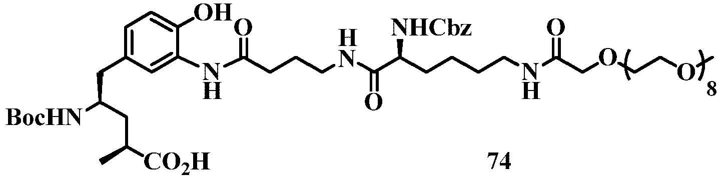

To a solution of compound **69** (6.4 g, 15.1 mmol, 1.0 eq.) in 40 mL of ethanol and 10 mL of 0.1 M NaH₂PO₄ was added compound **73** (12.7 g, 15.9 mmol, 1.05 eq.). The reaction mixture was stirred overnight, concentrated and re-dissolved in dichloromethane, dried over anhydrous Na₂SO₄, filtered and concentrated and purified by silica gel column (3-5% methanol/dichloromethane) to give a white foam (11.7 g, 70% yield). ESI m/z calcd for C₅₄H₈₈N₅O₁₉ [M + H]⁺: 1110.6, found:1110.6.

### Example 83 Synthesis of compound 75

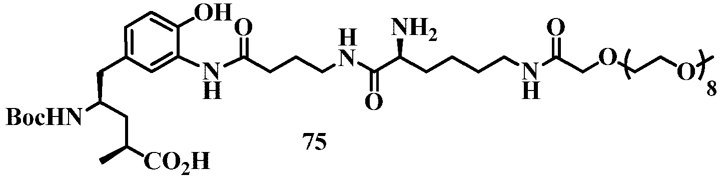

Compound **74** (4.2 g, 3.79 mmol, 1.0 eq.) and Pd/C (0.4 g, 10 wt %) were mixed in 5 mL of methanol. The mixture was stirred under a H₂ balloon at room temperature overnight, and then the catalyst was filtered off and washed with methanol. The filtrate was concentrated to give 0.32 g of crude product, which was used without further purification (87% yield). ESI m/z calcd for C₄₆H₈₂N₅O₁₇ [M+H]+: 1997.1, found: 1997.1.

### Example 84 Synthesis of compound 76

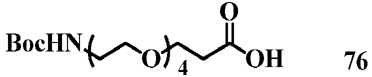

In a 500 mL flask, H₂N-PEG₄-CH₂CH₂CO₂H (3.0 g, 11.3 mmol, 1.0 eq.) and K₂CO₃ (4.7 g,33.93 mmol,3.0 eq.) was dissolved in 50 mL of water and cooled in an ice water bath, and then the Boc₂O (3.2 g, 14.7 mmol, 1.3) in 50 mL of tetrahydrofuran was added dropwise. The reaction was heated to room temperature and stirred overnight, 1 N KHSO ₄ was added until pH 4-5 was reached. The mixture was extracted with dichloromethane (200 mL × 1, 100 mL × 3), washed with water (500 mL × 1) and brine (500 mL × 1), dried with anhydrous Na₂SO₄ and concentrated. The residue was dissolved in a small amount of dichloromethane and then loaded onto a silica gel column, eluted with 2-4% methanol/dichloromethane, combined and concentrated to give a colorless oil 3.8 g (93% yield). ESI m/z calcd for C₁₆H₃₂NO₈ [M+H]⁺: 366.2, found: 366.2

### Example 85 Synthesis of compound 77

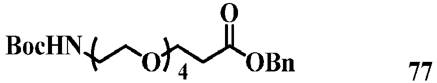

In a 50 mL single-necked flask, BocHN-PEG₄-CH₂CH₂CO₂H (0.81 g, 2.22 mmol, 1.0 eq.), K₂CO₃ (0.92 g,6.66 mmol,3.0 eq.), NaI (0.033 g,0.222 mmol,0.1 eq.) were mixed in 10 mL DMF and cooled over an ice water bath. BnBr (0.57 g, 3.33 mmol, 1.5 eq.) was added dropwise and the mixture was warmed to room temperature and stirred overnight, diluted with 100 mL of water, extracted with dichloromethane (100 mL × 2), washed with water (200 mL × 1) and brine (200 mL × 1), dried over anhydrous Na₂SO₄, and concentrated. The residue was dissolved in a small amount of dichloromethane, loaded onto a silica gel column, eluted with 70-90% ethyl acetate/petroleum ether to give 0.69 g of a colorless oil (69% yield). ESI m/z calcd for C₂₃H₃₈NO₈ [M+H]⁺: 446.3, found:446.3.

### Example 86 Synthesis of compound 78

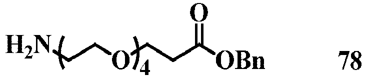

A solution of BocHN-PEG₄-CH₂CH₂CO₂Bn (0.69 g, 1.5 mmol, 1.0 eq.) in 6 mL dichloromethane and 3mL TFA was stirred at room temperature for 30 minutes. The solvent was removed, and the residue was co-evaporated with dichloromethane for three times, and place on a high vacuum pump. The product was directly used for the next reaction step. ESI m/z calcd for C₁₈H₃₀NO₆ [M+H]+:356.2, found 356.2.

### Example 87 Synthesis of compound 79

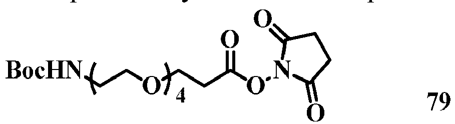

To a solution of BocHN-PEG₄-CH₂CH₂CO₂H (3.8 g, 10.4 mmol, 1.0 eq. ) in 50 mL anhydrous dichloromethane were added NHS (1.4 g, 12.5 mmol, 1.2 eq.) and EDC (10.0 g, 52.0 mmol, 5.0 eq.). The mixture was stirred at room temperature overnight and then washed with water (50 mL × 2), brine (100 mL × 1), dried over anhydrous Na₂SO₄, and concentrated. The crude product is used directly for the next step. ESI m/z calcd for C₂₀H₃₅N₂O₁₀[M+H]⁺ : 463.2, found463.2.

### Example 88 Synthesis of compound 80

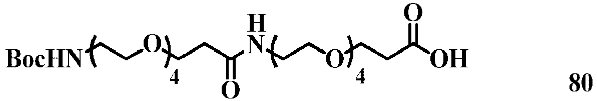

In a 300 mL flask H₂N-PEG₄-CH₂CH₂CO₂H (2.8 g, 10.4 mmol, 1.0 eq.) and K₂CO₃ (4.3 g, 31.2 mmol, 3.0 eq.) were dissolved in 40 mL water and cooled in an ice water bath, to which a solution of compound **79** (3.8 g, 10.4 mmol, 1.0 eq.) in tetrahydrofuran (40 mL) was added dropwise. The mixture was warmed to room temperature and stirred overnight. IN KHSO₄ was added until pH 4-5 was reached, the mixture was extracted with dichloromethane (150 mL × 1, 100 mL × 2), washed with water (200 mL × 1) and brine (200 mL × 1), dried over anhydrous Na₂SO₄, and concentrated. The residue was dissolved in a small amount of dichloromethane and loaded on a silica gel column and eluted with 4-6% methanol/dichloromethane to give a colorless oil (5.18 g, 81% yield). ESI m/z calcd for C₂₇H₅₃N₂O₁₃ [M+H]⁺: 613.3, found613.3.

### Example 89 Synthesis of compound 81

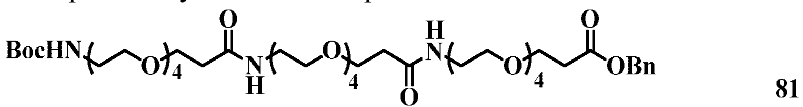

H₂N-PEG₄-CH₂CH₂CO₂Bn (crude product from the previous step) was dissolved in 3 mL DMF, cooled in an ice/water bath, DIPEA (0.78 g, 6.0 mmol, 4.0 eq.) was added dropwise, and then compound **80** (0.93 g, 1.5 mmol, 1.0 eq.) in DMF (7 mL) solution and HATU (1.72 g, 4.5 mmol, 3.0 eq.) was added. The mixture was stirred on an ice bath for 2 hours and diluted with 100 mL of water, extracted with dichloromethane (100 mL × 3), washed with IN KHSO₄ (200 mL × 1), saturated by sodium bicarbonate (200 mL × 1), and brine (200 mL × 1), dried over anhydrous Na₂SO₄, and concentrated. The residue was dissolved in a small amount of dichloromethane, loaded on a silica gel column, and eluted with 0-5% methanol/dichloromethane. The fractions were combined and concentrated to give a light yellow oil (1.0 g,71% yield). ESI m/z calcd for C₄₅H₈₀N₃O₁₈ [M+H]⁺:950.5, found 950.5.

### Example 90 Synthesis of compound 82

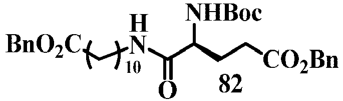

To a solution of 11-aminoundecanoate (2.91 g, 10.0 mmol) and Boc-Glu (OBzl) -OH (3.37 g, 10.0 mmol) in DMF (50 mL) were added EDC (1.91 g, 12.0 mmol) and triethylamine (3.5 mL, 25.0 mmol). The mixture was stirred at room temperature for 8 hours, diluted with water (100 mL) and extracted with ethyl acetate (3 × 100 mL). The combined organic phase was washed once with 100 mL of brine and then dried over anhydrous Na₂SO₄, filtered and concentrated. The residue was purified by silica gel column chromatography (ethyl acetate/dichloromethane, 1: 15) to give the title compound as a colorless oil (5.37 g, 88% yield).

### Example 91 Synthesis of compound 83

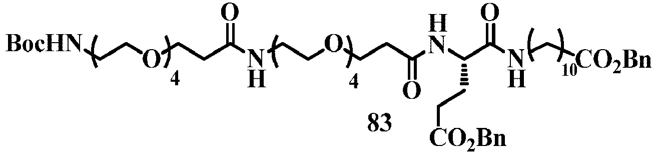

Compound **82** (0.64 g, 1.05 mmol, 1.0 eq.) was mixed with 5 mL dichloromethane and 2 mL TFA, stirred at room temperature for 2 hours, then concentrated. The residue was co-evaporated with dichloromethane three times and placed on a high vacuum pump. The crude product was re-dissolved in 3 mL DMF and cooled in an ice water bath. Compound **80** (0.64 g, 1.05 mmol, 1.0 eq.) in DMF (7 mL) was then added, followed by DIPEA (0.54 g, 4.20 mmol, 4.0 eq.) and HATU (1.2 g, 3.15 mmol, 3.0 eq.). The reaction mixture was stirred over an ice bath for 1 hour, then diluted with 100 mL of water and extracted with dichloromethane (150 mL × 1, 100 mL × 1). The organic phase was washed with IN KHSO₄ (200 mL × 1), saturated sodium bicarbonate (200 mL × 1) and brine (200 mL × 1), dried over anhydrous Na₂SO₄, filtered and concentrated. The crude product was dissolved in a small amount of dichloromethane and loaded on a silica gel column and then eluted with 0-10% MeOH/dichloromethane. The fractions were combined and concentrated to give 0.94 g of light yellow oil (81% yield). ESI m/z calcd for C₅₇H₉₂N₄O₁₇ [M+H]⁺ :1104.6,found:1104.6.

### Example 92 Synthesis of compound 84

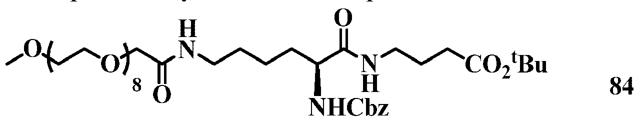

To a solution of tert-butyl 4-aminobutyric acid (1.03 g, 6.12 mmol) and compound **49** (3.91 g, 5.56 mmol) in DMF (18 mL) at 0°C were added HATU (2.32 g, 6.12 mmol) and TEA (1.2 mL, 8.34 mmol). The reaction mixture was stirred for 1 hour and then diluted with water (300 mL) and extracted with ethyl acetate (3 × 250 mL). The organic solution was washed with brine, dried over anhydrous Na₂SO₄, filtered, concentrated and purified by silica gel column chromatography (32: 1 dichloromethane/methanol) to afford the title compound (5.10 g, 99% yield). ESI MS m/z 846.50 ([M+H]⁺).

### Example 93 Synthesis of compound 85

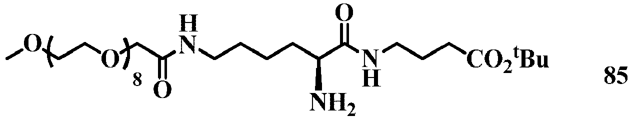

In a hydrogenation bottle, compound **84** (1.0 g, 1.18 mmol) and Pd/C (10 wt %, 0.10 g) were added to methanol (50 mL). The mixture was shaken for 2 hours, then filtered through Celite (filter aid), the filtrate was concentrated to give compound **85** (0.93 g, yield > 100%). ESI MS m/z 712.50 ([M+H]⁺).

### Example 94 Synthesis of compound 86

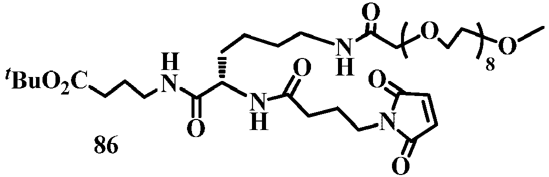

To a solution of compound **85** in 95% EtOH (50 mL) and NaH₂PO₄ (0.1M, pH 5.0, 10 mL) was added N-succinimidyl 4-maleimidobutyrate (0.50 g, 1.77 mmol, 1.5 eq.), and the mixture was stirred overnight, then concentrated, diluted with water (50 mL), extracted with dichloromethane (80 mL × 3), dried over anhydrous Na₂SO₄, filtered, concentrated, and purified by silica gel column chromatography (25: 1 dichloromethane/methanol) to give the title compound as a light yellow oil (0.82 g, 80%).

### Example 95 Synthesis of compound 87

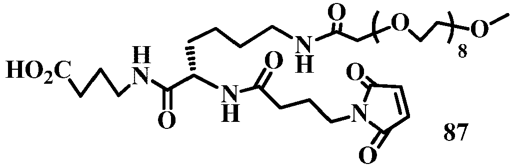

Compound **86** (0.82 g, 0.94 mmol) was dissolved in HCOOH (50 mL) and stirred at room temperature for 1 hour. The reaction mixture was concentrated and co-evaporated twice with toluene. The residue was placed on a vacuum pump to give compound **87** (0.80 g, crude product). ESI MS m/z ([M+H]⁺): 820.45.

### Example 96 Synthesis of compound 88

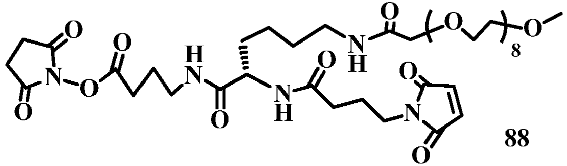

To a solution of compound **87** (0.80 g, crude, 0.94 mmol) in DMA (5.0mL) were added NHS (0.12 g, 1.03 mmol) and EDC·HCl (0.27 g, 1.41 mmol) and the reaction mixture was stirred for 2 hours and then diluted with water (15 mL) and extracted with ethyl acetate (3 × 10 mL). The combined organic phase was washed with brine (10 mL), dried over anhydrous Na₂SO₄, filtered and concentrated. The residue was purified by silica gel column (10-50% ethyl acetate/petroleum ether) to give a colorless oily compound (0.67 g, 78% yield). ESI MS m/z ([M + H] +) : 918.55.

### Example 97Synthesis of compound 89

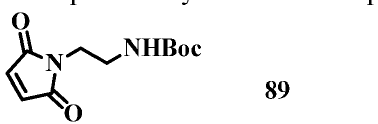

A mixture of N-Boc-ethylenediamine (5.6 mL, 35.4 mmol, 1.1 eq.) and saturated NaHCO₃ (60 mL) was cooled to 0°C and N-methoxycarbonyl maleimide (5.00 g, 32.2 mmol, 1.0 eq.) was added in portions. After stirring at 0°C for 30 minutes, the reaction was warmed to room temperature and stirred for 1 hour. The solid was collected by filtration, washed with cold water, then dissolved in ethyl acetate, washed with brine, dried over anhydrous Na₂SO₄, and concentrated to give a white solid (6.69 g, 87% yield). ESI MS m/z ([M+H]⁺) : 241.12.

### Example 98 Synthesis of compound 90

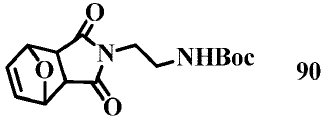

In a high pressure tube, a solution of compound **89** (6.00 g, 25.0 mmol), furan (18.0 mL) in toluene (120 mL) was heated to reflux and stirred for 16 hours. The colorless solution became yellow during the reaction and the mixture was then cooled to room temperature and concentrated. The resulting white solid was triturated with ether to give compound **90** (6.5 g, 84% yield). ESI MS m/z ([M+H]⁺) : 309.13.

### Example 99 Synthesis of compound 91

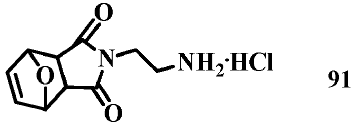

At room temperature, compound **90** (9.93 g, 32.2 mmol) in dioxane (15 mL) was treated with concentrated HCl (15 mL) for 3 hours. The resulting solid was collected by filtration, and washed with ethyl acetate, and then dried in an oven (50°C) overnight to give compound **91** (6.94 g, 88% yield). ESI MS m/z ([M + H] ⁺): 206.05.

### Example 100 Synthesis of compound 92

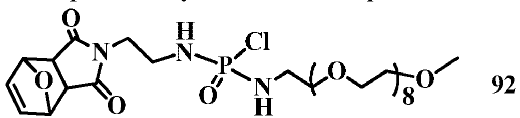

To a solution of compound **91** (1.22 g, 5 mmol) in tetrahydrofuran (10 mL) was added POCl₃ (0.47 mL, 5 mmol) at -10°C. After stirring for 10 minutes, 2,5, 8,11,14, 17,20,23, 26-nonoxydioctadecane-28-amine (2.14 g, 5 mmol) and DIPEA (0.87 mL, 5 mmol) were added. The reaction was warmed to 0°C, stirred for 3 hours and then concentrated. The residue was diluted with dichloromethane (10 mL), filtered over Celite, and the filtrate was used directly for the next step. ESI MS m/z ([M+H]⁺) :716.29.

### Example 101 Synthesis of compound 93

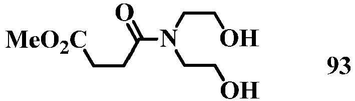

A mixture of dimethyl succinate (20.0 g, 136.9 mmol) and dihydroxyethyl amine (7.20 g, 68.7 mmol) in anhydrous toluene (500 mL) and pyridine (50 mL) was heated at 150°C for 28 hours. The mixture was concentrated and purified on a silica gel column and eluted with 5-25% ethyl acetate/dichloromethane to give the title compound (12.5 g, 83% yield). ESI MS m/z ([M+Na]⁺) :242.42.

### Example 102 Synthesis of compound 94

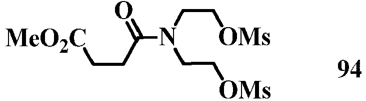

To a solution of compound **93** (12.0 g, 49.56 mmol) in anhydrous pyridine (350 mL) was added methanesulfonyl chloride (20.0 g, 175.4 mmol). After stirring overnight, the mixture was concentrated, diluted with ethyl acetate (350mL), washed with cold 1 M NaH₂PO₄ (2 × 300 mL), dried over MgSO4, filtered and concentrated to give a crude product (18.8 g, > 100% yield), which can be used for the next step without further purification.. ESI MS m/z ([M+H]+) : 376.06.

### Example 103 Synthesis of compound 95

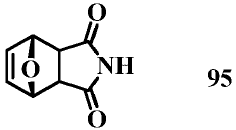

To a solution of maleimide (10.0 g, 103.0 mmol) in toluene (200 mL) was added furan (10.0 mL, 137.4 mmol). The mixture was heated at 100°C for 8 hours, cooled to room temperature, then concentrated and crystallized in ethyl acetate/hexane, and the resulting solid was washed with methanol to give 16.7 g of the title compound (99%). ¹H NMR (CDC₁₃): 11.12 (s, 1H), 6.68~6.64 (m, 2H), 5.18~5.13 (m, 2H), 2.97 ~2.92 (m, 2H). ESI MS m/z ([M+Na]⁺): 188.04.

### Example 104 Synthesis of compound 96

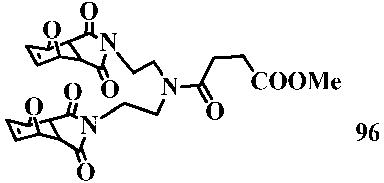

To a solution of compound **94** (freshly prepared, 90% pure, 8.5 g, about 20 mmol) in DMA (350 mL) were added compound 95 (10.2 g, 61.8 mmol), sodium carbonate (8.0 g, 75.5 mmol) and sodium iodide (0.3 g, 2.0 mmol). The mixture was stirred at room temperature overnight, concentrated, diluted with ethyl acetate (350 mL), washed with saturated NaHCO₃ solution (300 mL), saturated NaCl solution (300 mL), and 1 M NaH₂PO₄ (300 mL). The organic layer was dried over Na₂SO₄, filtered, concentrated, loaded on a silica gel column, eluted with 10-30% ethyl acetate/hexane to give the title compound (7.9 g, 77% yield). ESI MS m/z ([M + Na]⁺): 536.4.

### Example 105 Synthesis of compound 97

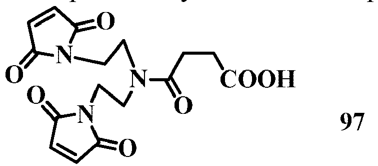

Compound **96** (3.0 g, 5.8 mmol) and trimethylstannane (4.8 g, 26.4 mmol) in 1,2-dichloroethane (150 mL) were refluxed at 80°C for 8 hours, and then cooled to room temperature, loaded on a short silica gel column, eluted with dichloromethane/methanol to remove excess trimethylammonium hydroxide. The fractions were combined, concentrated, diluted with DMA and toluene, and then heated to 120°C and stirred overnight. The reaction mixture was loaded on a silica gel column and eluted with 5-10% methanol/dichloromethane to give the title compound (1.62 g, 76% yield). ESI MS m/z ([M + Na]⁺) :386.2.

### Example 106 Synthesis of compound 98

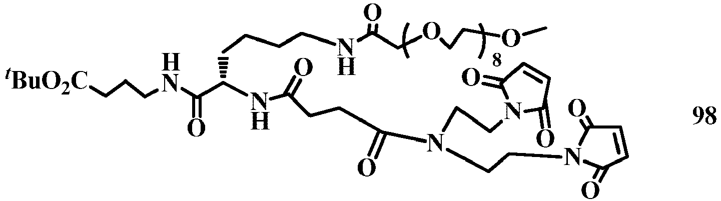

To a solution of compound **97** (1.62 g, 4.20 mmol) and compound **85** (2.71 g, 3.82 mmol) in DMA (201 g, 3.82 mmol) was added EDC·HCl (0.81 g,4.20 mmol). The reaction was stirred at room temperature overnight, then poured into water (50 mL) and extracted with ethyl acetate (3 × 40 mL). The combined organic phase was washed with brine (40 mL), dried over anhydrous Na₂SO₄, filtered and concentrated. The residue was purified by column chromatography (10-50% ethyl acetate/petroleum ether) to give a colorless oil (3.20 g, 80% yield). ESI MS m/z ([M+H]⁺) :1057.85.

### Example 107 Synthesis of compound 99

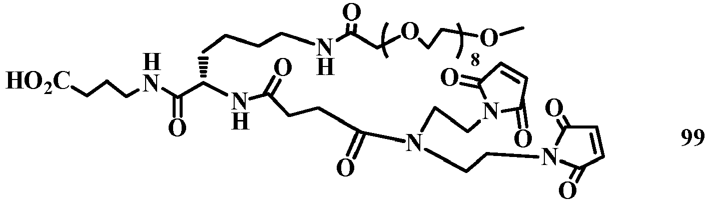

A solution of compound 98 (3.20 g, 3.03 mmol) in formic acid (10 mL) was stirred overnight at room temperature, and then co-evaported with toluene for three times to give a colorless oil (3.00 g, crude), which can be used without further purification. ESI MS m/z ([M+H]⁺) : 1001.50.

### Example 108 Synthesis of compound 100

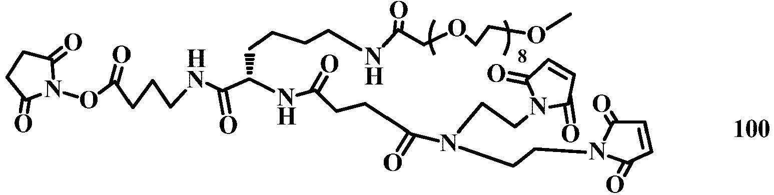

To a solution of compound **99** (3.00 g, crude product, 3.03 mmol) in DMA (15.0mL) were added NHS (0.38 g, 3.33 mmol) and EDC (0.87 g, 4.55 mmol). The reaction mixture was stirred at room temperature for 2 hours and then diluted with water (50 mL) and extracted with ethyl acetate (3 × 30 mL). The combined organic phase was washed with brine (30 mL), dried over anhydrous Na₂SO₄, filtered and concentrated. The residue was purified by silica gel column (10-50% ethyl acetate/petroleum ether) to give a colorless oil (2.90 g, 90% yield). ESI MS m/z ([M+H]⁺): 1098.50.

### Example 109 Synthesis of compound 101

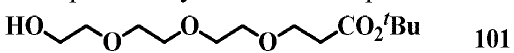

To a solution of 2,2'-(ethane-1,2-diylbis(oxy))diethanol (55.0 mL, 410.75 mmol, 3.0 eq.) in anhydrous THF (200 mL) was added sodium pieces (0.1 g). The mixture was stirred until sodium disappeared and then tert-butyl acrylate (20.0 mL, 137.79 mmol, 1.0 eq.) was added dropwise. The mixture was stirred overnight and then quenched by HCl solution (20.0 mL, IN) at 0 °C. THF was removed by rotary evaporation, brine (300 mL) was added and the resulting mixture was extracted with ethyl acetate (3 × 100 mL). The organic layers were washed with brine (3 × 300 mL), dried over anhydrous Na₂SO₄, filtered and concentrated to afford a colorless oil (30.20 g, 79.0% yield), which was used without further purification. MS ESI m/z ([M+H]⁺): 278.17.

### Example 110 Synthesis of compound 102

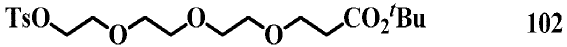

To a solution of tert-butyl 3-(2-(2-(2-hydroxyethoxy) ethoxy) ethoxy) propionate (30.20 g, 108.5 mmol, 1.0 eq.) in the anhydrous dichloromethane (220 mL), were added T_{S}Cl (41.37 g, 217.0 mmol, 2.0 eq.) and TEA (30.0 mL, 217.0 mmol, 2.0 eq.) at 0°C. The mixture was stirred overnight at room temperature and then washed with water (3 × 300 mL) and brine (300 mL), dried over anhydrous Na₂SO₄, filtered, concentrated and purified by silica gel column chromatography (3: 1 hexanes/ethyl acetate) to afford a colorless oil (39.4 g, yield 84.0%). MS ESI m/z ([M+H]⁺):433.28.

### Example 111 Synthesis of compound 103

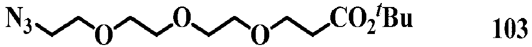

To a solution of *tert*-butyl 3-(2-(2-(2-(toluenesulfonyloxy) ethoxy) ethoxy) ethoxy) propionate (39.4 g, 91.1 mmol, 1.0 eq.) in DMF (100 mL) was added NaN₃ (20.67 g, 316.6 mmol, 3.5 eq.). The mixture was stirred at room temperature overnight, diluted with water (500 mL) and extracted with ethyl acetate (3 × 300 mL). The combined organic layers were washed with water (3 × 900 mL) and brine (900 mL), dried over anhydrous Na₂SO₄, filtered, concentrated and purified by silica gel column chromatography (5: 1 hexanes/ethyl acetate) to afford a colorless yellow oil (23.8 g, 85.53% yield). MS ESI m/z ([M + Na]⁺) :326.2.

### Example 112 Synthesis of compound 104

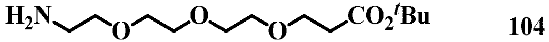

Raney-Ni (7.5 g, suspended in water) was washed with water (three times) and isopropanol (three times) and mixed with compound **103** (5.0 g, 16.5 mmol) in isopropanol. The mixture was stirred under a H₂ balloon at room temperature for 16 hours, then filtered over a Celite pad, washed with isopropyl alcohol and the filtrate was concentrated, and purified by column chromatography (5-25% methanol/dichloromethane) to give a light yellow oil (2.60 g, 57% yield). MS ESI m/z ([M+H]⁺) :279.19.

### Example 113 Synthesis of compound 105

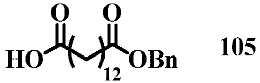

To a solution of tetradecanedioic acid (2.06 g, 8 mmol) in DMF (30 mL) was added K₂CO₃ (1.1 g, 8 mmol) and benzyl bromide (1.36 g, 8 mmol). The mixture was stirred at room temperature overnight and then concentrated and purified by column chromatography (ethyl acetate/petroleum ether) to give the title compound **105** (1.2 g, 45% yield). ESI MS m/z ([M+H]⁺):349.23.

### Example 114 Synthesis of compound 106

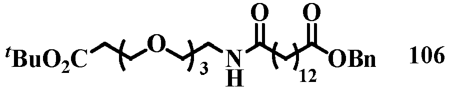

To a solution of compound **104** (2.60 g, 9.35 mmol) and compound **105** (3.91 g, 11.2 mmol) in dichloromethane (50 mL) were added EDC·HCl (2.15 g, 11.2 mmol) and DIPEA (3.6 mL, 20.6 mmol). The reaction mixture was stirred at room temperature for 1 hour and then diluted with 50 mL dichloromethane and poured into a separatory funnel containing 50 mL of water. The organic phase was separated, washed with brine (50 mL), dried over anhydrous Na₂SO₄, filtered and concentrated. The residue was purified by column chromatography (0-10% methanol/dichloromethane) to give the title compound (4.94 g, 87% yield). ESI m/z ([M+H]⁺):608.40.

### Example 115 Synthesis of compound 107

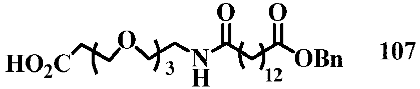

To a solution of compound **106** (4.94 g, 8.14 mmol) in dichloromethane (20 mL) was added TFA (20 mL). The reaction was stirred at room temperature for 1 hour, then concentrated to dryness and co-evaporated with dichloromethane twice, and the residue was placed on a vacuum pump to give compound **107** (4.50 g, crude product). ESI MS m/z ([M+H]⁺) :552.35.

### Example 116 Synthesis of compound 108

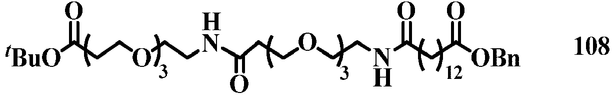

To a solution of compound **107** (4.50 g, crude product, 8.14 mmol) and compound **104** (1.95 g, 7.00 mmol) in dichloromethane (50 mL) were added EDC·HCl (1.56 g, 8.14 mmol) and DIPEA (2.7 mL, 15.4 mmol). The reaction mixture was stirred at room temperature for 1 hour and then diluted with 50 mL dichloromethane and poured into a separatory funnel containing 50 mL of water. The organic phase was separated, washed with brine (50 mL), dried over anhydrous Na₂SO₄, filtered and concentrated. The residue was purified by column chromatography (0-10% methanol/dichloromethane) to give the title compound **108** (5.22 g, 92% yield). ESI m/z ([M+H]⁺): 811.52.

### Example 117 Synthesis of compound 109

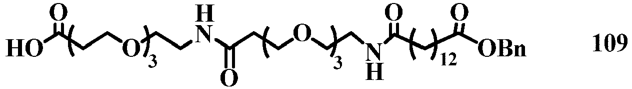

To a solution of compound **108** (5.22 g, 6.44 mmol) in dichloromethane (20 mL) was added TFA (5 mL). The reaction was stirred at room temperature for 1 hour, then concentrated to dryness and co-evaporated with dichloromethane twice, and the residue was placed on a vacuum pump to give compound **109** (4.90 g, crude). ESI MS m/z ([M+H]⁺) 755.46.

### Example 118 Synthesis of compound 110

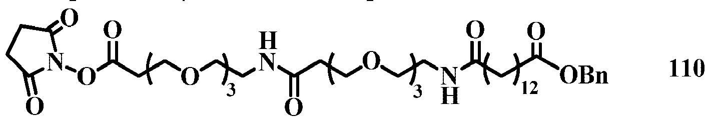

To a solution of compound **109** (4.90 g, crude product, 6.44 mmol) in dichloromethane (30M) were added NHS (0.81 g, 7.08 mmol), EDC·HCl (1.85 g, 9.66 mmol) and DIPEA (2.8 mL, 16.1 mmol). The reaction mixture was stirred at room temperature for 2 hours, then diluted with water (50 mL) and extracted with ethyl acetate (3 × 30 mL). The combined organic phase was washed with brine (30 mL), dried over anhydrous Na₂SO₄, filtered and concentrated. The residue was purified by silica gel column (10-50% ethyl acetate/petroleum ether) to give a colorless oil **110** (4.90 g, 90% yield). ESI MS m/z ([M+H]⁺) :852.48.

### Example 119 Synthesis of compound 111

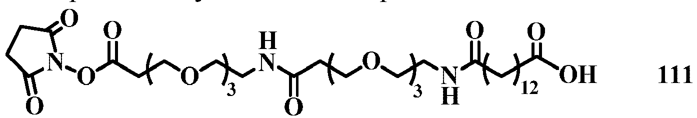

In a hydrogenated bottle, Pd/C (10 wt %, 0.20 g) was added to a solution of compound **110** (4.90 g, 5.75 mmol) in tetrahydrofuran (20 mL). The mixture was stirred overnight under H₂ (1 atm), filtered through Celite (filter aid) and the filterate was concentrated to give compound **111** (4.50 g, > 100% yield). ESI MS m/z ([M+H]⁺):762.44.

### Example 120 Synthesis of compound 112

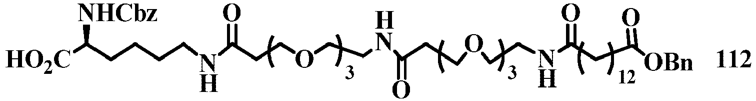

To a solution of compound **111** (1.00 g, 1.32 mmol) in dichloromethane (10 mL) were added HATU (0.50 g, 1.32 mmol) and triethylamine (0.06 mL, 1.32 mmol) at 0°C. The reaction was stirred at 0°C for 30 minutes, then Z-Lys-OH (0.40 g, 1.43 mmol) was added, stirred at room temperature for 1 hour. The reaction mixture was diluted with water (20 mL) and extracted with ethyl acetate (3 × 20 mL). The combined organic phase was washed with brine (30 mL), dried over anhydrous Na₂SO₄, filtered and concentrated. The residue was purified by silica gel column (0-10% methanol/dichloromethane) to give a colorless oil **112** (1.28 g, 95% yield). ESI MS m/z ([M+H]⁺):1017.60.

### Example 121 Synthesis of compound 113

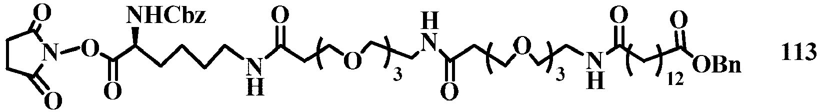

To a solution of compound **112** (1.28 g, 1.26 mmol) in dichloromethane (10 mL) were added NHS (0.17 g, 1.51 mmol) and EDC·HCl (0.29 g, 1.51 mmol), followed by triethylamine (0.38 mL, 2.77 mmol). The reaction was stirred at room temperature for 2 hours and then diluted with water (20 mL) and extracted with ethyl acetate (3 × 15 mL). The combined organic phase was washed with brine (30 mL), dried over anhydrous Na₂SO₄, filtered and concentrated. The residue was purified by silica gel column (0-10% MeOH/dichloromethane ) to give a colorless oil **113** (1.28 g, 91% yield). ESI MS m/z([M+H]⁺):1114.62.

### Example 122 Synthesis of compound 114

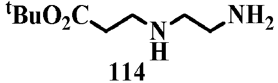

A solution of *tert*-butyl acrylate (12.81 g, 0.10 mmol) and ethyl -1,2-diamine (24.3 g, 0.40 mol) in tetrahydrofuran (150 mL) was stirred at 45°C for 24 hours. The mixture was concentrated and purified on an Al₂O₃ gel column and eluted with methanol/dichloromethane (triethylamine) (5% -15% - 80%) to give the title compound (17.50 g, 92% yield). ESI MS m/z ([M+H]⁺) : 189.20.

### Example 123 Synthesis of compound 115

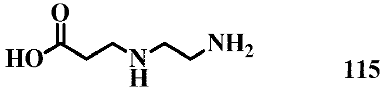

A mixture of *tert*-butyl 3-((2-aminoethyl) amino) propionate (17.00 g, 90.33 mmol) in 1,4-dioxane and concentrated HCl (15 mL) was stirred at room temperature for 30 minutes, then concentrated and diluted with pure water (150 mL) and ethyl acetate/hexane (40 mL, 1: 5). The mixture was separated and the organic layer was extracted with water (2 × 10 mL). The aqueous layer was concentrated and dried on a vacuum pump to give the title compound (18.70 g, 100% yield, 96% pure by LC-MS). ESI MS m/z ([M+H]⁺): 133.20.

### Example 124 Synthesis of compound 116

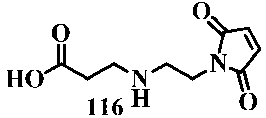

To a solution of 3-((2-aminoethyl) amino) propionic acid (18.70 g, 90.33 mmol) in tetrahydrofuran (150 mL) at 0°C was added maleic anhydride (8.85 g, 90.33 mmol). The mixture was stirred at 0-4°C for 4 hours, concentrated to give (Z)-4-(2-((2-carboxyethyl) amino) ethyl) amino)-4-oxo-2-enoic acid, to which toluene (150 mL) and DMA (50 mL) were added. The mixture was then stirred at 90°C and refluxed under a Dean-Stark trap. After 30 mL of solvent was collected in the trap, HMDS (hexamethyldisilazane, 9.0 mL, 43. 15 mmol) and ZnCl₂ (16 mL, 1.0 M ether solution) were added, and the mixture was further heated to 115-125°C and toluene was collected by Dean-Stark trap. The reaction mixture was heated at 120°C for 6 hours and 2 × 40 mL of anhydrous toluene was added to maintain the volume of about 50 mL. The reaction mixture was then cooled and 1 mL of 1: 10 concentrated HCl/MeOH was added. The mixture was concentrated, purified on a silica gel column, eluted with water/acetonitrile (1: 15). The fractions were concentrated and dried under a vacuum pump to give 14.75 g of the title compound (77.0% yield). ESI MS m/z ([M+H]⁺): 213.10.

### Example 125 Synthesis of compound 117

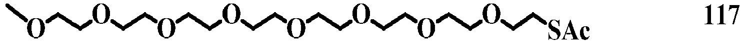

To a mixture of tetrahydrofuran (300 mL), DIPEA (50 mL) and HSAc (10.0 g, 0.131 mol) was added compound **60** (57.30 g, 0.106 mol). The mixture was stirred overnight, concentrated and purified on a silica gel column, eluted with ethyl acetate/MeOH (1: 2 to 4: 1), concentrated, and dried under a vacuum pump to give the title compound (40.51 g, 86% yield). ESI MS m/z ([M+H]⁺):443.35.

### Example 126 Synthesis of compound 118

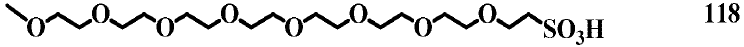

Compound **117** (40.40 g, 0.091 mol) was added to a mixture of acetic acid (200 mL) and 30% H₂O₂ (100 mL). The reaction mixture was stirred at 35°C overnight and then concentrated, diluted with pure water (200 mL) and toluene (150 mL). After phase separation, the organic layer was extracted with water (2 × 25 mL). The aqueous solutions were combined, concentrated and dried over a vacuum pump to give a title compound 40.50 g (99% yield, 95% pure by LC-MS). ESI MS m/z ([M + H] ⁺) : 449.30.

### Example 127 Synthesis of compound 119

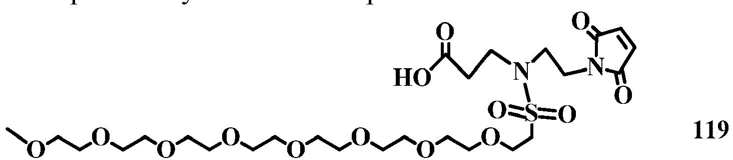

To a mixture of the compound **118** (20.0 g, 44.62 mmol) in tetrahydrofuran (100 mL) and dichloromethane (100 mL) were added oxalyl chloride (25.21 g, 200.19 mmol) and DMF (0.0151 mmol) in sequence. The mixture was stirred at room temperature for 2 hours, concentrated, co-evaporated with dichloromethane/toluene (1: 1, 2 × 50 mL) and then dissolved in tetrahydrofuran (50 mL). Compound **116** (7.50 g, 35.36 mmol) in tetrahydrofuran (100 mL) was added and the mixture was stirred overnight, concentrated in vacuo and purified on a silica gel column, eluted with MeOH/dichloromethane (1: 6 to 1: 5), concentrated and dried under a vacuum pump to give the title compound (14.76 g, 65% yield). ESI MS m/z ([M+H]⁺):643.35.

### Example 128 Synthesis of compound 120

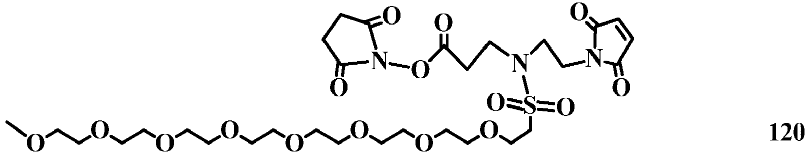

A solution of compound **119** (7.50 g, 11.67 mmol), N-hydroxysuccinimide (1.50 g, 13.04 mmol) and EDC (10.10 g, 52.60 mmol) in tetrahydrofuran (100 mL) was stirred overnight, concentrated in vacuo and purified on a silica gel column, eluted with ethyl acetate/dichloromethane (1: 4 to 2: 1), concentrated and dried under a vacuum pump to give the title compound (6.30 g, 73% yield). ESI MS m/z ([M+H]⁺):740.40.

### Example 129 Synthesis of compound 121

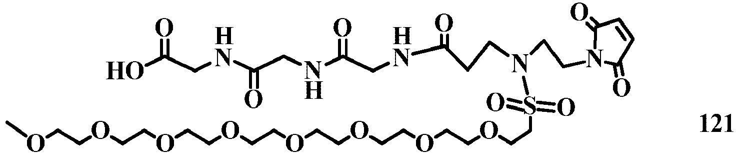

To a solution of 2-(2-(2-(2-aminoacetamido) acetamido) acetamido) acetic acid (Gly-Gly-Gly) (0.50 g, 2.03 mmol) and compound **120** (1.65 g, 2.22 mmol) in DMF (15 mL) was added DIPEA at 0°C. The reaction mixture was stirred at 0°C for 0.5 hours and then room temperature for 4 hours. The reaction mixture was then concentrated and purified by silica gel chromatography (mobile phase: acetonitrile/water = 95: 5, containing 0.1% formic acid) to give the title compound **121** (1.04 g, 63% yield). MS-ESI m/z calcd for C₃₂H₅₆N₅O₁₇S [M+H]⁺ 14.33,found 814.46.

### Example 130 Synthesis of compound 122

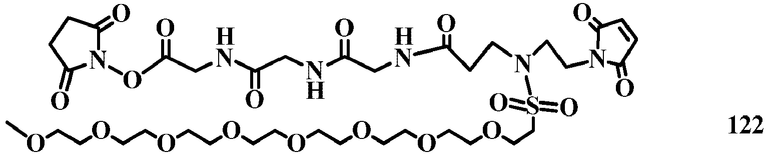

Compound **121** (0.70 g, 0.86 mmol), N-hydroxysuccinimide (0.20 g, 1.73 mmol) and EDC (1.21 g, 6.36 mmol) in tetrahydrofuran (201 mL) were stirred at room temperature overnight, concentrated in vacuo and purified on a silica gel column, and then eluted with ethyl acetate/dichloromethane (1: 4 to 2: 1) and dried under a vacuum pump to afford 0.540 g (69% yield) of the title compound. MS-ESI m/z calcd for C₃₆H₅₉N₆O19S[M+H]⁺:911.34,found 911.42.

### Example 131 Synthesis of compound 123

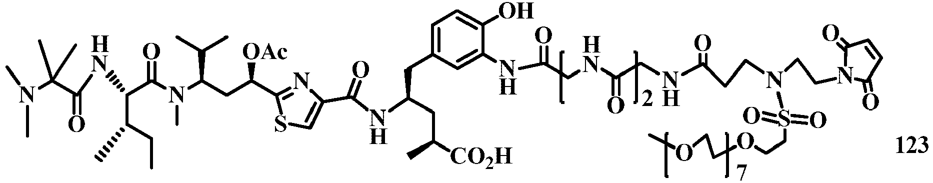

To a solution of (2S,4R)-5-(3-amino-4-hydroxyphenyl)-4-(2-((6S,9R, 11R)-6-((S)-sec-butyl)-9-isopropyl-solution -2,3,3,8-tetramethyl -4,7,13-trioxy -12-oxa -2,5,8-triaza-undecane-11-yl) thiazole-4-formamido)-2-methylpentanoic acid (**Tub-039**, R Zhao, et al. PCT CN 2017/120454; R Zhao, et al. 14th PEGS Boston, Boston, Mass. USA, 3 rd May 2018) (83 mg, 0.106 mmol) and compound **122** (122 mg, 0.134 mmol) in DMF (82 mg) was added DIPEA (2 mL). The reaction mixture was stirred at 0°C for 0.5 hours and then room temperature for 4 hours, then concentrated and purified by preparative HPLC (mobile phase: acetonitrile/water = 10% to 80%, containing 0.1% formic acid) to give compound **123** (95.5 mg, 58% yield). MS-ESI m/z: [M+H]⁺ calcd for C₆₉H₁₁₂N₁₁O₂₄S, 1542.72; found, 1542.76.

### Example 132 Synthesis of compound 124

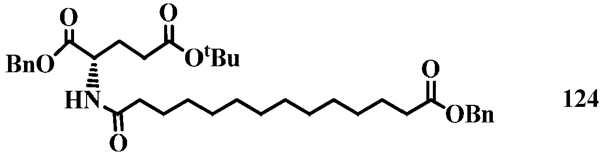

A solution of (S)-1-benzyl 5-*tert*-butyl 2-aminopentanedioate hydrochloride salt (8.70 g, 26.39 mmol), 14-(benzyloxy)-14-oxotetradecanoic acid (9.19 mmol), DIPEA (8.0 ml, 46.0 mmol) and EDC (15.3 g, 80.50 mmol) in CH₂Cl₂ (200 mL) was stirred at room temperature for 6 hours, and then diluted with water (100 mL) and phases were sepearated. The aqueous phase was extracted with CH₂Cl₂ (100 mL). The organic phases were combined, washed with brine, dried over Na₂SO₄, filtered, concentrated and purified on a silica gel column (dichloromethane/ethyl acetate = 20:1 to 5:1) to give the title compound **314** (13.65 g, 83% yield). MS ESI m/z: [M+H]⁺ calcd for C₃₇H₅₄NO₇, 624.38; found, 624.38.

### Example 133 Synthesis of compound 125

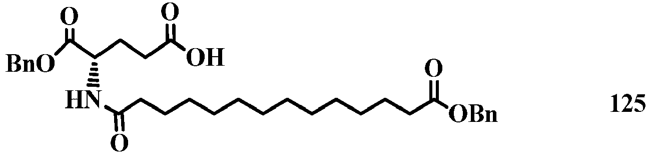

Compound **124** (12.50 g, 20.05 mmol) was dissolved in dioxane (30 mL) at 4 °C, and treated with hydrochloric acid (10 mL, 36% conc) for 0.5 hours. The reaction mixture was diluted with toluene (20 ml) and DMF (20 ml), concentrated at 15 °C to give the title compound (11.26 g, 99% yield). MS-ESI m/z: [M+H]⁺ calcd for C₃₃H₄₆NO₇, 568.32; found, 568.34.

### Example 134 Synthesis of compound 126

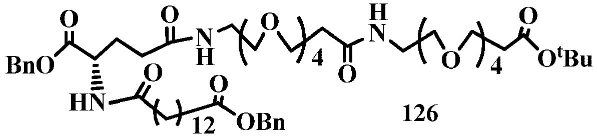

A mixture of compound **215** (10.70 g, 18.86 mmol), *tert*-butyl 1-amino-15-oxo-3,6,9,12,19,22,25,28-octaoxa-16-azahentriacontan-31-oate hydrochloride salt (11.45 g, 18.93 mmol), EDC (9.51 g, 50.01 mmol) and DIPEA (4.00 ml, 23.00 mol) in CH₂Cl₂ (200 ml) was stirred overnight, diluted with brine (100 ml) and phases were separated. The aqueous phase was extracted with CH₂Cl₂ (100 ml). The organic phases were combined, washed with brine, dried over Na₂SO₄, filtered, concentrated and purified on a silica gel column (dichloromethane/ethyl acetate = 10:1 to 4:1) to give the title compound **316** (18.15 g, 86% yield). MS-ESI m/z: [M+H]⁺ calcd for C₅₉H₉₆N₃O₁₇, 1118.67; found, 1118.80.

### Example 135 Synthesis of compound 127

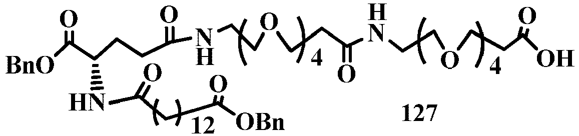

Compound **126** (10.50 g, 9.39 mmol) was dissolved in dioxane (45 mL) at 4 °C, and treated with hydrochloric acid (15 mL, 36% conc) for 0.5 hours. The reaction mixture was diluted with toluene (20 ml) and DMF (20 ml), concentrated at 15 °C and purified on a silica gel column (dichloromethane/MeOH= 10:1 to 6:1) to give the title compound (8.67 g, 87% yield). MS-ESI m/z: [M+H]⁺ calcd for C₅₅H₈₈N₃O₁₇, 1062.60; found, 1062.68.

### Example 136 Synthesis of compound 128

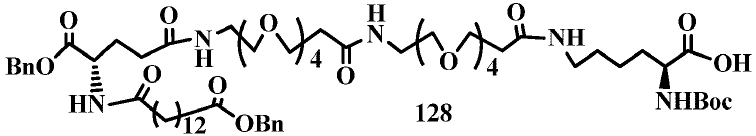

A solution of compound **127** (8.50 g, 8.01 mmol), N-hydroxysuccinimide (3.20 g, 27.82 mmol), EDC (10.28 g, 54.10 mmol) and DIPEA (6.00 mL, 34.51 mmol) in THF (150 ml) was stirred for 6 h and evaporated in vacuo to give a crude N-succinimidyl ester, which was used in next step without purification. To a solution of (S)-6-amino-2-((*tert*-butoxycarbonyl)amino)hexanoic acid hydrochloride salt (2.75 g, 9.73 mmol) in DMF (100 mL) and 1.0 M Na₂PO₄ (pH 7.5, 55 mL), and the above prepared N-succinimidyl ester was added in four portions in 1 h. The reaction mixture was stirred at room temperature for 3 hours. After concentration, the residue was purified on a silica gel column (dichloromethane/MeOH = 10:1 to 4:1) to give the title compound (8.16 g, 79% yield). MS-ESI m/z: [M+H]⁺ calcd for C₆₆H₁₀₈N₅O₂₀, 1289.75; found, 1289.90.

### Example 137 Synthesis of compound 129

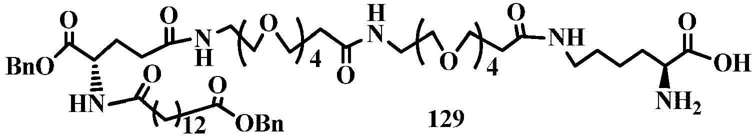

Compound **128** (8.10 g, 6.28 mmol) was dissolved in dioxane (40 mL) at 4 °C, and treated with hydrochloric acid (15 mL, 36% conc) for 0.5 hours. The reaction mixture was diluted with toluene (20 ml) and DMF (20 ml), concentrated at 15 °C to give the crude title compound (7.71 g, 100% yield), which was used in the next step without further purification. MS-ESI m/z: [M+H]⁺ calcd for C₆₁H₈₈N₃O₁₇, 1190.70; found, 1190.78.

### Example 138 Synthesis of compound 130

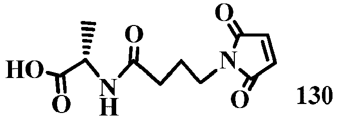

To a solution of 4-maleimide-butyric acid-N-succinamide ester (7.10 g, 25.35 mmol) and alanine (3.01 g, 33.80 mmol) in DMF (50 mL) at 0 °C, DIPEA (10 mL) was added. The reaction mixture was stirred at 0 °C for 0.5 h, and at room temperature for 1 h, concentrated and then purified on SiO₂ column (mobile phase: dichloromethane /MeOH = 10:1 with 0.1% formic acid) to afford compound **130** (5.21 g, 81% yield). MS-ESI m/z: [M+H]⁺ calcd for C₁₁H₁₄N₂O₅, 255.09; found, 255.15.

### Example 139 Synthesis of compound 131

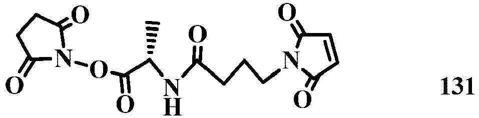

A solution of compound **130** (5.15 g, 20.26 mmol), N-hydroxysuccinimide (2.80 g, 24.34 mmol), EDC (10.28 g, 54.10 mmol) and DIPEA (5.50 ml, 31.63 mmol) in dichloromethane (70 mL) was stirred for 6 h, concentrated in vacuo and purified on SiO₂ column (mobile phase: dichloromethane /ethyl acetate = 10:1) to afford compound **131** (5.83 g, 82% yield). MS-ESI m/z: [M+H]⁺ calcd for C₁₅H₁₇N₃O₇, 351.11; found, 351.20.

### Example 140 Synthesis of compound 132

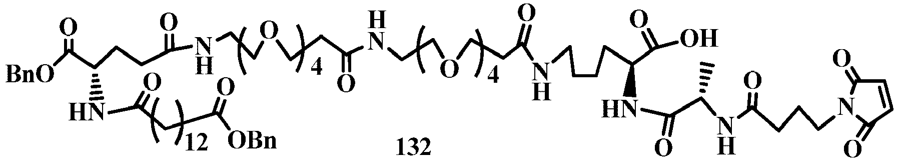

To a solution of compound **129** (7.61 g, 6.39 mmol) and compound **131** (2.90 g, 8.280 mmol) in DMF (40 mL) at 0 °C, DIPEA (7 mL) was added. The reaction mixture was stirred at 0 °C for 0.5 h, at room temperature for 1 h, concentrated and purified on SiO₂ column (mobile phase: dichloromethane /MeOH = 10:1 with 0.1% formic acid) to afford compound **132** (7.10 g, 78% yield). MS-ESI m/z: [M+H]⁺ calcd for C₇₂H₁₁₂N₇O₂₂, 1426.7782; found, 1426.7820.

### Example 141 Synthesis of compound 133

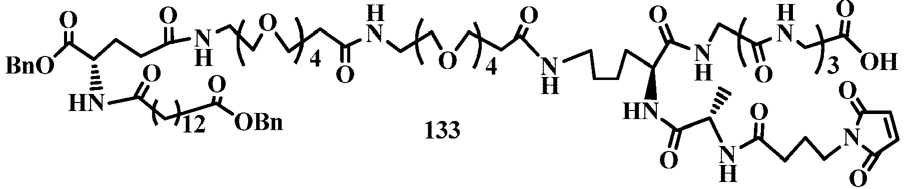

A solution of compound **132** (7.05 g, 4.94 mmol), N-hydroxysuccinimide (0.92 g, 8.00 mmol), EDC (3.01 g, 15.84 mmol) and DIPEA (1.00 ml, 5.75 mmol) in THF (50 ml) was stirred for 6 h and evaporated in vacuo to give a NHS ester, which is used in the next step without purification.

To a solution of 2-(2-(2-aminoacetamido)acetamido)acetic acid (gly-gly-gly) hydrochloride salt (1.67 g, 7.40 mmol) in DMF (40 mL) and 1.0 M Na₂PO₄ (pH 7.5, 15 mL), the above compound was added in four portions in 1 h. The reaction mixture was stirred at room temperature for 3 hours. After concentration, the residue was purified on a silica gel column (dichloromethane /MeOH = 10:1 to 7:1) to give the title compound (8.16 g, 79% yield). MS-ESI m/z: [M+H]⁺ calcd for C₇₈H₁₂₁N₁₀O₂₅, 1597.8426; found, 1597.8495.

### Example 142 Synthesis of compound 134

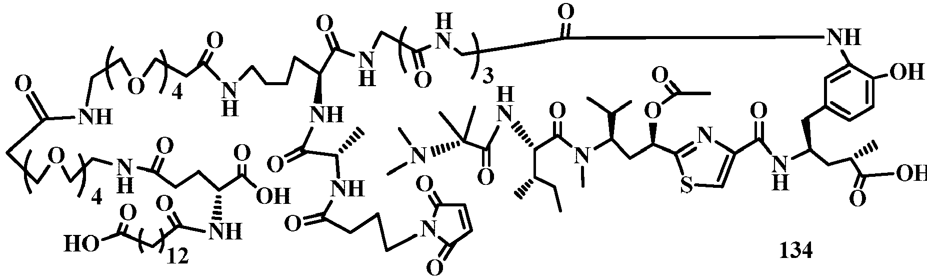

To a solution of compound **133** (150.3 mg, 0.0935 mmol), **Tub-039** (60.2 mg, 0.0769 mmol), and DIPEA (0.030 ml, 0.172 mmol) in DMA (5 ml), EDC (100 mg, 0.526 mmol) was added. The reaction mixture was stirred at room temperature for 6 h, concentrated in vacuo, re-dissolved in MeOH/dichloromethane (0.5 ml: 3 ml) and passed through a short silica gel column with elution of MeOH/ dichloromethane (1:3), concentrated in vacuo to afford a crude compound for next step. MS-ESI m/z: 2326.25.

The above crude compound was dissolved in dichloromethane (1 mL) and treated with TFA (3 mL) for 1 hour, diluted with toluene (3 mL) and DMF (3 mL), concentrated, and purified by prep-HPLC (mobile phase: 2% to 50% of acetonitrile in water, containing 0.1% formic acid) to afford compound **322** (69.0 mg, 72% yield). MS-ESI m/z: [M+H]⁺ calcd for C₈₈H₁₂₈FN₁₃O₂₈, 2146.1497; found, 2146.1588.

### Example 143 Synthesis of compound 135

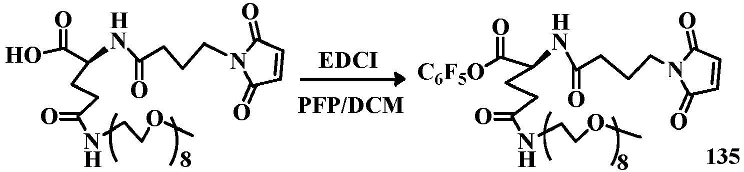

To a solution of (S)-30-(4-(2,5-dioxo-2,5-dihydro-1H-pyrrol-1-yl) butanamido)-27-oxo-2,5,8,11,14,17,20,23-octaoxa-26-azahentriacontan-31-oic acid (20 mg, 0.029 mmol) in dichloromethane (5 mL), were added EDC (11 mg, 0.059 mmol) and pentafluorophenol (10.8 mg, 0.059 mmol). The reaction mixture was stirred at room temperature for 2 hours, concentrated and purified on SiO₂ column with elution of ethyl acetate/dichloromethane (1:4) to give the title compound **246** (24 mg, 100% yield). MS-ESI m/z: [M+H]⁺ calcd for C₃₆H₅₀F₅N₃O₁₄, 844.32; found, 844.32.

### Example 144 Synthesis of compound 136

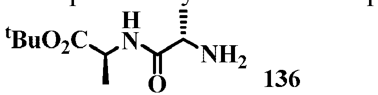

A solution of (S)-tert-butyl 2-((S)-2-(((benzyloxy)carbonyl)amino) propanamido)propanoate (10 g, 0.028 mol) in methanol (100 mL) and 10% Pd/C (1.0 g) were stirred under H₂ (5 psi) for 3 hours. The solid was filtered off and the filtrate was concentrated to give a colorless oil (6.1 g, 100% yield). ESI m/z: [M+H]⁺ calcd for C₁₀H₂₀N₂O₃, 217.15; found, 217.15.

### Example 145 Synthesis of compound 137

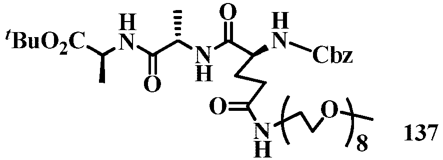

To a solution of (S)-30-(((benzyloxy)carbonyl)amino)-27-oxo-2,5,8,11,14,17,20,23-octaoxa-26-azahentriacontan-31-oic acid (**250)** (100 mg, 0.154 mmol) in dichloromethane (5 mL), EDC (59 mg, 0.309 mmol) and pentafluorophenol (PFP) (57 mg, 0.309 mmol) were added. The mixture was stirred at room temperature for 2 hours, diluted with dichloromethane (20 mL), washed with water (5 mL), dried over Na₂SO₄, filtered, and concentrated. The residue was re-dissolved in DMF (5 mL), after addition of compound **136** (49 mg, 0.23 mmol) and DIPEA (90 mg, 0.69 mmol), was stirred at room temperature for 1 hour, concentrated, and purified on a short SiO₂ column with elution of MeOH/CH₂Cl₂ (1:10) to give the title compound **137** (80 mg, 61% yield). ESI m/z: [M+H] ⁺ calcd for C₄₀H₆₈N₄O₁₅, 845.47; found, 845.47.

### Example 146 Synthesis of compound 138

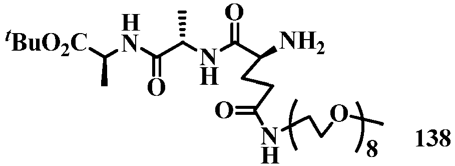

A solution of compound **137** (80 mg, 0.094 mmol) in methanol (5 mL) and 10% palladium carbon (10 mg) were stirred under H₂ (5 psi) for 2 hours. The solid was filtered off and filtrate was concentrated to give a colorless oil (66 mg, 100% yield) for the next step without further purification. MS-ESI m/z: [M+H]⁺ calcd for C₃₂H₆₂N₄O₁₃, 711.43; found, 711.43.

### Example 147 Synthesis of compound 139

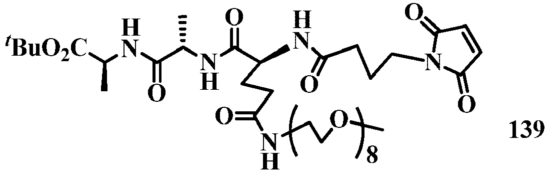

To a solution of compound **138** (66 mg, 0.094 mmol) in ethanol (5 mL), 2,5-dioxopyrrolidin-1-yl 4-(2,5-dioxo-2,5-dihydro-1H-pyrrol-1-yl)butanoate (39 mg, 0.141 mmol) and PBS (0.1 M, pH 7.5, 1.0 mL) were added. The reaction mixture was stirred overnight, concentrated and purified on a silica gel column (dichloromethane/MeOH = 100:0 to 10:1) to afford the title compound **139** (37 mg, 45% yield). ESI m/z: [M+H]⁺ calcd for C₄₀H₆₉N₅O₁₆, 876.47; found, 876.47.

### Example 148 Synthesis of compound 140

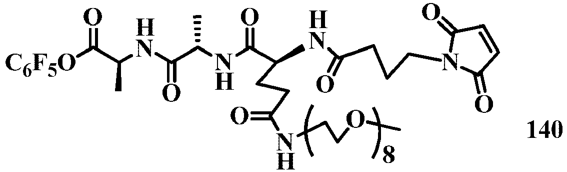

Compound **139** (50 mg, 0.057 mmol) in dichloromethane (3 mL) was treated with TFA (1 mL) at room temperature for 2 hours. The reaction mixture was evaporated to dryness and then re-dissolved in dichloromethane (5 mL), to which EDC (16 mg, 0.084 mmol) and pentafluorophenol (15 mg, 0.084 mmol) were added. The mixture was stirred at room temperature for 4 hours, concentrated, and purified on a silica gel column (dichloromethane/ethyl acetate = 100:10 to 3:1) to give the title compound **140** (41 mg, 73% yield). ESI m/z: [M+H]⁺ calcd for C₄₂H₆₀F₅N₅O₁₆, 986.40; found, 986.42.

### Example 149 Synthesis of compound 141

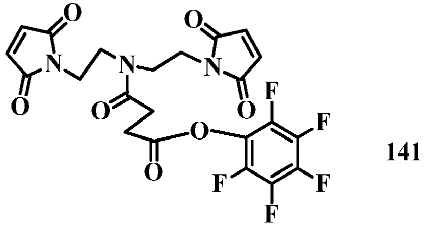

To a solution of 4-(bis(2-(2,5-dioxo-2,5-dihydro-1H-pyrrol-1-yl)ethyl) amino)-4-oxobutanoic acid (100 mg, 0.27 mmol) in dichloromethane (5 mL), EDC (210 mg, 1.10 mmol) and pentafluorophenol (101 mg, 0.55 mmol) were added. The mixture was stirred at room temperature for 3 hours, concentrated and purified on a silica gel column (dichloromethane/ethyl acetate = 20:1 to 5:1) to give the title compound **141** (114 mg, 80% yield). MS-ESI m/z: [M+H]⁺ calcd for C₂₂H₁₆F₅N₃O₇, 530.09; found, 530.09.

### Example 150 General method of conjugating reduced antibody disulfide bond with tubulysin derivatives

To HER2 antibody (2.0 mL, 10 mg/mL, pH of 6.0-8.0 ) were added 0.70 mL -2.0 mL, 100 mM, pH 6.5-8.5 phosphate buffer (PBS) and TCEP (16-20 µL, 20 mM aqueous solution). After incubation at room temperature to 37.5°C for 0.5-4 hours, and the same equivalent of azide compound (azido benzoic acid, or 2-(2-(2-hydroxyethoxy) ethoxy) ethoxy azide) was added at room temperature to 37.5 °C and incubated at room temperature to 37.5 °C for 1-4 hours. A Tubulysin derivative with a thiol-reactive group (28-32 µL, 20 mM DMA solution) (eg, compounds 39, 57, 72, 123 or 134) was added and incubated at room temperature to 37.5°C for 2 to 18 hours, and then DHAA (135µL, 50 mm) was added and incubated at room temperture overnight. The mixture was purified by G-25 column, cation or anion chromatography column, and eluated with pH 6~ 7.5, 10-100 mM phosphoric acid, or citric acid buffer system, with 50-200 mM NaCl to afford the conjugate (75% -99% yield). The mixture can also be purified by diafiltration, using pH 6~ 7.5, 10 -100 mM phosphoric acid, or citric acid buffer system, with 50-200 mM NaCl. The volume was 3-30 times of the sample volume, to yiled the conjugate (75% -99% yield). The drug/antibody ratio (DAR) was 3.1-4.9 as determined by HPLC-MS; HPLC analysis indicated 95-99% monomer (Tosoh Bioscience, Tskgel G3000SW, 7.8 mm × 30 cm, 0.5 ml/min, 100 min). The structures of the prepared conjugates 39, 57, 72, 123, or 134 are as follows:

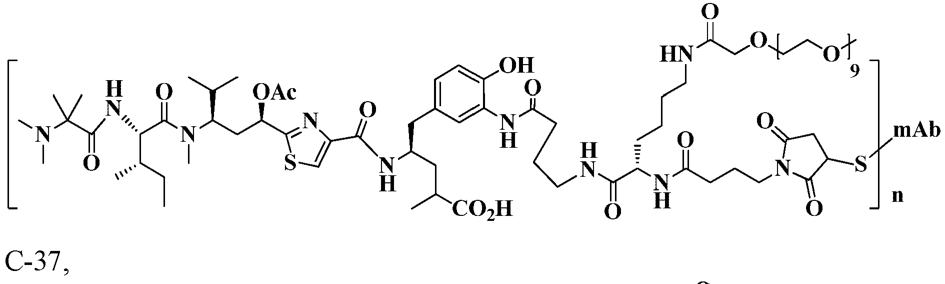

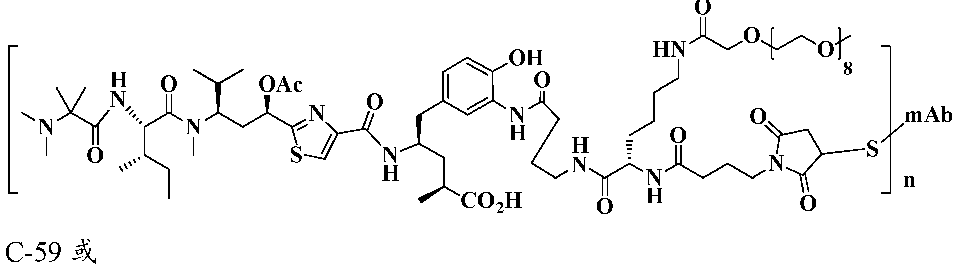

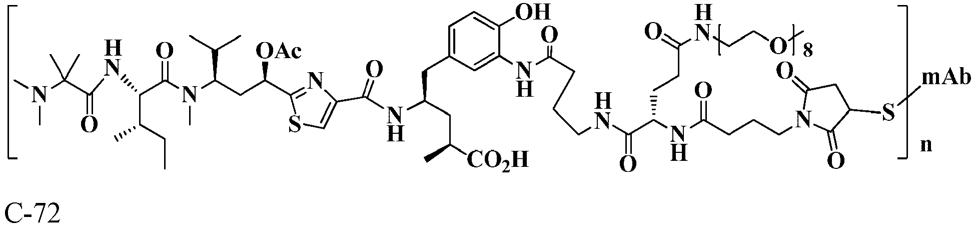

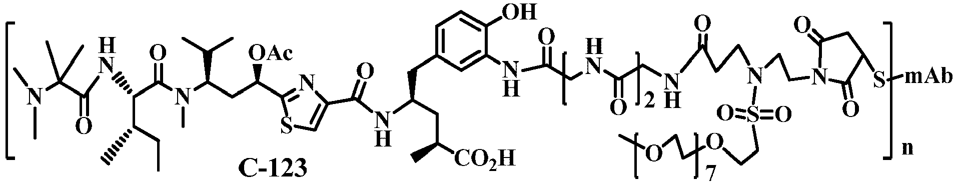

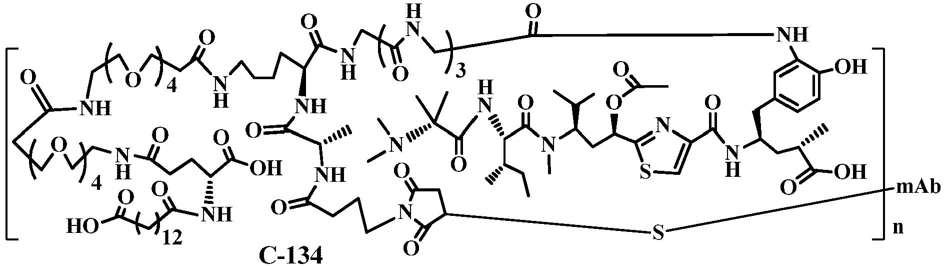

### Example 151. Other method of preparing the conjugates

Cell binding molecules (antibodies) can be conjugated with the compounds of the invention by amide, thioether or disulfide bonds. Antibodies (> 5 mg/mL) were diluted with pH 8.0 PBS buffer with 50 mM sodium metaborate, treated with dithiothreitol (final concentration of 10 mM) at 35°C for 30 minutes, to generate free thiol groups. The mixture was purrfied by G-25 gel filtration chromatography (1 mM EDTA in PBS buffer). About 8 thiol groups per antibody were detected by Ellman's reagent [5,5'- dithiobis (2-nitrobenzoic acid)]. Antibodies may react with Traut's reagent (2-iminothiophene) (Jue, R., et al. Biochem. 1978, 17 (25): 5399-5405) or with SATP (N-succinimido-S-acetylthiopropionate) or N-succinimide-S-acetyl (thiotetraacetic acid) (SAT (PEG) 4) under pH 7-8, to form thiol groups (Duncan, R, et al, Anal. Biochem. 1983, 132, 68-73; Fuji, N. et al, Chem. Pharm. Bull. 1985, 33, 362-367). In general, 5-9 thiol groups can be generated in one antibody molecule.

At 4°C, to a cold dimethylacetamide (DMA) solution of antibody containing free thiol groups (2-20% v/v) was added a drug molecule with maleimide or bromoacetamide group (molar ratio of drug to thiol group was 1.2-1.5: 1)) (the alkylation reaction between antibody and bromoacetamide usually requires 0.5 M of sodium borate solution (pH 9)). After 1-2 hours, excess cysteine was added to quench the reaction; ultrafiltration, gel chromatography (G-25, PBS buffer solution), sterile filtration afforded a concentrated conjugation product. Protein concentration and the number of drug linked to each antibody were determined by measuring absorbance at 280 nm and 252 nm. Molecular exclusion HPLC was used to determine the propotion of the monomer form of the conjugate, and the free drug less than 0.5% was determinded by RP-HPLC. For the monomeric conjugate formed by thioether bond linkage, each antibody molecule are linked with 3.2-4.8 tubulysin derivatives on average.

The types of linker include dimethyl (phenyl) silyl (DMPS), SMDP, 4-succinimidyl-methyl-α(2-pyridyl disulfide) toluene (SMPT), N-succinimidyl-4-(2- pyridyldithio) propionate (SPP), N-succinimidyl-4-(2-pyridyldithio) butyrate (SPDP), N-succinimide-4-(2- pyridyldithio) butyrate (SMCC), N-hydroxysuccinimide-(polyethylene glycol) N-maleimide (SM (PEG) ₙ), and the like. Antibody (> 5 mg/mL) was dissolved in buffer (pH 6.5~7.5, 5 mM PBS, 50 mM NaCl, 1 mM EDTA), reacted with the linker for 2 hours, while the ratio of the linker to the antibody was 6-10 or more. The reaction mixture was purified by Sephadex G25 gel chromatography and lower molecular weight molecules was removed. The mixture can also be purified by a cationic chromatography or an anion chromatography with a buffer solution of pH 6-7.5, 10-100 mM phosphoric acid, or citric acid buffer, 50-200 mM NaCl to produce a conjugate (75% -99% yield). The mixture can also be purified by diafiltration with pH 6-7.5, 10-100 mM phosphoric acid, or citric acid buffer, 50-200 mM NaCl. The volume was 3-30 times of the sample volume, to yiled a conjugate (75% -99% yield). The concentration of the antibody was determined by spectrophotometry, and the linker comprises a pyridyldithiol group. The extinction coefficient of antibody was 2067550 M⁻¹ cm⁻¹ at 280 nm. The modified antibody was treated with excess dithiothreitol (more than 20 equivalences), the extinction coefficients of the released 2-thiopyridyl at 343 and 280 nm were 8080 and 5100 M⁻¹ cm⁻¹, respectively. To a modified antibody, 1.2 to 1.5 eq. of tubulysin derivative molecule with a thiol group was added. After the reaction proceeded at room temperature for 5-18 hours, the reaction mixture was chromatographed on Sephadex G 25 to remove unlinked drugs or other low molecular weight substances. The concentration of the product was then determined by measuring absorbances at 280 nm and 252 nm. The products were in the form of monomer and each antibody molecule was linked to 3.2-4.8 drug molecules.

### Example 152. In vitro cytotoxicity evaluation of Her2 conjugates C-37, C-59, C-72, C-123 and C-134 in comparison withT-DM1

The cell line used in the cytotoxicity assays was NCI-N87, a human gastric carcinoma cell line. Cells grew in RPMI-1640 with 10% FBS. To run the assay, the cells (180 µL, 6000 cells) were added to each well of 96-well plates and incubated for 24 hours at 37°C in 5% CO₂. Next, the cells were treated with test compounds (20 µL) at various concentrations in appropriate cell culture medium (total volume 0.2 mL). The control wells contain cells and the medium but not the test compounds. The plates were incubated for 120 hours at 37°C in 5% CO₂. MTT (5 mg/ml) was then added to the wells (20 µL) and the plates were incubated for 1.5 hr at 37°C. The medium was carefully removed and DMSO (180 µL) was added afterward. After the plates were shaken for 15min, the absorbances were measured at 490 nm and 570 nm with a reference of 620 nm. The inhibition% was calculated according to the following equation: inhibition% = [1-(assay-blank)/(control-blank)] × 100. The results are listed in Table 1.

**Table 1. Cytotoxicity of Her2-Tubulysin analogue conjugate in this invention**

| Conjugate | DAR (Drug/Antidody) | IC₅₀ of NCI-N87 cells (nM) |
|---|---|---|
| **C-37** | 3.9 | 0.22 |
| **C-59** | 4.0 | 0.11 |
| **C-72** | 3.8 | 0.10 |
| **C-123** | 7.8 | 0.15 |
| **C-134** | 3.9 | 0.25 |
| **T-DM1** | 3.5 | 0.32 |

### Example 153 Study of in vivo antitumor activity in BALB/c nude mice bearing NCI-N87 xenograft tumor

The *in vivo* efficacy of conjugates **C-37, C-49, C-72, C-123** and **C-134** along with T-DM1 was evaluated in a human gastric carcinoma N-87 cell line tumor xenograft model. Five-week-old female BALB/c nude mice (60 animals) were inoculated subcutaneously in the area under the right shoulder with N-87 carcinoma cells (5 × 10⁶ cells/mouse) in 0.1 mL of serum-free medium. After the tumor grew for 8 days to an average size of 140 mm³, the animals were then randomly divided into 10 groups (6 animals per group). The first group of mice served as the control group and was treated with the phosphate-buffered saline (PBS) vehicle. 6 groups were administered intravenously with conjugates **C-37, C-49, C-72, C-123,C-134** and T-DM1 respectively at dose of 6 mg/Kg. Three dimensions of the tumor were measured every 3 or 4 days and the tumor volumes were calculated using the formula: tumor volume =1/2(length × width × height). The weight of the animals was also measured at the same time. A mouse was sacrificed when any one of the following criteria was met: (1) loss of body weight of more than 20% of pre-treatment weight, (2) tumor volume larger than 1500 mm³, (3) too sick to reach food and water, or (4) skin necrosis. A mouse was considered to be tumor-free if no tumor was palpable.

The results were plotted in Figures 7. All the 6 conjugates did not cause the weight loss of the animals. Compared with PBS control group, all conjugates demonstrated anti-tumor activity. All tested Tubulysin conjugates also demonstrated better anti-tumor activity than T-DM1.

### Example 154 Cytotoxicity evaluation of Her2-Tubulysin B derivate conjugates in comparison withT-DM1

The weight change (usually decrease) of an animal reflects the toxicity of the drugs. Fifty six female ICR mice, 6-7 weeks old, were separated into 7 groups. Each group included 8 mice and each animal was given 150 mg/Kg of the **C-37, C-49, C-72, C-123, C-134** and T-DM1 in a single I.V. injection accordingly. Control groups (8 animals) was administered with phosphate-buffered saline (PBS) vehicle. As shown in Fig. 8, in the 12-day experiment, the weight loss of the mice in all conjugate groups, except for the control group and the T-DM1 group, was less than 5%. In contrast, the animal body weight in the T-DM1 group decreased continuously, with a maximum reduction of 24%, and the recovery trend was not observed at the end of the study. The weight change result shows that Her2-tubulysin B derivative conjugates containing branched linkers were better tolerated, than T-DM 1 with conventional single linkers in animals.

The foregoing examples shows only the principles of the invention, and it is to be understood that the scope of the invention is not intended to be limited to the exemples described herein, but should include all currently known and future to be developed equivalents. In addition, it should be noted that improvements and modifications can be made without deviation from the technical principles of the present invention, and these improvements and modifications should also be regarded as within the scope of the present invention.

## Claims

1. An antibody-Tubulysin B derivative (analog) conjugate, and the conjugate has structure of Formula (I): 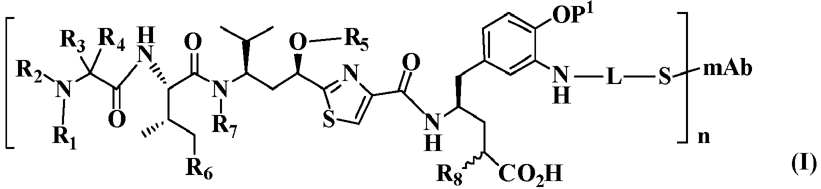 or its pharmaceutically acceptable salt, hydrate or hydrated salt; or a polymorphic crystalline structure represented by Formula (I); or an optical isomer of the structure represented by Formula (I); or its derivative with one or more hydrogen (¹H) atoms being stustituted by one or mored euterium (²H) atoms, or its derivative with one or more ¹²C atoms being stustituted by one or more ¹³C atoms;
wherein P¹ is H,COCH₃,COH,PO(OH)₂, CH₂OPO(OH)₂, CONHCH₃,CON(CH₃)₂, CON(CH₂CH₂)₂NCH₃, CON(CH₂CH₃)₂ or CON(CH₂CH₂)₂CHN(CH₂CH₂)₂CH₂;
wherein R₁, R₂, R₃ and R₄ are independently H, C₁-C₆ alkyl, C₁-C₆ alkenyl, C₁-C₆ alkoxy, C₁-C₆ alkylcarbonyl, C₁-C₆ alkylester, C₁-C₆ alkylcarboxy or C₁-C₆ alkylamido group;
or R₁ and R₂, R₁ and R₃, R₂ and R₃, or R₃ and R₄ form a C₂-C₇ heterocyclic or C₂-C₇ cycloalkyl structure;
R₅ is H, O-C₁-C₆ alkyl, C(O)-H, C(O)-C₁-C₆ linear or branched alkyl, C(O)-NH-C₁-C₆ linear or branched alkyl or C(O)-N(C₁-C₆ linear or branched alkyl)₂;
R₆, R₇ and R₈ are independently H, C₁-C₆ alkyl, C₁-C alkoxy, C₁-C₆ alkylcarbonyl, C₁-C₆ alkyl ester, C₁-C₆ alkylcarboxy or C₁-C₆ alkylamido group; preferably R₆, R₇ and R₈ are independently H or CH₃;
mAb is an antibody, antibody fragment, monoclonal antibody, polyclonal antibody, nanobody, prodrug antibody, or an antibody and antibody fragment that is modified by a synthetic molecule or protein;
L is a linker containing a hydrophilic branched chain, which is composed of a C₂-C₁₀₀ peptide unit (1-12 natural or non-natural amino acids), a hydrazone, a disulfide, an ester, an oxime, an amide or a thioether bond;
n = 1-30.

2. The conjugate according to **Claim 1**, wherein L has the structure below: 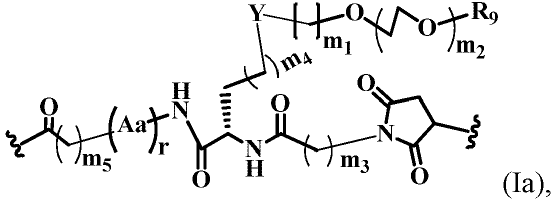 wherin Aa is a L- or D- natural or non-natural amino acid;
r is an integer between 0 and 12; when R is not 0, (Aa)r is a peptide unit composed of the same or different amino acids;
m₁ is an integer between 1 and 18; m₂ is an integer between 1 and 100; m₃ is an integer between 1 and 8; m₄ is an integer between 0 and 8; m₅ is an integer between 1 and 8;
Y is NHC(=O), NHS(O₂), NH(SO), NHS(O₂)NH, NHP(O)(OH)NH or C(O)NH;
R₉ is H, (O=)CR₁, (O=)CNHR₁, R₁COOH, R₁(COCH₂NH)ₘ₂H, R₁(Aa)ᵣ or R₁(COCH₂NCH₃)ₘ₂H; and
R₁, m₂ and (Aa)ᵣ are defined the same as in **Claim 1**.

3. The conjugate according to **Claim 1** or **Claim 2**, wherein the synthesis of the conjugate comprises one or more of the following steps: 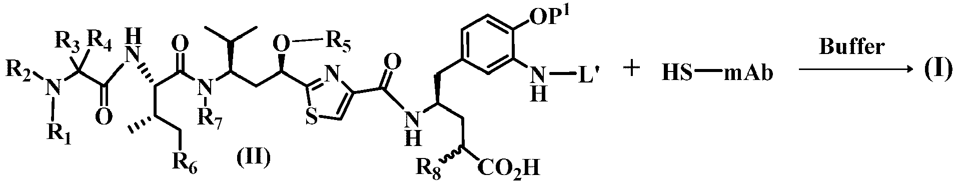 wherein P¹, " 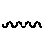 ", R₁, R₂, R₃, R₄, R₅, R₆, R₇ and R₈ in Formula (II) and mAb are defined the same as in **Claims 1** or **Claim 2**;
the structure of L' is as below: 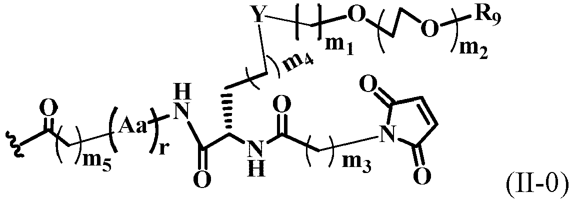 wherein m₁, m₂, m₃, m₄, m₅, Aa, r and R₉ are defined the same as in **Claims 1** or **Claim 2**.

4. The conjugate according to **Claim 3**, wherein the structure of L'is shown as below: 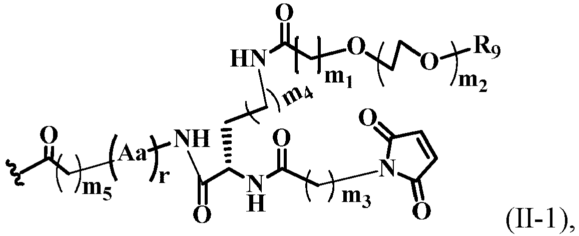 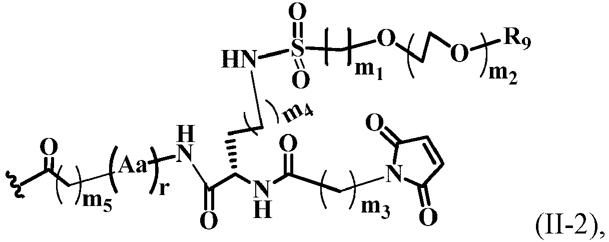 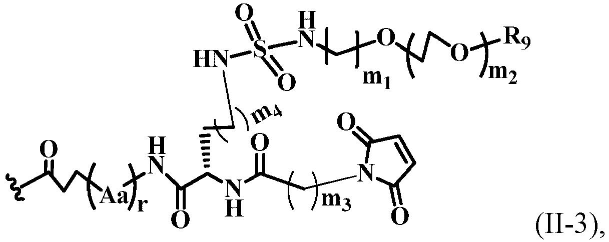 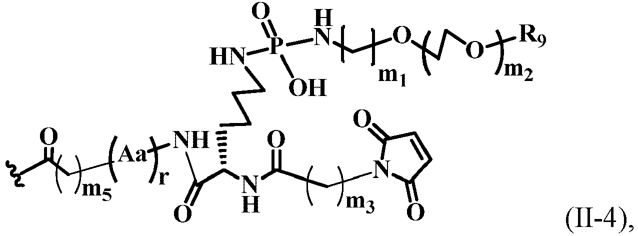 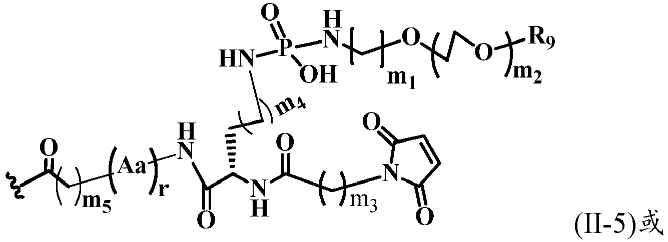 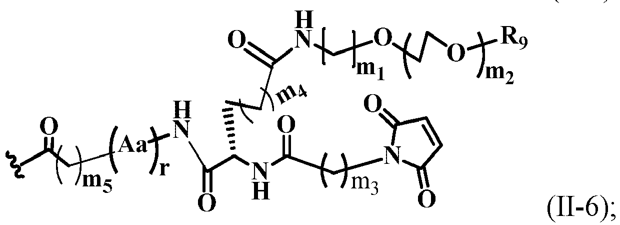 wherein m₁, m₂, m₃, m₄, m₅, Aa, r and R₉ are defined the same as in **Claims 1** or **Claim 2**.

5. The conjugate according to **Claims 3 ~ 4**, wherein the preparation of mAb-SH comprises any method of the following:
a) Reduction of the disulfide bonds between heavy and light chains, heavy and heavy chains or intra-disulfide bonds of an antibody, antibody fragment, monoclonal antibody, polyclonal antibody, nanobody, probody or antibody and antibody fragment modified by synthetic molecules or proteins, by reducing agents (preferablely, tris (2-carboxyethyl) phosphine (TCEP ), dithiothreitol (DTT), dithioerythritol (DTE), L-glutathione (GSH), 2-mercaptoethylamine (β-MEA), or/and β-mercaptoethanol (β-ME, 2-ME));
b) Generation of a sulfhydryl group by reaction of the amino group of antibody with Traut's reagent or thiolactone; 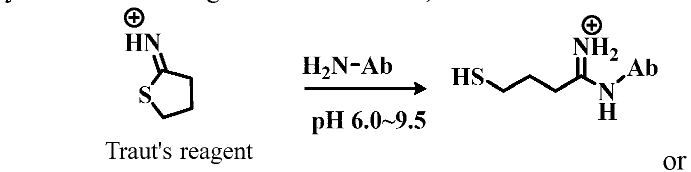 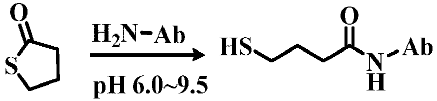
c) Incorporation of an easily reduced disulfide bond into the antibody by biochemical reaction in a buffer system, followed by reduction by TCEP, DTT, GSH, β-MEA or β-ME:

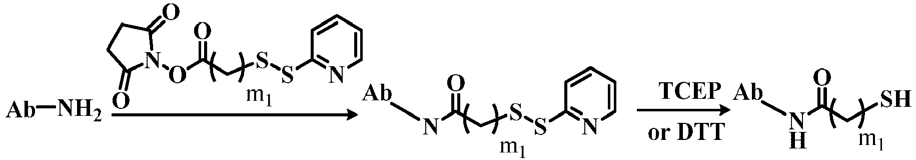

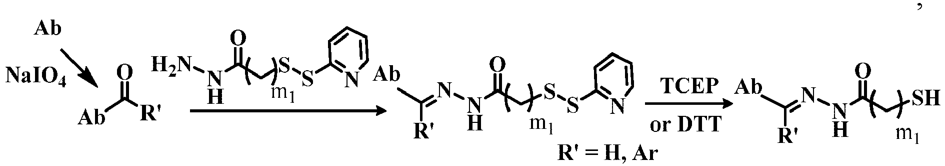

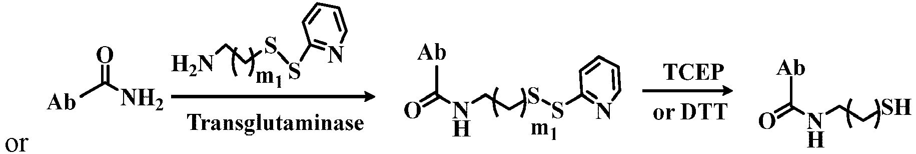

6. The conjugate according to **Claims 3 ~ 5**, wherein the buffer system used in the synthesis of the conjugate is pH 5.0-9.5, 1 mM ~ 1000 mM phosphoric acid, acetic acid, citric acid, boric acid, carbonic acid, barbituric acid, Tris (trimethylaminomethane), benzoic acid or triethanolamine system, or a mixed buffer solution thereof, and contains 0 to 35% water-soluble organic solvent of methanol, ethanol, n-propanol, isopropanol, n-butanol, isobutanol, acetonitrile, acetone, DMF, DMA or DMSO; the reaction temperature is 0°C to 45°C, reaction time 5 minutes to 96 hours.

7. The conjugate according to **Claims 3 ~ 6**, wherein the conjugate of Formula (I) is yielded by ultrafiltration or column chromatography purification after the conjugation reaction is completed.

8. The conjugate according to **Claim 7**, wherein the purification column comprises a molecular sieve column, a cationic column, an anionic column, a hydrophobic (HIC) column, a reverse phase column or a protein A or G affinity column.

9. The conjugate according to **Claim 3 ~ 8**, wherein the structure of Formula (II) is yielded by the condensation reaction of a Tubulysin B derivative of Formula (III) with a compound of Formula (L'): 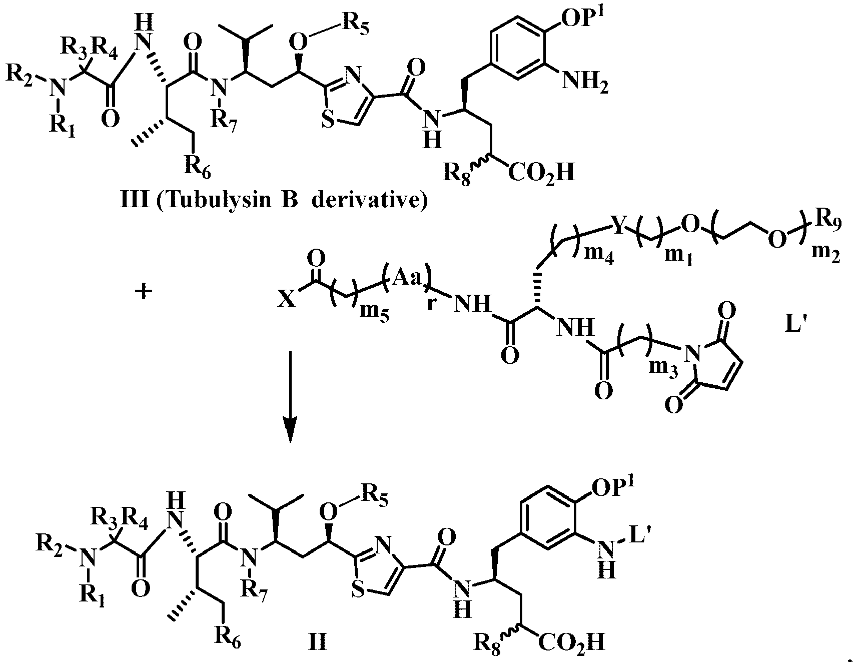 wherein X is OH, halogen (F, Cl, Br, or I), phenoxy, pentachlorophenoxy, trifluoromethanesulfonyl, imidazole, dichlorophenoxy, tetrachlorophenoxy, 1-hdroxbenzotriazole, p-toluenesulfonyl, methanesulfonyl, 2-ethyl-5-phenyl isoxazole -3'- sulfonyl group, 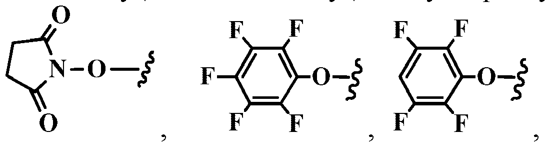 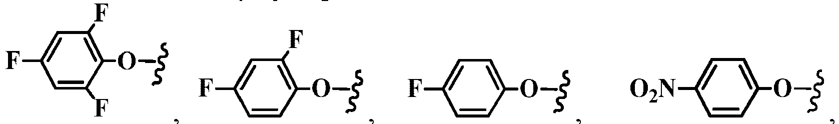 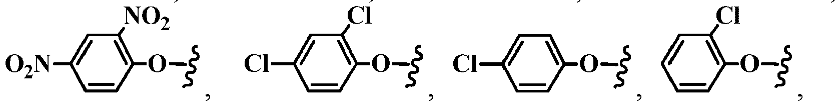 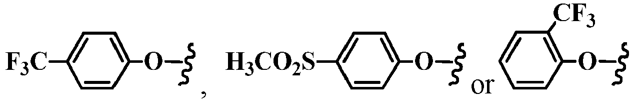 , an anhydride formed by itself or with other anhydrides, such as acetyl anhydride and formic anhydride; or a peptide coupling reaction intermediate or a Mitsunobu reaction intermediate;
wherein the condensation reaction is carried out in dichloroethane, DMF, DMA, tetrahydrofuran (THF), DMSO, acetone, isopropanol, n-butanol or acetonitrile, or above two or three mixed solvents, containing 1 to 100% pyridine, triethylamine or diisopropylethylamine; with or without an inert gas (nitrogen, argon, helium) protection, at -20 to 150 °C, for 5 minutes to 120 hours;
Alternatively, the condensation reaction is conducted in the following buffer system and under the following conditions. The buffer system is pH 5.0 ~ 9.5, 1 mM ~ 1000 mM phosphoric acid, acetic acid, citric acid, boric acid, carbonic acid, barbituric acid, Tris (Tris-hydroxymethyl aminomethane), benzoic acid or triethanolamine system, or a mixture thereof, containing 0 to 35% miscible organic solvents, such as methanol, ethanol, n-propanol, isopropanol, n-butanol, isobutanol, acetonitrile, acetone, DMF, DMA or DMSO. The reaction temperature is 0 to 45°C and reaction time is from 5 minutes to 96 hours.

10. The conjugate according to **Claim 9**, wherein the NH₂ group of formula (III) participates in the conjugation reaction in a form of salt, with trifluoroacetic acid, hydrochloride acid, formic acid, acetatic acid, sulfuric acid, phosphoric acid, nitric acid, citric acid, succinic acid, benzoic acid or sulfonic acid.

11. The conjugate according to **Claim 9 ~ 10**, when X is OH, the proceeding of the condensation reaction requires a condensation reagent, selected from (N-(3 -Dimethylaminopropyl)-N'-ethylcarbodiimide) (EDC), dicyclohexylcarbodiimide (DCC), N, N'-diisopropylcarbodiimide (DIC), N-Cyclohexyl-N'-(2-morpholino-ethyl)carbodiimide metho-p-toluenesulfonate (CMC or CME-CDI), carbonyldiimidazole (CDI), O-benzotriazole-N, N, N', N'-tetramethyl urea tetrafluoroborate (TBTU), O-benzotriazole-tetramethyl urea hexafluorophosphate (HBTU), (Benzotriazol-1-yloxy)tris(dimethylamino)-phosphonium hexafluorophosphate (BOP), (Benzotriazol-1-yloxy)tripyrrolidinophosphonium hexafluorophosphate (PyBOP), diethyl pyrocarbonate (DEPC), N, N, N', N'-tetramethylchlorobenzamidine hexafluorophosphate, 2-(7-oxobenztriazole)-N, N, N', N'-tetramethyluronium hexafluorophosphate (HATU), 1-[(dimethylamino) (morpholinyl) methylene ] -1 [1,2,3] triazolo [4,5-b] 1-pyridin-3-oxyhexafluorophosphate (HDMA), 2-chloro-1,3-dimethylimidazolium hexafluorophosphate (CIP), chlorotripyrrolidinylphosphonium hexafluorophosphate Bis (tetramethylene) fluoroformamide (BTFFH), N, N, N', N'-tetramethyl-sulfur-(1-oxo-2-pyridinyl) thiuronium hexafluorophosphate, 2-(2-pyridone-1-yl) -1,1,3,3-tetramethyl urea tetrafluoroborate (TPTU), sulfur-(1-oxo-2-pyridinyl)-N, N, N', N'-tetramethylthiourea hexafluorophosphate, O-[(ethoxycarbonyl) cyanamide ]-N, N, N', N'-tetramethylthiourea hexafluorophosphate (HOTU), (1-cyano-2-ethoxy-2-oxoiminoyloxy) dimethylamino-morpholine-carbenium hexafluorophosphate (COMU), N-benzyl- N'-cyclohexylcarbodiimide (or on solid support), N-benzyl- N'-cyclohexyl carbodiimide (or on solid support), di-pyrrolidinyl (N-succinimido-methyl) pyrrolidine hexafluorophosphate (CIB), 2-chloro -1,3-dimethylimidazolium tetrafluoroborate (CIB), (benzotriazol-1-yloxy) dipiperidinyl carbon hexafluorophosphate (TCTU), tris (dimethylamino) phosphine hexafluorophosphate (RRHA), 1-n-propylphosphorus anhydride (PPACA, T3P^{®}) , 2-isocyanoethylmorpholine (MEI), N,N,N',N'-tetramethylurea-oxy-(N-succinimidyl) hexafluorophosphate (HSTU), 2-bromo -1-Ethylpyridine tetrafluoroborate (BEP), oxygen-[(ethoxycarbonyl)cyanomethylamine]-N,N,N',N'-tetramethylthiourea tetrafluoroborate (TOTU), 4-(4,6-dimethoxytriazin-2-yl)-4-methylmorpholine hydrochloride (MMTM, DMTMM), 2-succinimidyl -1,1,3,3-tetramethyl urea tetrafluoroborate (TSTU), N, N, N', N'-tetramethyl-O-(3,4-dihydro-4-oxo -1, 2) 3-benzotriazine-3-yl) urea tetrafluoroborate (TDBTU), azodicarbonyldipiperidinyl (ADD), bis (4-chlorobenzyl) azodicarboxylate (DCAD), di-tert-butyl azodicarboxylate (DBAD), diisopropyl azodicarboxylate (DIAD) or diethyl azodicarboxylate (DEAD).

12. The conjugate according to **Claims 9 ~ 11**, wherein the synthesis of the Tubulysin B derivatives of formula (III) comprises one or more of the following steps: 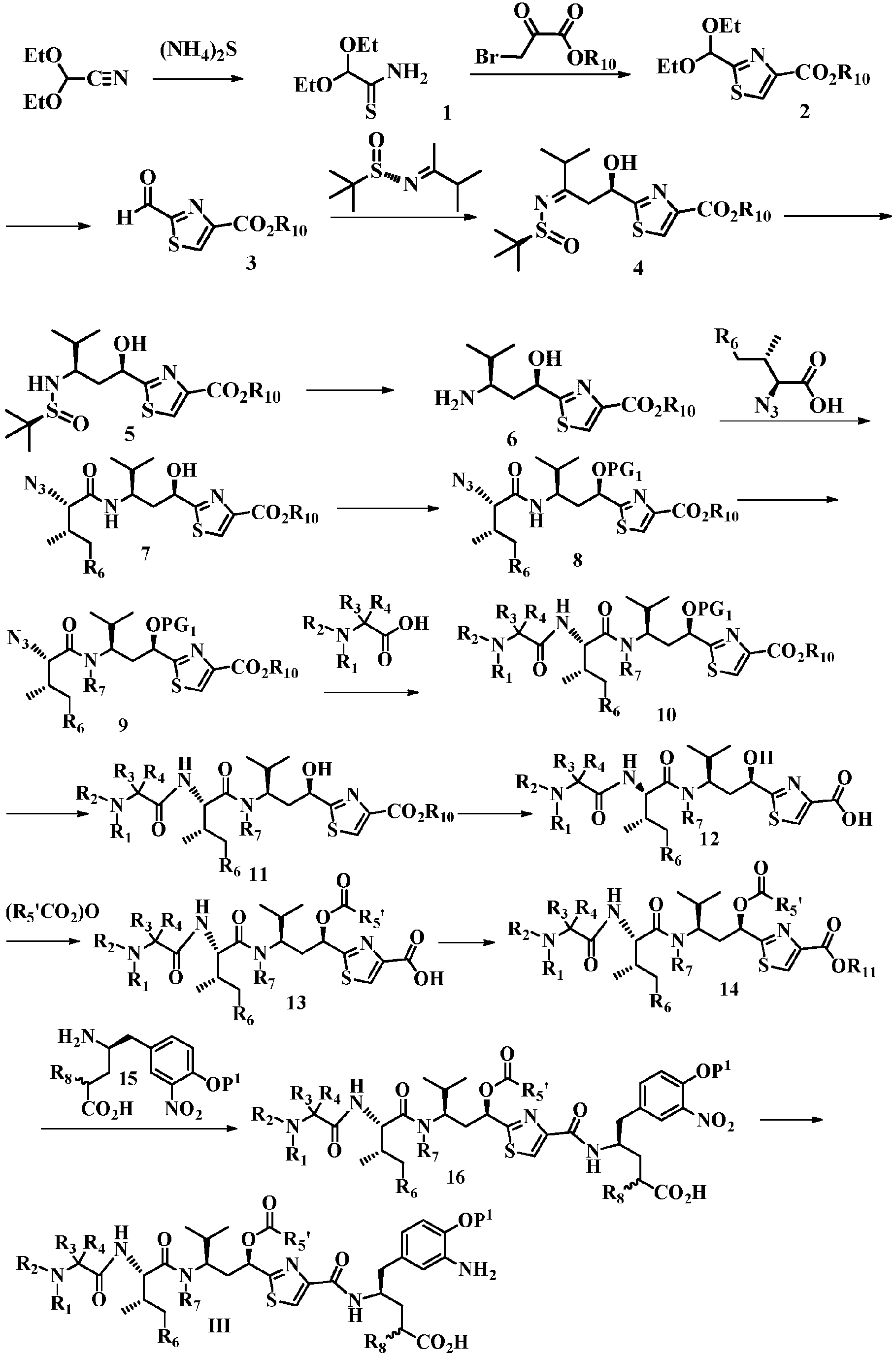 R₅' is H, C₁-C₆ alkyl group, C₁-C₆ alkenyl group, or C₁-C₆ linear or branched aminoalkyl group.

13. The conjugate according to **Claim 12**, wherein the synthesis of the Tubulysin B derivatives of formula (III) comprises one or more of the following steps:
Step 1: Diethoxyacetonitrile and aqueous ammonium sulfide are stirred at room temperature to yield compound **1**, 2,2-diethoxythioacetamide; 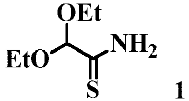
Step 2: Compound **1** and bromopyruvate in an anhydrous solvent (such as anhydrous tetrahydrofuran, dichloromethane, acetonitrile, N, N-dimethylformamide, methanol or isopropanol) are heated and condensed to yield compound **2**; 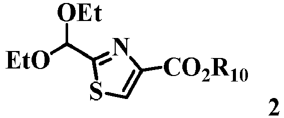
Step 3: Compound **2** is dissolved in a solvent (such as tetrahydrofuran, dichloromethane, ethyl acetate, n-heptane, dioxane, acetonitrile) and hydrolyzed in the presence of a Lewis acid or protonic acid (such as hydrochloric acid, sulfuric acid, phosphoric acid, methanesulfonic acid, formic acid, oxalic acid, acetic acid, p-toluenesulfonic acid, pyridinium p-toluenesulfonate , AlCl₃, FeCl₃, ZnCl₂, BF₃, BCl₃, BBr₃, TiCl₄, ZnBr₂ or LiBF₄), to yield compound **3**; 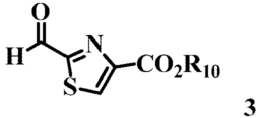
Step 4: The sulfinamide is deprotonated by n-butyllithium under low temperature (such as -45 to -78°C), and then condensed with compound **3** in the presence of a Lewis acid, to yield compound **4** (Adol reaction); 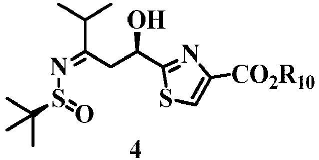
Step 5: Compound **4** is selectively reduced at low temperature (for example, -45 to -78°C) by a reducing reagent (such as NaBH₄, LiBH₄, Na(OAc)₃BH, Na(CN)BH₃ etc.), to yield compound **5**, and in the reaction a Lewis acid (for example Ti(OEt)₄) is added to control the stereochemistry outcome. 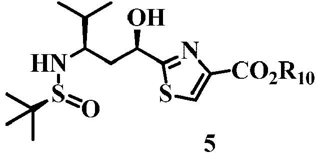
Step 6: Compound **5** is dissolved in a solvent (such as methanol, ethanol, isopropanol, tetrahydrofuran or acetonitrile), and tert-butylsulfinyl group is removed by acid such as hydrochloric acid, sulfuric acid and phosphoric acid, to yield compound **6**. 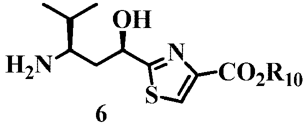
Step 7: In presence of a condensation reagent (such as DIC/HOBt, DCC/HOBt, EDC/HOBt, HATU, BOP, T3P or BrOP), compound **6** and azido acid in a solvent (for example, n-heptane, tetrahydrofuran, dichloromethane, N, N-dimethylformamide) is condensed, to yield compound **7**;
Alternatively, azido acid reacts with isobutyl chloroformate in THF, in the presence of an organic base (such as triethylamine, diisopropylethylamine, N-methylmorpholine, etc.), to yield a mixed anhydride, which condenses with the hydrochloride salt of compound **6** to afford **7**;
Alternatively, azido acid in a solvent (such as n-heptane, n-hexane, dichloromethane or tetrahydrofuran) reacts with oxalyl chloride, in presence of triethylamine and DMF (catalytic amount), to produce acyl chloride, which then condenses with the hydrochloride salt of compound **6** to afford **7**. 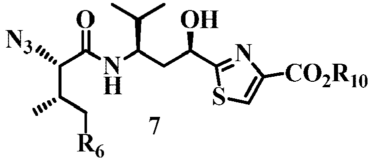
Step 8: In a solvent (such as dichloromethane, tetrahydrofuran or acetonitrile), the hydroxyl group of compound **7** reacts with a hydroxyl protection reagent (such as using TESCl), in the presence of an organic base (such as imidazole, triethylamine or pyridine), to yield the protected compound **8**; 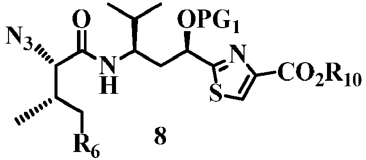
Step 9: Compound **8** in a solvent (such as tetrahydrofuran, dichloromethane or acetonitrile) is deprotonated with added base (such as KHMDS, LiHMDS, NaHMDS, KOtBu, NaH or KH), and then alkylated with iodomethane, bromomethane, dimethyl sulfate, methyl trifluoromethanesulfonate, iodoethane and the like, to yield compound **9**; 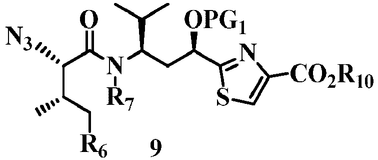
Step 10: Compound **9** is dissolved in a solvent (such as tetrahydrofuran, dichloromethane or ethyl acetate), wherein the azido group is reduced to an amino group under certain conditions, such as H₂ and Pd/C, triphenylphosphine and water (Staudinger reaction) and then condensed with an acid or an acid derivative having similar reactivity, to afford compound **10**; 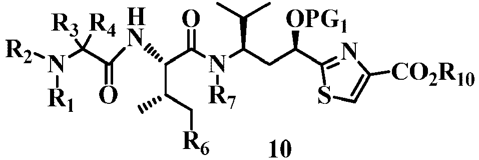
Step 11: The hydroxyl protecting group PG₁ of compound **10** can be deprotected under appropriate conditions (for example, TES protecting group may be deprotected in HCl, THF/MeOH/AcOH, *ⁿ*Bu₄NF or HF-pyridine in THF) to yield compound 11; 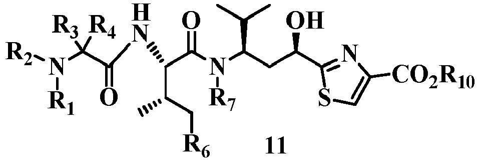
Step 12: The ester compound **11** is converted to acid **12**, being subjected to a base (such as LiOH, NaOH or KOH) or other suitable conditions (such as methyl ester can be converted to carboxylic acid with LiCl, LiI, Me₃SiOK, and the like); 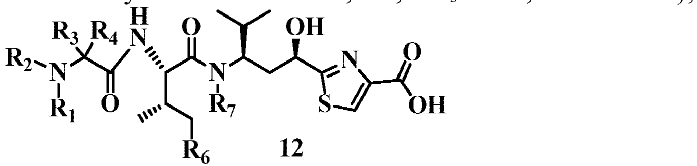
Step 13: In the presence of a base (such as triethylamine, N, N-diisopropylethylamine or pyridine) and a catalyst (such as DMAP), compound **12** reacts with anhydride, such as acetic anhydride, propionic anhydride, iso-propionic anhydride, or acyl halide, such as acetyl halide, propionyl halide, carbamoyl halide, methylcarbamoyl halide, ethylcarbamoyl halide, dimethylcarbamoyl halide, at certain temperature (such as 0°C to 23°C), to yield compound **13**, and the reaction may take place without base or catalyst; 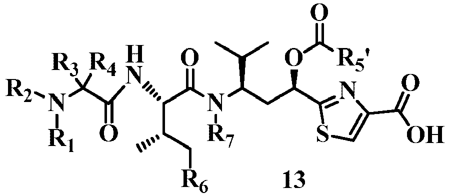
Step 14: Compound **13** condenses with hydroxyl-containing compound such as pentafluorophenol or N-hydroxysuccinimide in the presence of a condensation reagent, such as EDC, DIC, DCC, HATU, HBTU, to yield a reactive ester compound **14**; 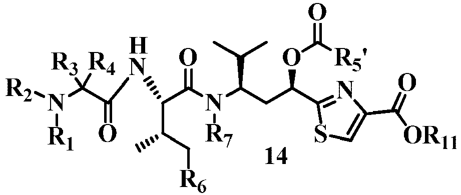
Step 15: Compound **15** and compound **14** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **16**. Optionally a base is not required for the reaction to proceed, but the reaction temperature (from °C to 23°C) and reaction time (from 30 minutes to 18 hours) need to be tightly controlled; 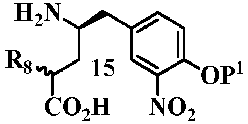 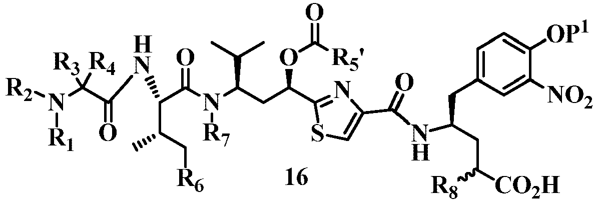
Step 16: The nitro group of compound **16** is reduced to an amino group under reduction conditions, such as hydrogen and Pd/C catalyst, hydrazine hydrate and FeCl₃, iron powder and acetic acid, and the like, to yield compound III.

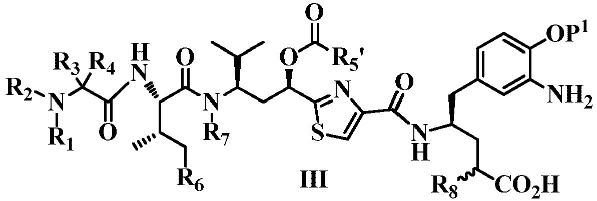

14. The conjugate according to **Claim 9 ~ 13**, wherein the synthesis of compounds of Formula (L') comprises one or more of the following steps:

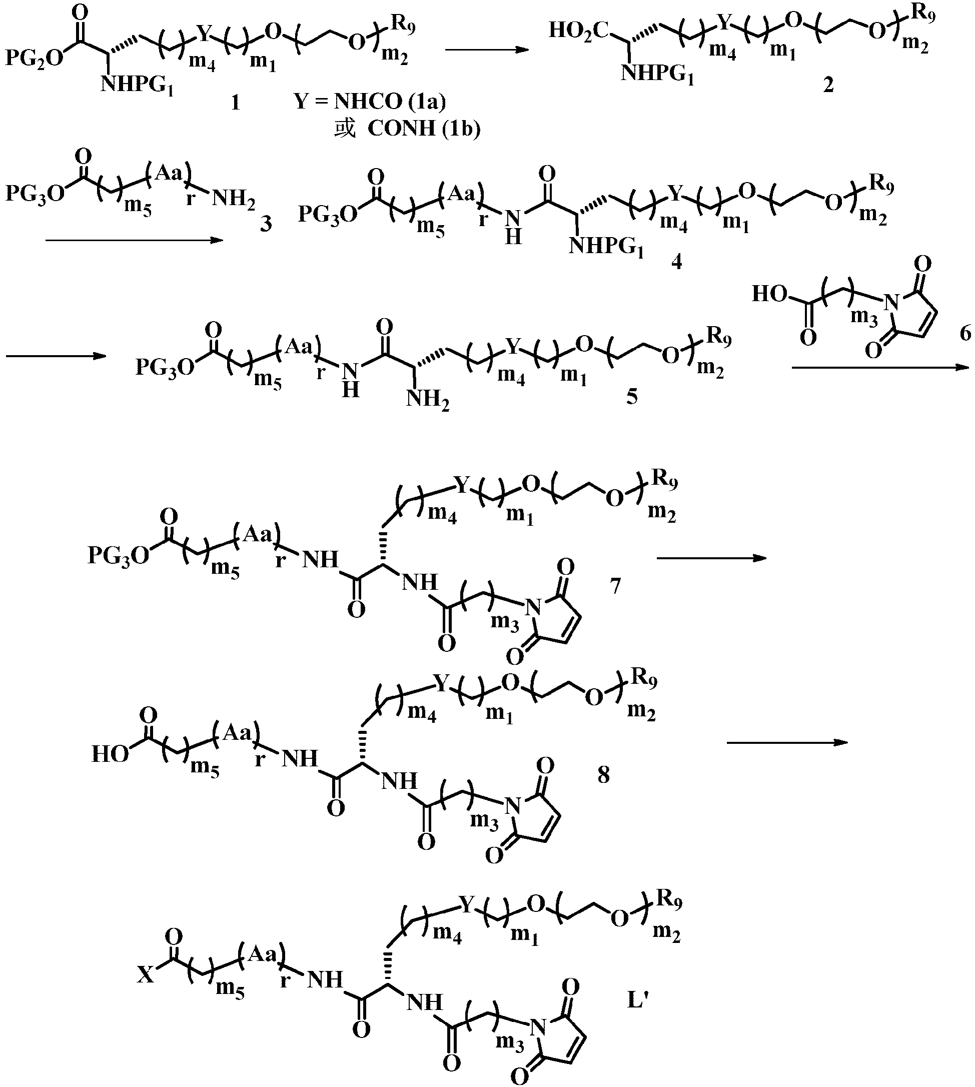

15. The conjugate of **Claim 14**, wherein the synthesis of the compound of Formula (L') comprises one or more of the following steps:
Step 1: Compound **1-1** and compound **1-2** condense directly in the presence of a condensation reagent (such as EDC, HATU, DIC or DCC), or by reacting compound **1-2** with pentafluorophenol, nitrophenol or N-hydroxysuccinimide to yield correposnding active eater, in the presence of a condensation reagent such as DIC or EDC, and then reacting with compound **1-1**, to yield **1a**;
Alternatively, compound **1-3** and compound **1-4** condense directly, in the presence of a condensation reagent (such as EDC, HATU, DIC or DCC), or by other indirect condensation reaction routes, to yield compound **1b**; 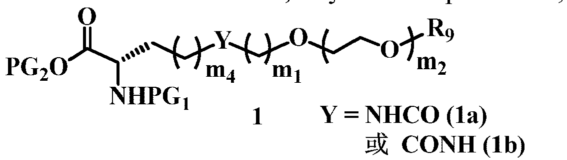 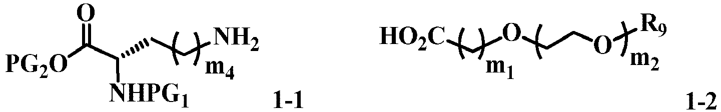 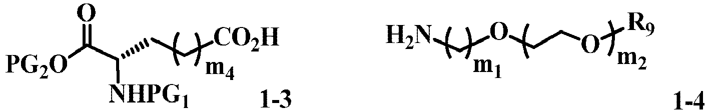
Step 2: The carboxyl protecting group PG₂ of compound **1** is removed by a deprotection reagent (such as to remove tert-butyl ester group by an acid), to yield compound **2**; 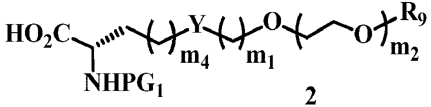
Step 3: An acid compound **2** and an amine compound **3** condense in the presence of a condensation reagent (such as EDC, HATU, DIC or DCC), or by other indirect condensation reaction routes, to yield compound **4**; 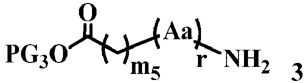 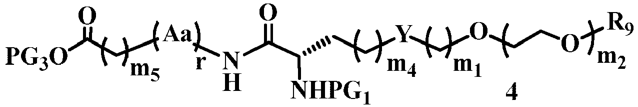
Step 4: The amino protecting group PG₁ of compound **4** is removed under deprotection conditions, such as H₂ and Pd/C catalyst for Cbz protecting group, and acidic conditions for Boc protecting group, to yield compound **5**; 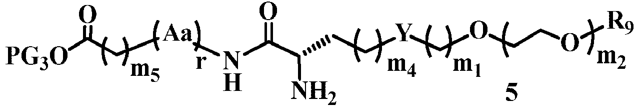
Step 5: Carboxylic acid **6** and amine **5** condense in the presence of a condensation reagent (such as EDC, HATU, DIC or DCC), or by other indirect condensation reaction routes, to yield compound **7**; 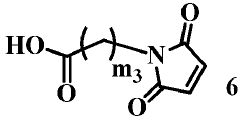 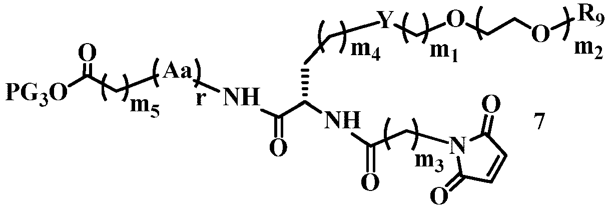
Step 6: The carboxyl protecting group PG₃ of compound **7** is removed under deprotection conditions, such as acid (formic acid, acetic acid, trifluoroacetic acid, hydrochloric acid, phosphoric acid and the like) for tert-butyl ester protecting group, to yield compound **8**; 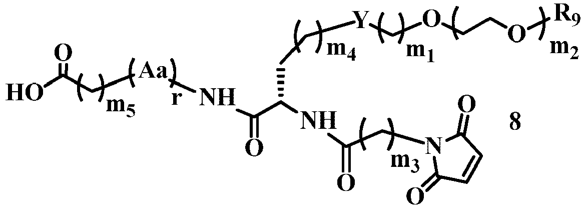
Step 7: Compound **8** reacts with a compound containing a hydroxyl group (such as pentafluorophenol or N-hydroxysuccinimide), in the presence of a condensation reagent (such as EDC, HATU, DIC or DCC), or reacts with other carboxylic acid activating compounds, to yield a reactive ester compound L'.

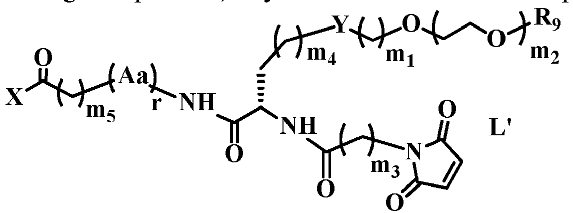

16. The conjugate according to **Claims 9 ~ 13**, wherein the synthesis of compounds of Formula (L') comprises one or more of the following steps:

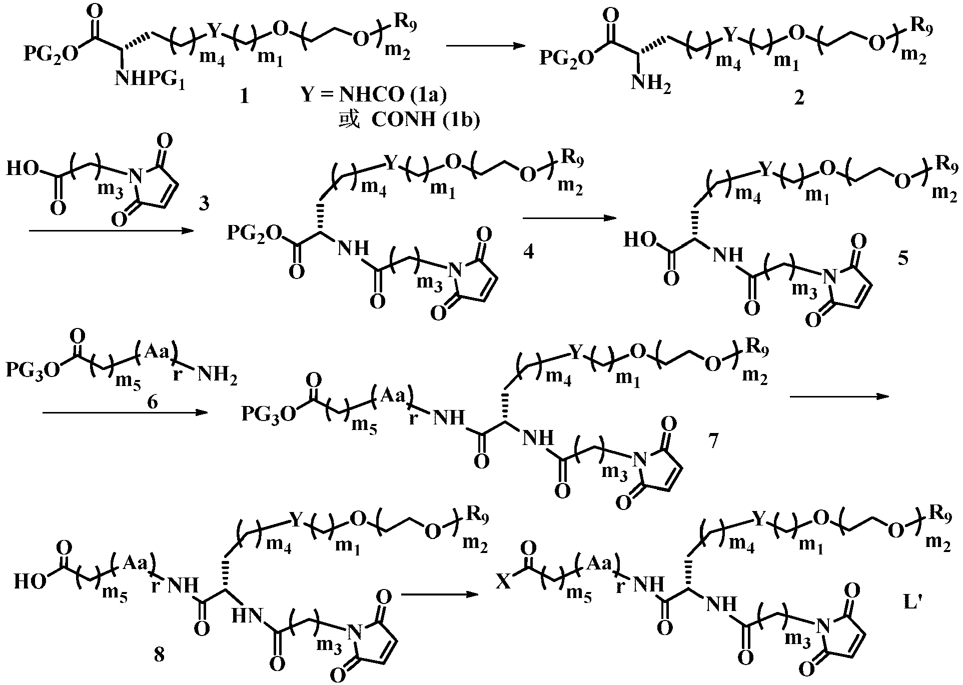

17. The conjugate according to **Claim 16**, wherein the synthesis of compounds of Formula (L') comprises one or more of the following steps:
Step 1: The amino protecting group PG₁ on compound **1** is removed under deprotection condition, such as H₂ and Pd/C catalyst for Cbz protecting group, and acidic conditions for Boc protecting group, to yield compound **2**; 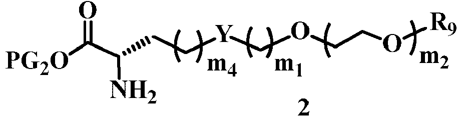
Step 2: Amino compound **2** and carboxylic acid **3** condense in the presence of a condensation reagent (such as EDC, HATU, DIC or DCC), or by other indirect condensation reaction routes, to yield compound **4**; 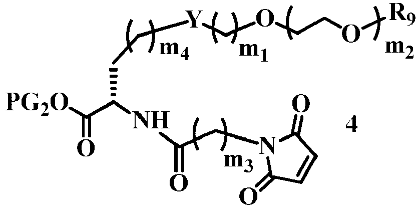
Step 3: The carboxyl protecting group PG₂ of compound **4** is removed under deprotection conditions, such as acid (formic acid, acetic acid, trifluoroacetic acid, hydrochloric acid, phosphoric acid and the like) for the cleavage of tert-butyl ester protecting group, to yield compound **5**; 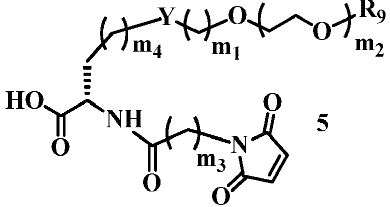
Step 4: Carboxylic acid **5** and amine **6** condense in the presence of a condensation reagent (such as EDC, HATU, DIC or DCC), or by other indirect condensation reaction routes, to yield compound **7**; 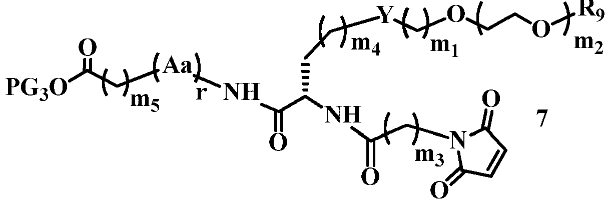
Step 5: The carboxyl protecting group PG₃ of compound **7** is removed under deprotection conditions, such as acid (formic acid, acetic acid, trifluoroacetic acid, hydrochloric acid, phosphoric acid and the like) for the cleavage of tert-butyl ester protecting group, to yield compound **8**; 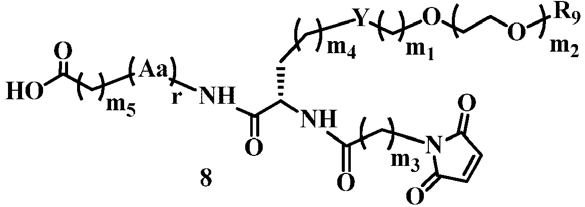
Step 6: Compound **8** reacts with a compound containing a hydroxyl group (such as pentafluorophenol or N-hydroxysuccinimide), in the presence of a condensation reagent (such as EDC, HATU, DIC or DCC), or reacts with other carboxylic acid activating compounds, to yield a reactive ester compound **9**;

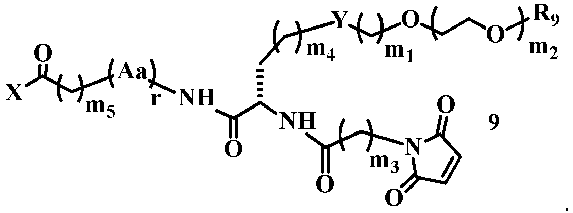

18. The conjugate according to **Claims 3 ~ 11**, wherein the synthesis of compounds of Formula (II) is achieved by the condensation reaction of compounds of Formula (IV) and Formula (V): 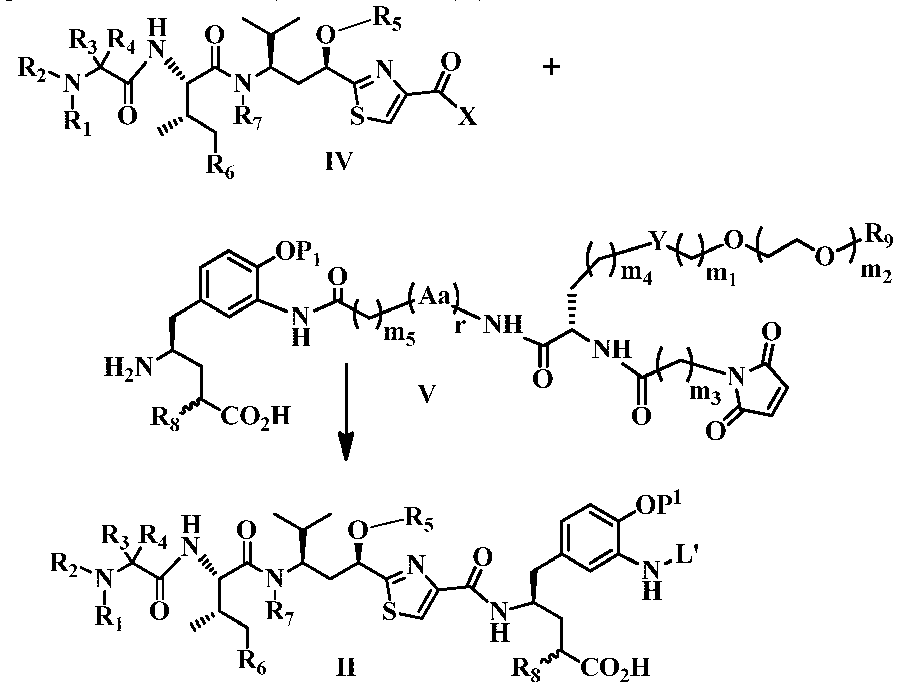 wherein the definition of X and the condensation reaction conditions are the same as in **Claim 9~11**.

19. The conjugate according to Claim 18, wherein the NH₂ group of Formula (V) participates in the conjugation reaction in a form of salt, with trifluoroacetic acid, hydrochloride acid, formic acid, acetatic acid, sulfuric acid, phosphoric acid, nitric acid, citric acid, succinic acid, benzoic acid or sulfonic acid.

20. The conjugate according to **Claim 18 ~ 19**, wherein the synthesis of the compound of Formula (IV) comprises one or more of the following steps:

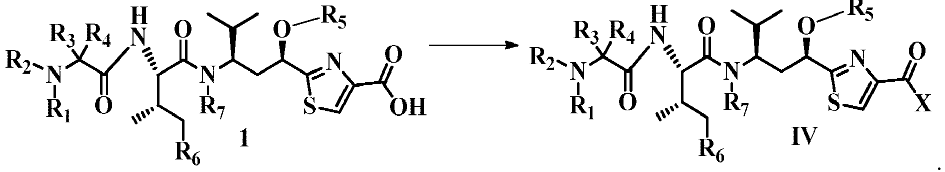

21. The conjugate according to **Claim 20**, wherein the synthesis of compounds of Formula (IV) comprises the following steps:
Carboxylic acid **1** and compound with a hydroxyl group (such as pentafluorophenol or N-hydroxysuccinimide) condense in the presence of a condensation reagent (such as EDC, DIC, DCC, HATU or HBTU) to yield a reactive ester;
Alternatively, carboxylic acid **1** reacts with ethyl chloroformate, isobutyl chloroformate, etc., in the presence of an organic base (such as N-methylmorpholine, triethylamine, diisopropylethylamine, etc.) to yield a reactive mixed anhydride;
Alternatively, carboxylic acid **1** reacts with oxalyl chloride, in the presence of an organic base such as trimethylamine, and catalytic amount of DMF (such as 0.01 eq. to 0.5 eq.) to yield an acyl chloride.

22. The conjugate according to **Claim 18 ~ 21**, wherein the synthesis of the compound of Formula (V) comprises one or more of the following steps:

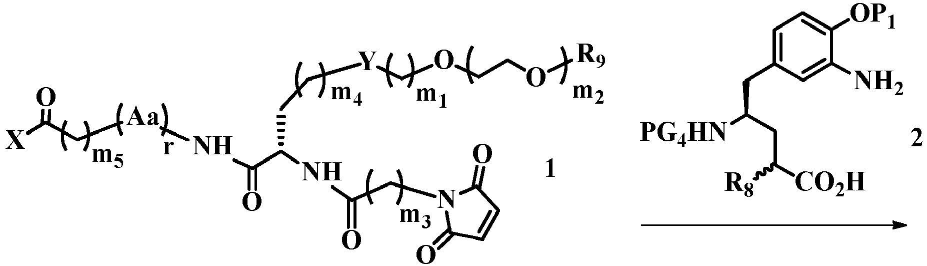

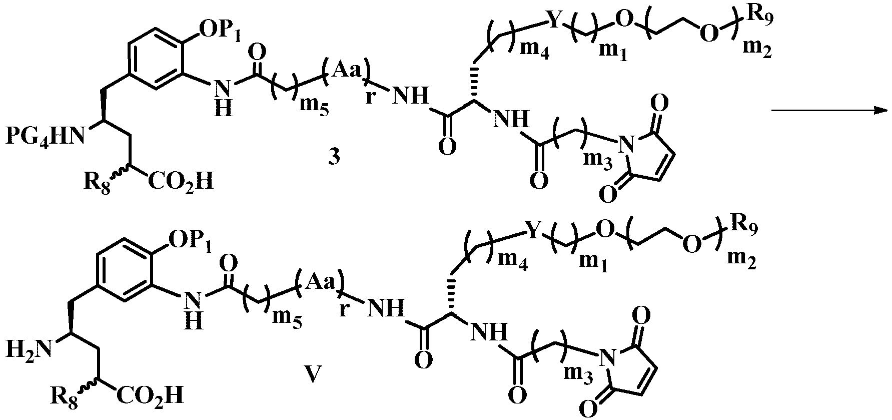

23. The conjugate of **Claim 22**, wherein the synthesis of compounds of Formula (V) comprises one or more of the following steps:
Step 1. Compound **1** and compound **2** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **3**. Optionally a base is not required for the reaction to proceed, but the reaction temperature and reaction time need to be tightly controlled; 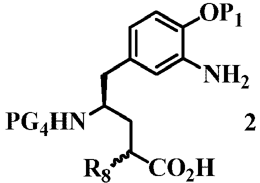 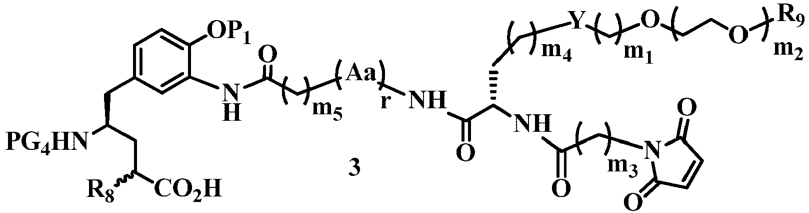
Step 2. The amino protecting group PG₄ of compound **3** is removed under deprotection conditions, such as H₂ and Pd/C catalyst for Cbz protecting group, and acidic conditions for Boc protecting group, to yield compound V;

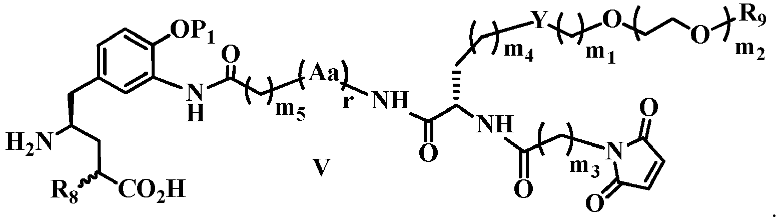

24. The conjugate according to **Claim 22 ~ 23**, wherein the synthesis of compound 2 comprises one or more of the following steps: 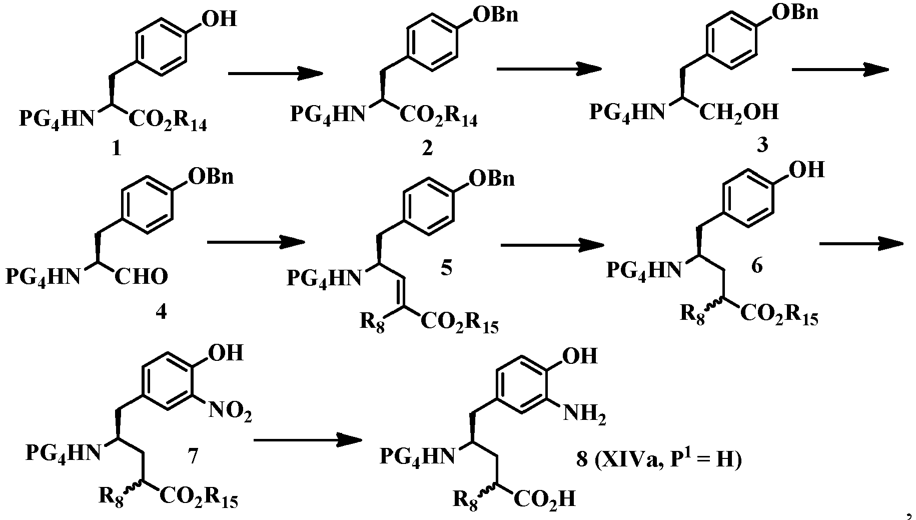 wherein compound **8** (compound XIVa) is compound **2** as in **Claim 23**.

25. The conjugate of **Claim 24**, wherein the synthesis of compound **8** comprises one or more of the following steps:
Step 1. To an ester derivative of L-tyrosine (**1**) in an appropriate solvent (such as acetone, tetrahydrofuran, acetonitrile, methylene chloride, etc.) or a solvent mixture of any above solvent and water, is added benzyl chloride, benzyl bromide or other benzyl compound at 0 ~ 60°C, followed by an organic or inorganic base, such as sodium carbonate, potassium carbonate, sodium bicarbonate, potassium bicarbonate, sodium hydroxide, potassium hydroxide, triethylamine, DBU, sodium hydride and the like, and optionally an appropriate additive, such as sodium iodide or a phase transfer catalyst, such as benzyltriethylammonium chloride (TEBA), tetrabutylammonium bromide (TBAB) , tetrabutylammonium ammonium chloride, tetrabutylammonium bisulfate, trioctylmethylammonium chloride, dodecyl trimethyl ammonium chloride, tetradecyl trimethyl ammonium chloride and the like, to yield compound **2**;
Step 2. Compound **2** is dissolved in an organic solvent (such as dichloromethane, tetrahydrofuran, methanol, ethanol, ether and the like), and then react with a reducing reagent, such as LiAlH₄, DIBAL, NaBH₄, LiBH₄, sodium bis(2-methoxyethoxy)aluminumhydride (Red-Al), diborane boroethane, etc., optionally in the presence of an addctive (such as I₂, FeCl₃, ZnCl₂, MgCl₂, LiCl or CaCl₂) to tune the activity of the reducing reagent, to yield compound **3**;
Step 3. Alcohol **3** is oxidized, under oxidation conditions such as Swern oxidation (oxalyl chloride, DMSO, triethylamine), Parikh-Doering oxidation (sulfur trioxide), Dess-Martin oxidation and the like, to yield aldehyde **4**;
Step 4. Aldehyde **4** reacts with a phosphate ester (Horner-Wadsworth-Emmons reaction) or a phosphorus ylide (Wittig reaction) to elongate the carbon chain and yield compound **5**;
Step 5. The double bond of compound **5** is reduced, in the presense of a homogeneous or heterogeneous catalyst, wherein the benzyl group is also removed, to yield a stereochemically pure compound, or a mixture of two diastereomers; the heterogeneous catalysts include Pd/C, Pd(OH)₂/C, Pd/BaSO₄, PtO₂, Pt/Al₂O₃, Ru/C, Raney Ni and the like, and the homogeneous asymmetric hydrogenation catalysts include Crabtree catalyst, [Ru (II)-(BINAP) ]-type catalyst, [(Ph₃P)CuH]₆, and the like;
Step 6. Compound **6** is dissolved in an organic solvent, such as tetrahydrofuran, acetonitrile, dichloromethane, and undergoes nitration. The nitration reagents include nitric acid, nitric acid/acetic acid, potassium nitrate/sulfuric acid, tert-butyl nitrite, nitric acid/trifluoroacetic anhydride, NO₂BF₄, nitropyridine, and the like;
Step 7. The nitro group of compound **7** is reduced to an amino group, under the conditions, including H₂/Pd/C, Fe or Zn/HOAc, SnCl₂/HCl.

26. The conjugate according to **Claim 22 ~ 23**, wherein the synthesis of compound 2 comprises one or more of the following steps: 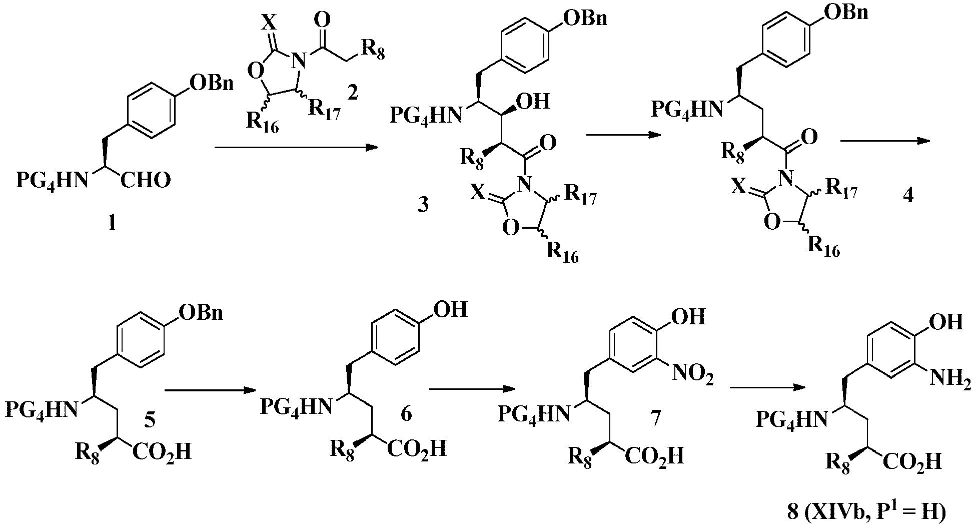 wherein compound **8** (Compound XIVb) is compound **2** in **Claim 22 - 23**.

27. The conjugate of **Claim 26**, wherein the synthesis of compound **2** comprises one or more of the following steps:
Step 1: Compound **1** undergoes Aldol reaction with Evans' chiral N-acyl oxazolidinone or thioketone **2** at -78 °C to -45°C, to yield a stereochemically pure compound **3**, wherein X = O or S, R₁₆ = H, methyl, phenyl, R₁₇ = H, methyl, isopropyl, phenyl, benzyl, and the like;
Step 2: The hydroxyl group of compound **3** is deoxygenated under Barton-McCombie deoxygenation conditions, i.e. the alcohol is first converted to a thiocarbonyl derivative, such as alkyl xanthate, phenyl carbothioate , imidazole carbothioate, and then treated with Bu₃SnH to undergo radical cleavage and afford the dehydrogenation product. The conditions of radical cleavage include *n*-Bu₃SnH/AIBN, *n*-Bu₃SnH/AIBN/*n*-BuOH/PMHS and (Bu₄N)₂S₂O₈/HCO₂Na;
Step 3: Compound **4** is dissolved in tetrahydrofuran and the Evans chiral auxiliary group is cleaved by LiOH/H₂O₂, to yield the corresponding acid **5**;
Step 4: Compound **5** is dissolved in an organic solvent (such as ethyl acetate, methanol, dichloromethane, ethanol or acetic acid, etc.) and hydrogenated in the presence of Pd/C catalyst, wherein the benzyl group is also removed, to yield compound **6**;
Step 5: Compound **6** is dissolved in an organic solvent, such as tetrahydrofuran, acetonitrile, dichloromethane, and undergoes nitration. The nitration reagents include nitric acid, nitric acid/acetic acid, potassium nitrate/sulfuric acid, tert-butyl nitrite, nitric acid/trifluoroacetic anhydride, NO₂BF₄, nitropyridine, and the like;
Step 6 : The nitro group of compound **7** is converted to amino group, under the condition of H₂/Pd/C, Fe or Zn/HOAc or SnCl₂/HCl, to yield stereochemically pure compound **8**.

28. The conjugate according to **Claim 3 ~ 11**, wherein the compound of formula (II) is obtained by condensation reaction of compounds of Formula (VI) and Formula (VII): 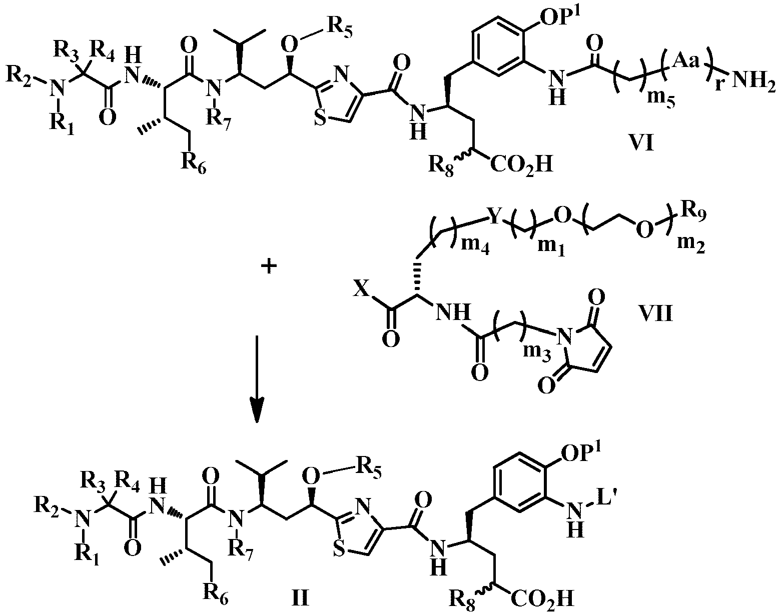 wherein the defination of X and condensation reaction conditions are the same as in **Claim 9 ~ 11**.

29. The conjugate of **Claim 28**, wherein the synthesis of compounds of formula (VI) comprises one or more of the following steps:

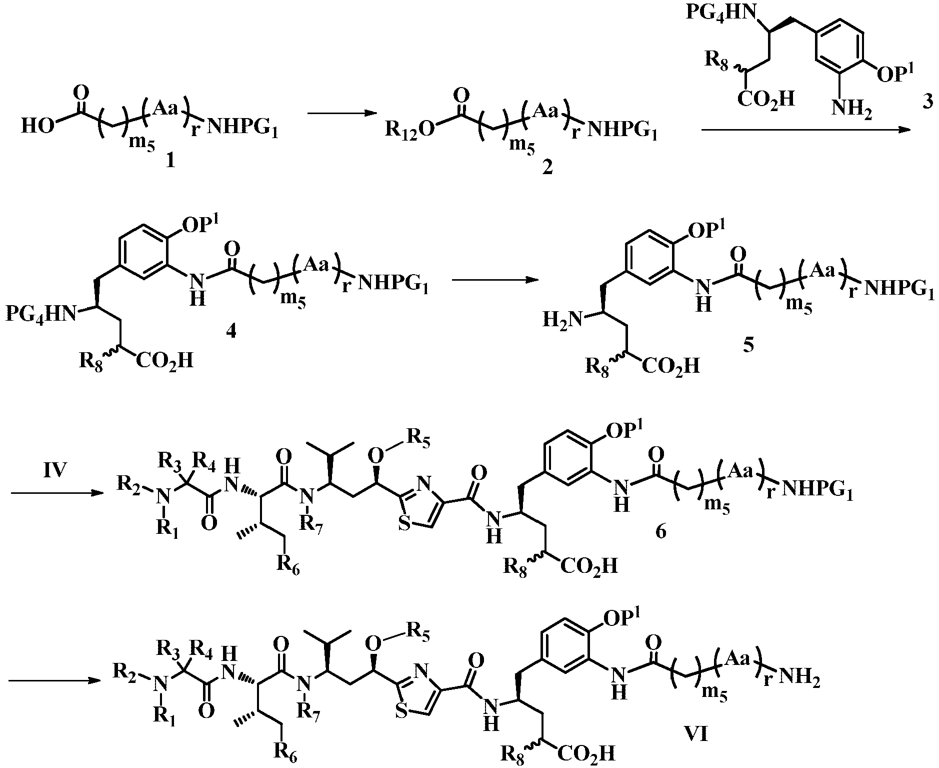

30. The conjugate according to **Claim 29**, wherein the synthesis of compounds of Formula (VI) comprises one or more of the following steps:
Step 1: Compound **1** reacts with a compound containing a hydroxyl group (such as pentafluorophenol or N-hydroxysuccinimide), in the presence of a condensation reagent, to yield a reactive carboxylic acid derivative compound **9**; 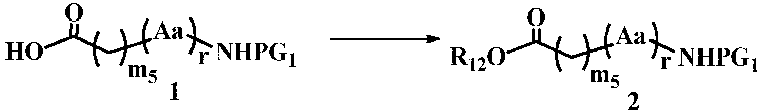
Step 2: Compound **2** and compound **3** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **4**. Optionally a base is not required for the reaction to proceed, but the reaction temperature and reaction time need to be tightly controlled; 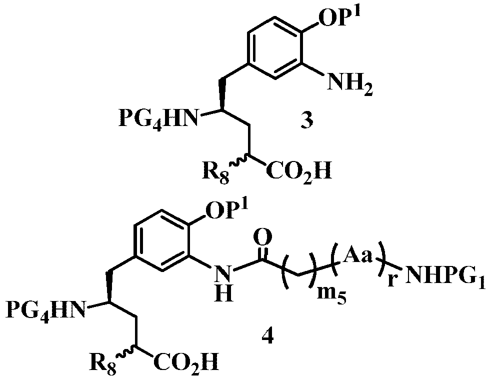
Step 3: The amino protecting group PG₄ of compound **4** is removed selectively under appropriate deprotection conditions, such as H₂ and Pd/C catalyst for Cbz protecting group, and acidic conditions for Boc protecting group, to yield compound **5**; 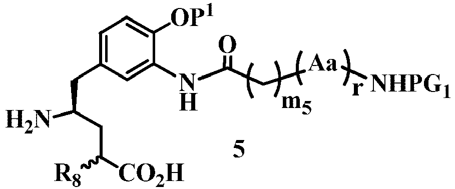
Step 4: Compound **5** and compound of Formula (IV) condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **6**. Optionally a base is not required for the reaction to proceed, but the reaction temperature and reaction time need to be tightly controlled; 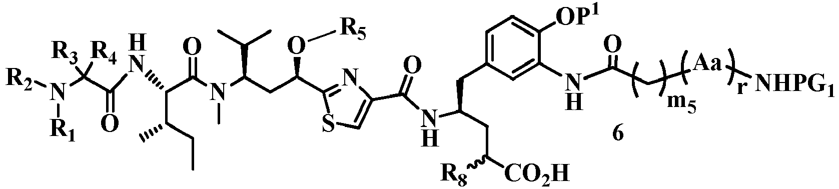
Step 5: The amino protecting group PG₁ of compound **6** is removed under deprotection conditions, such as H₂ and Pd/C catalyst for Cbz protecting group, and acidic conditions for Boc protecting group, to yield compound **VI;**

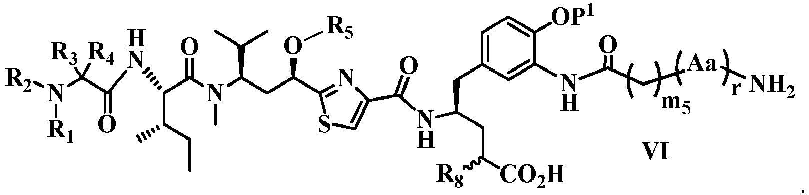

31. The conjugate according to **Claim 28 ~ 30**, wherein the synthesis of compounds of Formula (VII) comprises one or more of the following steps:

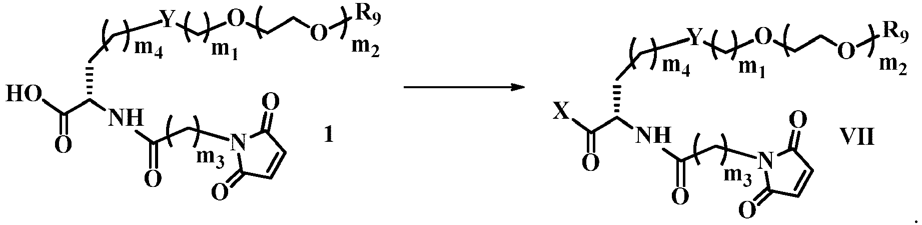

32. The conjugate according to **Claim 31**, wherein the synthesis of the compounds of Formula (VII) comprises one or more of the following steps:
Carboxylic acid **1** and compound with a hydroxyl group (such as pentafluorophenol or N-hydroxysuccinimide) condense in the presence of a condensation reagent (such as EDC, HATU, DIC or DCC) to yield a reactive ester;
Alternatively, carboxylic acid **1** reacts with ethyl chloroformate, isobutyl chloroformate, etc., in the presence of an organic base (such as N-methylmorpholine, triethylamine, diisopropylethylamine, etc.) to yield a reactive mixed anhydride;
Alternatively, carboxylic acid **1** reacts with oxalyl chloride, in the presence of an organic base such as trimethylamine, and catalytic amount of DMF (such as 0.01 eq. to 0.5 eq.) to yield an acyl chloride.

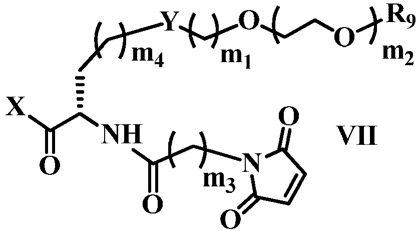

33. The conjugate according to **Claim 3 ~ 11**, wherein the compound of Formula (II) is obtained by condensation reaction of compounds of Formula (VIII) and Formula (IX): 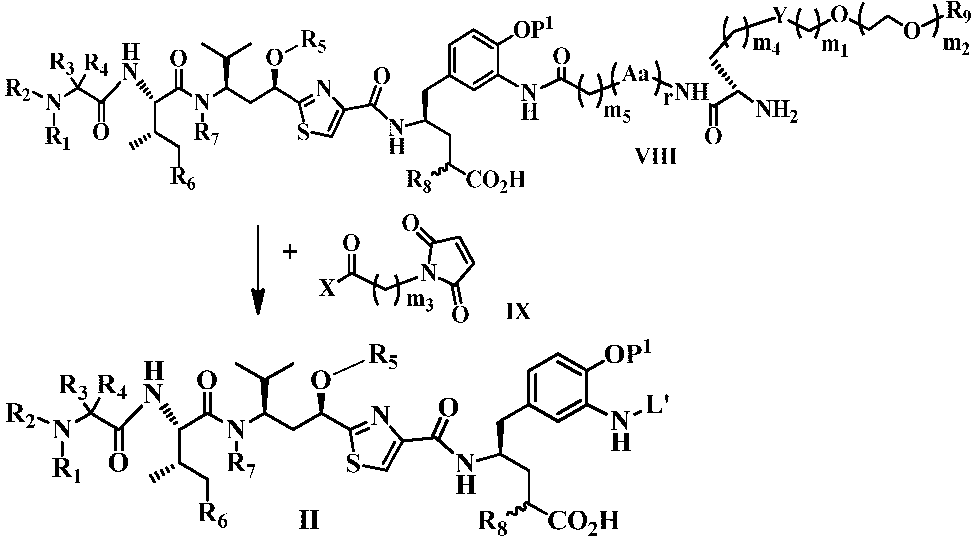 wherein the definition of X and the condensation reaction conditions are the same as in **Claim 9 ∼ 11**.

34. The conjugate according to **Claim 33**, wherein the NH₂ group of formula (VIII) participates in the conjugation reaction in a form of salt, with trifluoroacetic acid, hydrochloride acid, formic acid, acetatic acid, sulfuric acid, phosphoric acid, nitric acid, citric acid, succinic acid, benzoic acid or sulfonic acid.

35. The conjugate according to **Claim 33 ~ 34**, wherein the synthesis of compounds of Formula (VIII) comprises one or more of the following steps:

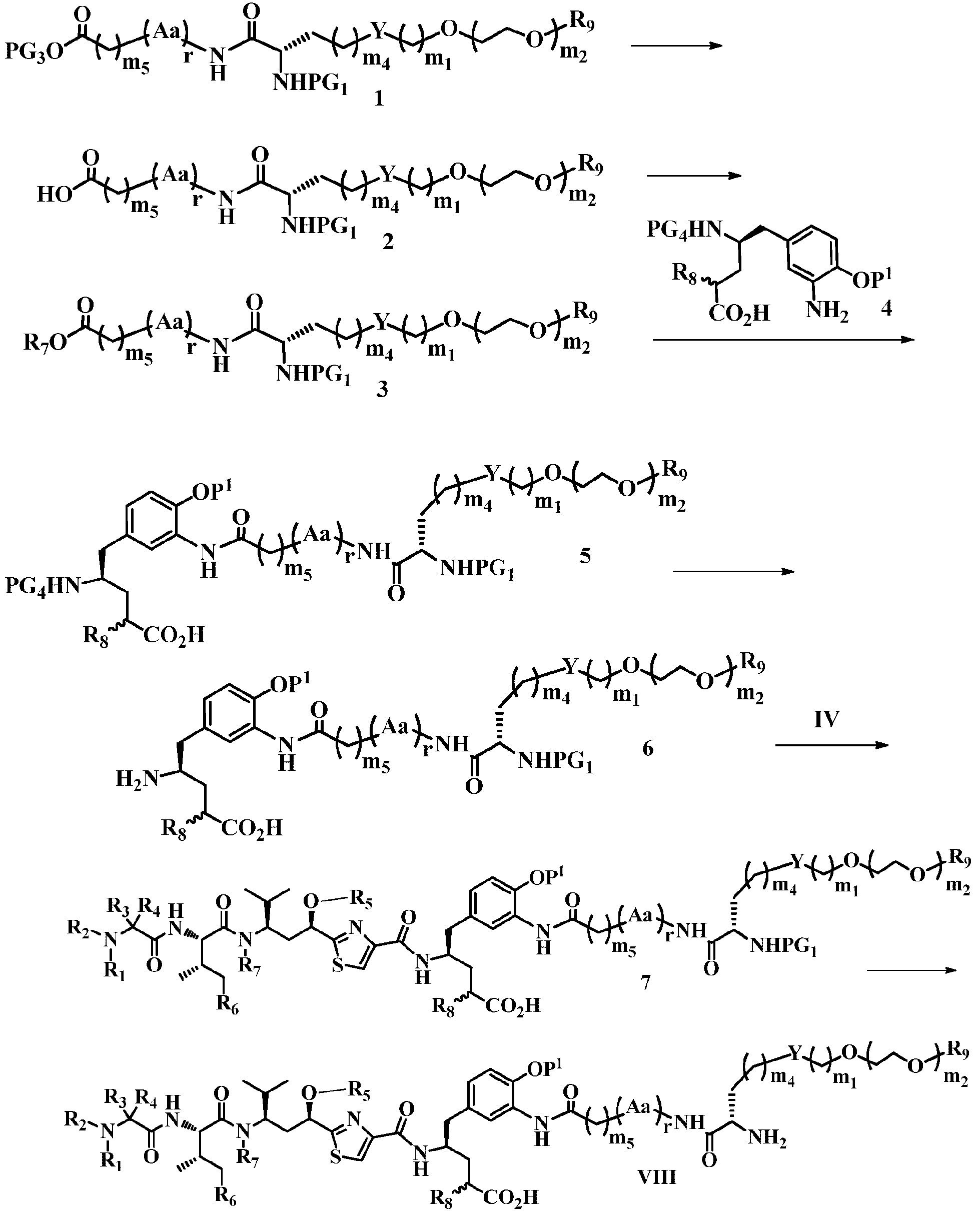

36. The conjugate according to **Claim 35**, wherein the synthesis of compounds of Formula (VIII) comprises one or more of the following steps:
Step 1: The carboxyl protecting group PG₃ of compound 1 is removed under deprotection conditions, such as acid (formic acid, acetic acid, trifluoroacetic acid, hydrochloric acid, phosphoric acid and the like) for the cleavage of tert-butyl ester protecting group, to yield compound **2**; 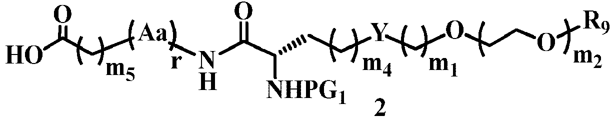
Step 2: Compound **2** reacts with a compound containing a hydroxyl group (such as pentafluorophenol or N-hydroxysuccinimide), in the presence of a condensation reagent (such as EDC, HATU, DIC or DCC), to yield a reactive ester compound **3**; 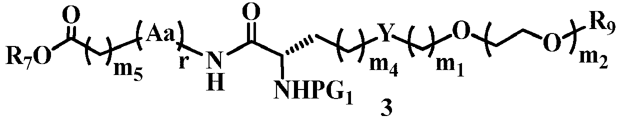
Step 3: Compound **3** and compound **4** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **5**. Optionally a base is not required for the reaction to proceed, but the reaction temperature and reaction time need to be tightly controlled; 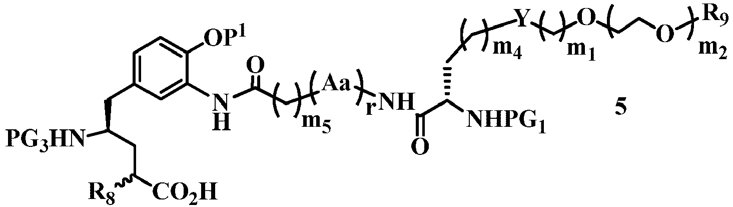
Step 4: The amino protecting group PG₄ of compound **5** is removed under deprotection conditions, such as H₂ and Pd/C catalyst for Cbz protecting group, and acidic conditions for Boc protecting group, to yield compound **6**; 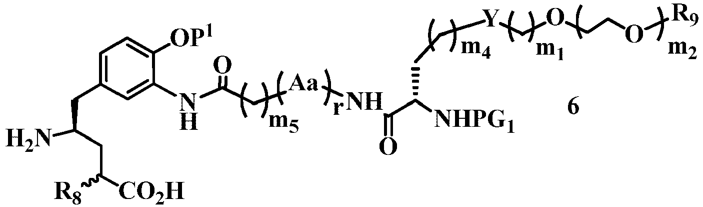
Step 5: Compound **6** and a compound of Formula (IV) in **Claim 18 ~ 21**, condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **7**. Optionally a base is not required for the reaction to proceed, but the reaction temperature and reaction time need to be tightly controlled; 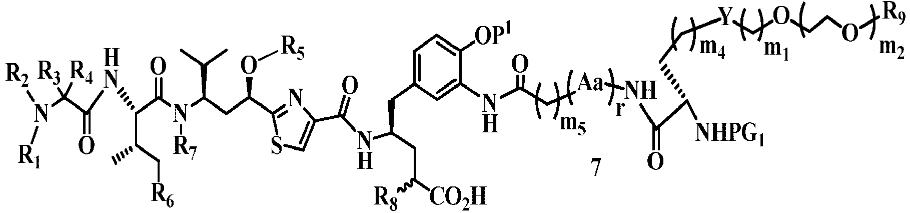
Step 6. The amino protecting group PG₁ of compound **7** is removed under deprotection conditions, such as H₂ and Pd/C catalyst for Cbz protecting group, and acidic conditions for Boc protecting group, to yield compound VIII;

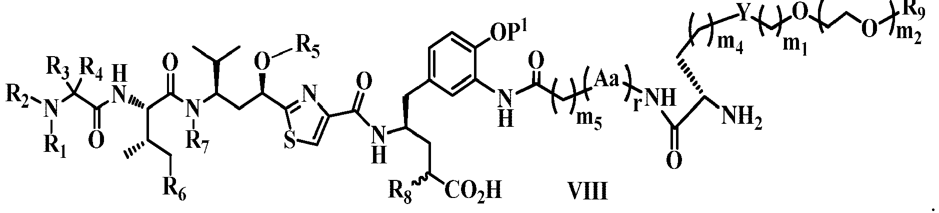

37. The conjugate according to **Claim 33** ~ **36**, wherein the synthesis of compounds of Formula (IX) comprises one or more of the following steps:
Carboxylic acid **1** and compound with a hydroxyl group (such as pentafluorophenol or N-hydroxysuccinimide) condense in the presence of a condensation reagent to yield a reactive ester IX;
Alternatively, carboxylic acid **1** reacts with ethyl chloroformate, isobutyl chloroformate, etc., in the presence of an organic base (such as N-methylmorpholine, triethylamine, diisopropylethylamine, etc.) to yield a reactive mixed anhydride IX;
Alternatively, carboxylic acid **1** reacts with oxalyl chloride, in the presence of an organic base such as trimethylamine, and catalytic amount of DMF (such as 0.01 eq. to 0.5 eq.) to yield an acyl chloride IX.

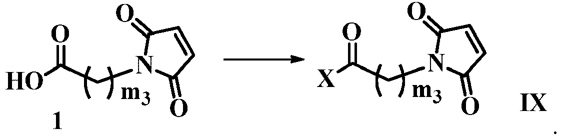

38. The conjugate according to **Claim 3** ~ **11**, wherein the compound of Formula (II) is obtained by condensation reaction of compounds of Formula (X) and Formula (XI): 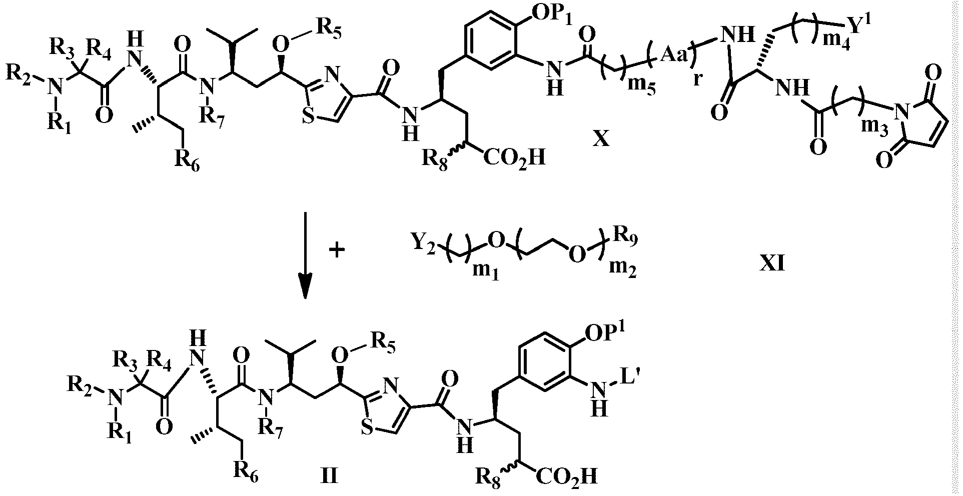 wherein Y¹ and Y² condense to form Y group; Y¹ and Y² are respectively NH₂, -⁺NH₃, COOH, COX, SO₂Cl, P(O)Cl₂, NHCOX, NHSO₂Cl, NHP(O)Cl₂, NHP(O)(OH)Cl,

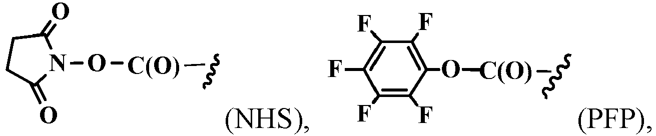

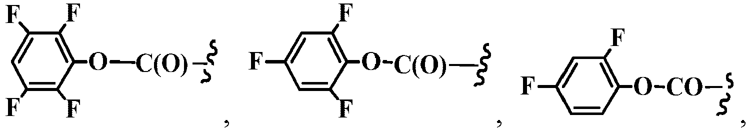

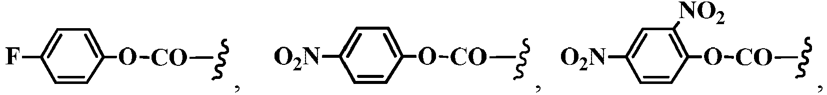

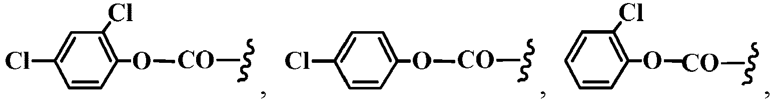

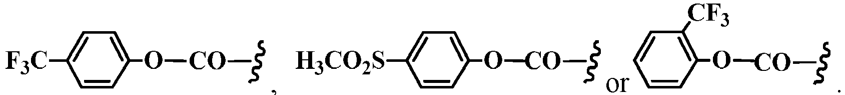

39. The conjugate of **Claim 38,** wherein the synthesis of compounds of Formula (X) comprises one or more of the following steps:

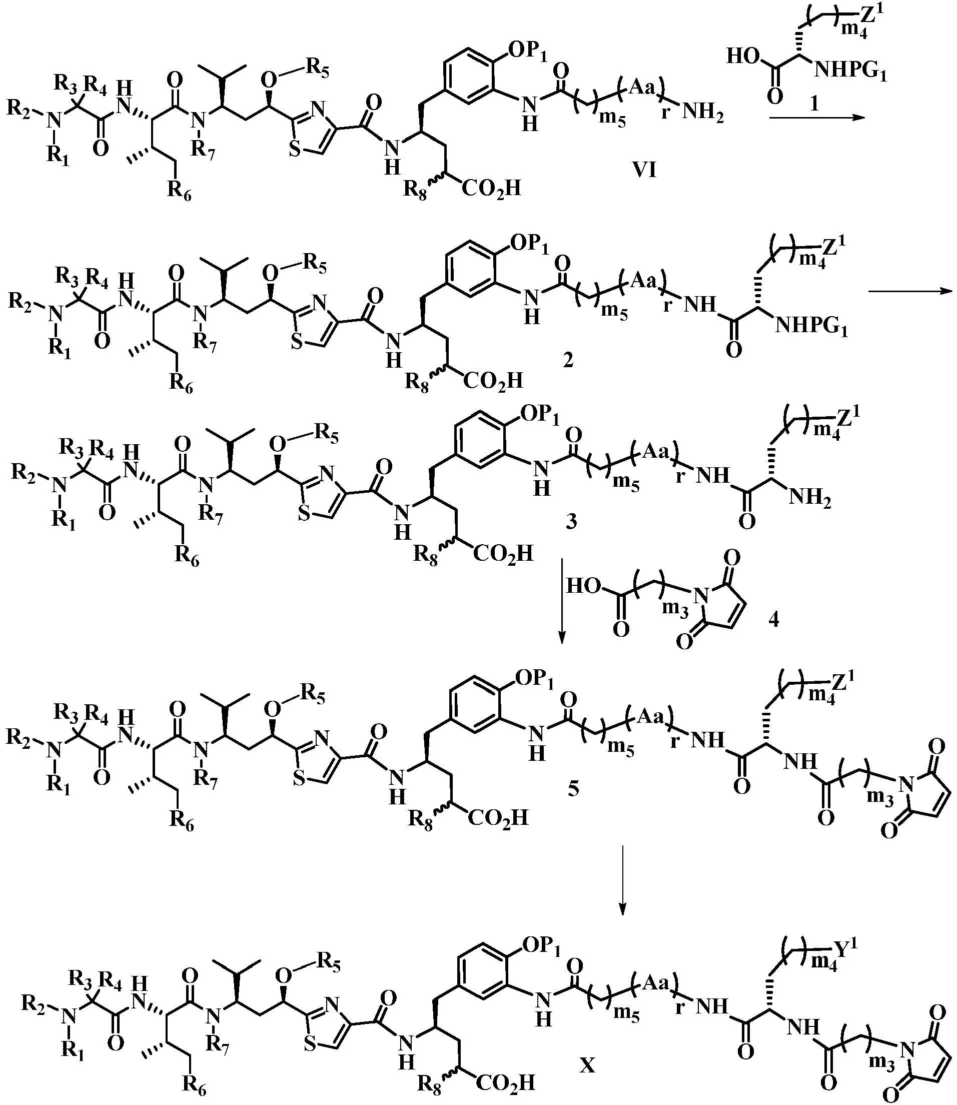

40. The conjugate according to **Claim 39**, wherein the synthesis of compounds of Formula (X) comprises one or more of the following steps:
Step 1: Carboxylic acid **1** and compound **VI** condense in the presence of a condensation reagent (such as EDC, HATU, DIC or DCC), or by other indirect condensation reaction routes, to yield compound **2**, wherein Z¹ is a precursor of Y¹, such as protected amino, protected carboxylic acid, amide, phosphoramide, sulfonamide, carboxylate, phosphate, phosphonate, etc.; 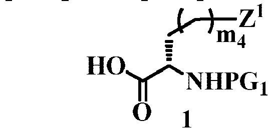 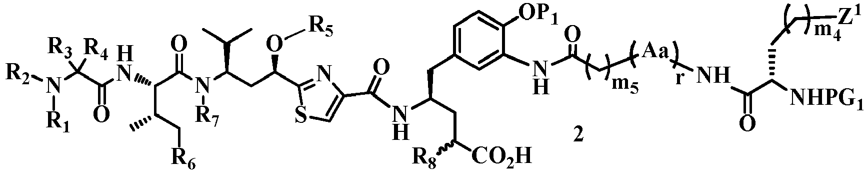
Step 2: The amino protecting group PG₁ on compound **2** is removed under deprotection condition, such as H₂ and Pd/C catalyst for Cbz protecting group, and acidic conditions for Boc protecting group, to yield compound **3**; 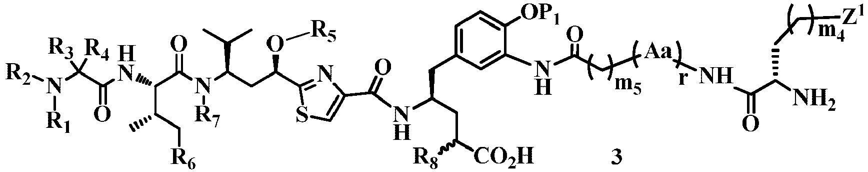
Step 3: Carboxylic acid **4** and amine **3** condense in the presence of a condensation reagent, or by other indirect condensation reaction routes, to yield compound **5**; 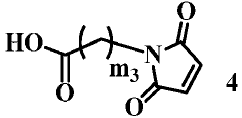 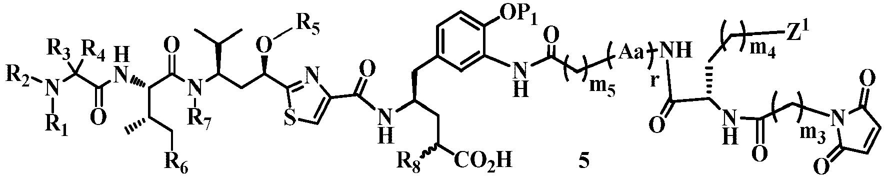
Step 4: The functional group Z¹ of compound **5** is converted to functional group Y¹ by appropriate chemical manipulations, such as deprotection of carboxylic acids and amines, leading to the formation of compound X;

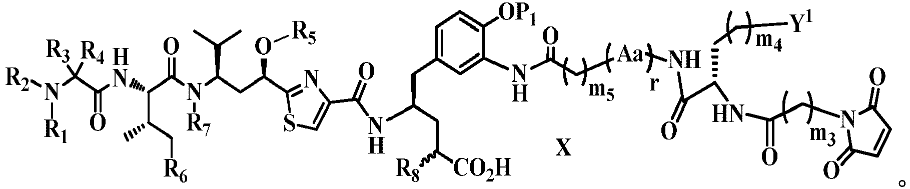

41. The conjugate according to **Claim 38** ~ **40**, wherein the synthesis of compounds of Formula (XI) comprises one or more of the following steps:

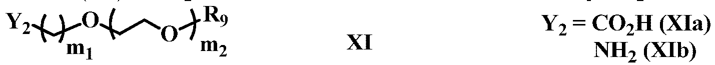

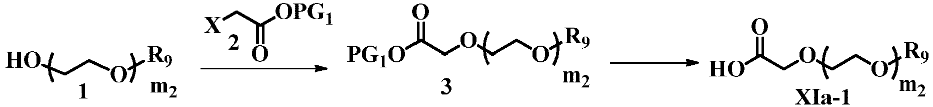

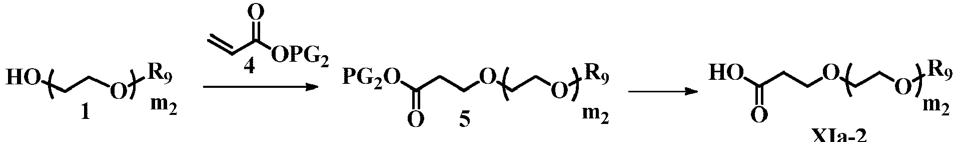

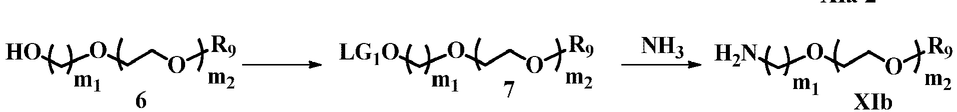

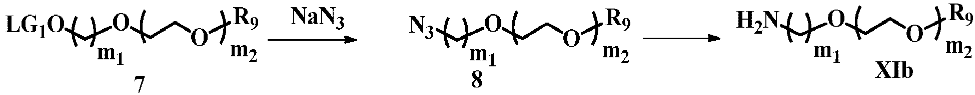

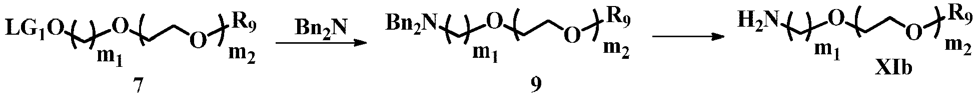

42. The conjugate of **Claim 41**, wherein the synthesis of compounds of Formula (XI) comprises one or more of the following steps:
Step 1: Compound **1** is dissolved in an organic solvent, such as tetrahydrofuran, dichloromethane, N, N-dimethylformamide, dimethyl sulfoxide, and then deprotonated with a base, such as sodium hydride, sodium, sodium hydroxide, and the like, and then stirred with compound **2** (wherein X is chlorine, bromine, iodine and the like or other leaving groups) at appropriate temperature to yield compound **3**;
Step 2: The carboxyl protecting group PG₁ of compound **3** is removed under deprotection conditions, for example, the tert-butyl ester protecting group can be removed by formic acid, acetic acid, trifluoroacetic acid, hydrochloric acid, phosphoric acid and the like, to yield compound XIa-1;
Step 3: Compound **1** is dissolved in an organic solvent, such as tetrahydrofuran, dichloromethane, N, N-dimethylformamide, dimethyl sulfoxide, and then deprotonated with a base, such as sodium hydride, sodium, sodium hydroxide, and the like, and then stirred with compound **4** at appropriate temperature to yield compound **5**;
Step 4: The carboxyl protecting group PG₁ of compound **5** is removed under deprotection conditions, for example, the tert-butyl ester protecting group can be removed by formic acid, acetic acid, trifluoroacetic acid, hydrochloric acid, phosphoric acid and the like, to yield compound **XIa-2**;
Step 5: Compound **6** is dissolved in an organic solvent, such as tetrahydrofuran, dichloromethane, N, N-dimethylformamide, dimethyl sulfoxide and the like, and in the presence of an appropriate organic base such as triethylamine, N, N-diisopropylethylamine, pyridine and the like, reacts with methylsulfonyl chloride, 4-toluenesulfonyl chloride and the like, at 0-5°C to yield compound **7**;
Step 6: Compound **7** and ammonia solution react in water or an organic solvent, such as methanol, ethanol, acetonitrile, tetrahydrofuran, dioxane and the like, optionally under heat, to yield compound **XIb**;
Step 7: Compound **7** and sodium azide react in an organic solvent, such as tetrahydrofuran, dichloromethane, N, N-dimethylformamide, dimethyl sulfoxide and the like, to yield compound **8**;
Step 8: The azide **8** is reduced, under hydrogenation condition (with Pd/C), or under the condition of triphenylphosphine and water, to give compound **XIb**;
Step 9: Compound **7** and dibenzylamine in an organic solvent, such as tetrahydrofuran, dichloromethane, N, N-dimethylformamide, dimethyl sulfoxide, and the like, preferably N, N-dimethylformamide, are heated to 100° C, to yield compound **9**;
Step 10: Compound **9** is dissolved in an organic solvent, such as ethyl acetate, methanol, ethanol, acetic acid, tetrahydrofuran and the like, hydrogenated under H₂, in the presence of Pd/C catalyst, to yield the compound **XIb.** Optionally the reaction can be heated to 45°C.

43. The conjugate according to **Claim 3** ~ **11**, wherein the compound of Formula (II) is obtained by condensation reaction of the compounds of Formula (XII) and Formula (XIII): 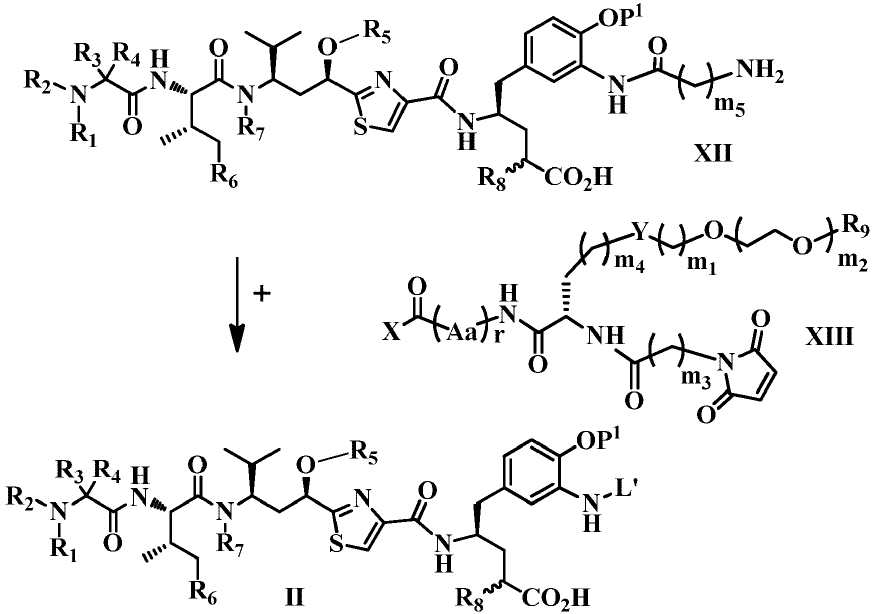 wherein the definition of X and condensation reaction conditions are the same as in **Claim 9** ~ **11**.

44. The conjugate of **Claim 43**, wherein the NH₂ group of Formula (XII) participates in the conjugation reaction in a form of salt, with trifluoroacetic acid, hydrochloride acid, formic acid, acetatic acid, sulfuric acid, phosphoric acid, nitric acid, citric acid, succinic acid, benzoic acid or sulfonic acid.

45. The conjugate according to **Claim 43** ~ **44**, wherein the synthesis of the compound of Formula (XII) comprises one or more of the following steps:

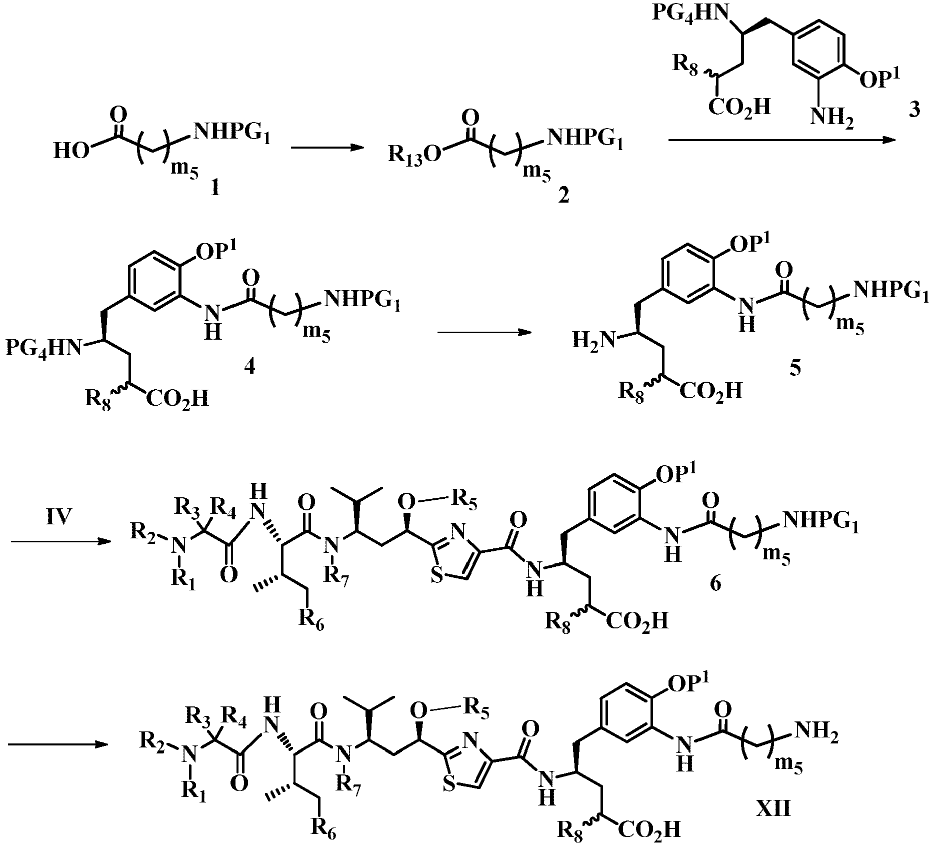

46. The conjugate according to **Claim 45**, wherein the synthesis of compounds of Formula (XII) comprises one or more of the following steps:
Step 1: Compound **1** reacts with a compound containing a hydroxyl group (such as pentafluorophenol or N-hydroxysuccinimide), in the presence of a condensation reagent, to yield a reactive carboxylic acid derivative compound **2**; 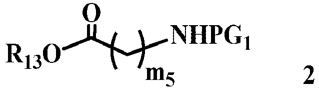
Step 2: Compound **2** and compound **3** condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **4**. Optionally a base is not required for the reaction to proceed, but the reaction temperature and reaction time need to be tightly controlled; 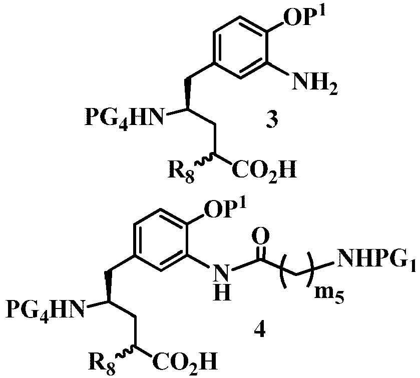
Step 3: The amino protecting group PG₄ of compound **4** is removed selectively under appropriate deprotection conditions, such as H₂ and Pd/C catalyst for Cbz protecting group, and acidic conditions for Boc protecting group, to yield compound **5**; 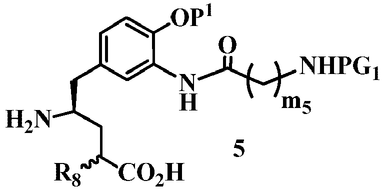
Step 4: Compound **5** and a compound of Formula (IV) in **Claim 18** ~ **21**, condense in an aqueous solution of suitable pH (such as pH = 5.0-8.0), or an organic solution, in the presence of an organic base (such as TEA, DBU or DIPEA) or an inorganic base (such as Na₂CO₃, Cs₂CO₃, K₂CO₃ or NaHCO₃), to yield compound **6**. Optionally a base is not required for the reaction to proceed, but the reaction temperature and reaction time need to be tightly controlled; 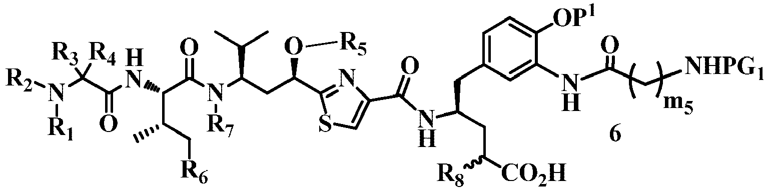
Step 5: The amino protecting group PG₁ of compound **7** is removed under deprotection conditions, such as H₂ and Pd/C catalyst for Cbz protecting group, and acidic conditions for Boc protecting group, to yield compound XIII.

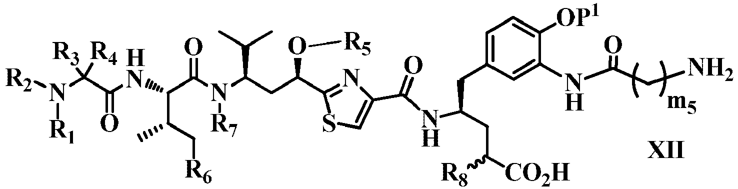

47. The conjugate according to **Claim 43** ~ **46**, wherein the synthesis of compounds of Formula (XIII) comprises one or more of the following steps:

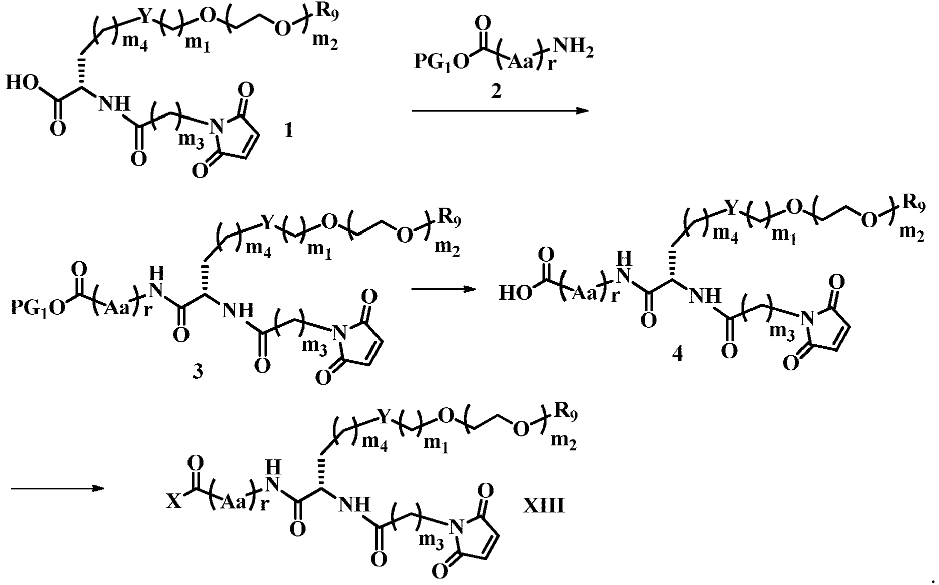

48. The conjugate according to **Claim 47**, wherein the synthesis of compounds of Formula (XIII) comprises one or more of the following steps:
Step 1: Carboxylic acid **1** and amine **2** condense in the presence of a condensation reagent (such as EDC, HATU, DIC or DCC), or by other indirect condensation reaction routes, to yield compound **3**; 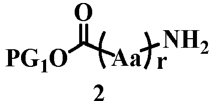 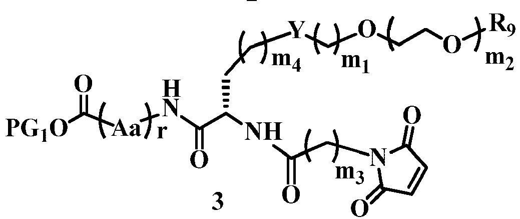
Step 2: The carboxyl protecting group PG₁ of compound **3** is removed under deprotection conditions, such as acid (formic acid, acetic acid, trifluoroacetic acid, hydrochloric acid, phosphoric acid and the like) for the cleavage of tert-butyl ester protecting group, to yield compound **4;** 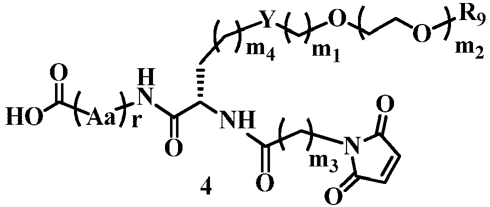
Step 3: Carboxylic acid **4** reacts with a compound containing a hydroxyl group (such as pentafluorophenol or N-hydroxysuccinimide), in the presence of a condensation reagent, to yield a reactive ester compound **XIII;**
Alternatively, carboxylic acid **4** reacts with ethyl chloroformate, isobutyl chloroformate, etc., in the presence of an organic base (such as N-methylmorpholine, triethylamine, diisopropylethylamine, etc.) to yield a reactive mixed anhydride **XIII;**
Alternatively, carboxylic acid **4** reacts with oxalyl chloride, in the presence of an organic base such as trimethylamine, and catalytic amount of DMF (such as 0.01 eq. to 0.5 eq.) to yield an acyl chloride **XIII.**

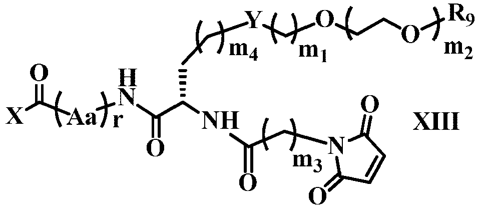

49. The conjugate according to **Claim 1** ~ **48,** wherein the compounds of Formula (I) have the following structures:

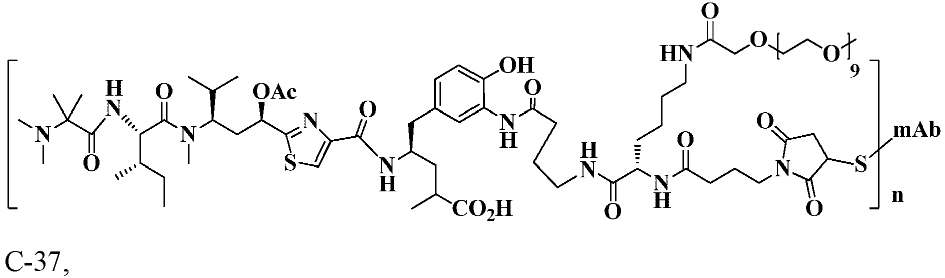

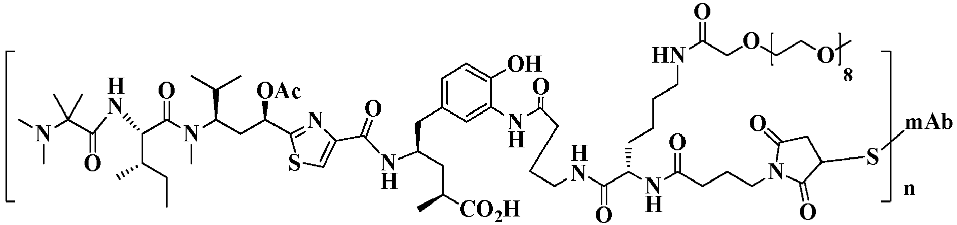

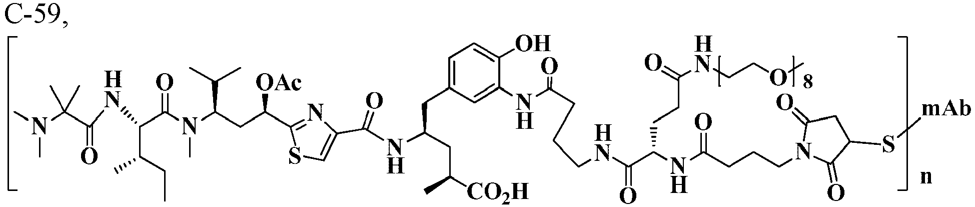

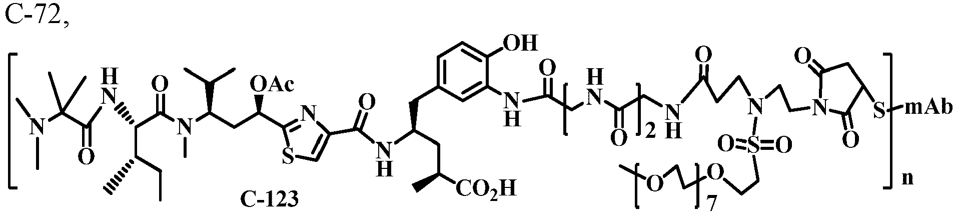

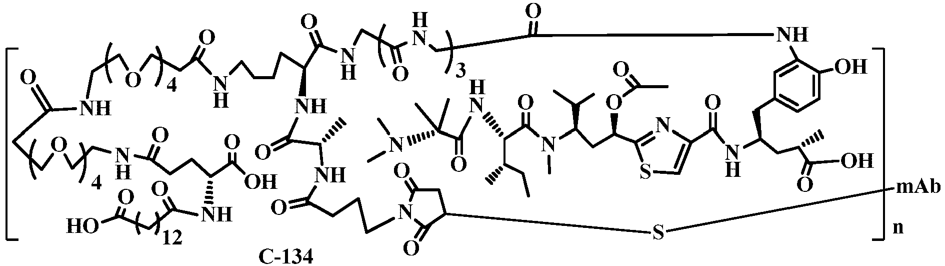

50. A compound of Formula (II), having the following structures:

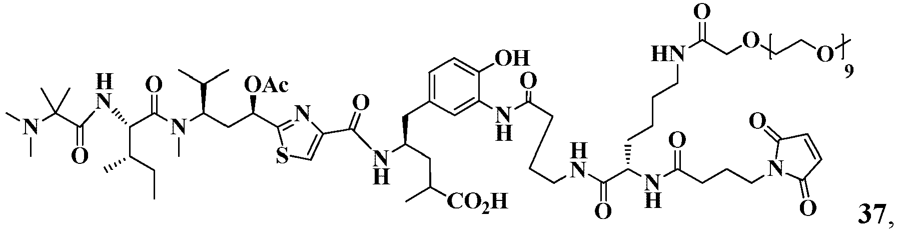

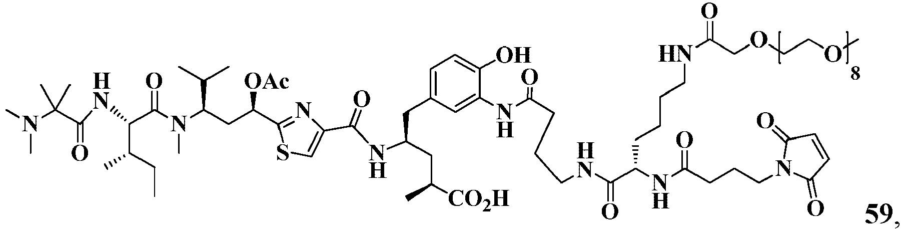

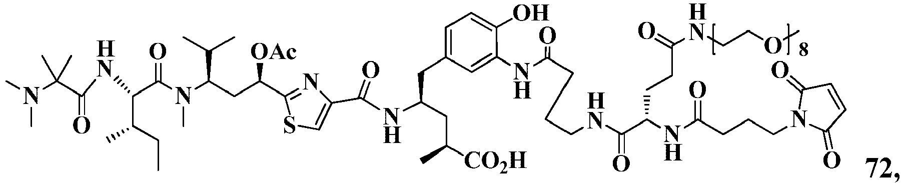

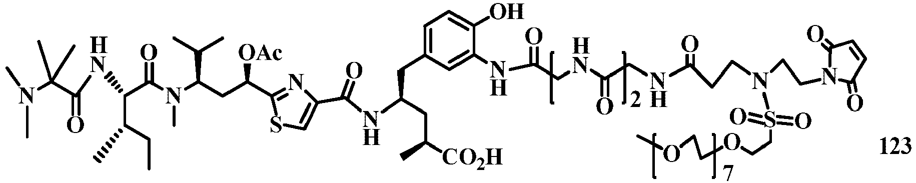

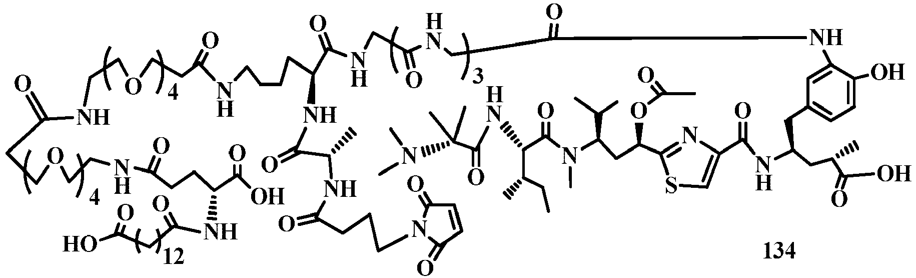

51. A pharmaceutical composition comprising conjugates of **Claim 1** ~ **49** or conjugate derived from compounds of **Claim 50,** and pharmaceutically acceptable excipients thereof.

52. Use of the conjugate according to **Claim 1** ~ **49** in the manufacture of drugs for treating cancer, infection or autoimmune diseases.

53. Use of the compounds according to **Claim 50** in the manufacture of drugs for treating cancer, infection or autoimmune diseases.

54. Use of the pharmaceutical compositions according to **Claim 51** in the manufacture of drugs for treating cancer, infection or autoimmune diseases.
